(19) Europäisches Patentamt
European Patent Office
Office européen des brevets

(11) EP 4 474 377 A1

(12) EUROPEAN PATENT APPLICATION
published in accordance with Art. 153(4) EPC

(43) Date of publication:
**11.12.2024 Bulletin 2024/50**

(21) Application number: **23762968.8**

(22) Date of filing: **02.03.2023**

(51) International Patent Classification (IPC):
***C07D 231/38*** (2006.01) ***C07D 471/04*** (2006.01)
***C07D 487/04*** (2006.01) ***A61K 31/522*** (2006.01)
***A61K 31/437*** (2006.01) ***A61K 31/53*** (2006.01)
***A61K 31/519*** (2006.01) ***A61P 35/00*** (2006.01)
***A61P 37/02*** (2006.01) ***A61P 35/02*** (2006.01)
***A61P 29/00*** (2006.01) ***A61P 19/02*** (2006.01)

(52) Cooperative Patent Classification (CPC):
**A61K 31/415; A61K 31/437; A61K 31/4985; A61K 31/5025; A61K 31/519; A61K 31/52; A61K 31/522; A61K 31/53; A61P 1/00; A61P 1/16; A61P 3/10; A61P 7/06; A61P 11/00; A61P 17/00; A61P 17/06;** (Cont.)

(86) International application number:
**PCT/CN2023/079360**

(87) International publication number:
**WO 2023/165570 (07.09.2023 Gazette 2023/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.03.2022 CN 202210202713**

(71) Applicant: **Shenzhen TargetRx, Inc.**
**Shenzhen, Guangdong 518057 (CN)**

(72) Inventors:
- **WANG, Yihan**
**Shenzhen, Guangdong 518057 (CN)**
- **LI, Huanyin**
**Shenzhen, Guangdong 518057 (CN)**
- **AI, Yixin**
**Shenzhen, Guangdong 518057 (CN)**

(74) Representative: **Novitas Patent AB**
**P.O. Box 55557**
**102 04 Stockholm (SE)**

# (54) CYCLOALKYL OR HETEROCYCLYL SUBSTITUTED HETEROARYL COMPOUND, AND COMPOSITION AND USE THEREOF

(57) The present invention relates to a compound of formula (I) or a tautomer, stereoisomer, prodrug, crystal form, pharmaceutically acceptable salt, hydrate, or solvate thereof, a pharmaceutical composition thereof, and a use of the compound, the tautomer, stereoisomer, prodrug, crystal form, pharmaceutically acceptable salt, hydrate, or solvate thereof, and the pharmaceutical composition thereof in the treatment and/or prevention of wild and/or mutated BTK kinase-mediated diseases.

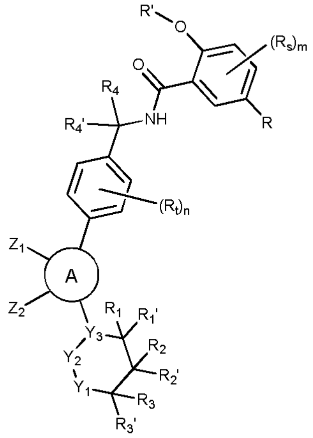

(I)

EP 4 474 377 A1

(52) Cooperative Patent Classification (CPC): (Cont.)
**A61P 17/14; A61P 19/02; A61P 19/10;**
**A61P 21/00; A61P 25/00; A61P 27/14;**
**A61P 29/00; A61P 35/00; A61P 35/02;**
**A61P 37/02; A61P 37/06; A61P 37/08;**
**C07D 231/38; C07D 405/04; C07D 471/04;**
**C07D 473/34; C07D 487/04**

## Description

### FIELD OF THE INVENTION

The present disclosure belongs to the technical field of medicine, and particularly relates to a highly selective substituted cycloalkyl or heterocyclyl substituted heteroaryl compound which has an inhibitory effect on Bruton's tyrosine kinase (BTK) and its C481 mutation, a pharmaceutical composition comprising the same, and a preparation method and use thereof.

### BACKGROUND OF THE INVENTION

Bruton's tyrosine kinase (BTK) belongs to the Tec family of cytoplasmic tyrosine kinases, which is the second largest family of non-receptor kinases in human. It is expressed in all cell lineages of hematopoietic system (except T cells), and it is located in bone marrow, spleen and lymph nodes. Inactive mutations in the gene encoding Btk lead to X-linked agammaglobulinemia (XLA) in humans and X-linked immunodeficiency (XID) in mice. These two diseases are characterized by major defects in the development and function of B cells, indicating that Btk plays a vital role in the development and function of B cells. In addition, the constitutive activation of Btk in B cells leads to the accumulation of autoreactive plasma cells. Preclinical studies show that Btk-deficient mice are resistant to the development of collagen-induced arthritis. In addition, the clinical study of Rituxan (a CD20 antibody that exhausts mature B cells) reveals the key role of B cells in many inflammatory diseases (such as rheumatoid arthritis, systemic lupus erythematosus and multiple sclerosis). Moreover, the abnormal activation of Btk plays an important role in the pathogenesis of B-cell lymphoma, indicating that the inhibition of Btk can be used to treat hematological malignancies.

Irutinib, a covalent Btk inhibitor, has been approved by the US Food and Drug Administration for the treatment of chronic lymphocytic leukemia (CLL), mantle cell lymphoma (MCL), waldenstrom's macroglobulinemia (WM) and chronic graft-versus-host disease (cGVHD). Despite its excellent efficacy and general tolerance, adverse events (such as bleeding, rash and diarrhea) that are not usually observed in patients with Btk deficiency have been reported.

These adverse reactions related to irutinib are thought to be mainly related to the off-target effect of irutinib, which proves to inhibit EGFR and Tec. Targeting EGFR can induce significant skin toxicity and gastrointestinal adverse reactions, because EGFR signaling cascade involves the biology of skin and gastrointestinal system. Both Btk and Tec belong to Tec family kinases. Platelets express Btk and Tec, which serve the downstream of glycoprotein VI(GPVI) signaling. Tec compensates for the loss of Btk in the downstream signal transduction of GPVI in rat platelets. The inhibition of Tec kinase by irutinib interferes with platelet aggregation and may contribute to the observed bleeding. Therefore, Btk inhibitors with high BTK inhibition and low EGFR and Tec inhibition are needed to reduce or avoid bleeding, rash and diarrhea.

Irutinib also irreversibly binds to interleukin-2-induced tyrosine kinase (ITK). ITK plays a key role in the natural killer function of NK cells stimulated by FcR required for antibody-dependent NK cell-mediated cytotoxicity (ADCC). ADCC is the standard of treatment for B-cell malignancies today when anti-CD20 antibodies such as rituximab are combined with chemotherapy, so it is desirable to have a Btk inhibitor that is more selective for Btk than ITK.

Irreversibly and covalently reversible BTK inhibitors specifically target the cysteine residue C481 within Btk. After treatment with irutinib, primary and secondary drug resistance occurred. Mutations in BTK such as C481S, C481Y, C481Y, C481R and C481F have been proved to clearly interfere with drug binding. It is predicted that the observed incidence of drug resistance will increase when clinical use other than clinical trials goes on over time.

Therefore, it is an object of the present disclosure to provide BTK inhibitors with different binding modes, more particularly reversible inhibitors. In addition, the present disclosure aims to provide Btk inhibitors, which have high selectivity for Btk inhibition compared with EGFR, Tec and ITK inhibition.

### SUMMARY OF THE INVENTION

The present disclosure provides a novel heteroaryl compound substituted by cycloalkyl or heterocyclyl, a composition containing the compound and use thereof, wherein the compound has better inhibitory activity and selectivity on Btk and C481 mutated BTK kinases, and has better pharmacodynamic and/or pharmacokinetic properties, and can treat diseases or disorders mediated by Btk kinases.

In this regard, the present disclosure adopts the following technical solutions:

In one aspect, the present disclosure relates to a compound of formula (I), or a tautomer, stereoisomer, prodrug, crystal form, pharmaceutically acceptable salt, hydrate or solvate thereof:

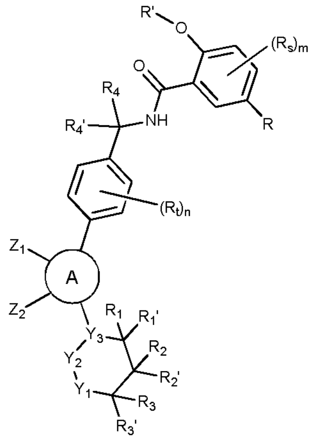

(I)

wherein

ring A is

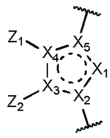 or 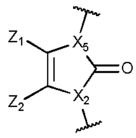 ;

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O, S, NH or $C(R_{Y1})_2$; wherein $R_{Y1}$ is independently H, D, halogen, $C_{1-6}$ alkyl or $C_{1-6}$ haloalkyl;
$Y_2$ is bond, $C(R_{Y2})_2$ or $C(R_{Y2})_2C(R_{Y2})_2$; wherein $R_{Y2}$ is independently H, D, halogen, $C_{1-6}$ alkyl or $C_{1-6}$ haloalkyl;
$Y_3$ is N, CD or CH;
$Z_1$ is $C(O)NH_2$;
$Z_2$ is $NH_2$;
or $Z_1$ and $Z_2$ are taken together with the atoms to which they are attached to form

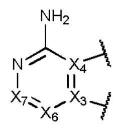 ;

wherein $X_6$ is N, CD, CH or C-OH;
$X_7$ is N, CD or CH;
$R_1$ is H, D, halogen or OH;
$R_1$' is H, D or halogen;
$R_2$ is H, D, OH or $NH_2$;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, halogen, $C_{1-6}$ alkyl, $C_{1-6}$ haloalkyl, $C_{3-6}$ cycloalkyl or 3- to 7-membered heterocyclyl; or $-C(R_3)(R_3')-C(R_{Y1})_2-$ or $-C(R_1)(R_1')-C(R_2)(R_2')-$ is taken together to form -CH=CH-;
or $R_1$ and $R_2$ are attached to form $C_{1-4}$ alkylene;
$R_4$ is H, D, halogen, $C_{1-6}$ alkyl or $C_{1-6}$ haloalkyl;
$R_4$' is H, D, halogen, $C_{1-6}$ alkyl or $C_{1-6}$ haloalkyl;
R is halogen;
R' is $C_{1-6}$ alkyl or $C_{1-6}$ haloalkyl;

$R_s$ is H, D, halogen, $C_{1-6}$ alkyl or $C_{1-6}$ haloalkyl;
$R_t$ is H, D, halogen, $C_{1-6}$ alkyl or $C_{1-6}$ haloalkyl;
m=0, 1, 2 or 3;
n=0, 1, 2, 3 or 4;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups can be substituted by D, up to full deuteration; provided that at least one of $R_2$ and $R_3$ is OH or $CH_2OR_a$.

In another aspect, the present disclosure relates to a pharmaceutical composition containing the compound of the present disclosure, or the tautomer, stereoisomer, prodrug, crystal form, pharmaceutically acceptable salt, hydrate or solvate thereof, and pharmaceutically acceptable excipients, and optionally, other therapeutic agents.

In another aspect, the present disclosure relates to use of the compound of the present disclosure, or the tautomer, stereoisomer, prodrug, crystal form, pharmaceutically acceptable salt, hydrate or solvate thereof, or the pharmaceutical composition of the present disclosure in the manufacture of a medicament for treating and/or preventing wild-type and/or mutated BTK kinase-mediated diseases; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

In another aspect, the present disclosure relates to a method for treating and/or preventing a disease mediated by wild-type and/or mutated BTK kinase in a subject, the method comprising administering to the subject the compound of the present disclosure or the pharmaceutically acceptable salt, stereoisomer, solvate, hydrate, polymorph, prodrug or isotopic variant thereof, or the pharmaceutical composition of the present disclosure; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

In another aspect, that present disclosure relate to the compound of the present disclosure, or the tautomer, stereoisomer, prodrug, crystal form, pharmaceutically acceptable salt, hydrate or solvate thereof, or the pharmaceutical composition of the present disclosure, for use in treating and/or preventing diseases mediated by wild-type and/or mutated BTK kinases; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

In a more specific aspect, the diseases mediated by BTK in the present disclosure are selected from allergic diseases, autoimmune diseases, inflammatory diseases and cancers.

In a more specific aspect, the BTK-mediated disease in the present disclosure is a B cell proliferative disease selected from chronic lymphocytic lymphoma, non-Hodgkin's lymphoma, diffuse large B cell lymphoma, mantle cell lymphoma, follicular lymphoma, chronic lymphocytic leukemia, acute lymphocytic leukemia, acute myeloid leukemia, acute promyelocytic leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplastic disorder, mixed lineage leukemia, myelodysplastic syndrome, myeloproliferative disorder, marginal zone lymphoma and waldenstrom's macroglobulinemia.

In a more specific aspect, the BTK-mediated disease of the present disclosure is multiple myeloma.

Other objects and advantages of the present disclosure will be apparent to those skilled in the art from the detailed description, examples and claims that follow.

## Definitions

### Chemical definitions

The definitions of specific functional groups and chemical terms are described in more detail below.

When a range of values is listed, it is intended to encompass each value and sub-range within the range. For example, "$C_{1-6}$ alkyl" is intended to encompass $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

"$C_{1-6}$ alkyl" refers to a linear or branched, saturated hydrocarbon group having 1 to 6 carbon atoms, and is also referred to herein as "lower alkyl". In some embodiments, $C_{1-4}$ alkyl is alternative. Examples of alkyl include, but are not limited to, methyl ($C_1$), ethyl ($C_2$), n-propyl ($C_3$), iso-propyl ($C_3$), n-butyl ($C_4$), t-butyl ($C_4$), sec-butyl ($C_4$), iso-butyl ($C_4$), n-pentyl ($C_5$), 3-pentyl ($C_5$), pentyl ($C_5$), neo-pentyl ($C_5$), 3-methyl-2-butyl ($C_5$), t-pentyl ($C_5$) and n-hexyl ($C_6$). Regardless of whether or not the alkyl group is modified with "substituted", each alkyl group is independently optionally substituted, for example, with 1 to 5 substituents, 1 to 3 substituents or 1 substituent. Appropriate substituents are defined as follows.

"Halo" or "halogen" refers to fluoro (F), chloro (Cl), bromo (Br) and iodo (I). In some embodiments, the halo group is F, Cl or Br. In some embodiments, the halo group is F or Cl. In some embodiments, the halo group is F.

Therefore, "$C_{1-6}$ haloalkyl" refers to the above "$C_{1-6}$ alkyl" substituted with one or more halo groups. In some

embodiments, $C_{1-4}$ haloalkyl is alternative, and $C_{1-2}$ haloalkyl is yet alternative. Examples of haloalkyl include, but are not limited to, $-CF_3$, $-CH_2F$, $-CHF_2$, - $CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc.

"$C_{3-10}$ cycloalkyl" refers to a non-aromatic cyclic hydrocarbon group having 3-10 ring carbon atoms and zero heteroatoms. In some embodiments, $C_{3-7}$ cycloalkyl is alternative, $C_{3-6}$ cycloalkyl is alternative, and $C_{5-6}$ cycloalkyl is yet alternative. Cycloalkyl also includes a ring system in which the above cycloalkyl ring is fused with one or more aryl or heteroaryl groups, wherein the point of attachment is on the cycloalkyl ring, and in such instances, the number of carbons continue to designate the number of carbons in the cycloalkyl system. Examples of cycloalkyl include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptyl ($C_7$), bicyclo[2.2.2]octyl ($C_8$), cyclononyl ($C_9$), cyclononenyl ($C_9$), cyclodecyl ($C_{10}$), cyclodecenyl ($C_{10}$), octahydro-1H-indenyl ($C_9$), decahydronaphthyl ($C_{10}$), spiro[4.5]decyl ($C_{10}$), etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"3- to 10-membered heterocyclyl" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon. In heterocyclyl groups that contain one or more nitrogen atoms, the point of attachment can be a carbon or nitrogen atom, as valency permits. In some embodiments, 3- to 7-membered heterocyclyl is alternative, and it is a 3- to 7-membered non-aromatic ring system having ring carbon atoms and 1-3 ring heteroatoms. In some embodiments, 3- to 6-membered heterocyclyl is alternative, and it is a 3- to 6-membered non-aromatic ring system having ring carbon atoms and 1-3 ring heteroatoms. 5- to 6-membered heterocyclyl is yet alternative, and it is a 5- to 6-membered non-aromatic ring system having ring carbon atoms and 1-3 ring heteroatoms. "Heterocyclyl" also includes ring systems wherein the heterocyclyl, as defined above, is fused with one or more cycloalkyl, aryl or heteroaryl groups wherein the point of attachment is on the heterocyclyl ring, and in such instances, the number of ring members continues to designate the number of ring members in the heterocyclyl ring system. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing three heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a $C_6$ aryl ring (also referred to herein as a 5,6-bicyclic heterocyclyl) include, without limitation, indolinyl, isoindolinyl, dihydrobenzofuranyl, dihydrobenzothienyl, benzoxazolinonyl, and the like. Exemplary 6-membered heterocyclyl groups fused to a $C_6$ aryl ring (also referred to herein as a 6,6-bicyclic heterocyclyl) include, without limitation, tetrahydroquinolinyl, tetrahydroisoquinolinyl, and the like.

Exemplary substituents on carbon atoms include, but are not limited to, halo, -CN, - $NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})R^{bb}$, -SH, - $SR^{aa}$, $-SSR^{cc}$, $-C(=O)R^{aa}$, $-CO_2H$, -CHO, $-C(OR^{CC})_2$, $-CO_2R^{aa}$, $-OC(=O)R^{aa}$, $-OCO_2R^{aa}$, - $C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, - $C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, - $OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, - $SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, - $C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, - $OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, - $P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl, heterocyclyl, aryl and heteroaryl, wherein each of alkyl, alkenyl, alkynyl, carbocyclyl, heterocyclyl, aryl and heteroaryl is independently substituted with 0, 1, 2, 3, 4 or 5 $R^{dd}$ groups;

or two geminal hydrogens on a carbon atom are replaced with the group =O, =S, $=NN(R^{bb})_2$, $=NNR^{bb}C(=O)R^{aa}$,

$=NNR^{bb}C(=O)OR^{aa}$, $=NNR^{bb}S(=O)_2R^{aa}$, $=NR^{bb}$ or $=NOR^{cc}$;

each $R^{aa}$ is independently selected from alkyl, haloalkyl, alkenyl, alkynyl, carbocyclyl, heterocyclyl, aryl and heteroaryl, or two $R^{aa}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{bb}$ is independently selected from: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{bb}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{cc}$ is independently selected from hydrogen, alkyl, haloalkyl, alkenyl, alkynyl, carbocyclyl, heterocyclyl, aryl and heteroaryl, or two $R^{cc}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{dd}$ is independently selected from: halo, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, carbocyclyl, heterocyclyl, aryl, and heteroaryl, wherein each of alkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be bound to form =O or =S;

each $R^{ee}$ is independently selected from alkyl, haloalkyl, alkenyl, alkynyl, carbocyclyl, aryl, heterocyclyl and heteroaryl, wherein each of alkyl, alkenyl, alkynyl, carbocyclyl, heterocyclyl, aryl and heteroaryl is independently substituted with 0, 1, 2, 3, 4 or 5 $R^{gg}$ groups;

each $R^{ff}$ is independently selected from hydrogen, alkyl, haloalkyl, alkenyl, alkynyl, carbocyclyl, heterocyclyl, aryl and heteroaryl, or two $R^{ff}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{gg}$ groups;

each $R^{gg}$ is independently: halo, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, $-C(=O)S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ carbocyclyl, $C_6$-$C_{10}$ aryl, $C_3$-$C_7$ heterocyclyl, or $C_5$-$C_{10}$ heteroaryl; or two geminal $R^{gg}$ substituents can be bound to form =O or =S, wherein $X^-$ is a counter ion.

Exemplary substituents on the nitrogen atom include but are not limited to, hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{cc}$ groups connected to the nitrogen atom are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as defined above.

"Deuteration", "deuterated" or "D substituted" means that one or more hydrogens in the group or in the compound are substituted by deuterium; Deuteration can be monosubstituted, disubstituted, polysubstituted or fully substituted. The terms "substituted with one or more deuteriums" and " deuterated one or more times" are used interchangeably.

"Non-deuterated compound" refers to a compound with deuterium atom content not higher than the natural deuterium isotope content (0.015%).

The deuterium isotope content of deuterium at the deuterated position is at least greater than the natural deuterium isotope content (0.015%), alternatively greater than 30%, yet alternatively greater than 50%, yet alternatively greater than 75%, yet alternatively greater than 95%, and yet alternatively greater than 99%.

The term "pharmaceutically acceptable salt" refers to those salts which are, within the scope of sound medical judgment, suitable for use in contact with the tissues of humans and lower animals without undue toxicity, irritation, allergic

response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

"Combination", "combined", and related terms refer to the simultaneous or sequential administration of the therapeutic agents of the present disclosure. For example, the compound disclosed herein may be administered with another therapeutic agent simultaneously or sequentially in separate unit dosage forms, or together in a single unit dosage form.

## DETAILED DESCRIPTION OF THE INVENTION

### Compounds

As used herein, "compound of the present disclosure" refers to a compound of formula (I) below (including subsets of each formula), or a tautomer, stereoisomer, prodrug, crystal form, pharmaceutically acceptable salt, hydrate or solvate thereof.

In one embodiment, the present disclosure relates to a compound of formula (I), or a tautomer, stereoisomer, prodrug, crystal form, pharmaceutically acceptable salt, hydrate or solvate thereof:

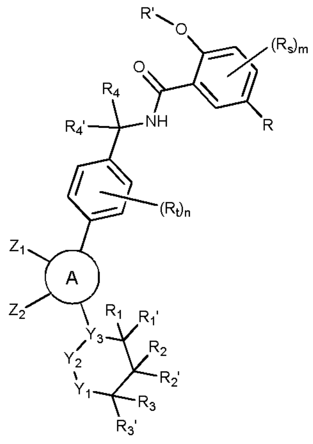

(I)

wherein

ring A is

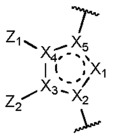 or 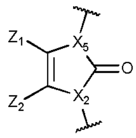 ;

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O, S, NH or $C(R_{Y1})_2$; wherein $R_{Y1}$ is independently H, D, halogen, $C_{1-6}$ alkyl or $C_{1-6}$ haloalkyl;
$Y_2$ is bond, $C(R_{Y2})_2$ or $C(R_{Y2})_2C(R_{Y2})_2$; wherein $R_{Y2}$ is independently H, D, halogen, $C_{1-6}$ alkyl or $C_{1-6}$ haloalkyl;
$Y_3$ is N, CD or CH;
$Z_1$ is $C(O)NH_2$;
$Z_2$ is $NH_2$;
or $Z_1$ and $Z_2$ are taken together with the atoms to which they are attached to form

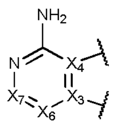 ;

wherein $X_6$ is N, CD, CH or C-OH;
$X_7$ is N, CD or CH;
$R_1$ is H, D, halogen or OH;
$R_1$' is H, D or halogen;
$R_2$ is H, D, OH or $NH_2$;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, halogen, $C_{1-6}$ alkyl, $C_{1-6}$ haloalkyl, $C_{3-6}$ cycloalkyl or 3- to 7-membered heterocyclyl; or $-C(R_3)(R_3')-C(R_{Y1})_2-$ or $-C(R_1)(R_1')-C(R_2)(R_2')-$ is taken together to form -CH=CH-;
or $R_1$ and $R_2$ are attached to form $C_{1-4}$ alkylene;
$R_4$ is H, D, halogen, $C_{1-6}$ alkyl or $C_{1-6}$ haloalkyl;
$R_4$' is H, D, halogen, $C_{1-6}$ alkyl or $C_{1-6}$ haloalkyl;
R is halogen;
R' is $C_{1-6}$ alkyl or $C_{1-6}$ haloalkyl;

$R_s$ is H, D, halogen, $C_{1-6}$ alkyl or $C_{1-6}$ haloalkyl;
$R_t$ is H, D, halogen, $C_{1-6}$ alkyl or $C_{1-6}$ haloalkyl;
m=0, 1, 2 or 3;
n=0, 1, 2, 3 or 4;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups can be substituted by D, up to full deuteration; provided that at least one of $R_2$ and $R_3$ is OH or $CH_2OR_a$.
Ring A and $X_1$-$X_5$

In one embodiment, ring A is

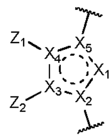
;

in another embodiment, ring A is

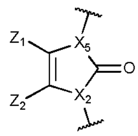

In a specific embodiment, $X_1$ is N; In another specific embodiment, $X_1$ is CD or CH. In a specific embodiment, $X_2$ is N atom; In another specific embodiment, $X_2$ is C atom. In a specific embodiment, $X_3$ is N atom; In another specific embodiment, $X_3$ is C atom. In a specific embodiment, $X_4$ is N atom; In another specific embodiment, $X_4$ is C atom. In a specific embodiment, $X_5$ is N atom; In another specific embodiment, $X_5$ is C atom.

In a more specific embodiment, ring A is

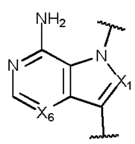 , 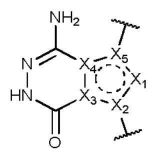 , 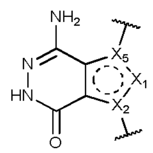 ,

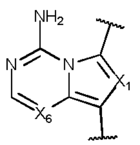 , 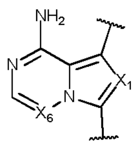 , 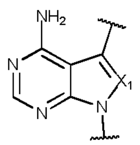 or 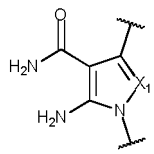 ,

alternatively is

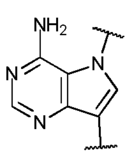 , 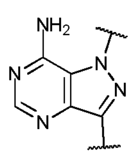 ,

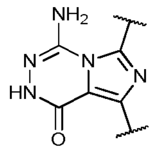 , 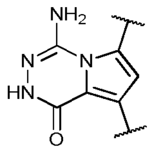 , 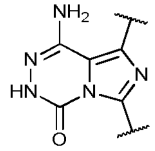 , 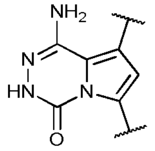 , 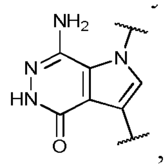 , 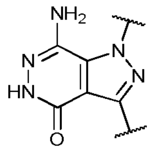 , 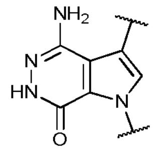 ,

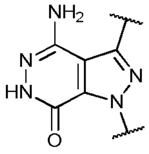, 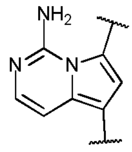, 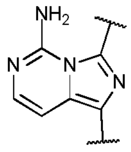, 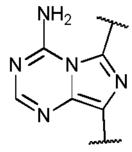, 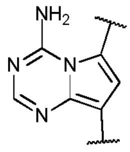, 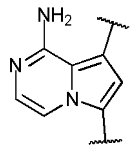, 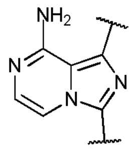, 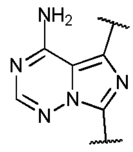,

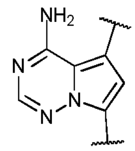, 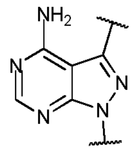, 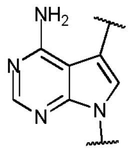, 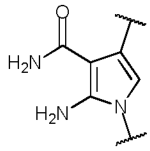 or 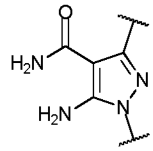

In a more specific embodiment, ring A is

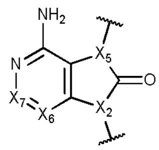, 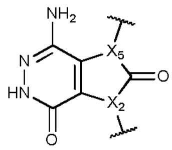, 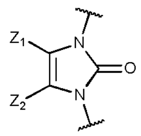

or

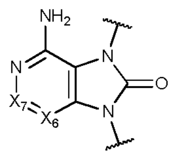,

alternatively is

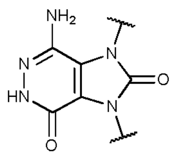, 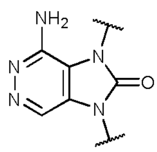, 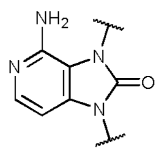 or 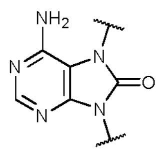

$Y_1$

In one embodiment, $Y_1$ is O; in another embodiment, $Y_1$ is S; in another embodiment, $Y_1$ is NH; in another embodiment, $Y_1$ is $C(R_{Y1})_2$.

In a specific embodiment, $Y_1$ is $C(R_{Y1})_2$, and wherein $R_{Y1}$ is independently H, D, halogen, $C_{1-6}$ alkyl or $C_{1-6}$ haloalkyl; In another specific embodiment, $Y_1$ is $C(R_{Y1})_2$, and wherein $R_{Y1}$ is independently H, D or halogen, alternatively $Y_1$ is $CH_2$.

$Y_2$

In one embodiment, $Y_2$ is bond; in another embodiment, $Y_2$ is $C(R_{Y2})_2$; in another embodiment, $Y_2$ is $C(R_{Y2})_2C(R_{Y2})_2$.

In a specific embodiment, $Y_2$ is $C(R_{Y2})_2$, and wherein $R_{Y2}$ is independently H, D, halogen, $C_{1-6}$ alkyl or $C_{1-6}$ haloalkyl; In another specific embodiment, $Y_2$ is $C(R_{Y2})_2$, and wherein $R_{Y2}$ is independently H, D or halogen, alternatively $Y_2$ is $CH_2$.

In a specific embodiment, $Y_2$ is $C(R_{Y2})_2C(R_{Y2})_2$, and wherein $R_{Y2}$ is independently H, D, halogen, $C_{1-6}$ alkyl or $C_{1-6}$ haloalkyl; In another specific embodiment, $Y_2$ is $C(R_{Y2})_2C(R_{Y2})_2$, and wherein $R_{Y2}$ is independently H, D or halogen, alternatively $Y_2$ is $CH_2CH_2$.

$Y_3$

In one embodiment, $Y_3$ is N; in another embodiment, $Y_3$ is CD; in another embodiment, $Y_3$ is CH.

$Z_1$ and $Z_2$

In one embodiment, $Z_1$ is $C(O)NH_2$, and $Z_2$ is $NH_2$; in another embodiment, $Z_1$ and $Z_2$ are taken together with the atoms to which they are attached to form

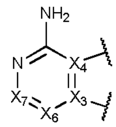

In a specific embodiment, $Z_1$ and $Z_2$ are taken together with the atoms to which they are attached to form

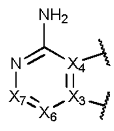,

and $X_6$ is N; In another specific embodiment, $Z_1$ and $Z_2$ are taken together with the atoms to which they are attached to form

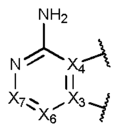,

and $X_6$ is CD or CH; In another specific embodiment, $Z_1$ and $Z_2$ are taken together with the atoms to which they are attached to form

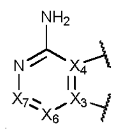,

and $X_6$ is C-OH; In another specific embodiment, $Z_1$ and $Z_2$ are taken together with the atoms to which they are attached to form

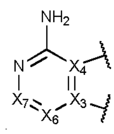,

and $X_7$ is N; In another specific embodiment, $Z_1$ and $Z_2$ are taken together with the atoms to which they are attached to form

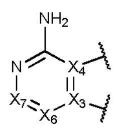,

and $X_7$ is CD or CH.

$R_1$ and $R_1$'

In one embodiment, $R_1$ is H; in another embodiment, $R_1$ is D; in another embodiment, $R_1$ is halogen; in another

embodiment, $R_1$ is OH.

In one embodiment, $R_1$' is H; in another embodiment, $R_1$' is D; in another embodiment, $R_1$' is halogen.

$R_2$ and $R_2$'

In one embodiment, $R_2$ is H; in another embodiment, $R_2$ is D; in another embodiment, $R_2$ is OH; in another embodiment, $R_2$ is $NH_2$.

In one embodiment, $R_2$' is H; in another embodiment, $R_2$' is D; in another embodiment, $R_2$' is $C_{1-6}$ alkyl; in another embodiment, $R_2$' is $C_{1-6}$ haloalkyl.

$R_3$ and $R_3$'

In one embodiment, $R_3$ is H; in another embodiment, $R_3$ is D; in another embodiment, $R_3$ is OH; in another embodiment, $R_3$ is $CH_2ORa$, and wherein $R_a$ is H, $C_{1-6}$ alkyl or $C_{1-6}$ haloalkyl; in another embodiment, $R_3$ is $CH_2OH$;

In one embodiment, $R_3$' is H; in another embodiment, $R_3$' is D; in another embodiment, $R_3$' is halogen; in another embodiment, $R_3$' is $C_{1-6}$ alkyl; in another embodiment, $R_3$' is $C_{1-6}$ haloalkyl; in another embodiment, $R_3$' is $C_{3-6}$ cycloalkyl; in another embodiment, $R_3$' is 3- to 7-membered heterocyclyl.

In a specific embodiment, $R_2$ is OH and $R_3$ is H; In another specific embodiment, $R_2$ is H and $R_3$ is OH; In another specific embodiment, $R_2$ is H and $R_3$ is $CH_2OH$.

In another specific embodiment, -C($R_3$)($R_3$')-C($R_{Y1}$)$_2$- is taken together to form - CH=CH-; In another specific embodiment, -C($R_1$)($R_1$')-C($R_2$)($R_2$')- is taken together to form - CH=CH-.

In another specific embodiment, $R_1$ and $R_2$ are attached to form $C_{1-4}$ alkylene, alternatively methylene.

$R_4$ and $R_4$'

In one embodiment, $R_4$ is H; in another embodiment, $R_4$ is D; in another embodiment, $R_4$ is halogen; in another embodiment, $R_4$ is $C_{1-6}$ alkyl; in another embodiment, $R_4$ is $C_{1-6}$ haloalkyl.

In one embodiment, $R_4$' is H; in another embodiment, $R_4$' is D; in another embodiment, $R_4$' is halogen; in another embodiment, $R_4$' is $C_{1-6}$ alkyl; in another embodiment, $R_4$' is $C_{1-6}$ haloalkyl.

Rand R'

In one embodiment, R is halogen, alternatively F.

In one embodiment, R' is $C_{1-6}$ alkyl; in another embodiment, R' is $C_{1-6}$ haloalkyl; in another embodiment, R' is methyl.

Any technical solution in any one of the above embodiments, or any combination thereof, may be combined with any technical solution in any one of other embodiments, or any combination thereof. For example, any technical solution of ring A, $Y_1$-$Y_3$, $Z_1$-$Z_2$, $R_1$ and $R_1$', $R_2$ and $R_2$', $R_3$ and $R_3$', R and R', or any combination thereof, may be combined. The present disclosure is intended to include all combination of such technical solutions, which are not exhaustively listed here to save space.

The present disclosure specifically relates to the following embodiments:

1. A compound of formula (I), or a tautomer, stereoisomer, prodrug, crystal form, pharmaceutically acceptable salt, hydrate or solvate thereof:

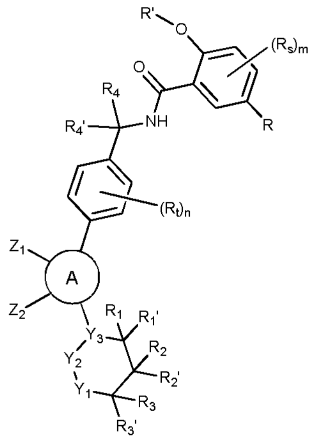

(I)

wherein

ring A is

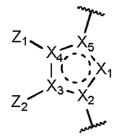 or 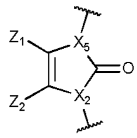 ;

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O, S, NH or $C(R_{Y1})_2$; wherein $R_{Y1}$ is independently H, D, halogen, $C_{1-6}$ alkyl or $C_{1-6}$ haloalkyl;
$Y_2$ is bond, $C(R_{Y2})_2$ or $C(R_{Y2})_2C(R_{Y2})_2$; wherein $R_{Y2}$ is independently H, D, halogen, $C_{1-6}$ alkyl or $C_{1-6}$ haloalkyl;
$Y_3$ is N, CD or CH;
$Z_1$ is $C(O)NH_2$;
$Z_2$ is $NH_2$;
or $Z_1$ and $Z_2$ are taken together with the atoms to which they are attached to form

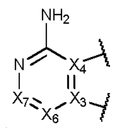 ;

wherein $X_6$ is N, CD, CH or C-OH;
$X_7$ is N, CD or CH;
$R_1$ is H, D, halogen or OH;
$R_1$' is H, D or halogen;
$R_2$ is H, D, OH or $NH_2$;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, halogen, $C_{1-6}$ alkyl, $C_{1-6}$ haloalkyl, $C_{3-6}$ cycloalkyl or 3- to 7-membered heterocyclyl; or $-C(R_3)(R_3')-C(R_{Y1})_2-$ or $-C(R_1)(R_1')-C(R_2)(R_2')-$ is taken together to form -CH=CH-;
or $R_1$ and $R_2$ are attached to form $C_{1-4}$ alkylene;
$R_4$ is H, D, halogen, $C_{1-6}$ alkyl or $C_{1-6}$ haloalkyl;
$R_4$' is H, D, halogen, $C_{1-6}$ alkyl or $C_{1-6}$ haloalkyl;
R is halogen;
R' is $C_{1-6}$ alkyl or $C_{1-6}$ haloalkyl;

$R_s$ is H, D, halogen, $C_{1-6}$ alkyl or $C_{1-6}$ haloalkyl;
$R_t$ is H, D, halogen, $C_{1-6}$ alkyl or $C_{1-6}$ haloalkyl;
m=0, 1, 2 or 3;
n=0, 1, 2, 3 or 4;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups can be substituted by D, up to full deuteration; provided that at least one of $R_2$ and $R_3$ is OH or $CH_2OR_a$.

2. The compound of embodiment 1, or a tautomer, stereoisomer, prodrug, crystal form, pharmaceutically acceptable salt, hydrate or solvate thereof, wherein ring A is

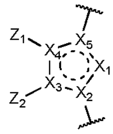,

alternatively is

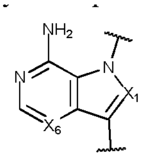, 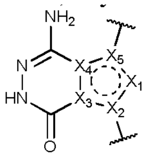, 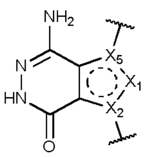, 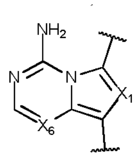, 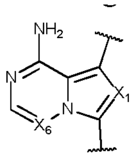, 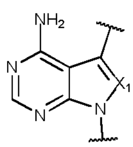

or

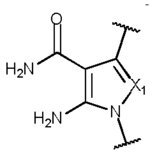,

yet alternatively is

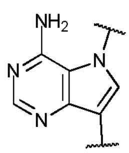, 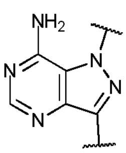, 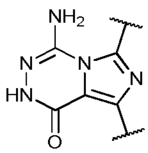, 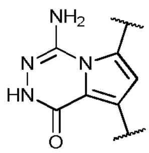, 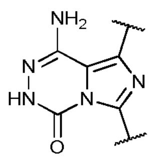,

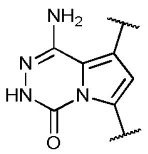, 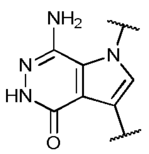, 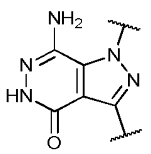, 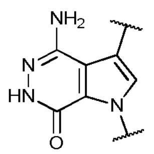, 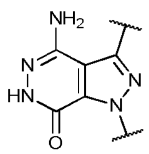, 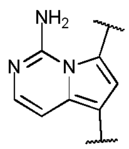, 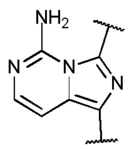,

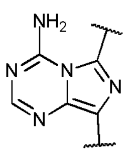, 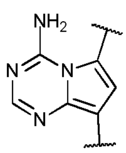, 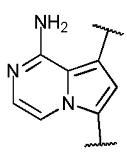, 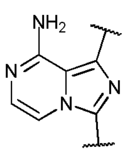, 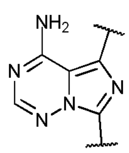, 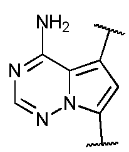, 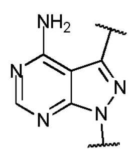, 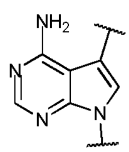,

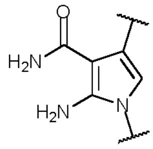 or 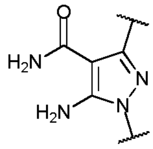

3. The compound of embodiment 1, or a tautomer, stereoisomer, prodrug, crystal form, pharmaceutically acceptable salt, hydrate or solvate thereof, wherein ring A is

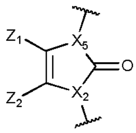,

alternatively is

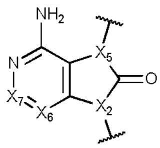, 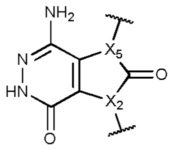, 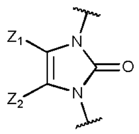 or 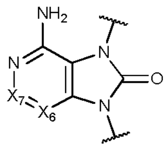,

yet alternatively is

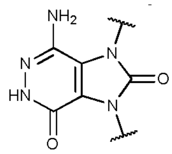, 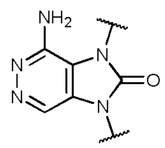, 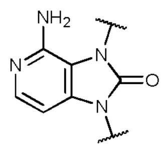 or 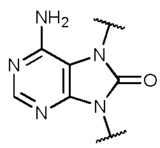

4. The compound of any one of embodiments 1-3, or a tautomer, stereoisomer, prodrug, crystal form, pharmaceutically acceptable salt, hydrate or solvate thereof, wherein $Y_1$ is O.

5. The compound of any one of embodiments 1-3, or a tautomer, stereoisomer, prodrug, crystal form, pharmaceutically acceptable salt, hydrate or solvate thereof, wherein $Y_1$ is $C(R_{Y1})_2$, alternatively $CH_2$.

6. The compound of any one of embodiments 1-5, or a tautomer, stereoisomer, prodrug, crystal form, pharmaceutically acceptable salt, hydrate or solvate thereof, wherein $Y_2$ is bond.

7. The compound of any one of embodiments 1-5, or a tautomer, stereoisomer, prodrug, crystal form, pharmaceutically acceptable salt, hydrate or solvate thereof, wherein $Y_2$ is $C(R_{Y2})_2$ or $C(R_{Y2})_2C(R_{Y2})_2$, alternatively $CH_2$ or $CH_2CH_2$.

8. The compound of any one of embodiments 1-7, or a tautomer, stereoisomer, prodrug, crystal form, pharmaceutically acceptable salt, hydrate or solvate thereof, wherein $Y_3$ is CH.

9. The compound of any one of embodiments 1-8, or a tautomer, stereoisomer, prodrug, crystal form, pharmaceutically acceptable salt, hydrate or solvate thereof, wherein $R_2$ is OH and $R_3$ is H, or $R_2$ is H and $R_3$ is OH, or $R_2$ is H and $R_3$ is $CH_2OH$.

10. The compound of any one of embodiments 1-9, or a tautomer, stereoisomer, prodrug, crystal form, pharmaceutically acceptable salt, hydrate or solvate thereof, wherein R is F.

11. The compound of any one of embodiments 1-10, or a tautomer, stereoisomer, prodrug, crystal form, pharmaceutically acceptable salt, hydrate or solvate thereof, wherein R' is methyl.

12. The compound of any one of embodiments 1-11, or a tautomer, stereoisomer, prodrug, crystal form, pharmaceutically acceptable salt, hydrate or solvate thereof, which has the following general formula:

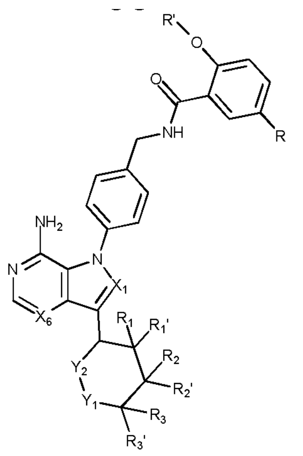

(II),

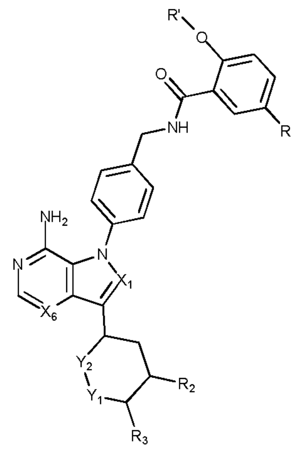

(II-1),

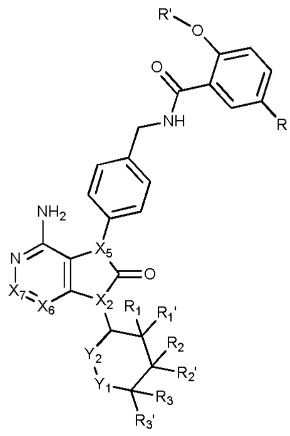

(III),

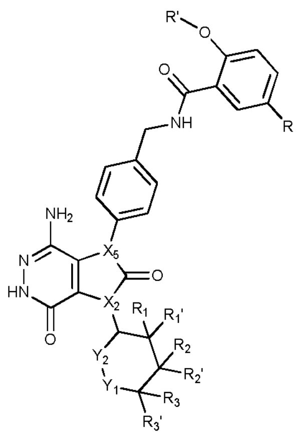

(III-1),

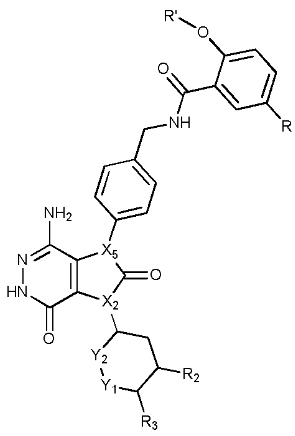

(III-2),

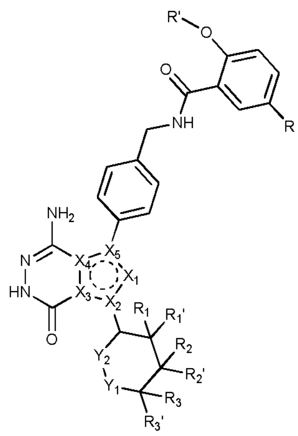

(IV),

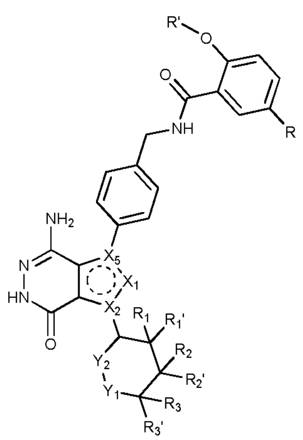

(IV-1),

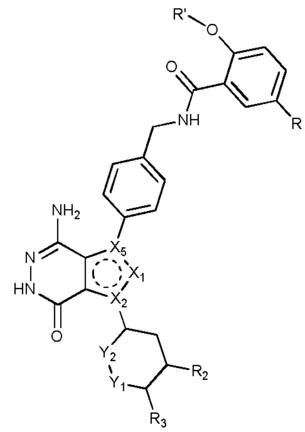

(IV-2),

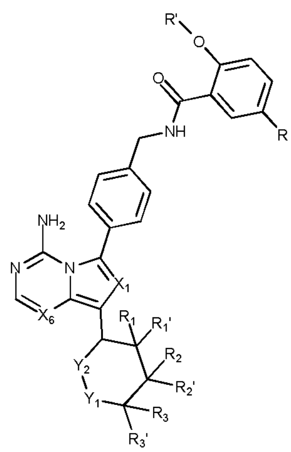

(V),

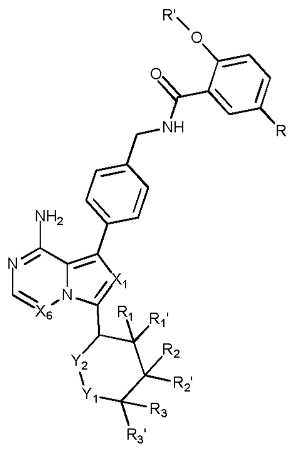 (VI), 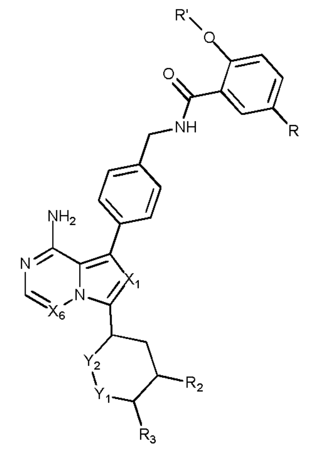 (VI-1), 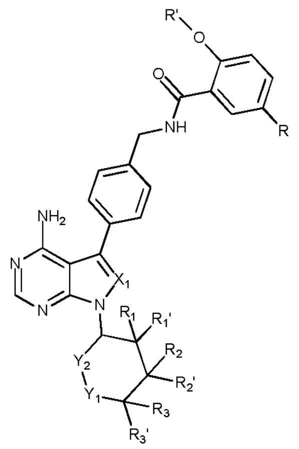 (VII),

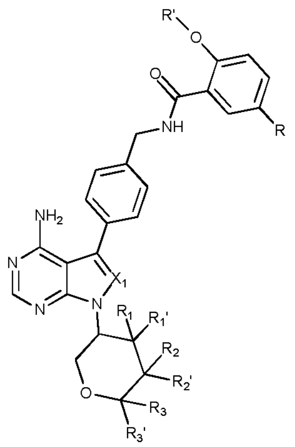 (VII-1), 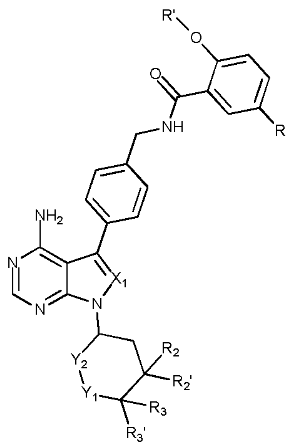 (VII-2), 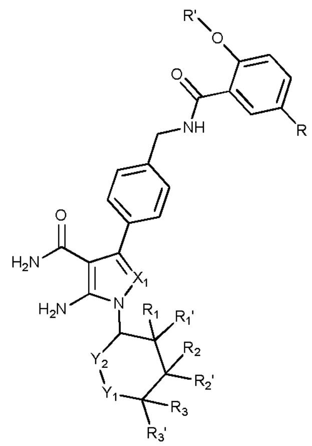

(VIII) or 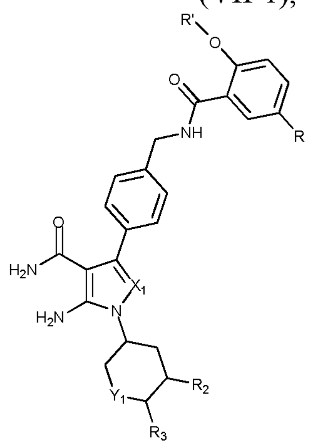 (VIII-1),

wherein each group is as defined in any one of embodiments 1-11.

13. The compound of embodiment 12, or a tautomer, stereoisomer, prodrug, crystal form, pharmaceutically acceptable salt, hydrate or solvate thereof, which is a compound of formula (II):

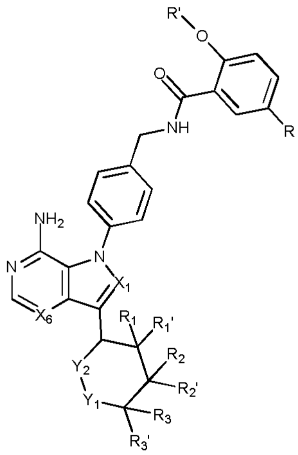

(II)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

14. The compound of embodiment 12, or a tautomer, stereoisomer, prodrug, crystal form, pharmaceutically acceptable salt, hydrate or solvate thereof, which is a compound of formula (II-1):

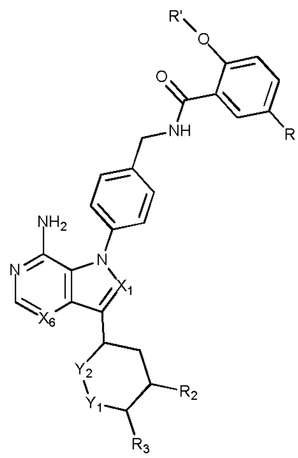

(II-1)

wherein

$X_1$ is N, CD or CH;
$X_6$ is N, CD or CH;
$Y_1$ is O or $C(R_{Y1})_2$; wherein $R_{Y1}$ is independently H, D or halogen;

$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that at least one of $R_2$ and $R_3$ is OH or $CH_2OH$.

15. The compound of embodiment 14, or a tautomer, stereoisomer, prodrug, crystal form, pharmaceutically acceptable salt, hydrate or solvate thereof, wherein:

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

16. The compound of embodiment 12, or a tautomer, stereoisomer, prodrug, crystal form, pharmaceutically acceptable salt, hydrate or solvate thereof, which is a compound of formula (III):

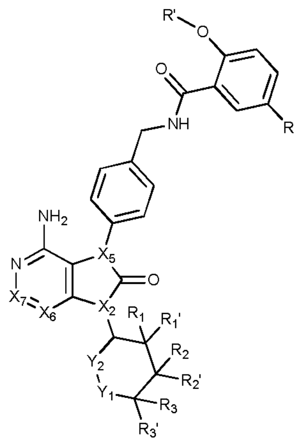

(III)

wherein

$X_2$ is N, CD or CH;
$X_5$ is N, CD or CH;
$X_6$ is N, CD, CH or C-OH;
$X_7$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D,

up to full deuteration;
provided that at least one of $R_2$ and $R_3$ is OH or $CH_2OR_a$.

17. The compound of embodiment 12, or a tautomer, stereoisomer, prodrug, crystal form, pharmaceutically acceptable salt, hydrate or solvate thereof, which is a compound of formula (III-1):

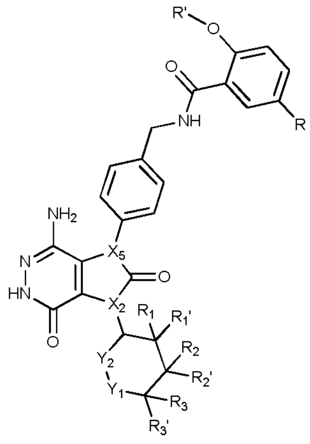

(III-1)

wherein

$X_2$ is N, CD or CH;
$X_5$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

18. The compound of embodiment 12, or a tautomer, stereoisomer, prodrug, crystal form, pharmaceutically acceptable salt, hydrate or solvate thereof, which is a compound of formula (III-2):

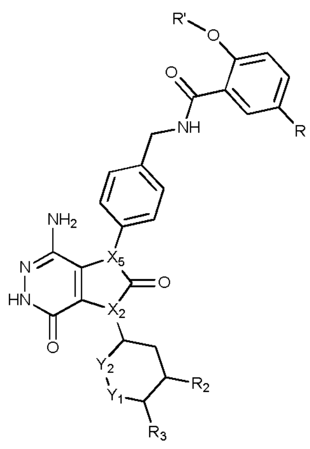

(III-2)

wherein

$X_2$ is N, CD or CH;
$X_5$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that at least one of $R_2$ and $R_3$ is OH or $CH_2OH$.

19. The compound of embodiment 18, or a tautomer, stereoisomer, prodrug, crystal form, pharmaceutically acceptable salt, hydrate or solvate thereof, wherein:

$X_2$ is N;
$X_5$ is N;
$Y_1$ is O or $C(R_{Y1})_2$; wherein $R_{Y1}$ is independently H, D or halogen;
$Y_2$ is $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

20. The compound of embodiment 12, or a tautomer, stereoisomer, prodrug, crystal form, pharmaceutically acceptable salt, hydrate or solvate thereof, which is a compound of formula (IV):

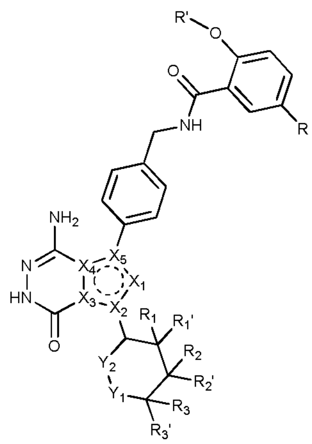

(IV)

wherein

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;

$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

21. The compound of embodiment 12, or a tautomer, stereoisomer, prodrug, crystal form, pharmaceutically acceptable salt, hydrate or solvate thereof, which is a compound of formula (IV-1):

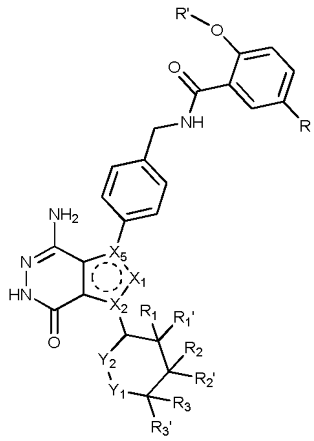

(IV-1)

wherein

the ring where $X_1$, $X_2$ and $X_5$ are located is an aromatic ring;
$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

22. The compound of embodiment 12, or a tautomer, stereoisomer, prodrug, crystal form, pharmaceutically acceptable salt, hydrate or solvate thereof, which is a compound of formula (IV-2):

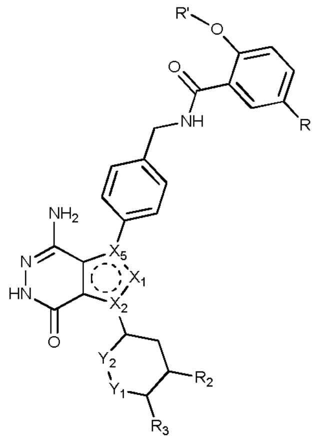

(IV-2)

wherein

the ring where $X_1$, $X_2$ and $X_5$ are located is an aromatic ring;
$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that at least one of $R_2$ and $R_3$ is OH or $CH_2OH$.

23. The compound of embodiment 22, or a tautomer, stereoisomer, prodrug, crystal form, pharmaceutically acceptable salt, hydrate or solvate thereof, wherein: the ring where $X_1$, $X_2$ and $X_5$ are located is an aromatic ring;

$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

24. The compound of embodiment 12, or a tautomer, stereoisomer, prodrug, crystal form, pharmaceutically acceptable salt, hydrate or solvate thereof, which is a compound of formula (V):

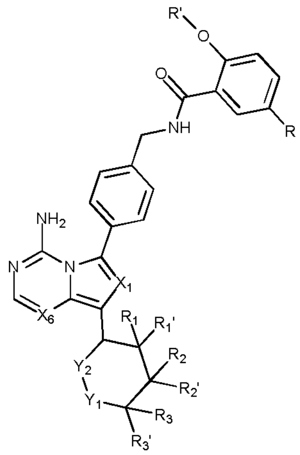

(V)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

25. The compound of embodiment 12, or a tautomer, stereoisomer, prodrug, crystal form, pharmaceutically acceptable salt, hydrate or solvate thereof, which is a compound of formula (VI):

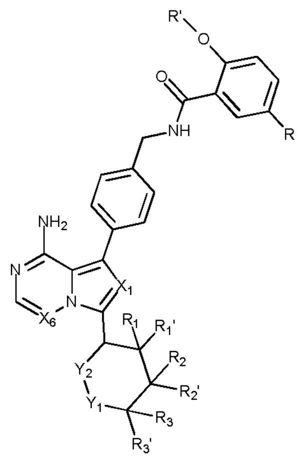

(VI)

wherein

$X_1$ is N, CD or CH;
$X_6$ is N, CD or CH;
$Y_1$ is O or $C(R_{Y1})_2$; wherein $R_{Y1}$ is independently H, D or halogen;

$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

26. The compound of embodiment 12, or a tautomer, stereoisomer, prodrug, crystal form, pharmaceutically acceptable salt, hydrate or solvate thereof, which is a compound of formula (VI-1):

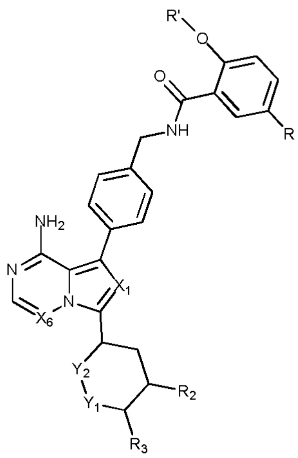

(VI-1)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that at least one of $R_2$ and $R_3$ is OH or $CH_2OH$.

27. The compound of embodiment 26, or a tautomer, stereoisomer, prodrug, crystal form, pharmaceutically acceptable salt, hydrate or solvate thereof, wherein:

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

28. The compound of embodiment 12, or a tautomer, stereoisomer, prodrug, crystal form, pharmaceutically acceptable salt, hydrate or solvate thereof, which is a compound of formula (VII):

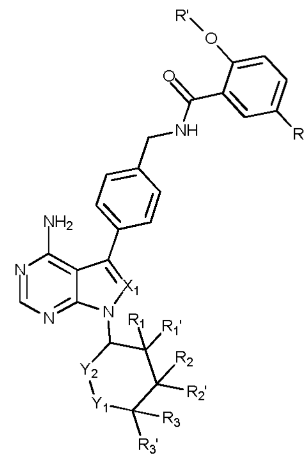

(VII)

wherein

$X_1$ is N, CD or CH;
$Y_1$ is O;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_1)(R_1')-C(R_2)(R_2')$ is taken together to form -CH=CH-;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl, $C_{3-6}$ cycloalkyl and $-CH_2-$ in the above groups can be substituted by D, up to full deuteration;
provided that at least one of $R_2$ and $R_3$ is OH or $CH_2OR_a$.

29. The compound of embodiment 12, or a tautomer, stereoisomer, prodrug, crystal form, pharmaceutically acceptable salt, hydrate or solvate thereof, which is a compound of formula (VII-1):

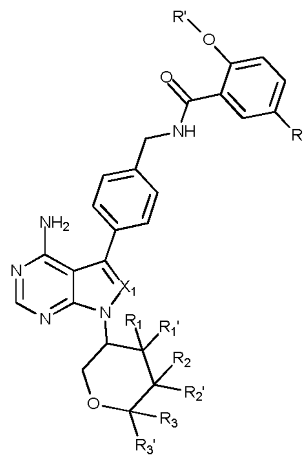

(VII-1)

wherein

$X_1$ is N, CD or CH;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OH$;
$R_3$' is H or D;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl and $-CH_2-$ in the above groups can be substituted by D, up to full deuteration;
provided that at least one of $R_2$ and $R_3$ is OH or $CH_2OH$.

30. The compound of embodiment 29, or a tautomer, stereoisomer, prodrug, crystal form, pharmaceutically acceptable salt, hydrate or solvate thereof, wherein:

$X_1$ is N;
$R_1$ is H, D, halogen or OH;
$R_1$' is H, D or halogen;
$R_2$ is H, D or OH;
$R_2$' is H or D;
$R_3$ is H, D, OH or $CH_2OH$;
$R_3$' is H or D;
or $R_1$ and $R_2$ are attached to form $-CH_2-$;
R is F;
R' is methyl;
provided that at least one of $R_2$ and $R_3$ is OH or $CH_2OH$.

31. The compound of embodiment 12, or a tautomer, stereoisomer, prodrug, crystal form, pharmaceutically acceptable salt, hydrate or solvate thereof, which is a compound of formula (VII):

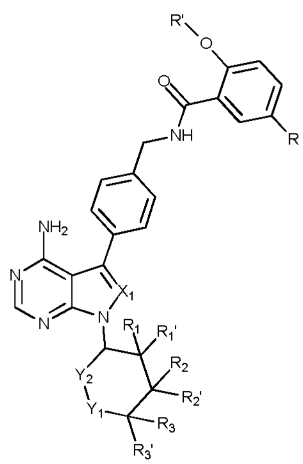
(VII)

wherein

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;

$R_3$' is H, D, $C_{1-6}$ alkyl, $C_{1-6}$ haloalkyl or $C_{3-6}$ cycloalkyl;
or -$C(R_3)(R_3')$-$C(R_{Y1})_2$- is taken together to form -CH=CH-;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

32. The compound of embodiment 12, or a tautomer, stereoisomer, prodrug, crystal form, pharmaceutically acceptable salt, hydrate or solvate thereof, which is a compound of formula (VII-2):

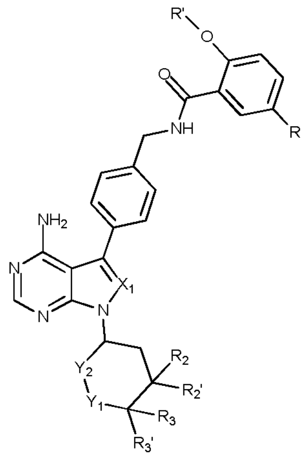

(VII-2)

wherein

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H or D;
or -$C(R_3)(R_3')$-$C(R_{Y1})_2$- is taken together to form -CH=CH-;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that at least one of $R_2$ and $R_3$ is OH or $CH_2OR_a$.

33. The compound of embodiment 32, or a tautomer, stereoisomer, prodrug, crystal form, pharmaceutically acceptable salt, hydrate or solvate thereof, wherein:

$X_1$ is N;
$Y_1$ is $C(R_{Y1})_2$; wherein $R_{Y1}$ is independently H or D;
$Y_2$ is bond, $C(R_{Y2})_2$ or $C(R_{Y2})_2C(R_{Y2})_2$; wherein $R_{Y2}$ is independently H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H or D;
or -$C(R_3)(R_3')$-$C(R_{Y1})_2$- is taken together to form -CH=CH-;
R is F;
R' is methyl;
wherein one or more H of $C_{1-6}$ alkyl and $C_{1-6}$ haloalkyl in the above groups can be substituted by D, up to full deuteration;

provided that at least one of $R_2$ and $R_3$ is OH or $CH_2OR_a$.

34. The compound of embodiment 12, or a tautomer, stereoisomer, prodrug, crystal form, pharmaceutically acceptable salt, hydrate or solvate thereof, which is a compound of formula (VIII):

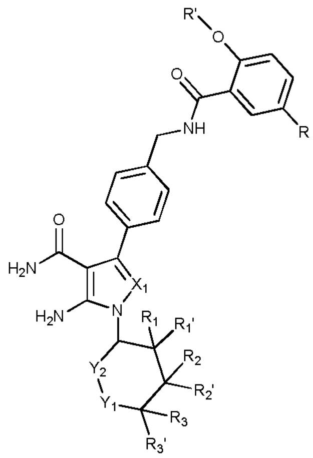

(VIII)

wherein

$X_1$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl, $C_{3-6}$ cycloalkyl or 3- to 7-membered heterocyclyl; or -C($R_3$)($R_3$')-C($R_{Y1}$)$_2$- or -C($R_1$)($R_1$')-C($R_2$)($R_2$')- is taken together to form -CH=CH-;
or $R_1$ and $R_2$ are attached to form $C_{1-4}$ alkylene;
R is halogen;
R' is $C_{1-6}$ alkyl or $C_{1-6}$ haloalkyl;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups can be substituted by D, up to full deuteration; provided that at least one of $R_2$ and $R_3$ is OH or $CH_2OR_a$.

35. The compound of embodiment 34, or a tautomer, stereoisomer, prodrug, crystal form, pharmaceutically acceptable salt, hydrate or solvate thereof, wherein:

$X_1$ is N, CD or CH;
$Y_1$ is O;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or -C($R_1$)($R_1$')-C($R_2$)($R_2$') is taken together to form -CH=CH-;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

36. The compound of embodiment 34, or a tautomer, stereoisomer, prodrug, crystal form, pharmaceutically acceptable salt, hydrate or solvate thereof, wherein:

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_3)(R_3')-C(R_{Y1})_2-$ is taken together to form -CH=CH-;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups can be substituted by D, up to full deuteration;
provided that at least one of $R_2$ and $R_3$ is OH or $CH_2OR_a$.

37. The compound of embodiment 12, or a tautomer, stereoisomer, prodrug, crystal form, pharmaceutically acceptable salt, hydrate or solvate thereof, which is a compound of formula (VIII-1):

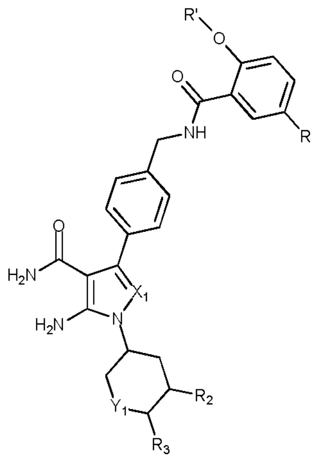

(VIII-1)

wherein

$X_1$ is N, CD or CH;
$Y_1$ is O, CHD, $CD_2$ or $CH_2$;
$R_2$ is H, D or OH;
$R_3$ is H, D or $CH_2OH$;
R is halogen;
R' is $C_{1-6}$ alkyl or $C_{1-6}$ haloalkyl;
wherein one or more H of $C_{1-6}$ alkyl and $C_{1-6}$ haloalkyl in the above groups can be substituted by D, up to full deuteration;
provided that $R_2$ and $R_3$ are not both H.

38. The compound of embodiment 37, or a tautomer, stereoisomer, prodrug, crystal form, pharmaceutically acceptable salt, hydrate or solvate thereof, wherein:

$X_1$ is N;
$Y_1$ is O, CHD, $CD_2$ or $CH_2$;
$R_2$ is H, D or OH;
$R_3$ is H, D or $CH_2OH$;
R is F;

R' is methyl;
provided that $R_2$ and $R_3$ are not both H.

39. A compound, or a tautomer, stereoisomer, prodrug, crystal form, pharmaceutically acceptable salt, hydrate or solvate thereof, wherein the compound is:

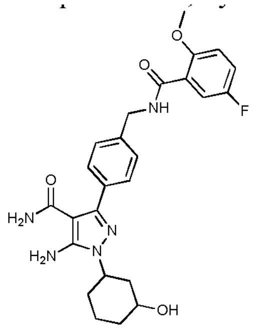 , 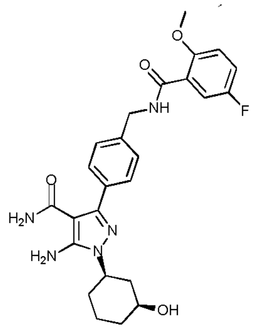 , 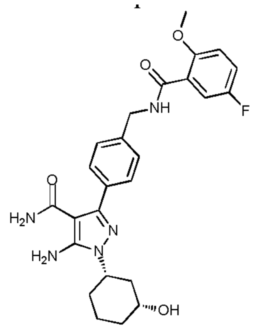 , 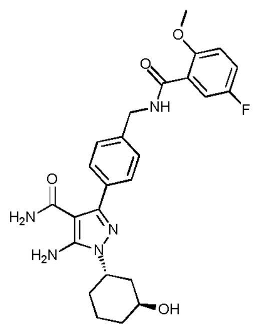 ,

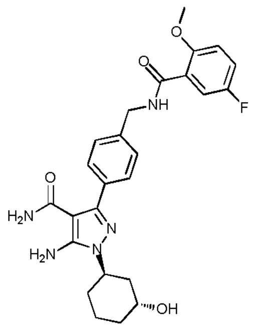 , 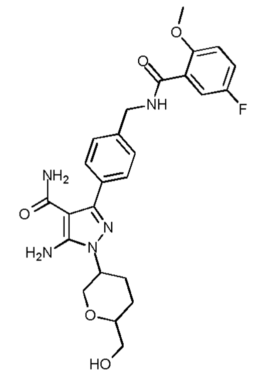 , 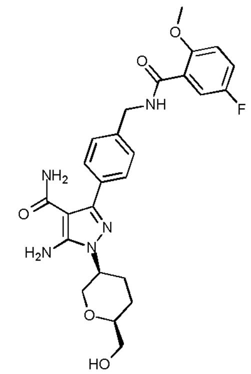 , 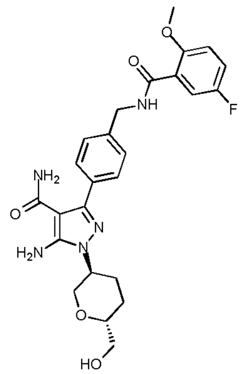 ,

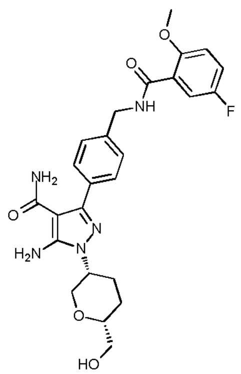 , 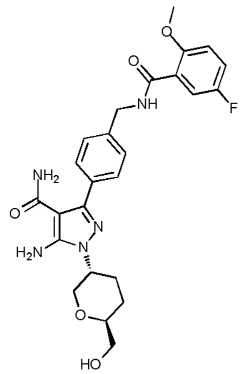 , 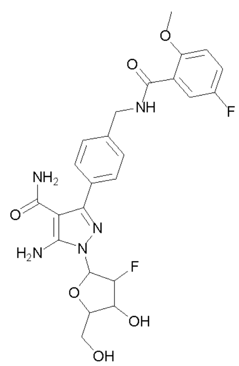 , 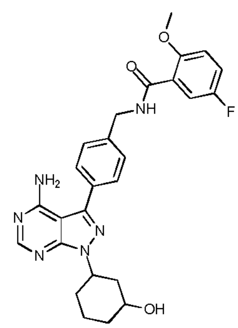 ,

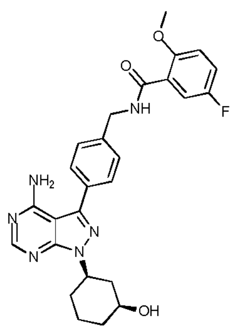 , 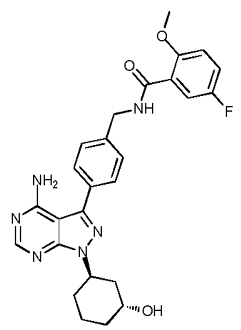 , 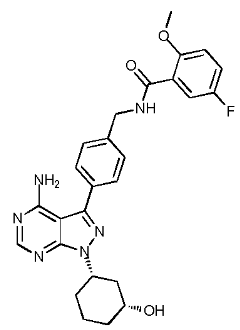 , 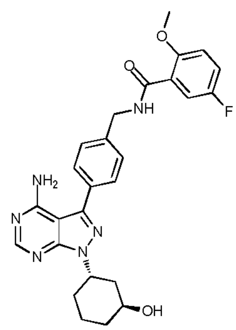 ,

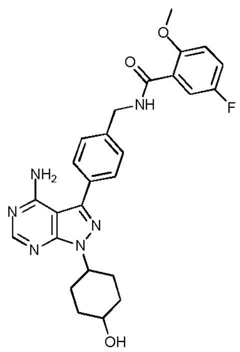 , 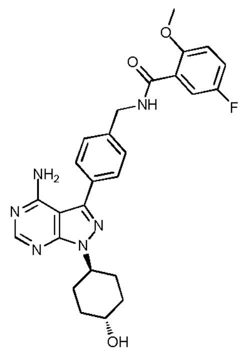 , 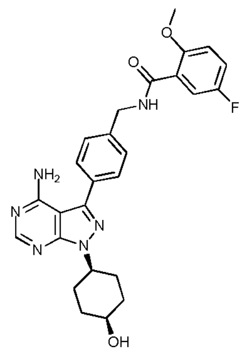 , 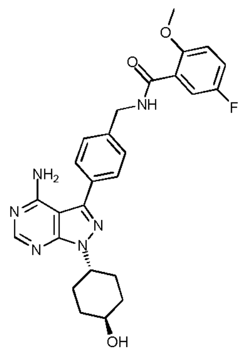 ,

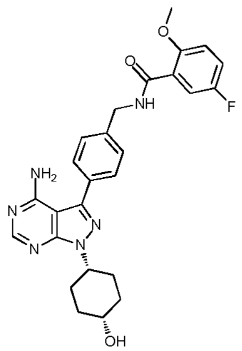 , 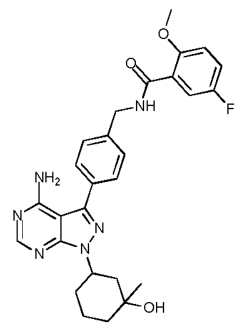 , 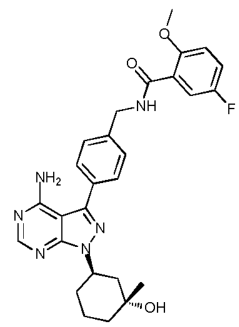 , 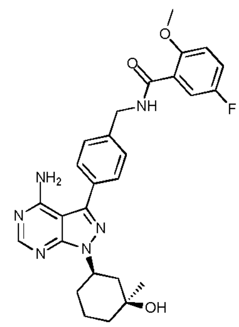 ,

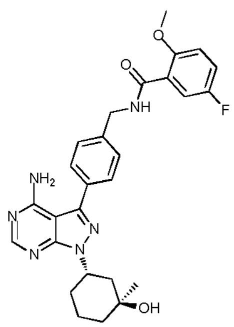 , 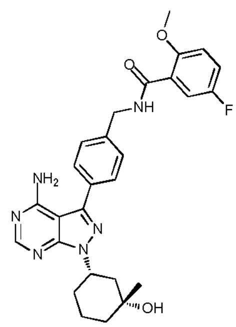 , 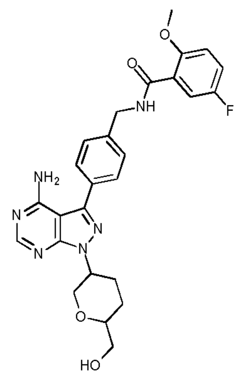 , 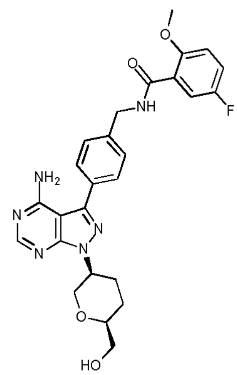 ,

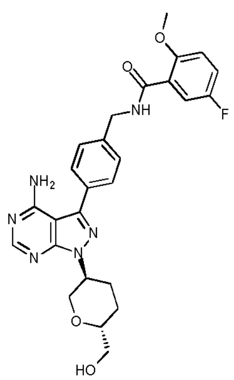 , 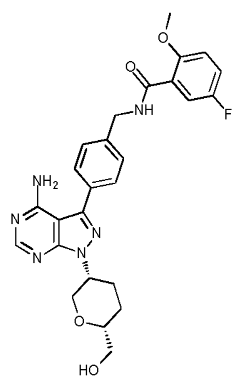 , 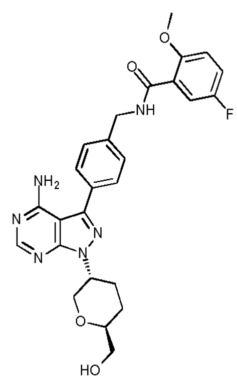 , 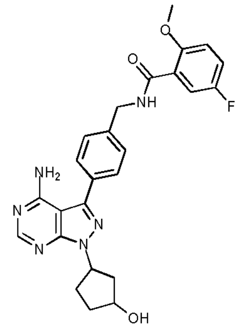 ,

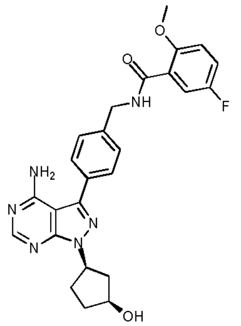
,
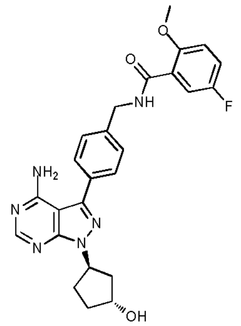
,
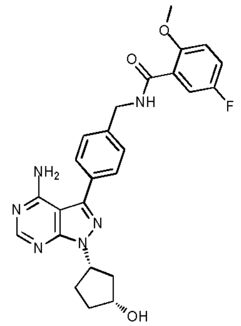
,
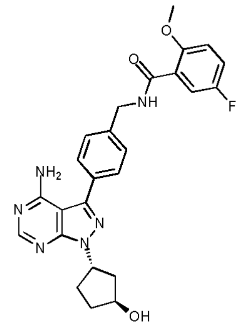
,

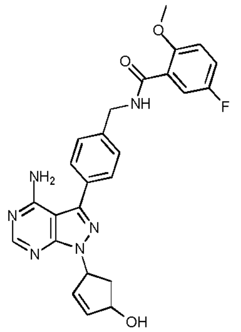
,
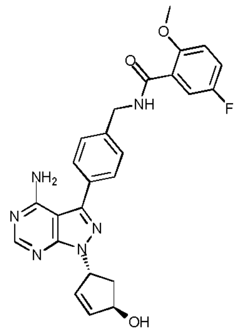
,
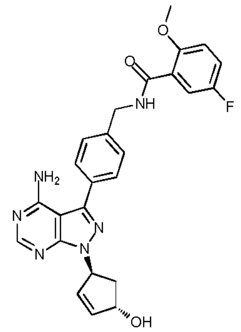
,
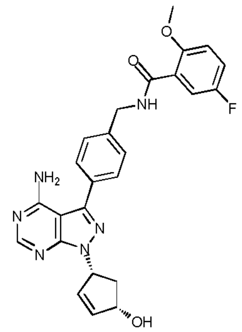
,

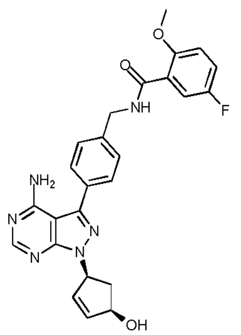
,
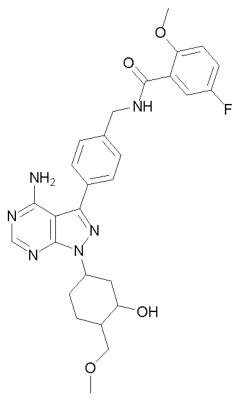
,
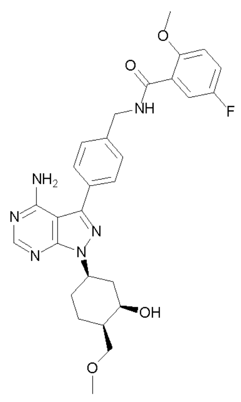
,
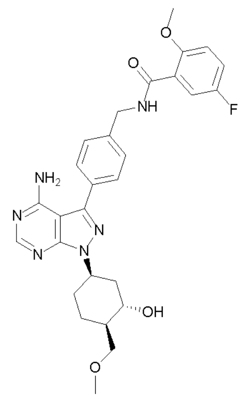
,

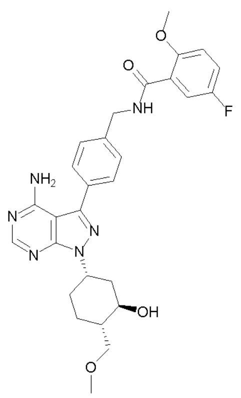
,
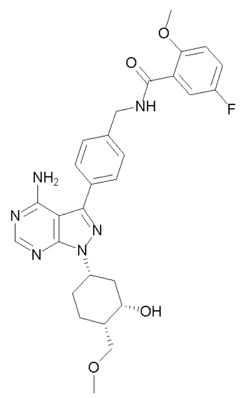
,
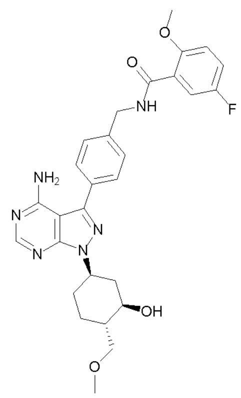
,
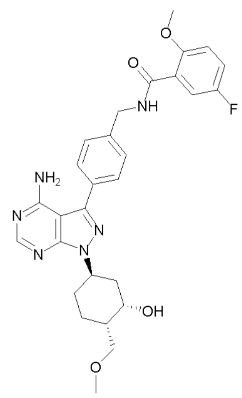
,

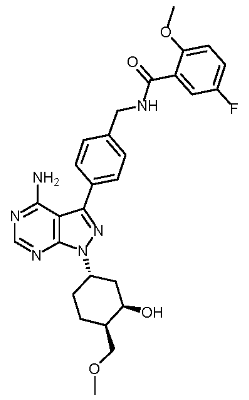 , 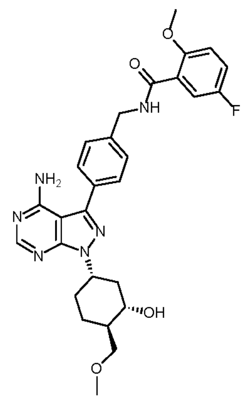 , 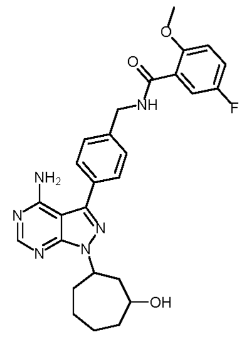 , 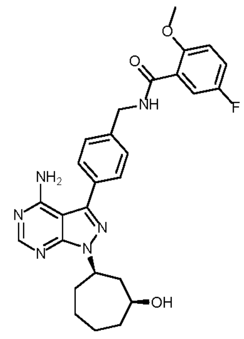 ,

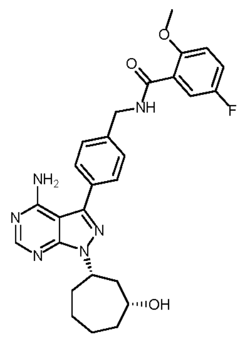 , 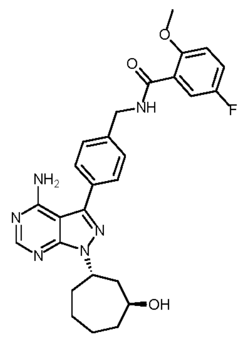 , 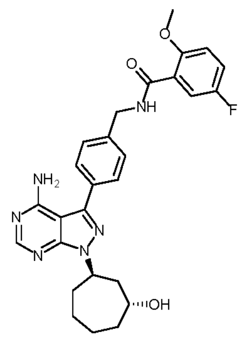 , 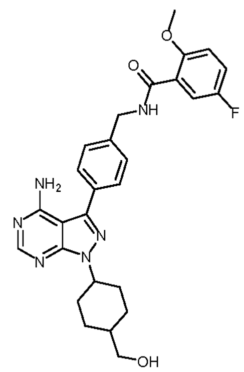 ,

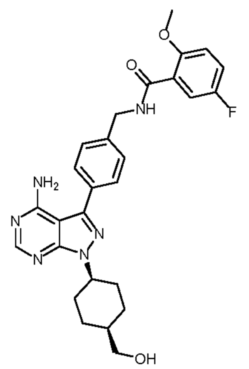 , 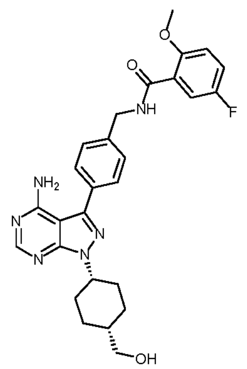 , 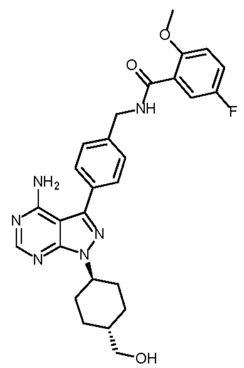 , 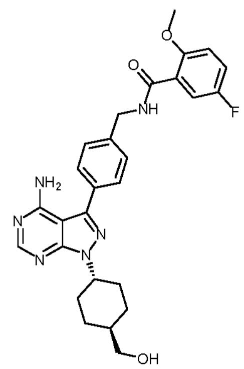 ,

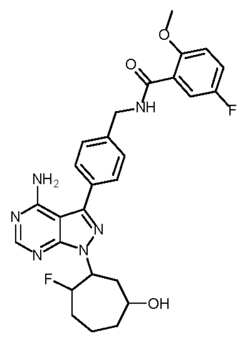 , 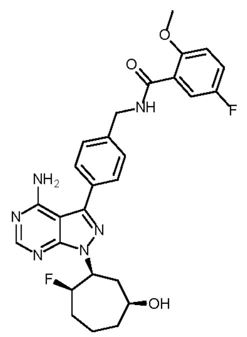 , 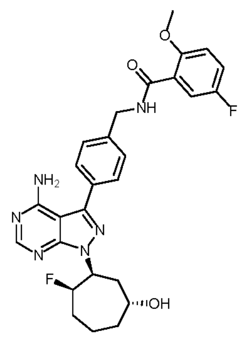 , 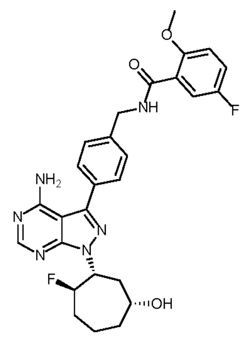 ,

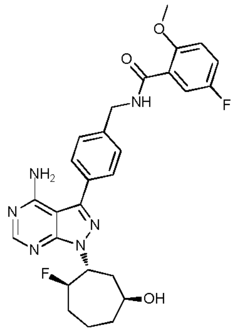 ,

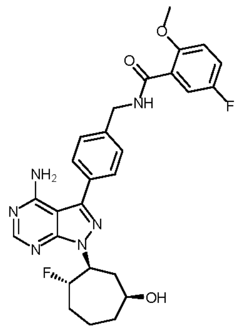 ,

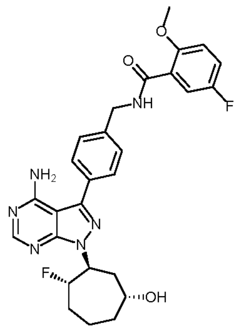 ,

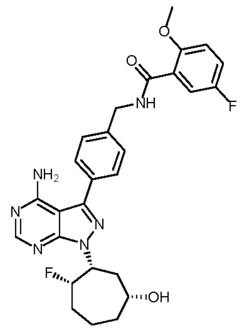 ,

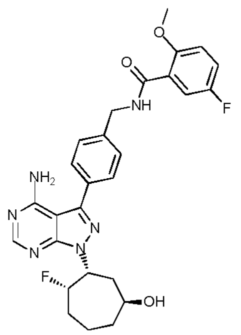 ,

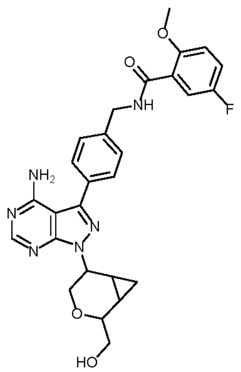 ,

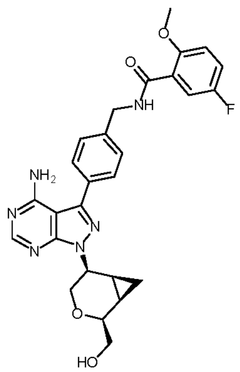 ,

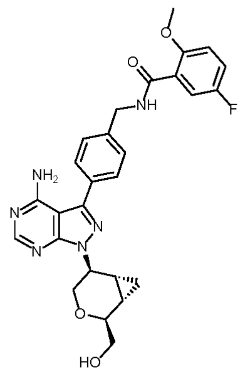 ,

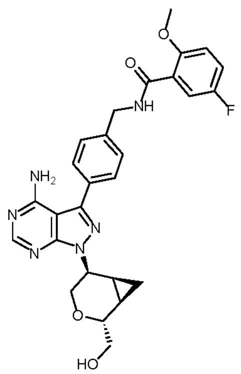 ,

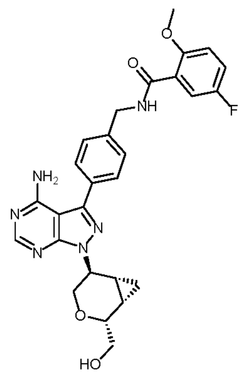 ,

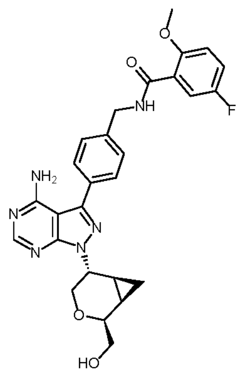 ,

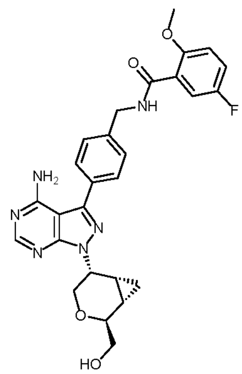 ,

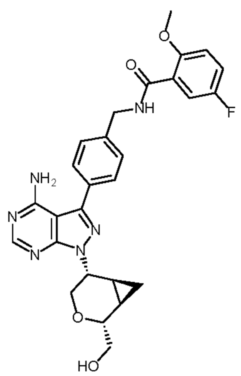 ,

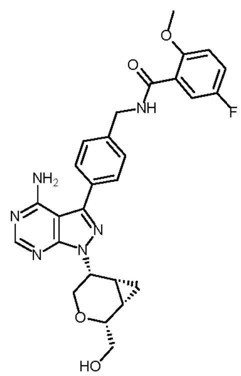 ,

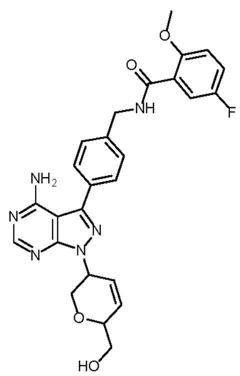 ,

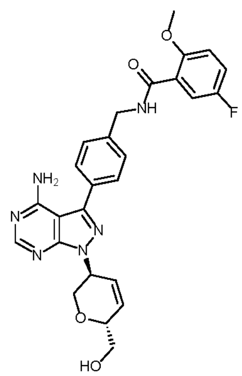 ,

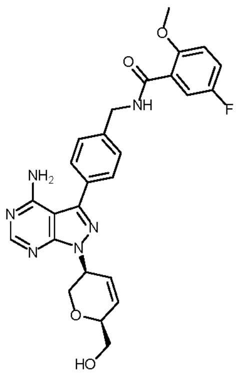 ,
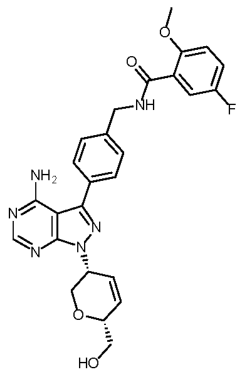 ,
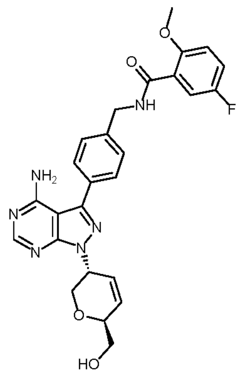 ,
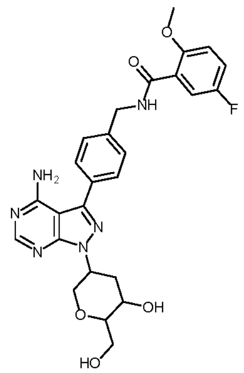 ,

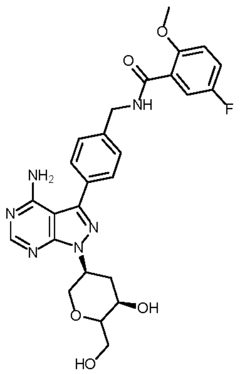 ,
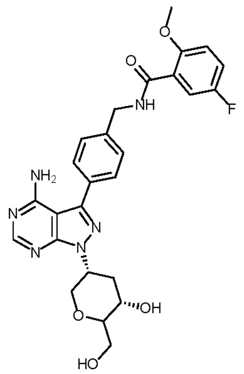 ,
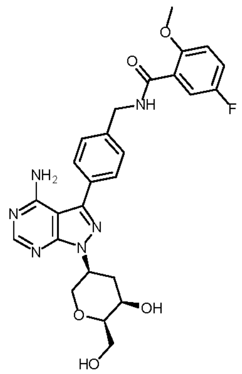 ,
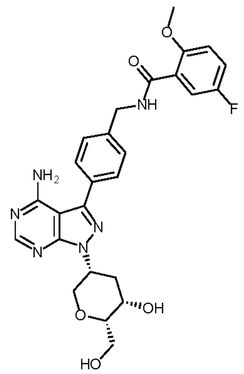 ,

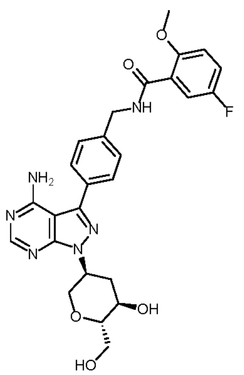 ,
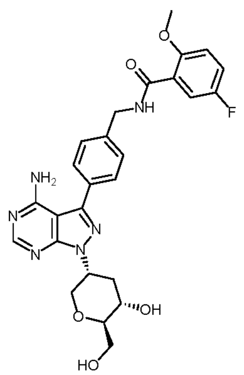 ,
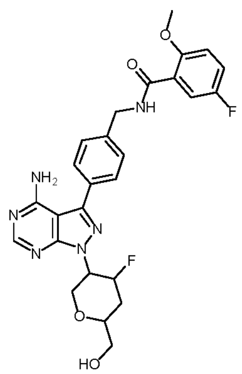 ,
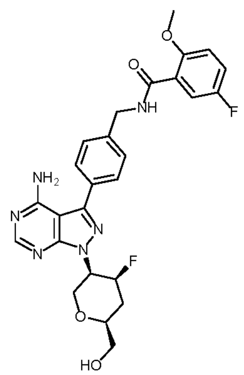 ,

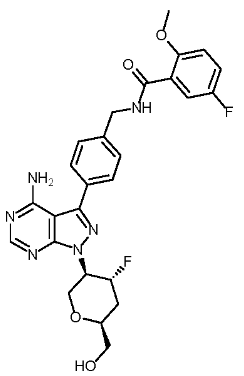 ,
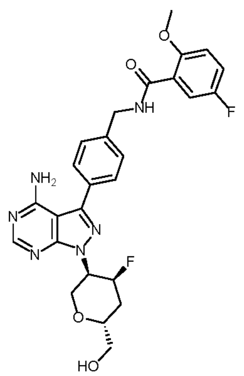 ,
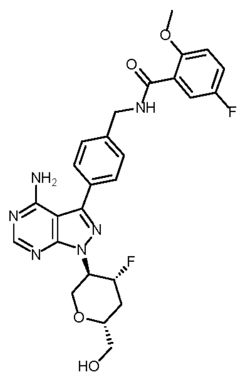 ,
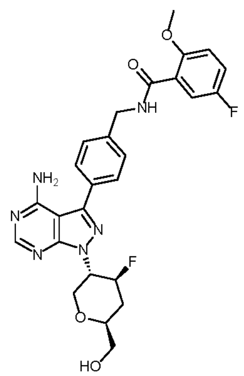 ,

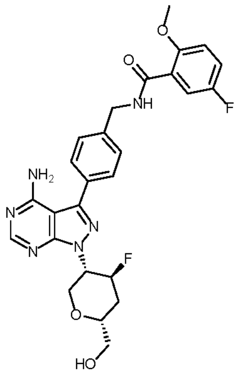 , 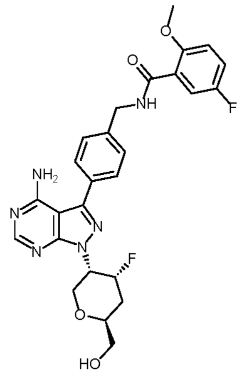 , 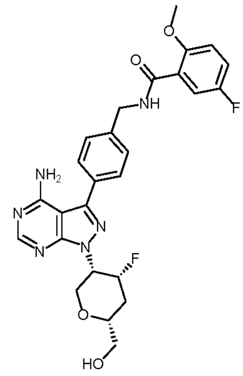 , 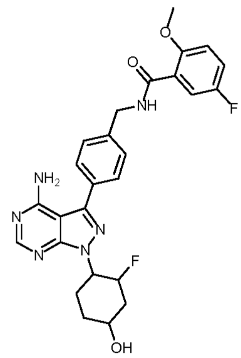 ,

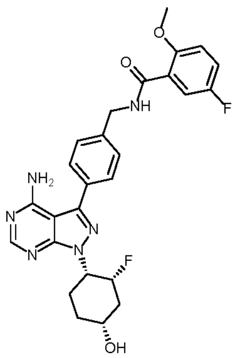 , 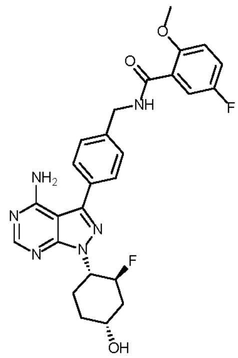 , 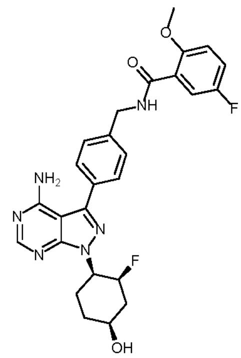 , 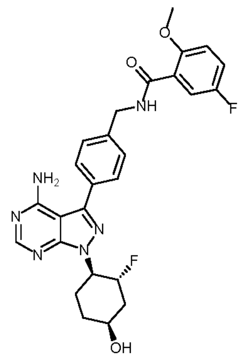 ,

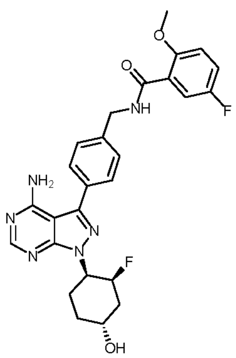 , 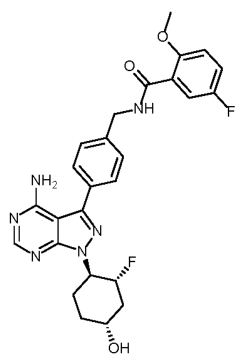 , 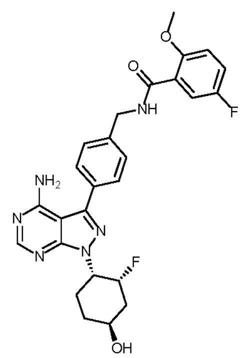 , 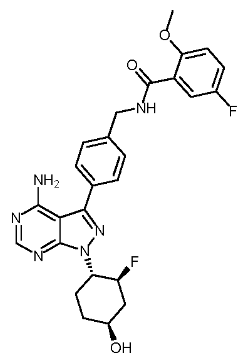 ,

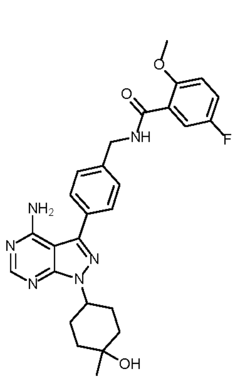 , 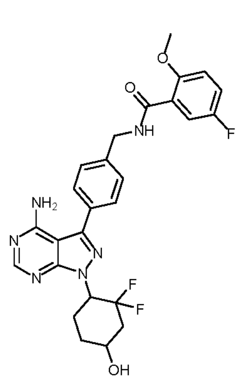 , 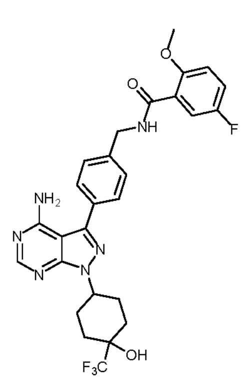 , 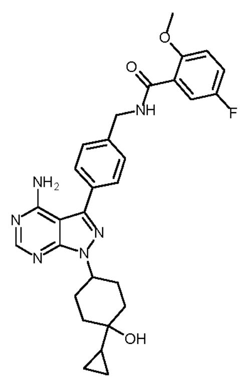 ,

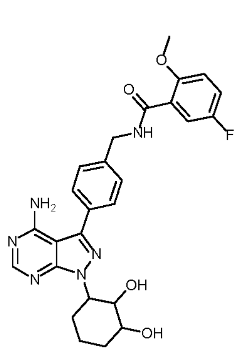 , 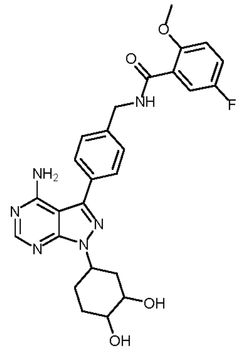 , 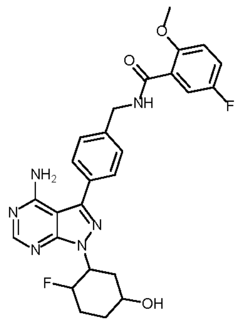 , 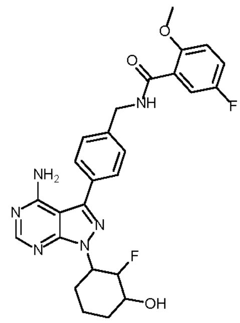 ,

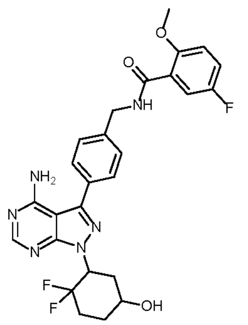 , 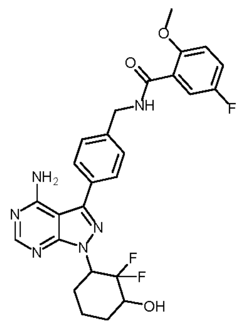 , 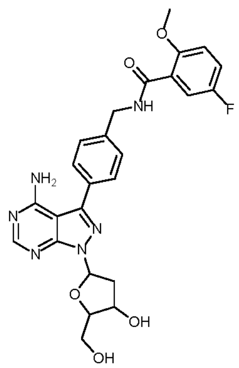 , 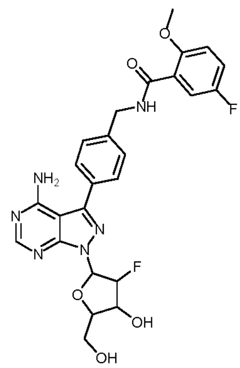 ,

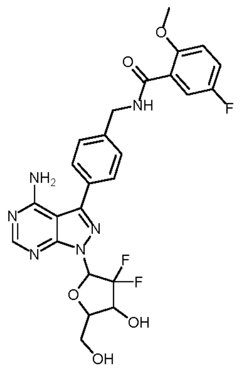 , 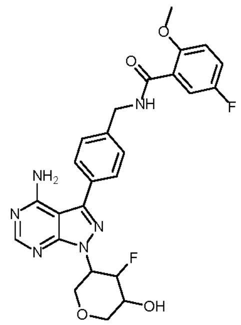 , 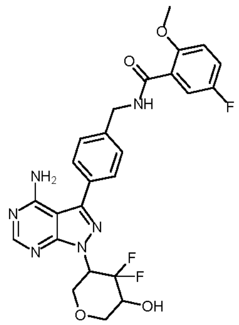 , 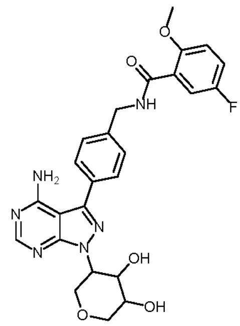 ,

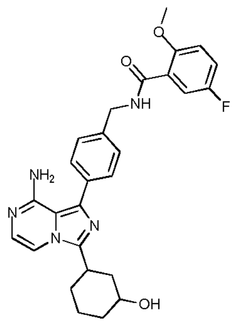 , 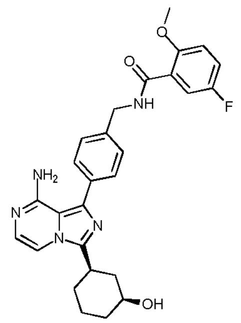 , 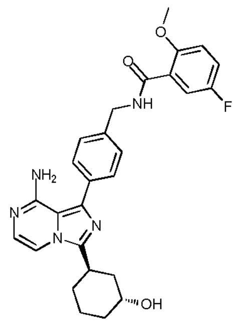 , 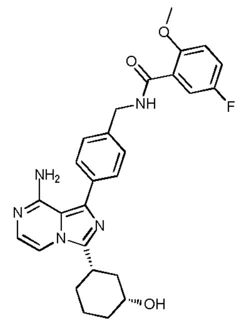 ,

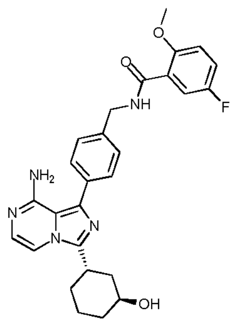 , 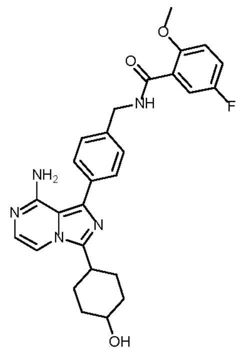 , 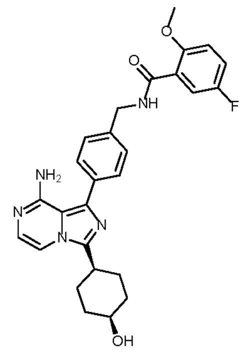 , 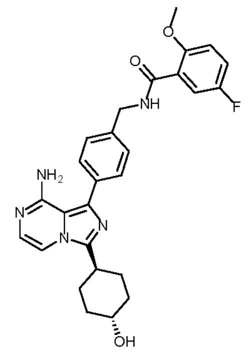 ,

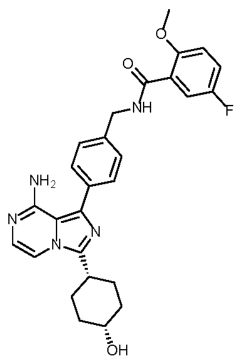 , 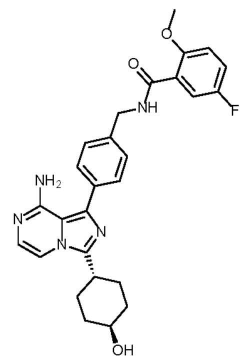 , 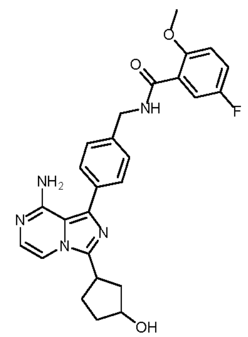 , 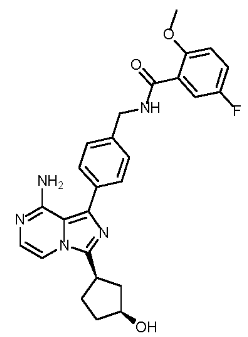 ,

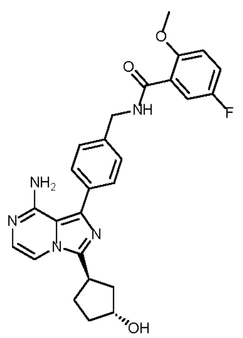 , 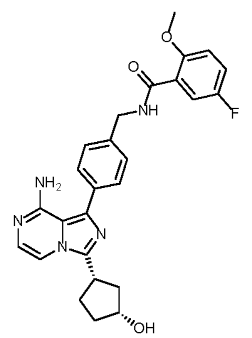 , 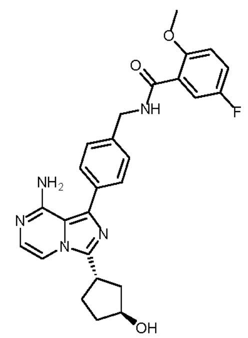 , 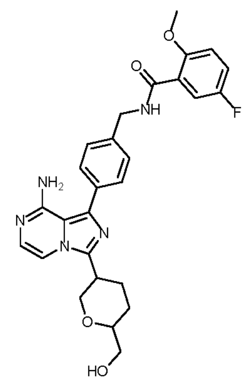 ,

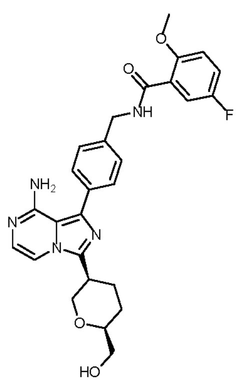 , 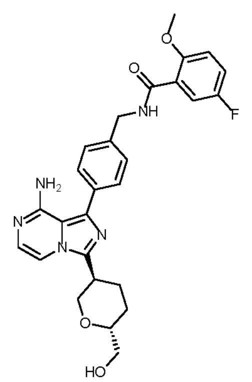 , 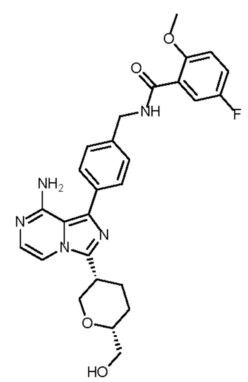 , 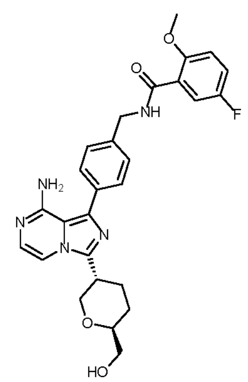 ,

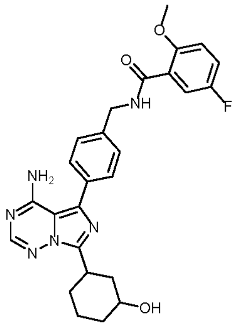 ,

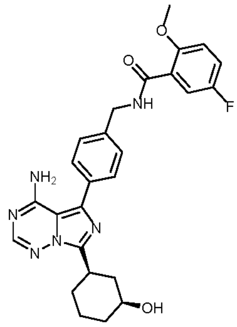 ,

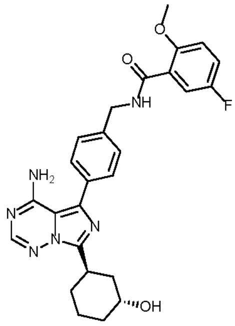 ,

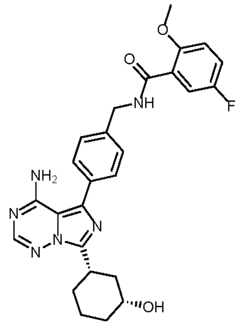 ,

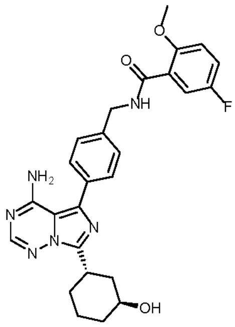 ,

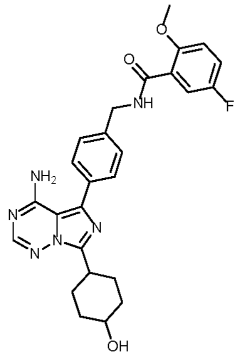 ,

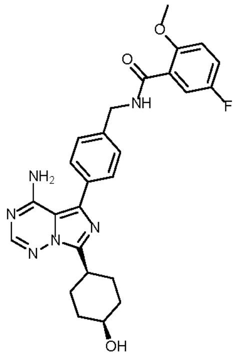 ,

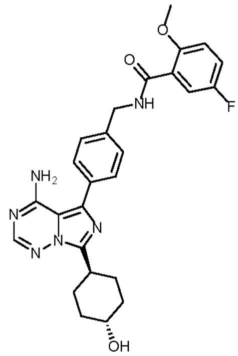 ,

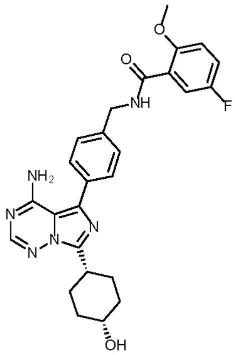 ,

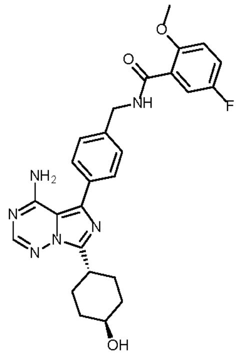 ,

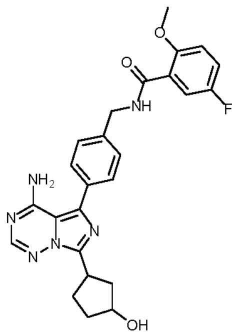 ,

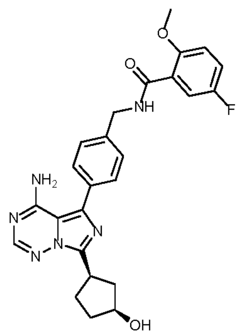 ,

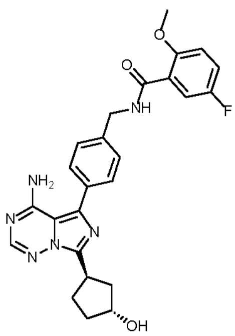 ,

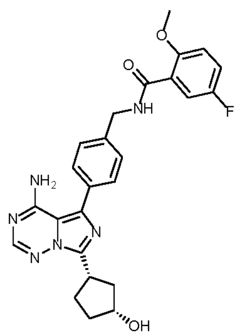 ,

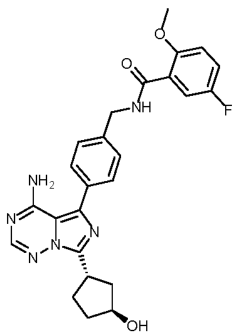 ,

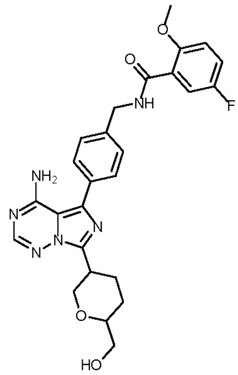 ,

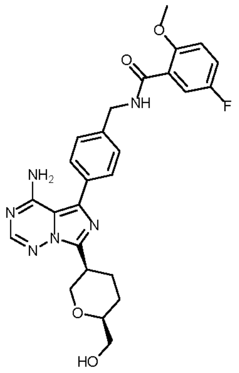,

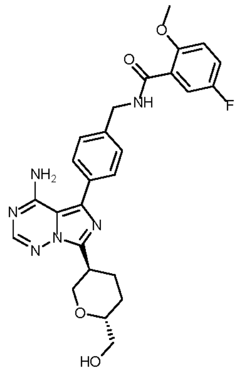,

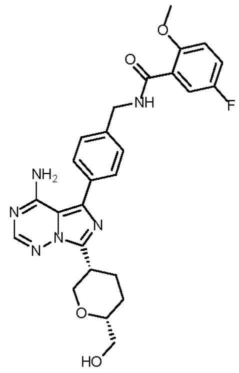,

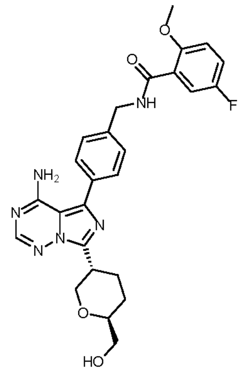,

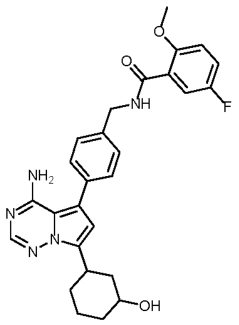,

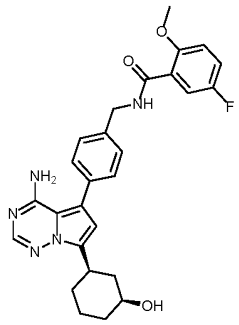,

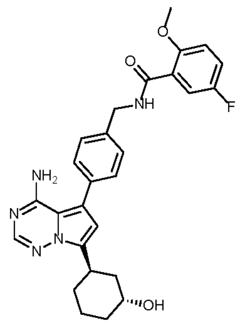,

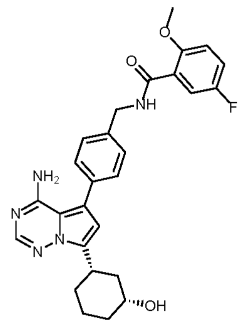,

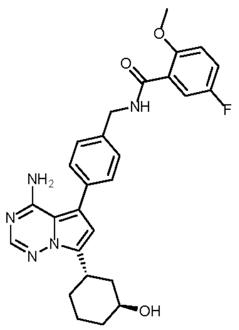,

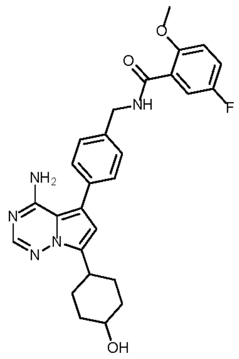,

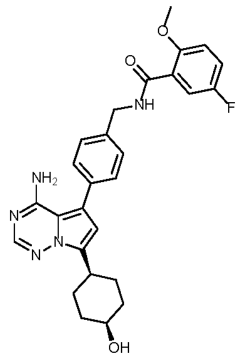,

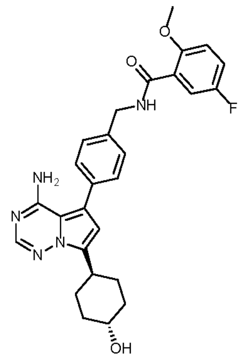,

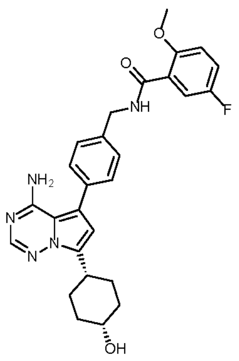,

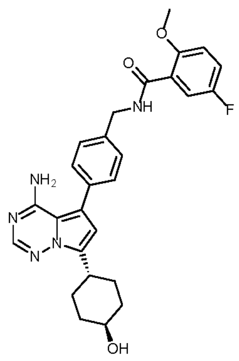,

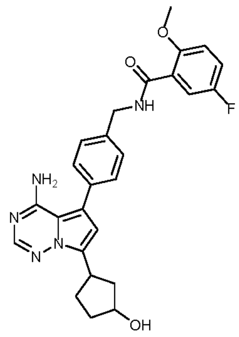,

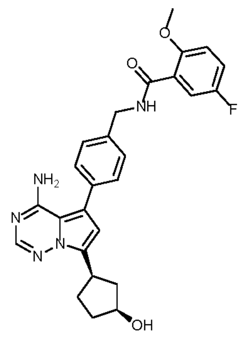,

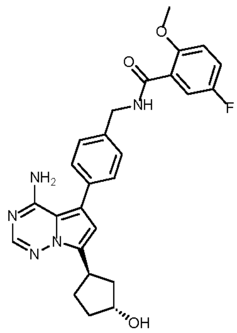 , 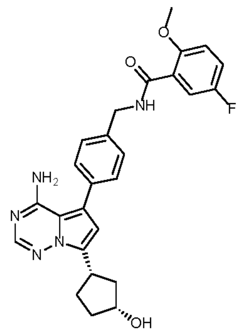 , 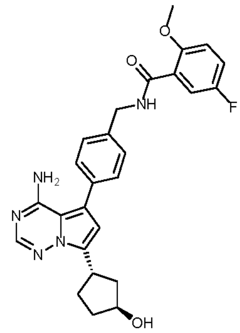 , 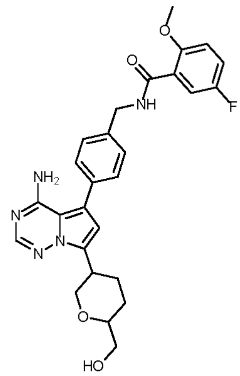 ,

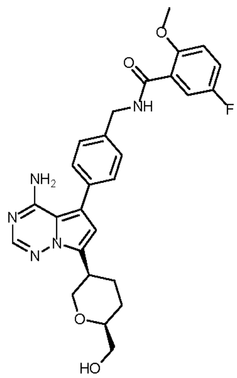 , 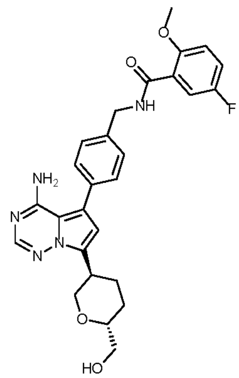 , 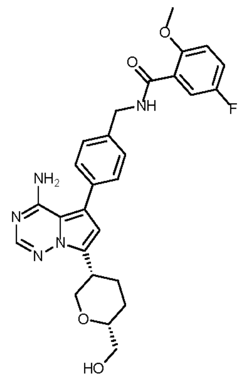 , 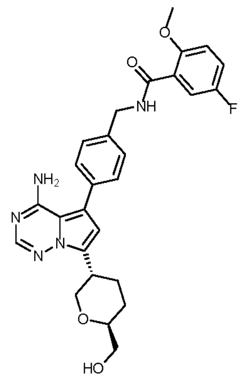 ,

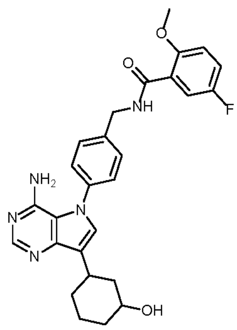 , 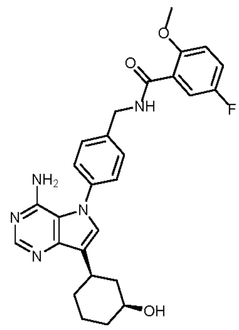 , 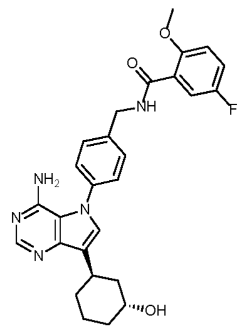 , 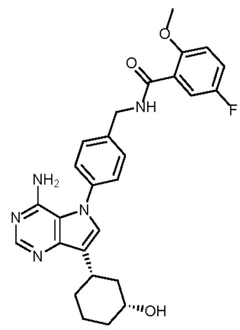 ,

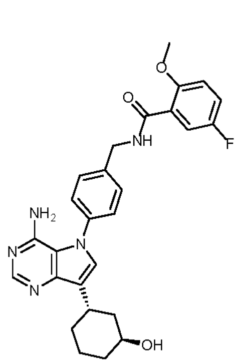 , 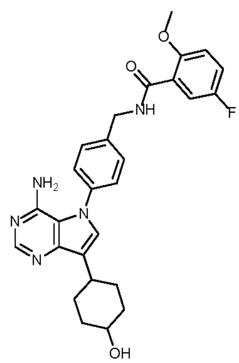 , 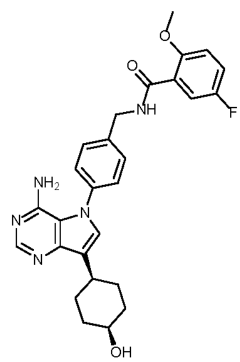 , 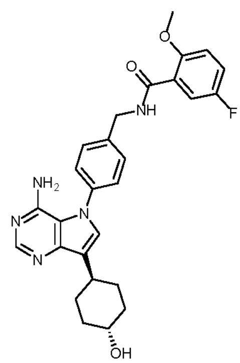 ,

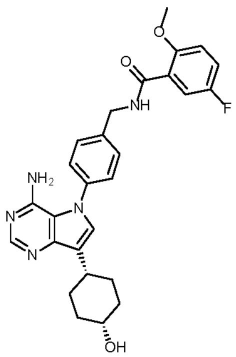 , 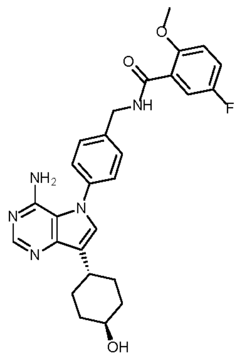 , 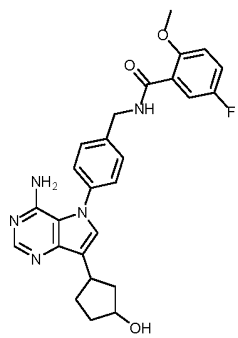 , 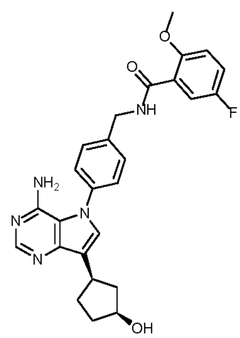 ,

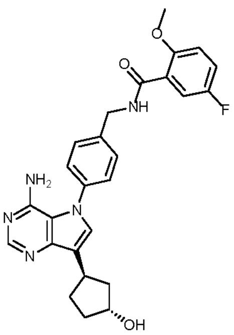 , 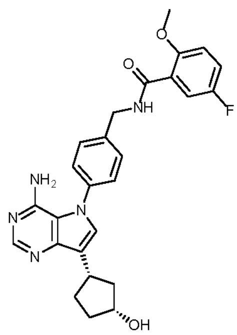 , 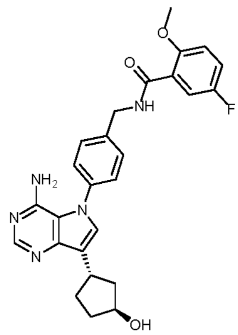 , 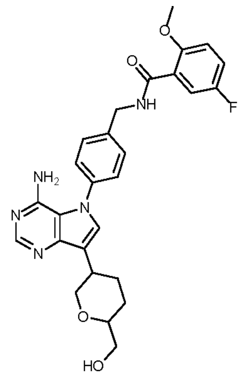 ,

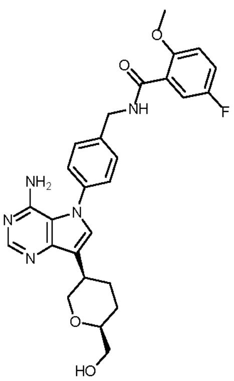 , 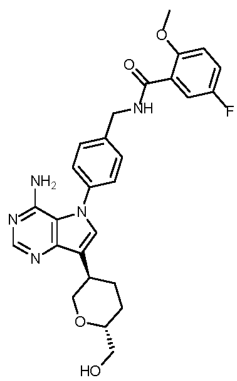 , 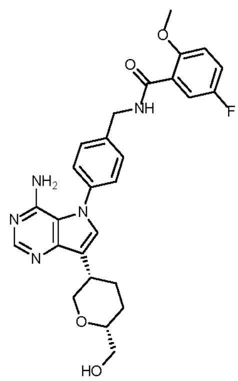 , 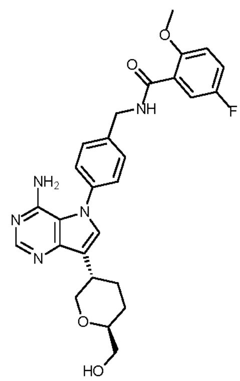 ,

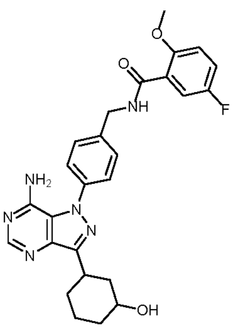 , 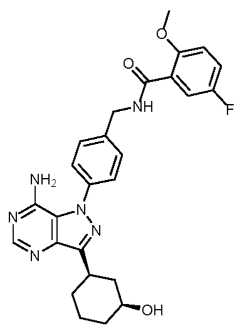 , 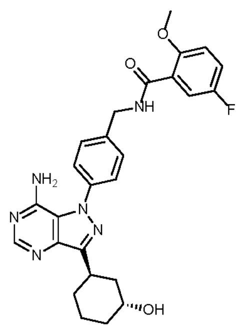 , 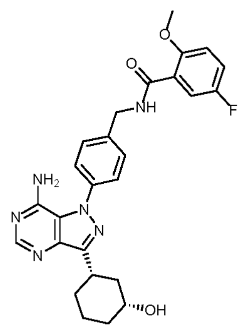 ,

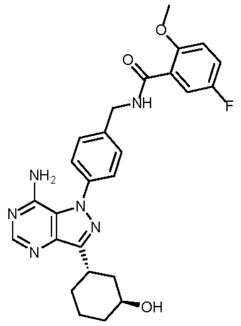, 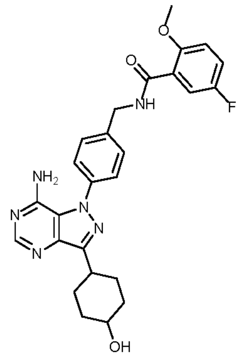, 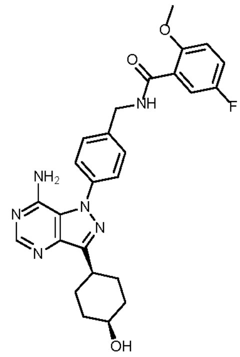, 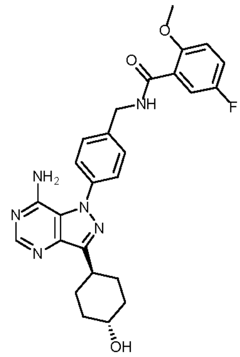,

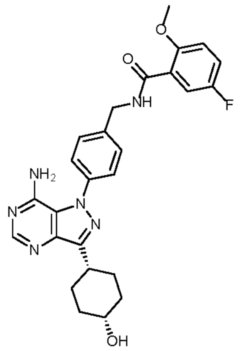, 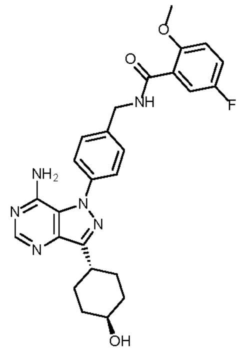, 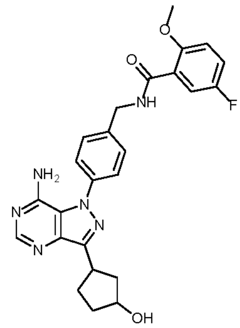, 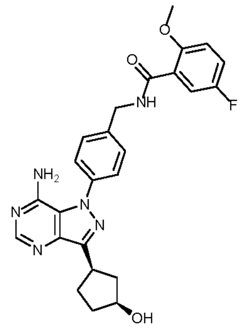,

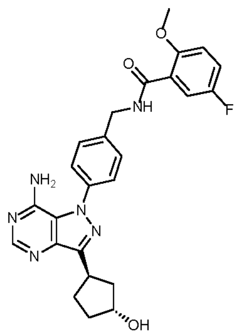, 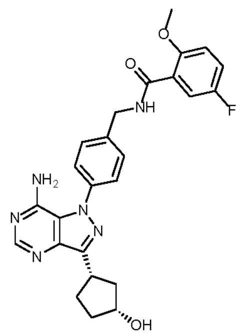, 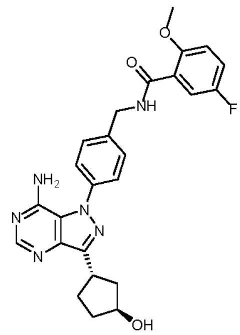, 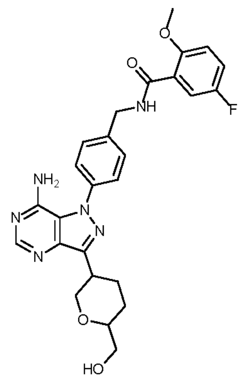,

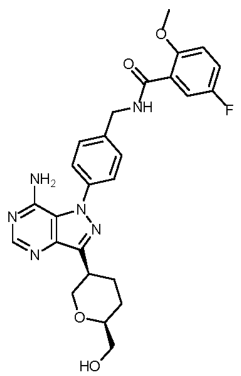, 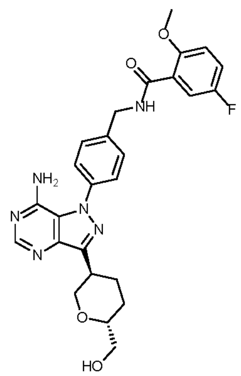, 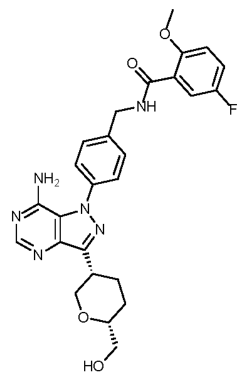, 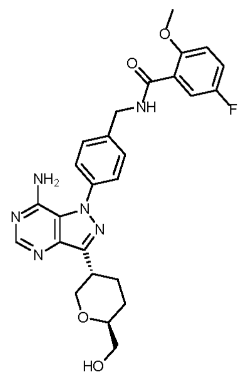,

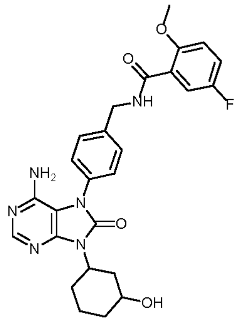 , 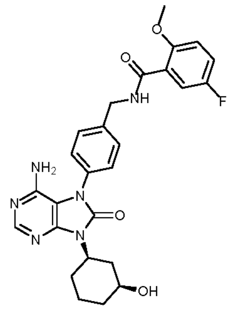 , 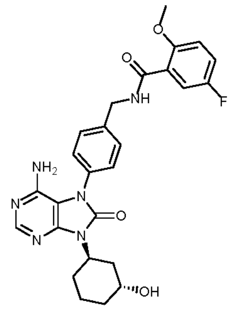 , 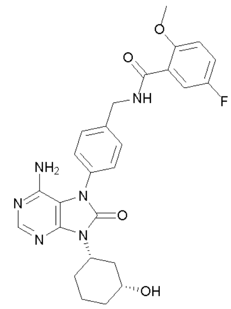 ,

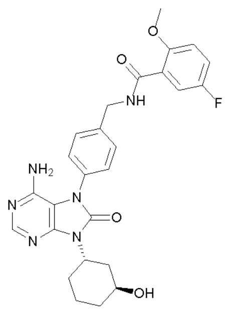 , 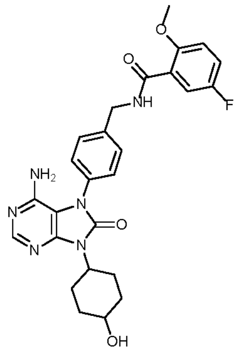 , 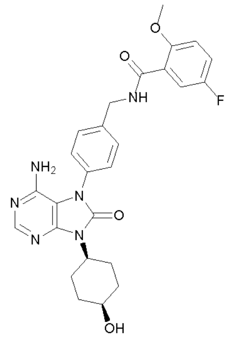 , 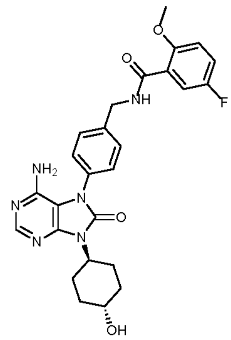 ,

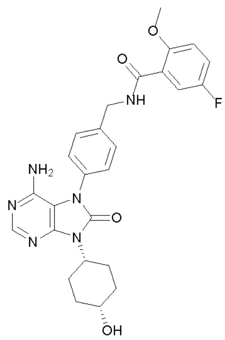 , 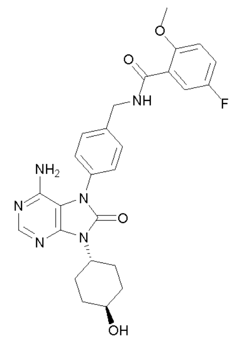 , 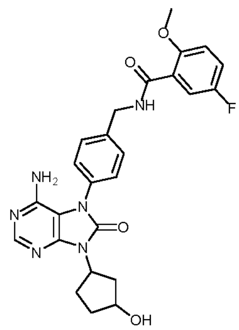 , 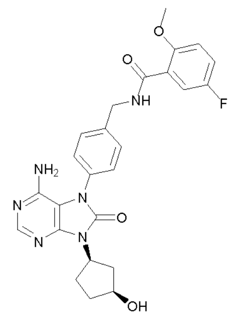 ,

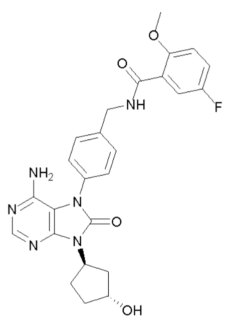 , 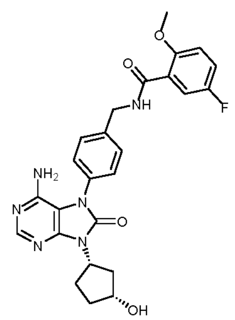 , 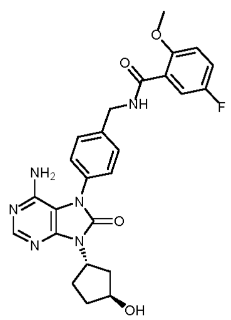 , 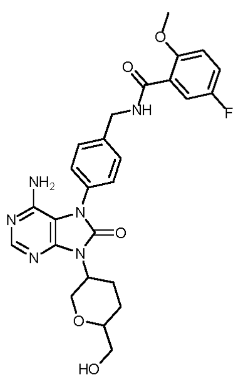 ,

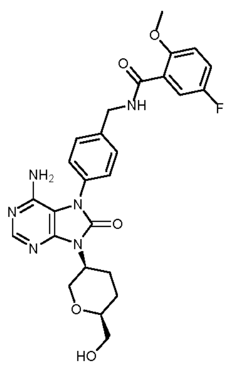
,
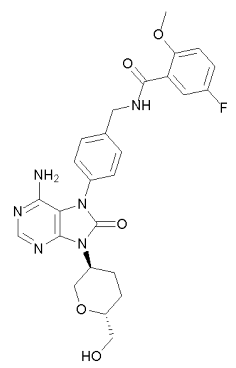
,
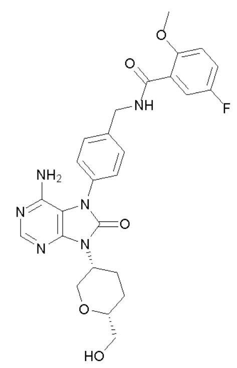
,
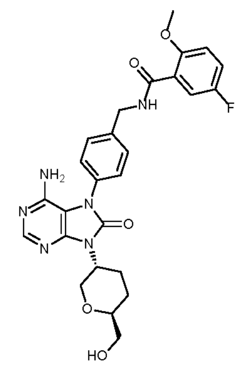
,

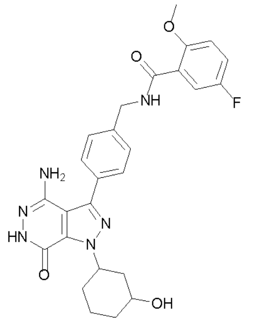
,
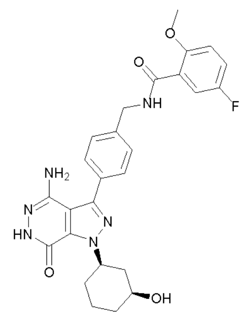
,
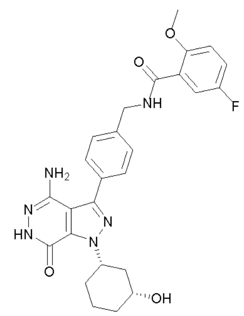
,
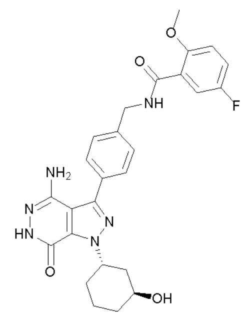
,

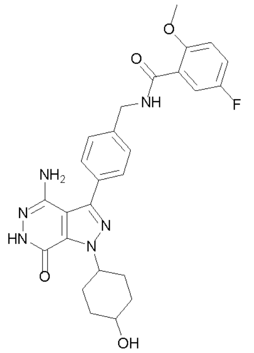
,
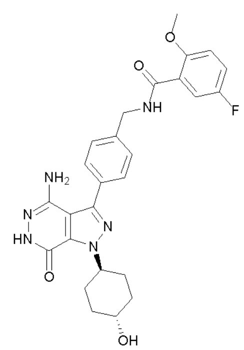
,
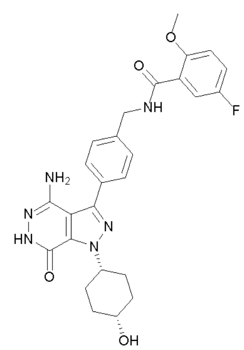
,
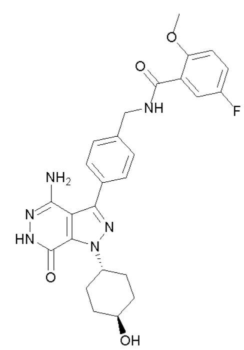
,

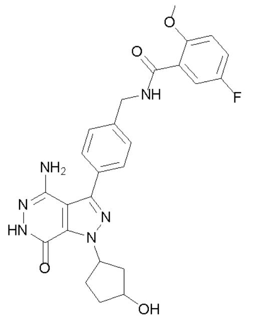
,
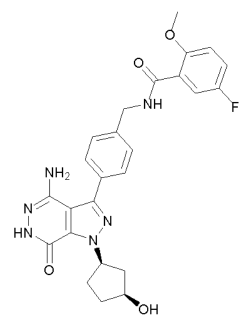
,
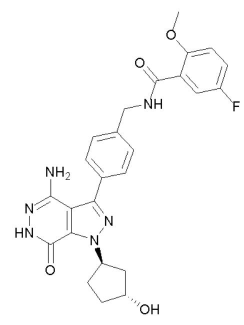
,
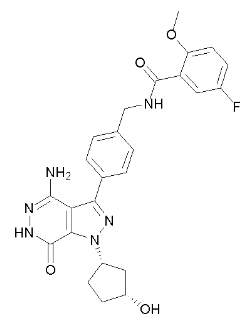
,

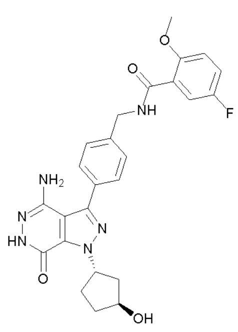 , 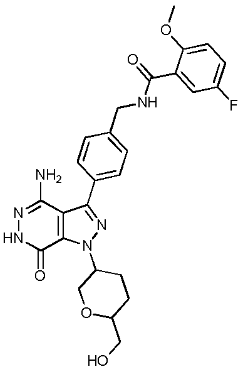 , 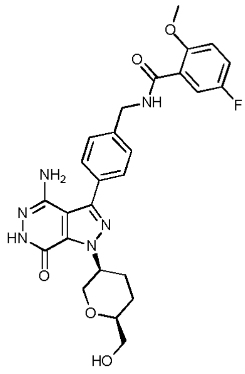 , 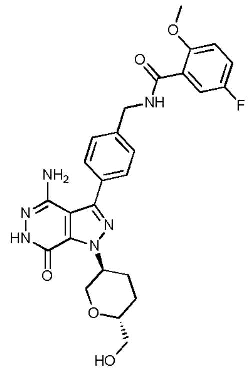 ,

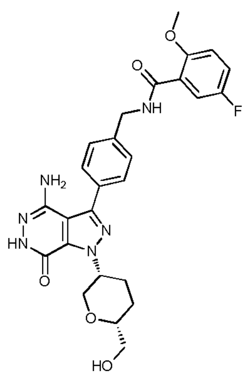 , 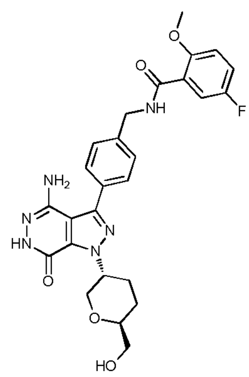 , 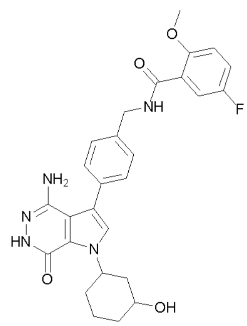 , 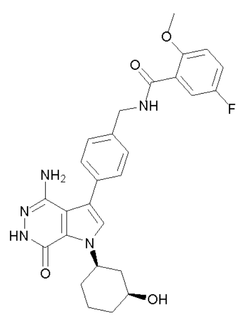 ,

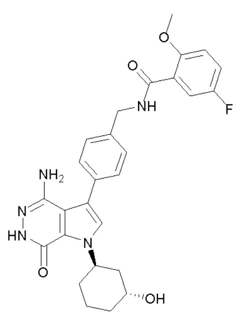 , 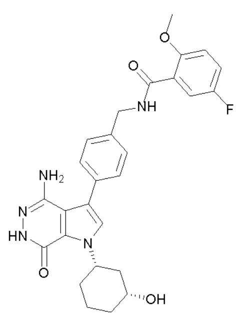 , 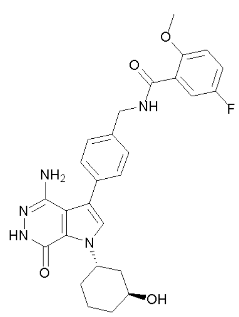 , 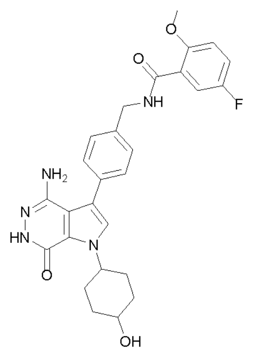 ,

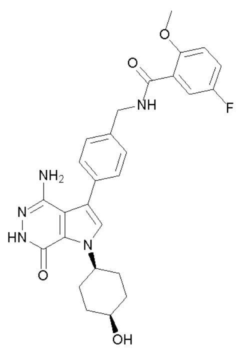 , 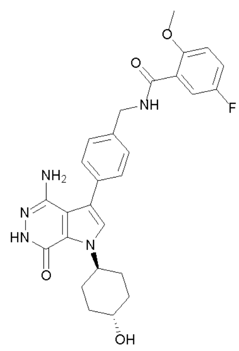 , 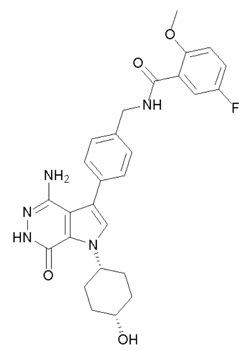 , 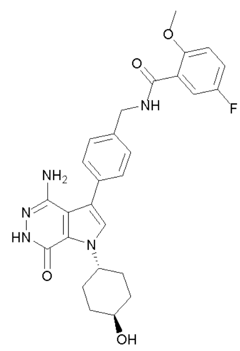 ,

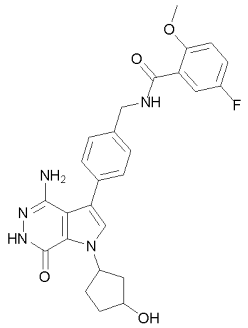 ,
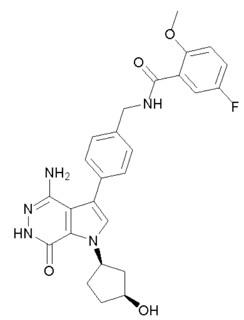 ,
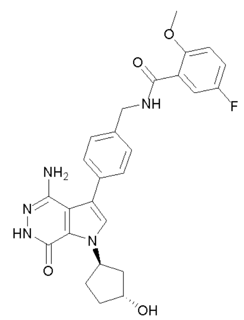 ,
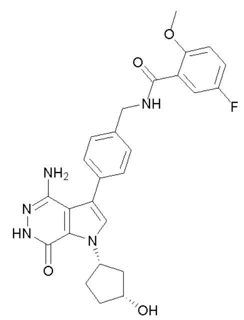 ,
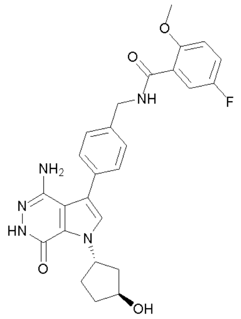 ,
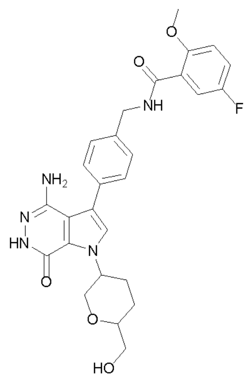 ,
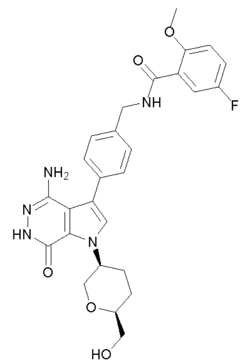 ,
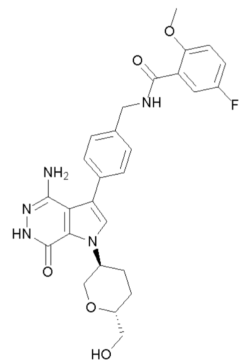 ,
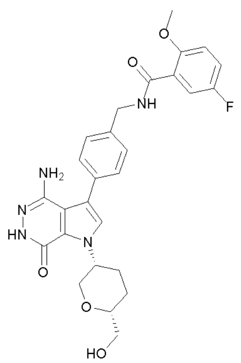 ,
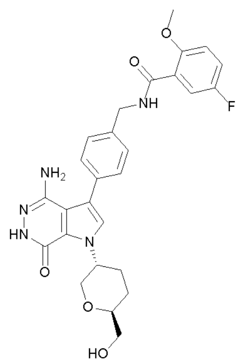 ,
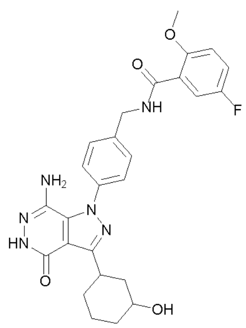 ,
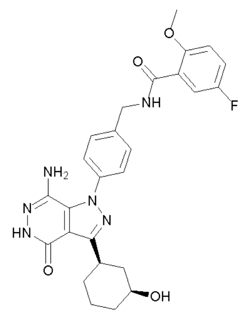 ,
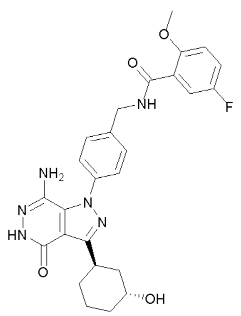 ,
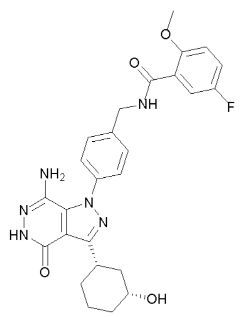 ,
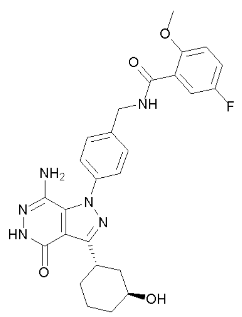 ,
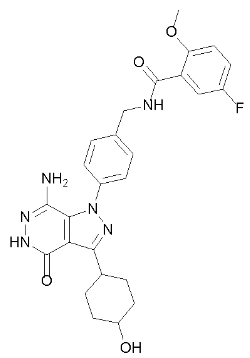 ,

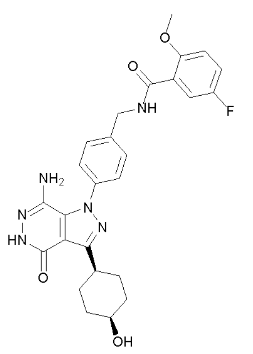 , 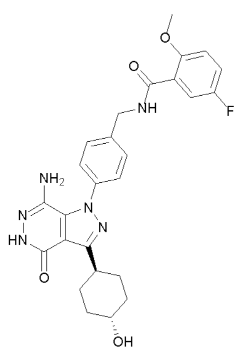 , 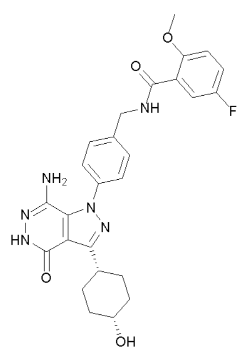 , 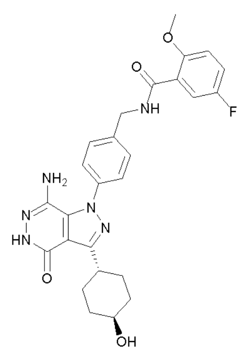 ,

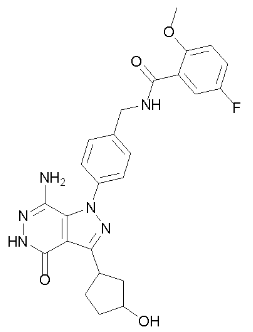 , 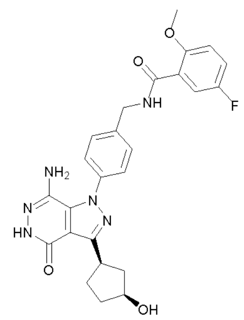 , 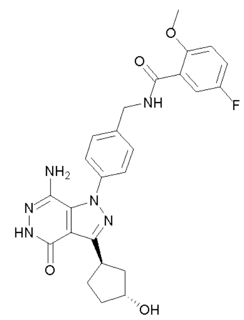 , 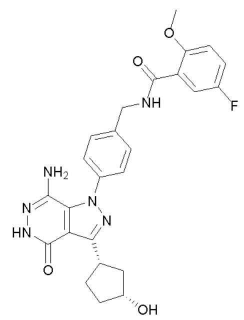 ,

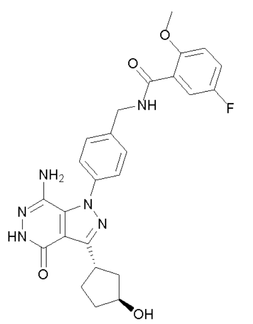 , 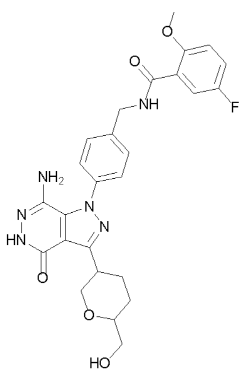 , 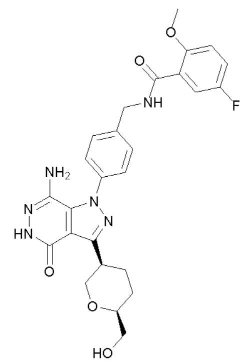 , 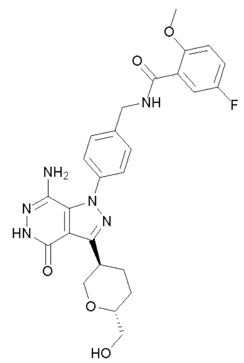 ,

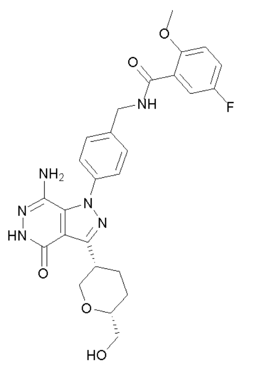 , 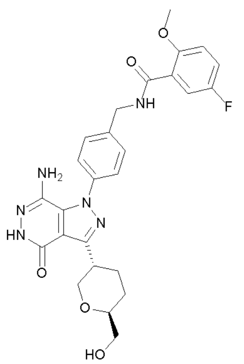 , 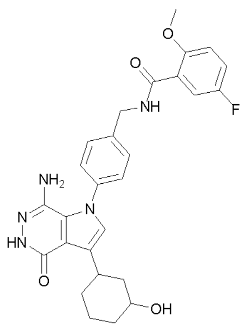 , 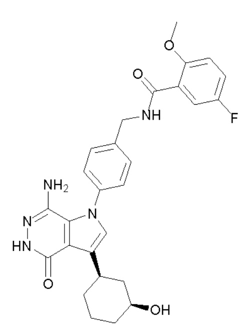 ,

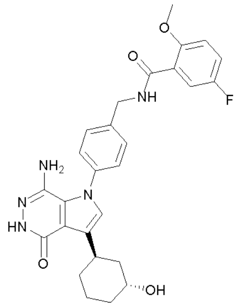
,
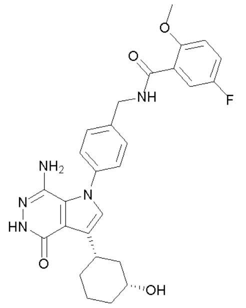
,
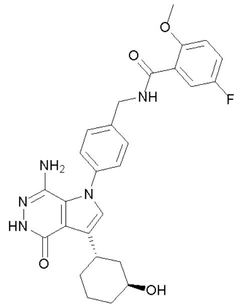
,
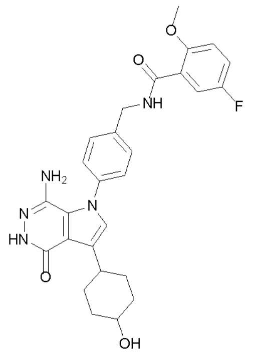
,
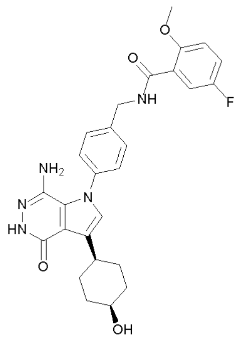
,
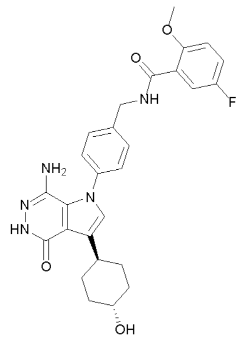
,
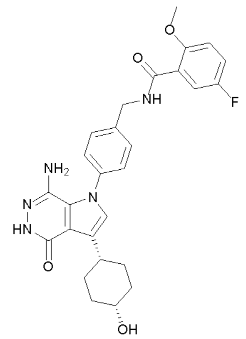
,
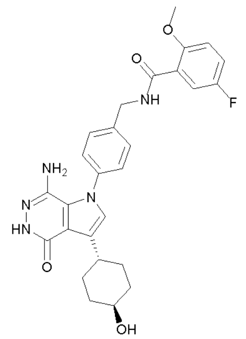
,
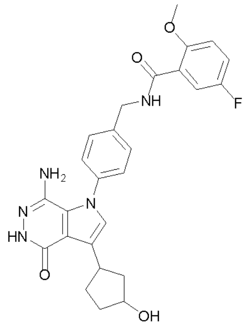
,
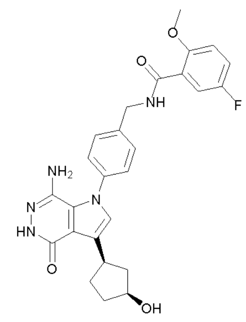
,
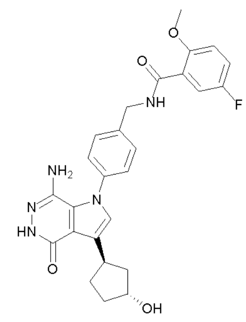
,
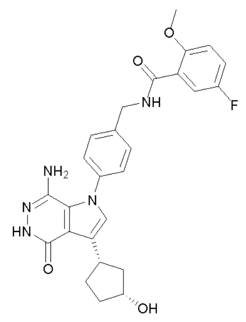
,
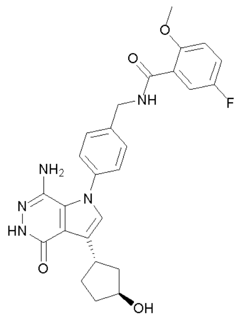
,
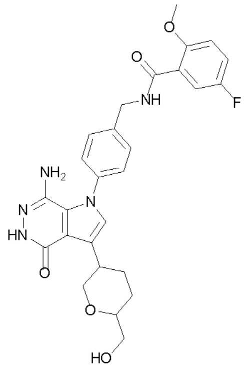
,
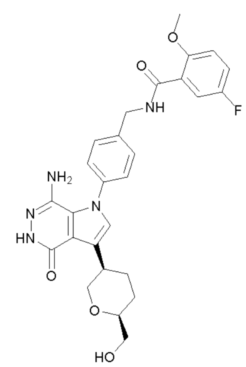
,
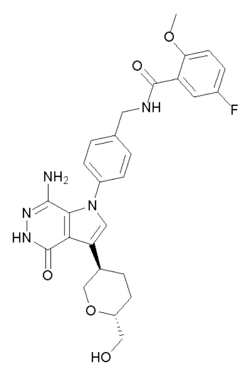
,

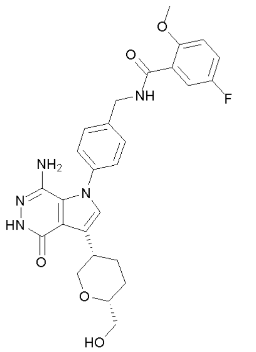 , 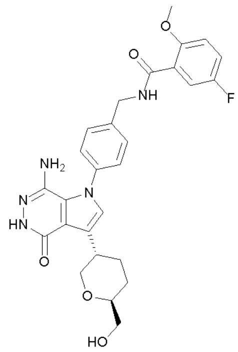 , 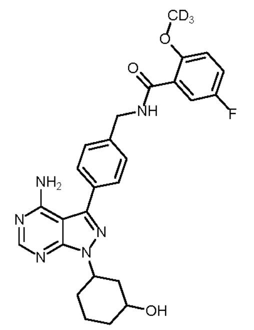 , 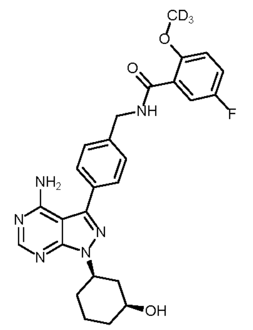 ,

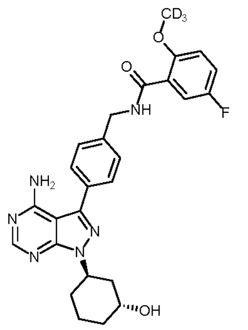 , 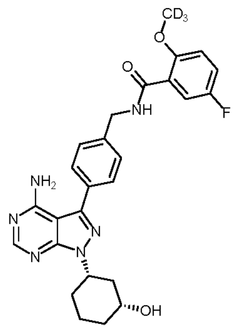 , 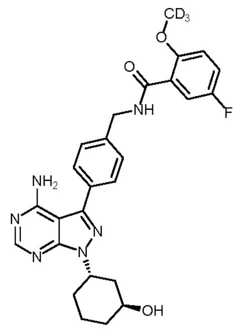 , 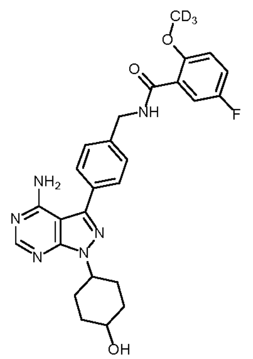 ,

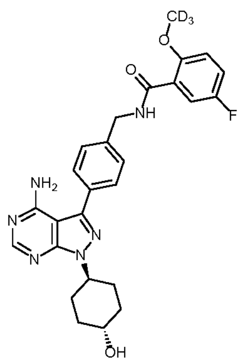 , 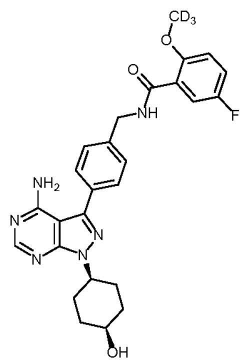 , 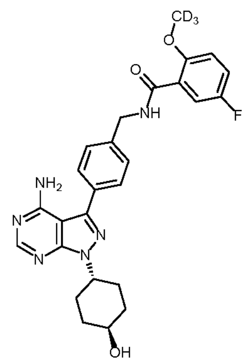 , 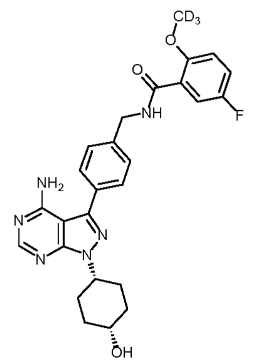 ,

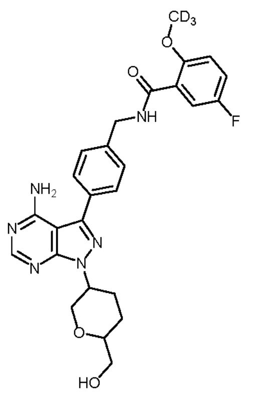 , 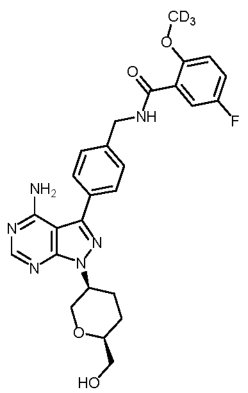 , 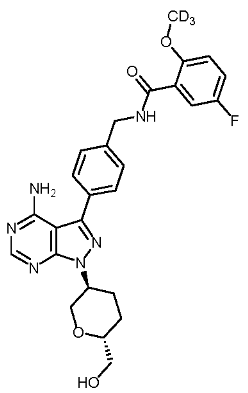 , 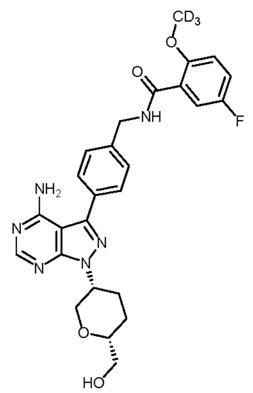

or

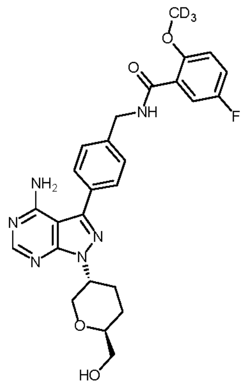

40. A pharmaceutical composition containing a compound of any one of embodiments 1-39, or a tautomer, stereoisomer, prodrug, crystal form, pharmaceutically acceptable salt, hydrate or solvate thereof, and pharmaceutically acceptable excipients, and optionally, other therapeutic agents.
41. Use of a compound of any one of embodiments 1 to 39, or a tautomer, stereoisomer, prodrug, crystal form, pharmaceutically acceptable salt, hydrate or solvate thereof, or a pharmaceutical composition according to embodiment 40 in the manufacture of a medicament for treating and/or preventing a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.
42. A method of treating and/or preventing a wild and/or mutated BTK kinase-mediated disease in a subject, the method comprising administering to the subject a compound of any one of embodiments 1-39, or a pharmaceutically acceptable salt, stereoisomer, solvate, hydrate, polymorph, prodrug, or isotopic variant thereof, or a pharmaceutical composition of embodiment 40; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.
43. A compound of any one of embodiments 1-39, or a tautomer, stereoisomer, prodrug, crystal form, pharmaceutically acceptable salt, hydrate or solvate thereof, or a pharmaceutical composition of embodiment 40, for use in the treatment and/or prevention of a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.
44. The use of embodiment 41, or the method of embodiment 42, or the compound or composition for use according to embodiment 43, wherein the BTK-mediated disease is selected from an allergic disease, an autoimmune disease, an inflammatory disease, and a cancer.
45. The use of embodiment 41, or the method of embodiment 42, or the compound or composition for use according to embodiment 43, wherein the BTK-mediated disease is a B-cell proliferative disease selected from chronic lymphocytic lymphoma, non-Hodgkin's lymphoma, diffuse large B-cell lymphoma, mantle cell lymphoma, follicular lymphoma, chronic lymphocytic leukemia, acute lymphocytic leukemia, acute myeloid leukemia, acute promyelocytic leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplastic disorder, mixed lineage leukemia, myelodysplastic syndrome, myeloproliferative disease, marginal zone lymphoma, and Waldenstrom's macroglobulinemia.
46. The use of embodiment 41 or the method of embodiment 42 or the compound or composition for use according to embodiment 43, wherein the BTK-mediated disease is multiple myeloma.

The compounds disclosed herein may include one or more asymmetric centers, and thus may exist in a variety of stereoisomeric forms, for example, enantiomers and/or diastereomers. For example, the compounds disclosed herein may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.
"Tautomer" refers to an isomer in which one functional group in a compound changes its structure into another functional group, wherein the compound and the isomer can quickly convert between each other, thus being in dynamic equilibrium; this two isomers are called tautomers.
It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the

compounds disclosed herein.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula Rx $H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R\cdot 0.5\ H_2O$)) and polyhydrates (x is a number greater than 1, for example, dihydrates ($R\cdot 2\ H_2O$) and hexahydrates ($R\cdot 6\ H_2O$)).

Compounds disclosed herein may be in an amorphous or crystalline form (crystal form or polymorph). Furthermore, the compounds disclosed herein may exist in one or more crystalline forms. Therefore, the present disclosure includes all amorphous or crystalline forms of the compounds disclosed herein within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes, which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds disclosed herein that comprise the above isotopes and/or other isotopes of other atoms, prodrugs thereof and pharmaceutically acceptable salts of said compounds or prodrugs all are within the scope of the present disclosure. Certain isotope-labeled compounds disclosed herein, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are alternative, because they are easy to prepare and detect. Furthermore, replaced by heavier isotopes, such as deuterium, which is $^{2}H$, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted *in vivo* into an active form that has medical effects by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

The prodrugs are any covalently bonded compounds disclosed herein, which release the parent compound *in vivo* when the prodrug is administered to a patient. Prodrugs are typically prepared by modifying functional groups in such a way that the modifications can be cleaved either by routine manipulation or decompose *in vivo* to yield the parent compound. Prodrugs include, for example, compounds disclosed herein wherein the hydroxy, amino or sulfhydryl groups are bonded to any group that, when administered to a patient, cleaves to form the hydroxy, amino or sulfhydryl groups. Thus, representative examples of prodrugs include (but are not limited to) the acetate/acetamide, formate/formamide and benzoate/benzamide derivatives of the hydroxy, amino or sulfhydryl functional groups of the compounds of formula (I). Furthermore, in the case of carboxylic acid (-COOH), esters such as methyl esters and ethyl esters, etc. can be employed. The ester itself may be active in their own and/or hydrolyzable under *in vivo* conditions in the human body. Suitable pharmaceutically acceptable *in vivo* hydrolysable ester groups include those groups that can readily break down in the human body to release the parent acids or salts thereof.

Pharmaceutical composition, formulation, and kit

In another aspect, the disclosure provides a pharmaceutical composition comprising a compound disclosed herein (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The pharmaceutical composition provided by the present disclosure may be administered by a variety of routes including, but not limited to, oral administration, parenteral administration, inhalation administration, topical administration, rectal administration, nasal administration, oral mucosal administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc.,* or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present disclosure may be further delivered using a variety of dosing methods. For example, in certain embodiments, the pharmaceutical composition may be given as a bolus, *e.g.,* in order to raise the concentration of the compound in the blood to an effective level rapidly. The placement of the bolus dose depends on the desired systemic levels of the active ingredient, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other

mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds disclosed herein can also be administered in sustained release forms or from sustained release drug delivery systems. A description of representative sustained release materials may be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are α-, β- and γ- cyclodextrins consisting of 6, 7 and 8 α-l,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol. See, *e.g.,* U.S. 5,376,645. In certain embodiments, the formulation comprises hexapropyl-β-cyclodextrin (e.g., 10-50% in water).

Indications

In another aspect, provided is the use of the compound of formula (I) disclosed herein (including all individual embodiments and generic subsets disclosed herein) or tautomer, stereoisomer, prodrug, crystal form, pharmaceutically acceptable salt, hydrate or solvate thereof as a drug.

The compounds of the present disclosure exhibit potent and selective BTK inhibition. For example, the compounds of the present disclosure exhibit nanomolar potency against wild-type BTK and BTK kinase encoded by a

BTK gene comprising a BTK kinase inhibitor resistance mutation; the mutation includes, for example, C481S, C481F, C481Y, C481R, C481T, C481G or C481W, alternatively, the mutation is C481S. In some embodiments, the inhibition of C481S is similar to the inhibition observed for wild-type BTK.

In some embodiments, the compounds of the present disclosure selectively target BTK kinase. For example, the compounds of the present disclosure can selectively target BTK kinase relative to another kinase or non-kinase target. In some embodiments, the compounds of the present disclosure can selectively target BTK kinase relative to one or more kinases of BRK, CSK, ERBB4, FYN, MEK1, MEK2, TEC, TXK, YES1, BMX, BLK, EGFR, ITK, SRC, JAK1, JAK2 and JAK3.

In some embodiments, the compounds of the present disclosure exhibit at least 30-fold selectivity for BTK kinase relative to another kinase. For example, relative to another kinase, the compounds of the present disclosure exhibit at least 40-fold selectivity for BTK kinase; at least 50-fold selectivity; at least 60-fold selectivity; at least 70-fold selectivity; at least 80-fold selectivity; at least 90-fold selectivity; at least 100-fold selectivity; at least 200-fold selectivity; at least 300-fold selectivity; at least 400-fold selectivity; at least 500-fold selectivity; at least 600-fold selectivity; at least 700-fold selectivity; at least 800-fold selectivity; at least 900-fold selectivity; or at least 1000-fold selectivity. In some embodiments, the compounds of the present disclosure exhibit at least 100-fold selectivity for BTK kinase relative to another kinase. In some embodiments, the selectivity for BTK kinase relative to another kinase is measured in a cellular assay (e.g., a cellular assay provided herein).

The compounds of the present disclosure are useful for treating diseases and conditions that are treated with BTK kinase inhibitors, such as diseases and conditions associated with BTK, such as proliferative diseases, such as cancers, including hematological cancers and solid tumors, and inflammatory conditions, immune diseases or fibrosis.

In some embodiments of any of the methods or uses described herein, the cancer (e.g., a BTK-related cancer) is a hematological cancer. In some embodiments of any of the methods or uses described herein, the cancer (e.g., a BTK-related cancer) is a solid tumor. In some embodiments of any of the methods or uses described herein, the cancer (e.g., a BTK-related cancer) is a B-cell malignancy. In some embodiments of any of the methods or uses described herein, the cancer (e.g., a BTK-related cancer) is Hodgkin lymphoma, diffuse large B-cell lymphoma, follicular lymphoma, mantle cell lymphoma, marginal zone lymphoma (e.g., splenic marginal zone lymphoma, extranodal marginal zone B-cell lymphoma), Burkitt's lymphoma, Waldenstrom's macroglobulinemia (lymphoplasmacytic lymphoma), primary central nervous system lymphoma, small lymphocytic lymphoma, chronic lymphocytic lymphoma, acute lymphocytic leukemia, B-cell prolymphocytic leukemia, precursor B-cell lymphoblastic leukemia, hairy cell leukemia, acute myeloid leukemia, chronic myeloid leukemia, multiple myeloma, plasma cell myeloma, plasmacytoma, bone cancer, bone metastasis, breast cancer, gastroesophageal cancer, pancreatic cancer, ovarian cancer, prostate cancer, lung cancer, colon cancer, head and neck cancer or glioma.

In some embodiments, the hematological cancer is selected from leukemia, non-Hodgkin's lymphoma, Hodgkin's lymphoma or myeloma, for example, acute lymphocytic leukemia (ALL), acute myeloid leukemia (AML), acute promyelocytic leukemia (APL), chronic lymphocytic lymphoma (CLL), chronic myeloid leukemia (CML), chronic myelomonocytic leukemia (CMML), chronic neutrophilic leukemia (CNL), acute undifferentiated leukemia (AML), anaplastic large cell lymphoma (ALCL), prolymphocytic leukemia (PML), juvenile myelomonocytic leukemia (JMML), adult T-cell leukemia (ALL), acute myeloid leukemia with trilineage myelodysplastic disorder (AML/TMDS), mixed lineage leukemia (MLL), myelodysplastic syndrome (MDS), myeloproliferative disorder (MPD), diffuse large B-cell lymphoma, Follicular lymphoma, mantle cell lymphoma, marginal zone lymphoma (e.g., splenic marginal zone lymphoma, extranodal marginal zone B-cell lymphoma), Burkitt's lymphoma, Waldenstrom's macroglobulinemia (lymphoplasmacytic lymphoma), primary central nervous system lymphoma, small lymphocytic lymphoma, precursor B-cell lymphoblastic leukemia, hairy cell leukemia, mucosa-associated lymphoid tissue lymphoma, plasma cell myeloma, plasmacytoma, and multiple myeloma. Other examples of hematological cancers include myelodysplastic disorders (MPD), such as polycythemia vera (PV), essential thrombocytopenia (ET), and idiopathic primary myelofibrosis (IMF/IPF/PMF). In some embodiments, the hematological cancer is mantle cell lymphoma, chronic lymphocytic lymphoma, small lymphocytic lymphoma, Waldenstrom's macroglobulinemia, or marginal zone lymphoma.

In some embodiments, the solid tumor is selected from bone cancer, bone metastasis, breast cancer, gastroesophageal cancer, pancreatic cancer, ovarian cancer, prostate cancer, lung cancer, colon cancer, and head and neck cancer.

In some embodiments, the immune disease is selected from arthritis, multiple sclerosis, osteoporosis, irritable bowel syndrome, inflammatory bowel disease, Crohn's disease, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barré syndrome, acute disseminated encephalomyelitis, Addison's disease, opsoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, warm autoimmune hemolytic anemia, Wegener's granulomatosis, psoriasis, Alopecia universalis, Behcet's disease, chronic fatigue, dysautonomia, endometriosis, interstitial cystitis, neuromyotonia, scleroderma and vulvodynia, asthma, appendicitis, blepharitis, bronchiolitis, bronchitis, bursitis, cervicitis, cholangitis, cholecystitis, colitis, conjunctivitis, colitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, inflammatory enteritis, suppurative inflammation, laryngitis, mastitis, meningitis, myelitis, myocarditis, myositis, nephritis, oophoritis, orchitis, Osteitis, otitis, pancreatitis, mumps, pericarditis, peritonitis, pharyngitis, pleurisy, phlebitis, pneumonia, pneumonitis, proctitis, prostatitis, pyelonephritis, rhinitis, salpingitis, sinusitis, stomatitis, synovitis, tendinitis, tonsillitis, uveitis, vaginitis, vasculitis, vulvitis graft-versus-host disease, transplantation, transfusion, anaphylaxis, allergic reaction, type I hypersensitivity, allergic conjunctivitis, allergic rhinitis and atopic dermatitis.

In some embodiments, the inflammatory disease is selected from arthritis, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis fibrositis, gastritis, gastroenteritis, hepatitis, hidradenitis suppurativa, laryngitis, mastitis, meningitis, osteomyelitis myocarditis, myositis, nephritis, oophoritis, orchitis, osteitis, otitis, pancreatitis, mumps, pericarditis, peritonitis, pharyngitis, pleurisy, phlebitis, pneumonia, lung disease, proctitis, prostatitis, Pyelonephritis, rhinitis, salpingitis, sinusitis, stomatitis, synovitis, tendinitis, tonsillitis, uveitis, vaginitis, vasculitis and vulvitis.

In some embodiments, the autoimmune disease is selected from lupus and Sjögren's syndrome, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barre syndrome, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter 's syndrome, Takayasu's arteritis, hemolytic anemia, Wegener's granulomatosis, psoriasis, alopecia universalis, Behcet's disease, chronic fatigue, dysautonomia, endometriosis, Interstitial cystitis, neuromyotonia, scleroderma and vulvodynia.

In some embodiments, the heteroimmune disease is selected from the group consisting of graft-versus-host disease, transplantation, transfusion, anaphylaxis, allergic reaction, type I hypersensitivity reaction, allergic conjunctivitis, allergic rhinitis, and atopic dermatitis.

In some embodiments, fibrosis is selected from pulmonary fibrosis, idiopathic pulmonary fibrosis (IPF), usual interstitial pneumonia (UIP), interstitial lung disease, cryptogenic fibrosing alveolitis (CFA), bronchiolitis obliterans, bronchiectasis, fatty liver disease, steatosis (e.g., nonalcoholic steatohepatitis (NASH), cholestatic liver disease (e.g., primary biliary cirrhosis (PBC)), cirrhosis, alcohol-induced liver fibrosis, bile duct damage, biliary fibrosis, cholestasis, and bile duct disease.

In the treatment methods of the present disclosure, "effective amount" is intended to mean an amount or dosage sufficient to produce the desired therapeutic benefit in an individual in need of such treatment. The effective amount or dosage of the compounds of the present disclosure can be determined by conventional methods (e.g., modeling, dose escalation, or clinical trials) and conventional factors (e.g., the mode or route of drug delivery, the pharmacokinetics of the agent, the severity and course of the infection, the individual's health status and weight, and the judgment of the treating physician). Exemplary dosages are in a range of about 0.1 mg to 1 g per day, or about 1 mg to 50 mg per day, or about 50 mg to 250 mg per day, or about 250 mg to 1 g per day. The total dosage may be in single or divided dosage units (e.g., BID, TID, QID).

After improvement of the patient's disease occurs, the dosage may be adjusted for preventive or maintenance treatment. For example, the dosage or frequency of administration, or both, may be reduced to an amount that maintains the desired therapeutic or preventive effect, depending on the symptoms. Of course, if the symptoms have been alleviated to an appropriate degree, then treatment may be discontinued. However, the patient may require long-term intermittent treatment upon recurrence of any symptom. The patient may also require long-term slow treatment.

Drug combinations

The compounds of the present disclosure described herein may be used in combination with one or more other active ingredients in pharmaceutical compositions or methods to treat the diseases and conditions described herein. Other additional active ingredients include other therapeutic agents or agents that mitigate the adverse effects of the therapeutic agent against the intended disease target. The combination may be used to increase efficacy, ameliorate other disease symptoms, reduce one or more negative effects, or reduce the required dosage of the compounds of the present disclosure. The additional active ingredients may be formulated as separate pharmaceutical compositions from the compounds of the present disclosure or may be included in a single pharmaceutical composition with the compounds of the present disclosure. The additional active ingredients may be administered simultaneously with, prior to, or after the administration of the compounds of the present disclosure.

Combination agents include those additional active ingredients known or observed to be effective in treating the

diseases and conditions described herein, including those effective against another target associated with the disease. For example, the compositions and formulations of the present disclosure, and methods of treatment may further comprise other drugs or medicaments, such as other active agents useful for treating or ameliorating the target disease or associated symptoms or conditions. For cancer indications, such other agents include, but are not limited to, kinase inhibitors, such as EGFR inhibitors (e.g., erlotinib, gefitinib); Raf inhibitors (e.g., vemurafenib), VEGFR inhibitors (e.g., sunitinib); standard chemotherapeutic agents, such as alkylating agents, antimetabolites, antitumor antibiotics, topoisomerase inhibitors, platinum drugs, mitotic inhibitors, antibodies, hormone therapy or corticosteroids. For pain indications, suitable combination agents include anti-inflammatory agents, such as NSAID. The pharmaceutical compositions of the present disclosure may additionally comprise one or more of such active agents, and methods of treatment may additionally comprise administering an effective amount of one or more of such active agents.

Example

The present disclosure will be further elaborated with specific examples. It should be understood that these examples are only used to illustrate the present disclosure and are not used to limit the scope of the present disclosure. The experimental methods without specific conditions in the following examples are usually in accordance with the conventional conditions or the conditions suggested by the manufacturer. Unless otherwise specified, parts and percentages are parts by weight and percentages by weight.

Generally, in the preparation process, each reaction is carried out in an inert solvent at room temperature to reflux temperature (such as 0 °C-100 °C, alternatively 0 °C-80 °C). The reaction time is usually 0.1-60 hours and alternatively 0.5-24 hours.

Abbreviations used herein have the following meanings:

TBDMSCl: tert butyldimethylsilyl chloride
TBDPSCl: tert butyldiphenylchlorosilane
Dess-Martin oxidant: 1,1,1-triacetoxy-1,1-dihydro-1,2-benziodoxol-3(1H)-one
EDCI: 1-ethyl-3-(3-dimethylaminopropyl)carbodiimide
CAN: $Ce(NH_4)_2(NO_3)_6$
DIAD: diisopropyl azodicarboxylate
PPTS or PyTs: pyridinium p-toluenesulfonate
$Pd(OAc)_2$: palladium acetate
Xphos: 2-dicyclohexylphosphino-2',4',6'-triisopropylbiphenyl
$Pd(dppf)Cl_2$: [1,1'-bis(diphenylphosphino)ferrocene]palladium dichloride
$Pd(OAc)_2$: palladium acetate
$Pd_2(dba)_3$: tris(dibenzylideneacetone)dipalladium (0)
DMAP: 4-dimethylaminopyridine
DIPEA: N,N-diisopropylethylamine
$OTf_2$: trifluoromethanesulfonic anhydride
$NaBH_3CN$: sodium cyanoborohydride
MsCl: methylsulfonyl chloride
NIS: N-iodosuccinimide
$T_3P$: n-propyl phosphonic acid cyclic anhydride
MTBE: methyl tert-butyl ether
TPP: triphenylphosphine

**Preparation of intermediate compound A-1 2-((4-bromophenyl)(methoxy)methylene)malononitrile**

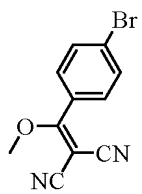

A-1

**The following synthetic route was adopted**

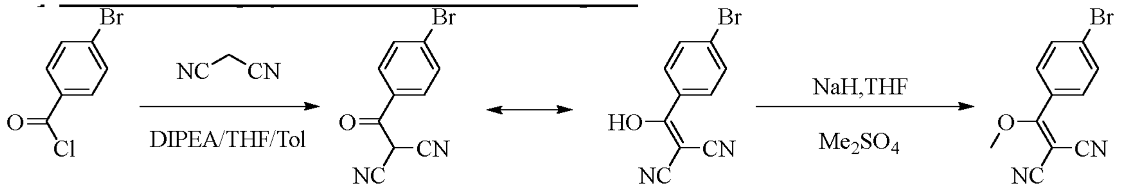

A-1

Step 1 Synthesis of Compound 2-((4-bromophenyl)(hydroxy)methylene)malononitrile

Toluene (100 mL), THF (20 mL) and malononitrile (compound 2, 6.3 mL, 100.3 mmol) were added to a 250 mL single-necked flask equipped with magnetic stirring in sequence, and stirred until dissolved. The solution was cooled to -10 °C, and 4-bromobenzoyl chloride (20 g, 91.1 mmol) was added. The mixture was stirred for 10 minutes, and DIPEA (31.8 mL, 182.3 mmol) was slowly added dropwise. The whole process took half an hour, and the internal temperature was controlled not to exceed -10 °C. After the addition was completed, the mixture was slowly raised to room temperature and stirred for 2 hours. Ethyl acetate (200 mL) and 1M aqueous hydrochloric acid solution (200 mL) were added, and the mixture was stirred for 10 minutes. The organic phase was separated, washed successively with 1M hydrochloric acid (100 mL) and saturated saline (100 mL), dried over anhydrous sodium sulfate, and filtered. The filtrate was concentrated to dryness. The residue was added to petroleum ether/ethyl acetate (100 mL, 20/1), stirred for 20 minutes, filtered, washed with petroleum ether, and air-dried to obtain 20 g of a gray solid with a yield of 88.1%. LC-MS(APCI): m/z=249.0$(M+1)^+$.

Step 2 Synthesis of intermediate compound A-1

Sodium hydride (2.48 g, 61.84 mmol, 60% by mass dispersed in silicone oil) was added to a 250 mL three-necked flask equipped with magnetic stirring and a condenser. The flask was evacuated and the atmosphere was replaced with nitrogen three times. Anhydrous THF (40 mL) was added under ice-water bath cooling. The mixture was dispersed with stirring. A solution of 2-((4-bromophenyl)(hydroxy)methylene)malononitrile (14 g, 56.2 mmol) in anhydrous THF (40 mL) was added dropwise. The mixture was stirred and reacted for 30 minutes under ice-water bath. Dimethyl sulfate (16.0 mL, 168.6 mmol) was added dropwise. After the addition was completed, the temperature was raised to 80 °C and the mixture was stirred and reacted overnight. The mixture was cooled to room temperature. Saturated saline (80 mL) was added to quench the reaction. The organic phase was separated, and the aqueous phase was extracted with ethyl acetate (50 mL × 2). The organic phases were combined, washed with saturated saline (50 mL), dried over anhydrous sodium sulfate, and filtered. The filtrate was concentrated and passed through a silica gel column to obtain 7.3 g of a white solid with a yield of 49.4%. LC-MS(APCI): m/z=23 1.0$(M+1)^+$.

**<u>Preparation of intermediate compound A-2 5-amino-3-(4-bromophenyl)-1-(3-hydroxycyclohexane)-1H-pyrazole-4-carbonitrile</u>**

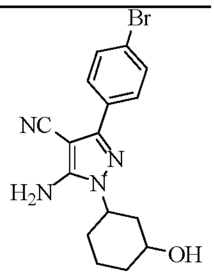
A-2

**The following synthetic route was adopted**

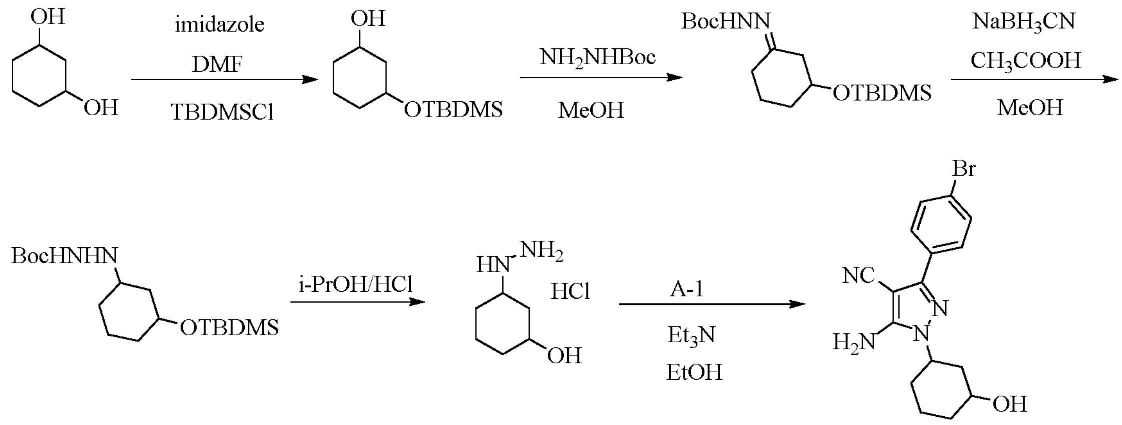


A-2

Step 1 Synthesis of Compound 3-((tert-butyldimethylsilyl)oxy)cyclohexane-1-ol

Cyclohexane-1,3-diol (3 g, 25.86 mmol), imidazole (2.64 g, 1.5 eq, 38.79 mmol) and DMF (50 mL) were successively added to a 250 mL two-necked flask equipped with magnetic stirring, and stirred until dissolved. The solution was cooled to 0 °C, and compound TBDMSCl (4.7g, 1.2eq, 31.03 mmol) was added in batches. The mixture was slowly warmed to room temperature and stirred overnight. Ethyl acetate (200 mL) and 1M aqueous hydrochloric acid (200 mL) were added. The mixture was stirred for 10 min. The organic phase was separated, washed with 1M hydrochloric acid (100 mL), washed with saturated saline (100 mL), dried with anhydrous sodium sulfate, and filtered. The filtrate was concentrated to dryness. The mixed sample was passed through a column to obtain 5g of colorless oil, yield: 84%.

Step 2 Synthesis of Compound tert-butyl 2-(3-((tertbutyldimethylsilyl)oxy)cyclohexylidene)hydrazine-1-formate

3-((tert-butyldimethylsilyl)oxy)cyclohexane-1-ol (1.79 g, 7.82 mmol) and MeOH (20 mL) were added to a 100 mL two-necked flask equipped with magnetic stirring, and stirred until dissolved. The solution was cooled to 0 °C, and compound $NH_2NHBoc$ (1.55g, 1.5eq, 11.73 mmol) was added. After the addition was completed, the mixture was naturally warmed to room temperature and stirred overnight. Dichloromethane (100 mL) and water (100 mL) were added and stirred for 5 minutes. The organic phase was separated, and the aqueous phase was extracted once more. The organic phases were combined, washed once with saturated saline, and rotary evaporated to dryness. The residue was passed through a silica gel column to obtain 1.67 g of the target product, yield: 62%. LC-MS(APCI): m/z=344.0$(M+1)^+$.

Step 3 Synthesis of Compound tert-butyl 2-(3-((tertbutyldimethylsilyl)oxy)cyclohexyl)hydrazine-1-formate

To a 100 mL three-necked flask equipped with magnetic stirring were added tert-butyl 2-(3-((tert-butyldimethylsilyl)oxy)cyclohexylidene)hydrazine-1-carboxylate (1.67 g, 4.87 mmol) and MeOH (30 mL), and stirred until dissolved. acetic acid (1.0 eq, 302 mg) was added. The mixture was stirred for 20 minutes, cooled to 0°C in an ice bath, NaBH3CN (2.0 eq, 604 mg) was added in batches, and stirred at room temperature overnight under nitrogen atmosphere. TLC showed that the reaction was complete. Dichloromethane (100 mL) and water (100 mL) were added and stirred for 5 minutes. The organic phase was separated, and the aqueous phase was extracted once more, and the organic phases were combined, washed once with saturated saline, and dried by rotary evaporation. The residue was passed through a silica gel column to obtain 1.6 g of the target product, yield: 95%. LC-MS(APCI): m/z=346.0$(M+1)^+$.

Step 4 Synthesis of Compound 3-hydrazinocyclohexane-1-ol hydrochloride

Tert-butyl 2-(3-((tert-butyldimethylsilyl)oxy)cyclohexyl)hydrazine-1-carboxylate (1.6 g, 4.62 mmol) was dissolved in 10 mL of dichloromethane. 10 mL of 5 M isopropanol hydrochloride was slowly added under ice bath cooling, and stirred at room temperature for 2 h. The mixture was directly rotary evaporated to dryness after TLC monitoration showed no raw material, and dried by suction for the next reaction.

Step 5 Synthesis of intermediate compound A-2

3-hydrazinocyclohexane-1-ol hydrochloride obtained in the previous step, intermediate compound A-1 (378 mg, 1.45 mmol) and anhydrous ethanol (20 mL) were added to a 100 mL two-necked flask equipped with magnetic stirring and

a condenser. Triethylamine (586 mg, 5.8 mmol) was added while stirring. The temperature was raised to 80 °C under a nitrogen atmosphere and the reaction was stirred overnight. The mixture was cooled to room temperature, and the solvent was evaporated under reduced pressure. Saturated saline (20 mL) and ethyl acetate (30 mL) were added. The mixture was stirred for 10 min. The organic phase was separated, and the aqueous phase was extracted with ethyl acetate (30 mLx2). The organic phases were combined, dried with anhydrous sodium sulfate, and filtered. The filtrate was concentrated and passed through a silica gel column to obtain 300mg of brown solid, LC-MS(APCI): m/z=361.0(M+1)$^+$.

**Preparation of intermediate compound A-3 trans-1-(3-((tert-butyldimethylsilyl)oxy)cyclohexyl)-3-iodo-1H-pyrazolo[3,4-d]pyrimidin-4-amine**

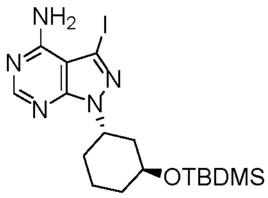

+

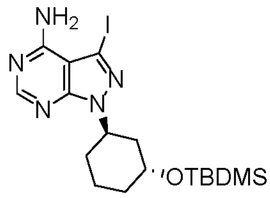

A-3

**The following synthetic route was adopted**

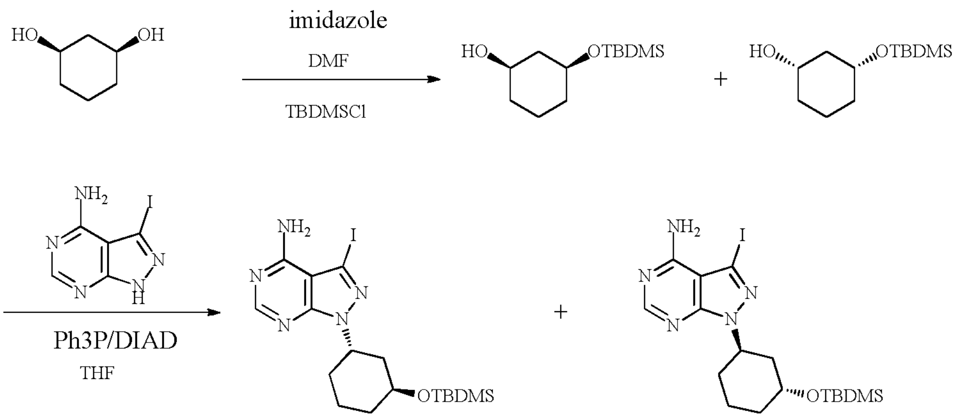


A-3

Step 1 Synthesis of compound cis-3-((tert-butyldimethylsilyl)oxy)cyclohexane-1-ol

Cis-cyclohexane-1,3-diol (3.0 g, 25.86 mmol), imidazole (2.64 g, 1.5 eq, 38.79 mmol) and DMF (40 mL) were added in sequence to a 250 mL two-necked flask equipped with magnetic stirring, and stirred until dissolved. The solution was cooled to 0 °C, and compound TBDMSCl (4.7 g, 31.03 mmol) was added in batches. The mixture was slowly warmed to room temperature and stirred overnight. Ethyl acetate (200 mL) and 1M aqueous hydrochloric acid (200 mL) were added. The mixture was stirred for 10 min. The organic phase was separated, washed with 1M hydrochloric acid (100 mL), washed with saturated saline (100 mL), dried with anhydrous sodium sulfate, and filtered. The filtrate was concentrated to dryness. A mixed sample was passed through a column to obtain 5.0 g of colorless oil, yield: 84%.

Step 2 Synthesis of intermediate compound A-3

Cis-3-((tert-butyldimethylsilyl)oxy)cyclohexane-1-ol (1.16 g, 5.02 mmol), 3-iodo-1H-pyrazolo[3,4-d]pyrimidin-4-amine (1.19 g, 4.57 mmol) and THF (20 mL) were added into a 100 mL two-necked flask equipped with magnetic stirring, and stirred until dissolved. The solution was cooled to 0 °C, and $Ph_3P$ (2.39 g, 9.13 mmol) was added. DIAD (1.84 g, 9.13 mmol) was then slowly added dropwise, naturally warmed to room temperature and stirred overnight. Ethyl acetate (50 mL) and water (50 mL) were added and stirred for 5 minutes. The organic phase was separated, and the aqueous phase was extracted once more. The organic phases were combined, washed with saturated saline once, and dried by rotary evaporation. The residue was passed through a silica gel column to obtain 2.3 g of a white solid, yield: 96.8%.

**Preparation of intermediate compound A-4 5-amino-3-(4-bromophenyl)-1-(6-(hydroxymethyl)tetrahydro-2H-pyran-3-yl)-1H-pyrazole-4-carbonitrile**

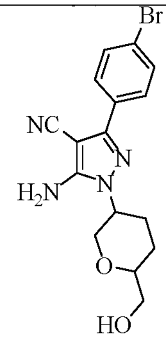

A-4

**The following synthetic route was adopted**

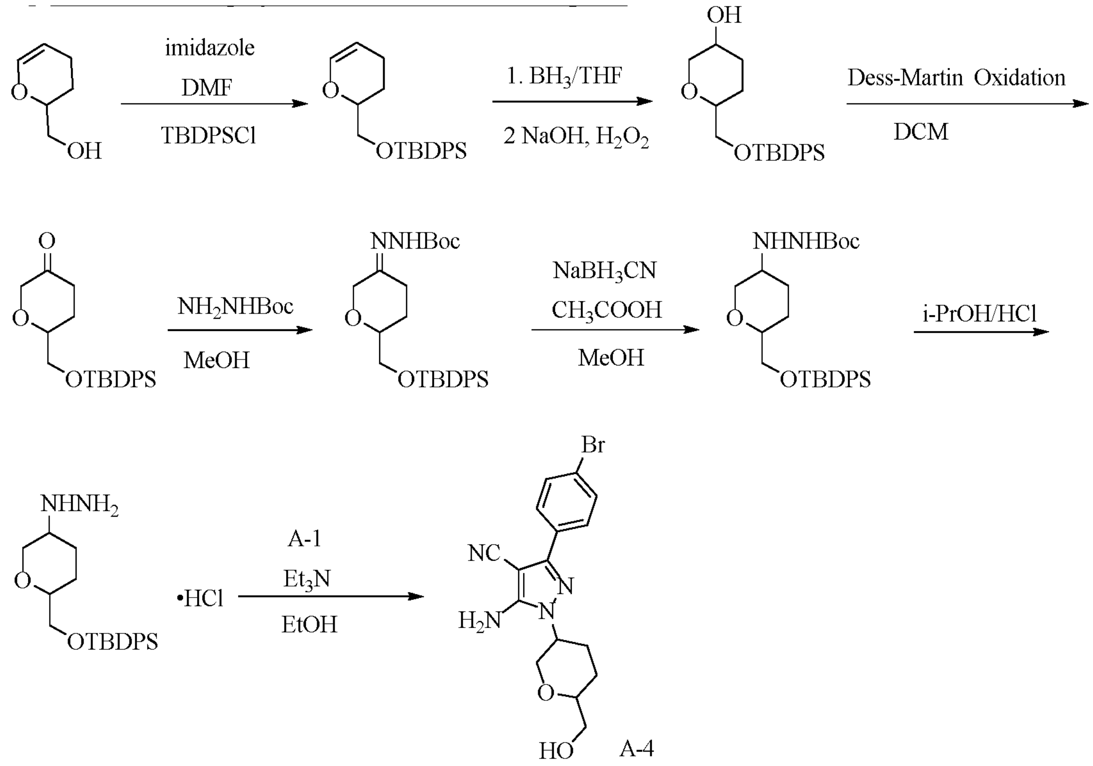


Step 1 Synthesis of compound ((3,4-dihydro-2H-pyran-2-yl)methoxy)(diphenyl)(tert-butyl)silane

(3,4-dihydro-2H-pyran-2-yl)methanol (3 g, 26.32 mmol), imidazole (2.7 g, 39.48 mmol) and DMF (40 mL) were added in sequence into a 250 mL two-necked flask equipped with magnetic stirring, and stirred until dissolved. The solution was cooled to 0 °C. Compound TBDPSCl(4.7 g, 28.95 mmol) was added in batches. The mixture was slowly warmed to room temperature and stirred overnight. Ethyl acetate (100 mL) and 1M aqueous hydrochloric acid (50 mL) were added. The mixture was stirred for 10 min. The organic phase was separated, washed with 1M hydrochloric acid (30 mL), washed with saturated saline (100 mL), dried with anhydrous sodium sulfate, and filtered. The filtrate was concentrated to dryness. The residue was passed through a silica gel column to obtain 7.4 g of a colorless oil, yield: 80%.

Step 2 Synthesis of Compound 6-(((tert-butyldiphenylsilyl)oxy)methyl)tetrahydro-2H-pyran-3-ol

Compound ((3,4-dihydro-2H-pyran-2-yl)methoxy)(diphenyl)(tert-butyl)silane (4 g, 11.33 mmol) and anhydrous

THF (20 mL) were added in sequence to a 100 mL two-necked flask equipped with magnetic stirring and a condenser, and stirred until dissolved. The flask was evacuated and the atmosphere was replaced with nitrogen three times. A $BH_3$-THF solution (23 mL, 1M) was slowly added dropwise under an ice bath. After the dropwise addition was completed, the mixture was stirred at room temperature overnight. The mixture was cooled in an ice bath. 4M NaOH aqueous solution (4M, 12 mL) was slowly added, then $H_2O_2$ solution (mass fraction: 33%, 20 mL) was added dropwise. The temperature was raised to 50 °C and the mixture was stirred for 1.5 h. The mixture was cooled to room temperature. Ethyl acetate (100 mL) and water (100 mL) were added, and stirred for 5 minutes. The organic phase was separated, and the aqueous phase was extracted with ethyl acetate (50 mLx2). The organic phases were combined, washed with saturated saline once, dried with anhydrous sodium sulfate, and filtered. The filtrate was concentrated. The residue was passed through a silica gel column to obtain 1.8 g of colorless oil, yield: 43.1%, LC-MS(APCI): m/z=371.2$(M+1)^+$.

Step 3 Synthesis of Compound 6-(((tert-butyldiphenylsilyl)oxy)methyl)dihydro-2H-pyran-3(4H)-one

In a 100 mL two-necked flask equipped with magnetic stirring, 6-(((tert-butyldiphenylsilyl)oxy)methyl)tetrahydro-2H-pyran-3-ol (1.5 g, 4.0 mmol) and DCM (20 mL) were added in sequence, and stirred until dissolved. The solution was cooled to 0 °C. Compound Dess-Martin oxidant (3.43 g, 8.0 mmol) was added in batches. After the addition, the mixture was warmed to room temperature and stirred overnight. Dichloromethane (50 mL) and water (50 mL) were added and stirred for 5 minutes. The organic phase was separated, and the aqueous phase was extracted with DCM (40 mL). The organic phases were combined, washed with saturated saline once, dried with anhydrous sodium sulfate, and filtered. The filtrate was concentrated. The residue was passed through a silica gel column to obtain 1.35 g of colorless oil, yield: 90.4%. LC-MS(APCI): m/z=369.2$(M+1)^+$.

Step 4 Synthesis of Compound tert-butyl 2-(6-(((tert-butyldiphenylsilyl)oxy)methyl)dihydro-2H-pyran-3(4H)-ylidene)hydrazine-1-formate

6-(((tert-butyldiphenylsilyl)oxy)methyl)dihydro-2H-pyran-3(4H)-one (1.35 g, 3.66 mmol) and MeOH (20 mL) were added in sequence into a 100 mL two-necked flask equipped with magnetic stirring, and stirred until dissolved. The solution was cooled to 0 °C. Compound $NH_2$NHBoc (724 mg, 5.49 mmol) was added. The mixture was warmed to room temperature and stirred overnight. The organic solvent was evaporated under reduced pressure, and the residue was extracted with dichloromethane (30 mLx3). The organic phases were combined, washed with saturated saline (20 mL), dried with anhydrous sodium sulfate, and filtered. The filtrate was concentrated. The residue was passed through a silica gel column to obtain 1.15 g of a white solid, yield: 65%. LC-MS(APCI): m/z=483.0$(M+1)^+$.

Step 5 Synthesis of Compound tert-butyl 2-(6-(((tert-butyldiphenylsilyl)oxy)methyl)tetrahydro-2H-pyran-3-yl)hydrazine-1-formate

Tert-butyl 2-(6-(((tert-butyldiphenylsilyl)oxy)methyl)dihydro-2H-pyran-3(4H)-ylidene)hydrazine-1-carboxylate (2.45 g, 5.07 mmol) and MeOH (30 mL) were added in sequence into a 100 mL three-necked flask equipped with magnetic stirring, and stirred until dissolved. Acetic acid (314 mg, 5.07 mmol) was added. The mixture was stirred for 20 minutes, and cooled to 0 °C in an ice bath. $NaBH_3CN$ (628 mg, 10.14 mmol) was added in batches, and stirred at room temperature overnight under nitrogen atmosphere. Saturated sodium bicarbonate solution (30 mL) was added to quench the reaction. The organic solvent was evaporated under reduced pressure, and the residue was extracted with DCM (30 mLx3). The organic phases were combined, washed with saturated saline once, dried with anhydrous sodium sulfate, and filtered. The filtrate was concentrated. The residue was passed through a silica gel column to obtain 2.3 g of a white solid, yield: 95%. LC-MS(APCI): m/z=485.0$(M+1)^+$.

Step 6 Synthesis of the hydrochloride salt of the compound (6-(((tert-butyldiphenylsilyl)oxy)methyl)tetrahydro-2H-pyran-3-yl)hydrazine

To a 50 mL single-necked flask equipped with magnetic stirring, were added tert-butyl 2-(6-(((tert-butyldiphenylsilyl)oxy)methyl)tetrahydro-2H-pyran-3-yl)hydrazine-1-carboxylate (2.3 g, 4.74 mmol) and dichloromethane (10 mL) in sequence, and stirred until dissolved. Isopropanol solution of hydrogen chloride (20 mL, 5 M) was added. The mixture was stirred at room temperature for 2 hours, and concentrated to dryness under reduced pressure for the next step.

Step 7 Synthesis of intermediate compound A-4

Intermediate compound A-1 (1.24 g, 4.74 mmol), the hydrochloride of (6-(((tert-butyldiphenylsilyl)oxy)methyl) tetrahydro-2H-pyran-3-yl)hydrazine obtained in the previous step, and anhydrous ethanol (20 mL) were added into a 100

mL two-necked flask equipped with magnetic stirring and a condenser. Triethylamine (1.44 g, 14.22 mmol) was added under stirring. The temperature was raised to 80 °C under a nitrogen atmosphere. The mixture was stirred and reacted overnight. After cooling to room temperature, the solvent was evaporated under reduced pressure, and saturated saline (20 mL) and ethyl acetate (30 mL) were added. The mixture was stirred for 10 min. The organic phase was separated, and the aqueous phase was extracted with ethyl acetate (30 mLx2). The organic phases were combined, dried with anhydrous sodium sulfate, and filtered. The filtrate was concentrated and passed through a silica gel column to obtain 1.1g of a white solid, yield: 61.6%, LC-MS(APCI): m/z=377.1(M+1)+.

**Preparation of intermediate compound A-5 3-(8-amino-1-bromoimidazo[1,5-a]pyrazin-3-yl)cyclohexane-1-ol**

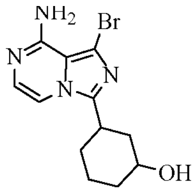

A-5

**The following synthetic route was adopted**

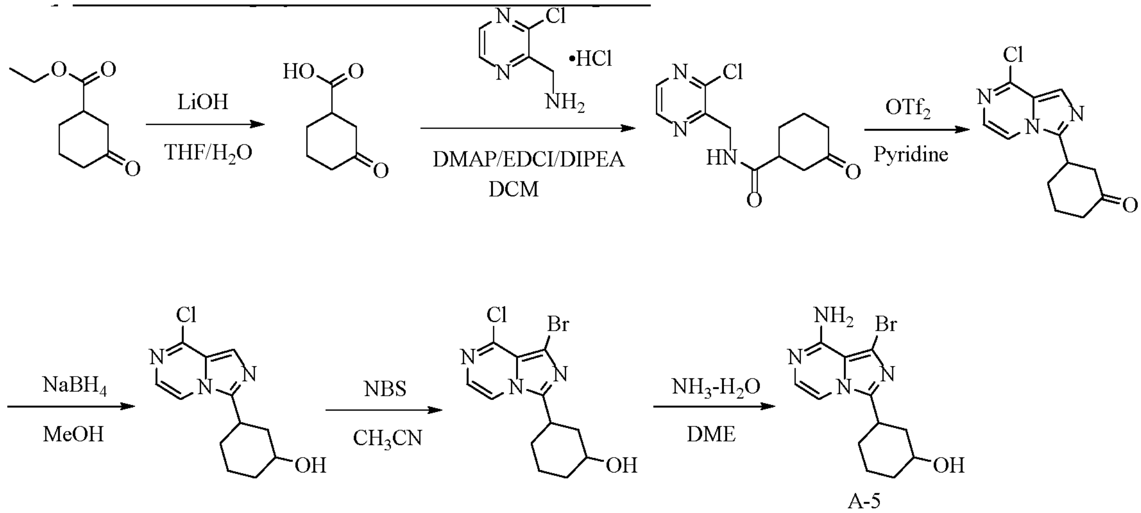


Step 1 Synthesis of Compound 3-oxocyclohexane-1-carboxylic acid

To a 100 mL single-necked flask equipped with magnetic stirring, were added ethyl 3-oxocyclohexane-1-carboxylate (1 g, 5.88 mmol), THF (10 mL), and $H_2O$ (10 mL) in sequence, and stirred until dissolved. LiOH (0.42 g, 17.64 mmol) was added. The mixture was stirred at room temperature overnight. The organic solvent was evaporated under reduced pressure. Dilute hydrochloric acid solution (1 M) was slowly added. pH was adjusted to ~2. A large amount of solid was precipitated, filtered, and washed with distilled water (10 mL). The filter cake was vacuum dried to obtain 800 mg of a white solid, yield: 86.1%.

Step 2 Synthesis of Compound N-((3-chloropyrazin-2-yl)methyl)-3-oxocyclohexane-1-carboxamide

(3-chloropyrazin-2-yl)methylamine hydrochloride (904 mg, 5.23 mmol), 3-oxocyclohexane-1-carboxylic acid (800 mg, 5.23 mmol) and dichloromethane (20 mL) were added in sequence into a 100 mL single-necked flask equipped with magnetic stirring. The mixture was cooled in an ice-water bath. DIPEA (1.69 g, 13.08 mmol), EDCI (1.3 eq, 6.80 mmol) and DMAP (124 mg, 1.05 mmol) were added under a nitrogen atmosphere. The ice bath was removed and the reaction was stirred at room temperature overnight. Water (50 mL) was added to quench the reaction and stirred for 3 minutes. The organic phase was separated, and the aqueous phase was extracted with dichloromethane (30 mLx2). The organic phases were combined, dried with anhydrous sodium sulfate, and filtered. The filtrate was concentrated and passed through a silica gel column to obtain 996 mg of an off-white solid, yield: 74.3%. LC-MS(APCI): m/z=268.2(M+1)+.

Step 3 Synthesis of Compound 3-(8-chloroimidazo[1,5-a]pyrazin-3-yl)cyclohexane-1-one

N-((3-chloropyrazin-2-yl)methyl)-3-oxocyclohexane-1-carboxamide (100 mg, 0.373 mmol), pyridine (88 mg, 1.12 mmol) and dichloromethane (10 mL) were added to a 50 mL two-necked flask equipped with magnetic stirring. The mixture was cooled in an ice-water bath and trifluoromethanesulfonic anhydride (316 mg, 1.12 mmol) was added dropwise under a nitrogen atmosphere. After the addition was completed, the ice bath was removed and the reaction was stirred at room temperature for 3 hours. Water (30 mL) was added to quench the reaction, and strirred for 5 min. The organic phase was separated, and the aqueous phase was extracted with dichloromethane (20 mLx2). The organic phases were combined, diluted with hydrochloric acid (1M, 10 mL), washed with saturated saline (20 mL), dried with anhydrous sodium sulfate, and filtered. The filtrate was concentrated and passed through a silica gel column to obtain 32 mg of an off-white solid, yield: 34.3%, LC-MS(APCI): m/z=250.1(M+1)$^+$.

Step 4 Synthesis of Compound 3-(8-chloroimidazo[1,5-a]pyrazin-3-yl)cyclohexyl-1-ol

3-(8-chloroimidazo[1,5-a]pyrazin-3-yl)cyclohexane-1-one (530 mg, 2.12 mmol) and MeOH (20 mL) were sequentially added to a 50 mL single-necked flask equipped with magnetic stirring, and stirred until dissolved. The solution was cooled in an ice-water bath, and $NaBH_4$ (4.24 mmol, 161 mg) was slowly added. After the addition was completed, the reaction was further stirred in an ice bath for 1 hour. Saturated aqueous sodium bicarbonate solution (30 mL) was added to quench the reaction, and strirred for 5 min. The organic solvent was evaporated under reduced pressure, and the residue was extracted with ethyl acetate (30 mLx3). The organic phases were combined, dried with anhydrous sodium sulfate, and filtered. The filtrate was concentrated to dryness. The residue was passed through a silica gel column to obtain 435 mg of an off-white solid, yield: 82.1%, LC-MS(APCI): m/z=250.1(M+1)$^+$.

Step 5 Synthesis of Compound 3-(1-bromo-8-chloroimidazo[1,5-a]pyrazin-3-yl)cyclohexyl-1-ol

3-(8-chloroimidazo[1,5-a]pyrazin-3-yl)cyclohexyl-1-ol (435 mg, 1.73 mmol) and acetonitrile (10 mL) were sequentially added to a 50 mL single-necked flask equipped with magnetic stirring. The mixture was cooled in an ice bath and NBS (323.3 mg, 1.82 mmol) was slowly added. After the addition was completed, the ice bath was removed and the reaction was stirred at room temperature for 2 hours. Saturated sodium bicarbonate aqueous solution (20 mL) was added to quench the reaction, and stirred for 3 minutes. Ethyl acetate (20 mL) was added. The organic phase was separated, and the aqueous phase was extracted with ethyl acetate (20 mLx2). The organic phases were combined, washed with saturated saline (20 mL), dried with anhydrous sodium sulfate, and filtered. The filtrate was concentrated to dryness. The residue was passed through a silica gel column to obtain 465 mg of an off-white solid, yield: 81.1%, LC-MS(APCI): m/z=369.1(M+1)$^+$.

Step 6 Synthesis of intermediate compound A-5

3-(1-bromo-8-chloroimidazo[1,5-a]pyrazin-3-yl)cyclohexyl-1-ol (465 mg, 1.4 mmol) and ethylene glycol monomethyl ether (5 mL) were added in sequence to a 100 mL sealed tube equipped with magnetic stirring, and stirred until dissolved. Ammonia water (10 mL) was added, sealed, and heated to 70°C. The reaction was stirred overnight. The mixture was cooled to room temperature. Ethyl acetate (50 mL) and water (50 mL) were added. The organic phase was separated, and the aqueous phase was extracted with ethyl acetate (30 mLx2). The organic phases were combined, dried with anhydrous sodium sulfate, and filtered. The filtrate was concentrated and passed through a silica gel column to obtain 400 mg of a white solid, yield: 92.0%, LC-MS(APCI): m/z=311.1(M+1)$^+$.

**Preparation of intermediate compound A-6 3-(4-amino-3-iodo-1H-pyrazolo[3,4-d]pyrimidin-1-yl)-1-methylcyclohexane-1-ol**

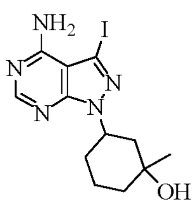

A-6

**The following synthetic route was adopted**

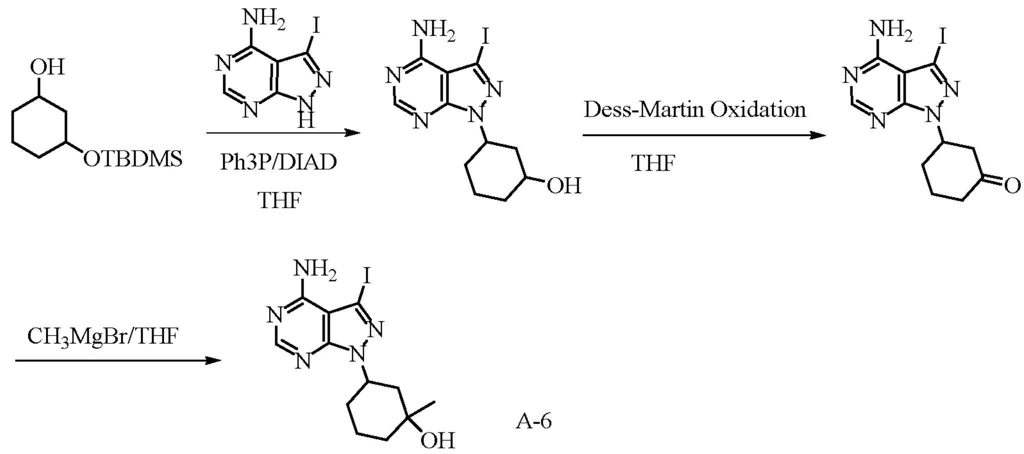


A-6

Step 1 Synthesis of Compound 3-(4-amino-3-iodo-1H-pyrazolo[3,4-d]pyrimidin-1-yl)cyclohexane-1-ol

3-((tert-butyldimethylsilyl)oxy)cyclohexane-1-ol (2 g, 8.69 mmol), triphenylphosphine (6 g, 22.90 mmol), 3-iodo-1H-pyrazolo[3,4-d]pyrimidin-4-amine (2 g, 7.66 mmol) were added to a 100 mL three-necked flask equipped with magnetic stirring and a condenser. The flask was evacuated and the atmosphere was replaced with nitrogen three times. Anhydrous THF (20 mL) was added under ice-water bath cooling. The mixture was stirred and dispersed. DIAD (3 g, 14.85 mmol) was added dropwise. After the addition was completed, the mixture was heated to room temperature and the reaction was stirred overnight. Ethyl acetate (50 mL) and water (50 mL) were added. The organic phase was separated, extracted with ethyl acetate (2x50mL), washed with saturated saline (2x50 mL), dried with anhydrous sodium sulfate, and filtered. 4M dioxane hydrochloride solution was added to the filtrate and stirred at room temperature for 30 min. The mixture was concentrated under reduced pressure to dryness. The residue was passed through a silica gel column to obtain 2.1 g of a white solid, yield: 76.36%. LC-MS(APCI): m/z=360.1$(M+1)^+$.

Step 2 Synthesis of Compound 3-(4-amino-3-iodo-1H-pyrazolo[3,4-d]pyrimidin-1-yl)cyclohexane-1-one

3-(4-amino-3-iodo-1H-pyrazolo[3,4-d]pyrimidin-1-yl)cyclohexane-1-ol (2.1 g, 5.85 mmol) was added to a 100 mL three-necked flask equipped with magnetic stirring and a condenser. The flask was evacuated and the atmosphere was replaced with nitrogen three times. Anhydrous THF (10 mL) was added under ice-water bath cooling, stirred and dispersed. Dess-martin oxidant (4.98 g, 11.74 mmol) in DCM (30 mL) was added dropwise, stirred and reacted under ice-water bath for 30 minutes. The mixture was warmed to room temperature, and stirred and reacted overnight. Saturated $Na_2S_2O_3$ (50 mL) was added to quench the reaction. The organic phase was separated, and the aqueous phase was extracted with DCM (2x50mL). The organic phases were combined, washed with saturated $NaHCO_3$ (2x50 mL), dried with anhydrous sodium sulfate, and filtered. The solvent was evaporated under reduced pressure. The residue was passed through a silica gel column to obtain 1 g of a white solid, yield: 47.89%. LC-MS(APCI): m/z=358.2$(M+1)^+$.

Step 3 Synthesis of intermediate compound A-6

Compound 3-(4-amino-3-iodo-1H-pyrazolo[3,4-d]pyrimidin-1-yl)cyclohexane-1-one (1 g, 2.80 mmol) was added to a 100 mL three-necked flask equipped with magnetic stirring and a condenser. The flask was evacuated and the atmosphere was replaced with nitrogen three times. Anhydrous THF (20 mL) was added under ice-water bath cooling, stirred and dispersed. 3M $CH_3MgBr$ (2.8 mL, 8.42 mmol) solution was added dropwise, stirred and reacted under ice-water bath for 30 minutes. The mixture was warmed to room temperature, and stirred and reacted for 2 hours. Water (50 mL) was added to quench the reaction. The mixture was extracted with ethyl acetate (3x50mL). The organic phases were combined, washed with saturated saline (2x50 mL), dried with anhydrous sodium sulfate, and filtered. The filtrate was concentrated under reduced pressure to dryness. The residue was passed through a silica gel column to obtain 924 mg of a white solid, yield: 88.56%. LC-MS(APCI): m/z=374.3$(M+1)^+$.

**Preparation of intermediate compound A-7 4-(4-amino-3-iodo-1H-pyrazolo[3,4-d]pyrimidin-1-yl)cyclohexane-1-ol**

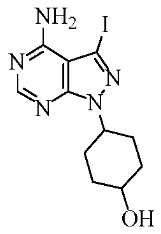

A-7

**The following synthetic route was adopted**

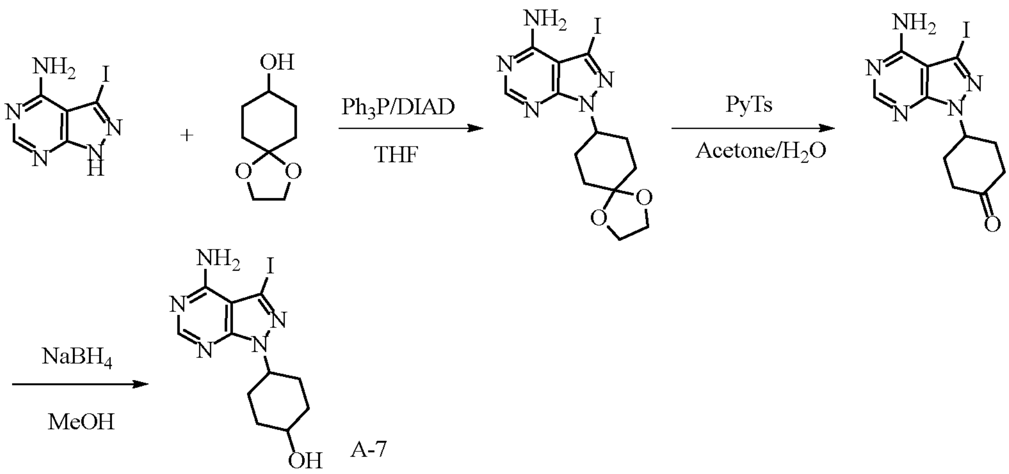


A-7

Step 1 Synthesis of Compound 3-iodo-1-(1,4-dioxaspiro[4.5]decane-8-yl)-1H-pyrazolo[3,4-d]pyrimidin-4-amine

4-dioxaspiro[4.5]decane-8-ol (2.9 g, 18.35 mmol), triphenylphosphine (12 g, 45.80 mmol), and 3-iodo-1H-pyrazolo[3,4-d]pyrimidin-4-amine (4 g, 15.32 mmol) were added into a 100 mL three-necked flask equipped with magnetic stirring and a condenser. The flask was evacuated and the atmosphere was replaced with nitrogen three times. Anhydrous THF (20 mL) was added under ice-water bath cooling, stirred and dispersed. DIAD (6.19 g, 30.64 mmol) was added dropwise. The reaction mixture was warmed to room temperature, and stirred and reacted overnight. Ethyl acetate (50 mL) and water (50 mL) were added. The organic phase was separated, extracted with ethyl acetate (2x50mL), washed with saturated saline (2x50 mL), dried with anhydrous sodium sulfate, and filtered. The filtrate was concentrated under reduced pressure to dryness. The residue was passed through a silica gel column to obtain 3.32 g of a white solid, yield: 54.07%. LC-MS(APCI): m/z=402.1$(M+1)^+$.

Step 2 Synthesis of Compound 4-(4-amino-3-iodo-1H-pyrazolo[3,4-d]pyrimidin-1-yl)cyclohexane-1-one

The compound 3-iodo-1-(1,4-dioxaspiro[4.5]decane-8-yl)-1H-pyrazolo[3,4-d]pyrimidin-4-amine (3.6 g, 89.78 mmol) was added to a 100 mL single-necked flask equipped with magnetic stirring and a condenser. Acetone (40 mL) and water (40 mL) were added at room temperature. PyTs (4.62 g, 183.84 mmol) was added. The mixture was stirred and dispersed. The flask was evacuated and the atmosphere was replaced with nitrogen three times. The mixture was heated to 65 °C, and stirred and reacted overnight. The mixture was cooled to room temperature and water (50 mL) was added. The mixture was extracted with ethyl acetate (3x50mL). The organic phases were combined, washed with saturated saline (2x50 mL), dried with anhydrous sodium sulfate, and filtered. The filtrate was concentrated under reduced pressure to dryness. The residue was passed through a silica gel column to obtain 2.8 g of a white solid, yield: 87.50%. LC-MS(APCI): m/z=358.5$(M+1)^+$.

Step 3 Synthesis of Intermediate Compound A-7

4-(4-amino-3-iodo-1H-pyrazolo[3,4-d]pyrimidin-1-yl)cyclohexan-1-one (1 g, 2.80 mmol) was added to a 50 mL three-necked flask equipped with magnetic stirring and a condenser tube. The flask was evacuated and the atmosphere was replaced with nitrogen three times. Anhydrous MeOH (10 mL) was added under ice-water bath cooling, stirred and dispersed. $NaBH_4$ (43 mg, 1.12 mmol) was added, and the reaction was stirred under ice-water bath for 30 minutes. The mixture was warmed to room temperature, and stirred for 1 hour. Water (50 mL) was added to quench the reaction. The mixture was extracted with ethyl acetate (3x50 mL). The organic phases were combined, washed with saturated saline (2x50 mL), dried with anhydrous sodium sulfate, and filtered. The filtrate was concentrated under reduced pressure to dryness. The residue was passed through a silica gel column to obtain 620 mg of a white solid, yield: 61.65%. LC-MS(APCI): m/z=360.3$(M+1)^+$.

**<u>Preparation of intermediate compound A-8 3-(4-amino-3-iodo-1H-pyrazolo[3,4-d]pyrimidin-1-yl)cyclopentan-1-ol</u>**

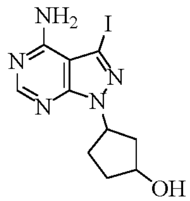

A-8

**<u>The following synthetic route was adopted</u>**

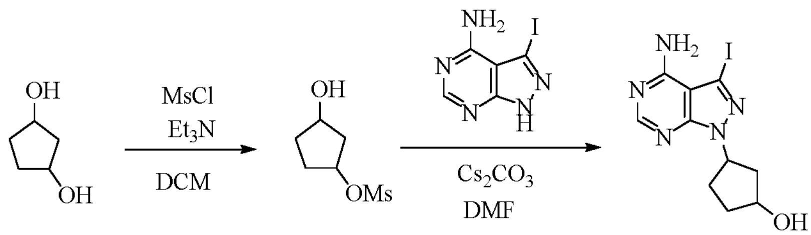


A-8

Step 1 Synthesis of 3-hydroxycyclopentyl methanesulfonate

Cyclopentane-1,3-diol (1 g, 9.80 mmol) and triethylamine (1.98 g, 19.60 mmol) were added to a 50 mL two-necked flask equipped with magnetic stirring and a condenser tube. The flask was evacuated and the atmosphere was replaced with nitrogen three times. Anhydrous DCM (10 mL) was added under ice-water bath cooling, stirred and dispersed. MsCl (1.12 g, 9.82 mmol) was added dropwise, and the mixture was warmed to room temperature. The mixture was stirred and reacted overnight while maintaining the temperature. Ethyl acetate (50 mL) and water (50 mL) were added. The organic phase was separated, extracted with ethyl acetate (2x50mL), washed with saturated sodium carbonate (2x50 mL), dried with anhydrous sodium sulfate, and filtered. The filtrate was concentrated under reduced pressure to dryness. 1.25 g of crude compound was obtained, yield: 70.83%.

Step 2 Synthesis of intermediate compound A-8

To a 50 mL single-necked flask equipped with magnetic stirring and a condenser tube, was added 3-iodo-1H-pyrazolo[3,4-d]pyrimidin-4-amine (750 mg, 2.87 mmol). DMF (10 mL) was added at room temperature. 3-hydroxycyclopentyl methanesulfonate (600 mg, 3.33 mmol) was added, and cesium carbonate (2.3 g, 7.08 mmol) was added. The mixture was stirred and dispersed. The flask was evacuated and the atmosphere was replaced with nitrogen three times. Temperature was raised to 100 °C. The mixture was stirred and reacted overnight while maintaining the temperature. The mixture was cooled to room temperature, and water (50 mL) was added. The mixture was extracted with ethyl acetate (3x50mL). The organic phases were combined, washed with saturated saline (2x50 mL), dried with anhydrous sodium sulfate, and filtered. The filtrate was concentrated under reduced pressure to dryness. The residue was passed through a silica gel column to obtain 706 mg of a white solid, yield: 71.36%. LC-MS(APCI): m/z=345.3$(M+1)^+$.

**Preparation of intermediate compound A-9 (trans-4-(4-amino-3-iodo-1H-pyrazolo[3,4-d]pyrimidin-1-yl)cyclopent-2-en-1-yl) acetate**

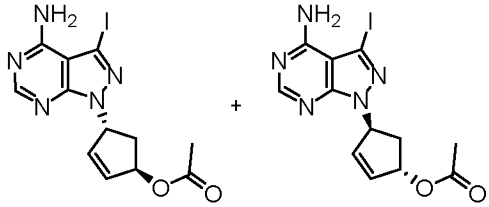

A-9

**The following synthetic route was adopted**

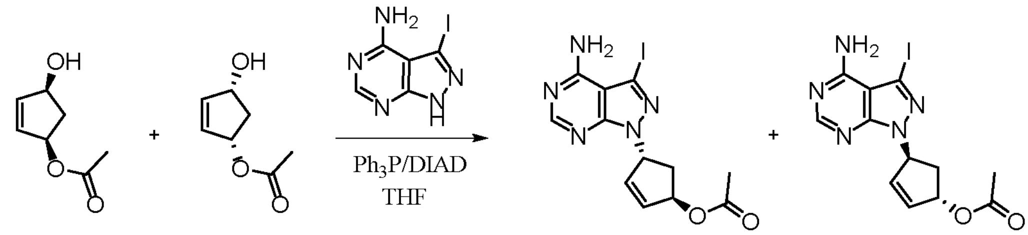


A-9

To a 100 mL three-necked flask equipped with magnetic stirring, were added 3-iodo-1H-pyrazolo[3,4-d]pyrimidin-4-amine (1.0 g, 7.03 mmol), (cis-4-hydroxycyclopent-2-en-1-yl) acetate (1.53 g, 5.86 mmol), $Ph_3P$ (4.61 g, 17.58 mmol), and THF (20 ml). The flask was evacuated and the atmosphere was replaced with nitrogen three times. DIAD (2.37 g, 11.72 mmol) was slowly added dropwise under ice-water bath cooling. After the dropwise addition was completed, the ice bath was removed, and the reaction was stirred at room temperature in nitrogen atmosphere overnight. Water (50 mL) was added to quench the reaction. The mixture was extracted with ethyl acetate (50 mL×2). The organic phases were combined, washed with saturated saline (50 mL), dried with anhydrous sodium sulfate, and filtered. The filtrate was concentrated and passed through a silica gel column to obtain 0.98 g of a white solid, yield: 43.4%. LC-MS(APCI): m/z=386.0$(M+1)^+$.

**Preparation of intermediate compound A-10 4-amino-3-(4-bromophenyl)-1-(4-hydroxycyclohexyl)-1,6-dihydro-7H-pyrazolo[3,4-d]pyridazin-7-one**

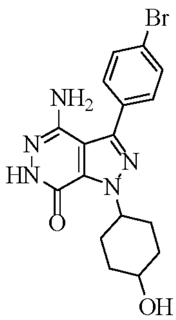

A-10

**The following synthetic route was adopted**

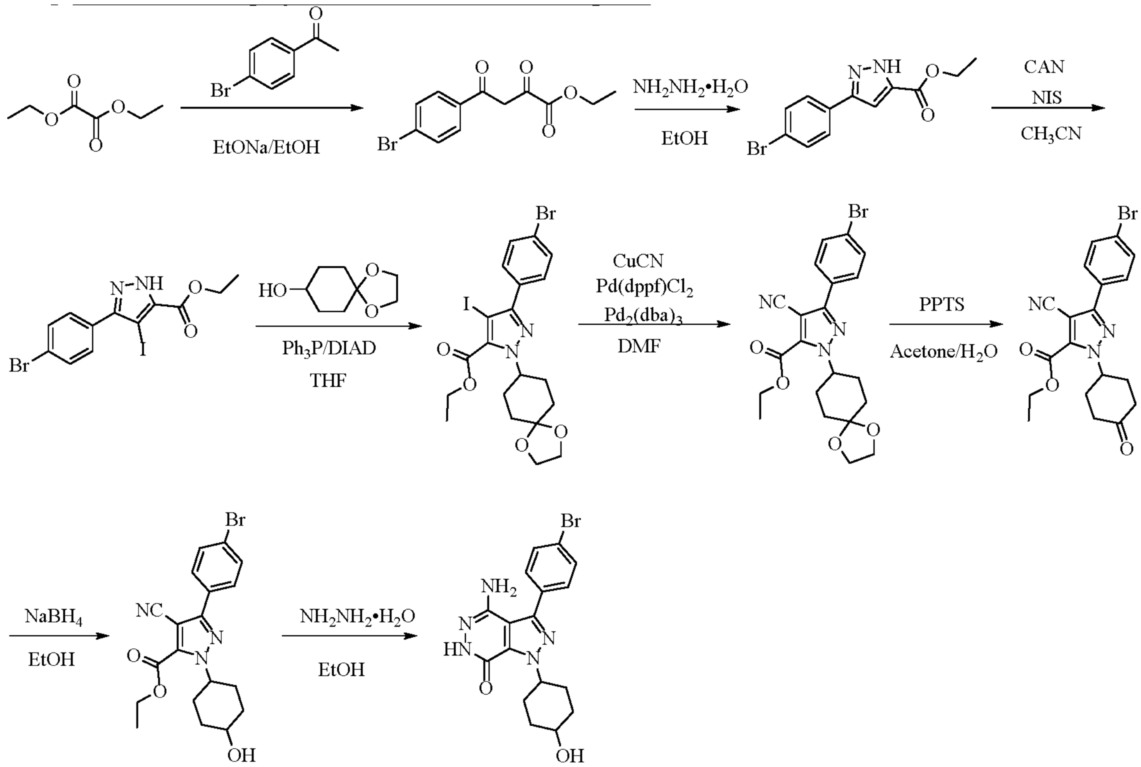


A-10

Step 1 Synthesis of compound ethyl 4-(4-bromophenyl)-2,4-dioxobutyrate

To a 250 mL two-necked flask equipped with magnetic stirring, were added ethanol (50 mL), sodium ethoxide solution (10.5 g, 20% wt), and diethyl oxalate (4.5 g, 30.1 mmol) in sequence. The flask was evacuated and the atmosphere was replaced with nitrogen three times. The mixture was reacted at room temperature for 10 minutes, and then a solution of 4-bromobenzophenone (5 g, 25 mmol) in ethanol (20 mL) was added dropwise to the above reaction solution under an ice bath. After the dropwise addition was completed, reaction was carried out at room temperature for 4 hours. Acetic acid (1.81 g, 30.1 mmol) was added to the reaction solution, and stirred at room temperature for 10 minutes. The reaction was quenched, and the reaction solution was directly retained for the next reaction.

Step 2 Synthesis of compound ethyl 3-(4-bromophenyl)-1H-pyrazole-5-formate

Hydrazine hydrate (1.25 g, 25 mmol) was directly added dropwise to the reaction solution quenched in Step 1, and reacted at room temperature overnight. Water (60 mL) and ethanol (100 mL) were added. The mixture was stirred at room temperature for 10 min. Ethanol was removed with rotary evaporator, and ethyl acetate (100 mLx3) was added for extraction. The organic phases were combined, washed with saturated saline (100 mL), dried with anhydrous sodium sulfate, and filtered. The filtrate was concentrated and passed through a silica gel column to obtain 7.0 g of a yellow oil. The combined yield of Step 1 and Step 2 was 94.4%. LC-MS(APCI): m/z=296.1$(M+1)^+$.

Step 3 Synthesis of compound ethyl 3-(4-bromophenyl)-4-iodo-1H-pyrazole-5-formate

Anhydrous acetonitrile (60 mL), ethyl 3-(4-bromophenyl)-1H-pyrazole-5-carboxylate (7 g, 23.7 mmol), NIS (5.87 g, 26.1 mmol), and CAN (1.3 g, 2.37 mmol) were sequentially added to a 150 mL two-necked flask equipped with magnetic stirring. The flask was evacuated and the atmosphere was replaced with nitrogen three times. The mixture was warmed to 90 °C. Water (60 mL) was added. The mixture was extracted with ethyl acetate (120 mL × 3). The organic phases were combined, dried with anhydrous sodium sulfate, and filtered. The filtrate was concentrated and passed through a silica gel column to obtain 8.3 g of a yellow oil, yield: 83%, LC-MS(APCI): m/z=421.2$(M+1)^+$.

Step 4 Synthesis of compound ethyl 3-(4-bromophenyl)-4-iodo-1-(1,4-dioxaspiro[4.5]decan-8-yl)-1H-pyrazole-5-carboxylate

Anhydrous THF (10 mL), ethyl 3-(4-bromophenyl)-4-iodo-1H-pyrazole-5-carboxylate (1 g, 2.38 mmol), 1,4-dioxaspiro[4.5]decan-8-ol (0.47 g, 2.97 mmol), and $Ph_3P$ (1.871 g, 7.14 mmol) were added to a 50 mL three-necked flask equipped with magnetic stirring. The flask was evacuated and the atmosphere was replaced with nitrogen three times. DIAD (0.962 g, 4.76 mmol) was added dropwise under ice-water bath cooling, stirred and dispersed. The reaction was stirred under an ice-water bath for 10 minutes, and slowly warmed to room temperature. The mixture was stirred and reacted overnight while maintaining the temperature. Water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 1.1 g of a white solid, yield: 82%. LC-MS(APCI): m/z=560.0 $(M+1)^+$.

Step 5 Synthesis of compound ethyl 3-(4-bromophenyl)-4-cyano-1-(1,4-dioxaspiro[4.5]decan-8-yl)-1H-pyrazole-5-carboxylate

Ethyl 3-(4-bromophenyl)-4-iodo-1-(1,4-dioxaspiro[4.5]decan-8-yl)-1H-pyrazole-5-carboxylate (1.1 g, 1.97 mmol) and DMF (10 mL) were charged to a 50 mL single-necked flask equipped with magnetic stirring and a condenser tube, and stirred until dissolved. Cuprous cyanide (0.26 g, 2.9 mmol), $Pd_2(dba)_3$ (0.18 g, 0.19 mmol), and $Pd(dppf)Cl_2$ (0.16 g, 0.19 mmol) were added. The flask was evacuated and the atmosphere was replaced with nitrogen three times. The mixture was heated to 100 °C under nitrogen atmosphere and the reaction was stirred overnight. At room temperature, water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 0.73g of a white solid, yield: 80%. LC-MS(APCI): m/z= 460.0$(M+1)^+$.

Step 6 Synthesis of ethyl compound 3-(4-bromophenyl)-4-cyano-1-(4-oxocyclohexanyl)-1H-pyrazole-5-carboxylate

Ethyl 3-(4-bromophenyl)-4-cyano-1-(1,4-dioxaspiro[4.5]decan-8-yl)-1H-pyrazole-5-carboxylate (735 mg, 1.60 mmol), acetone (5 mL), water (5 mL) and PPTS (805mg, 3.21 mmol) were charged to a 50 mL single-necked flask equipped with magnetic stirring and a condenser tube. The mixture was stirred until dissolved, and heated up to 90 °C under nitrogen atmosphere. The mixture was stirred and reacted overnight while maintaining the temperature. The reaction was cooled to room temperature. Water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 0.583 g of a white solid, yield: 87.74%. LC-MS(APCI): m/z= 416.0$(M+1)^+$.

Step 7 Synthesis of compound ethyl 3-(4-bromophenyl)-4-cyano-1-(4-hydroxycyclohexanyl)-1H-pyrazole-5-carboxylate

Ethyl 3-(4-bromophenyl)-4-cyano-1-(4-oxocyclohexanyl)-1H-pyrazole-5-carboxylate (583 mg, 1.40 mmol) and absolute ethanol (10 mL) were charged to a 50 mL single-necked flask equipped with magnetic stirring. Sodium borohydride (23 mg, 0.6 mmol) was added under stirring in an ice-water bath. The mixture was warmed to room temperature, and the reaction was stirred for 30 minutes. The solvent was evaporated under reduced pressure. The reaction was quenched by the addition of water (10 mL) and extracted by the addition of ethyl acetate (30 mL × 3). The organic phase was separated, washed with saturated saline (30 mL), dried with anhydrous sodium sulfate, and filtered. The filtrate was concentrated and passed through a silica gel column to obtain 0.492 g of a white solid, yield: 83.99%. LC-MS(APCI): m/z= 418.0$(M+1)^+$.

Step 8 Synthesis of Intermediate Compound A-10

Ethyl 3-(4-bromophenyl)-4-cyano-1-(4-hydroxycyclohexanyl)-1H-pyrazole-5-carboxylate (492 mg, 1.18 mmol) and ethanol (25 mL) were charged to a 50 mL single-necked flask equipped with magnetic stirring and a condenser tube, and stirred until dissolved. Hydrazine hydrate (8.5 mL) was added, and the temperature was raised to 80 °C under nitrogen atmosphere. The mixture was stirred and reacted overnight while maintaining the temperature. The reaction was cooled to room temperature. Water (30 mL) was added to quench the reaction. Ethyl acetate (30 mL × 3) was added for extraction. The organic phase was separated, washed with saturated saline (30 mL), dried with anhydrous sodium sulfate, and filtered. The filtrate was concentrated and passed through a silica gel column to obtain 0.171g (lower polarity) and 0.116g (higher polarity) of a white solid, yield: 60.36%. LC-MS(APCI): m/z= 404.0$(M+1)^+$.

**Preparation of intermediate compound A-11 4-amino-3-(4-bromophenyl)-1-(3-hydroxycyclopentanyl)-1,6-dihydro-7H-pyrazolo[3,4-d]pyridazin-7-one**

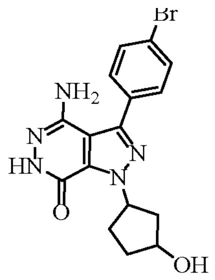

A-11

**The following synthetic route was adopted**

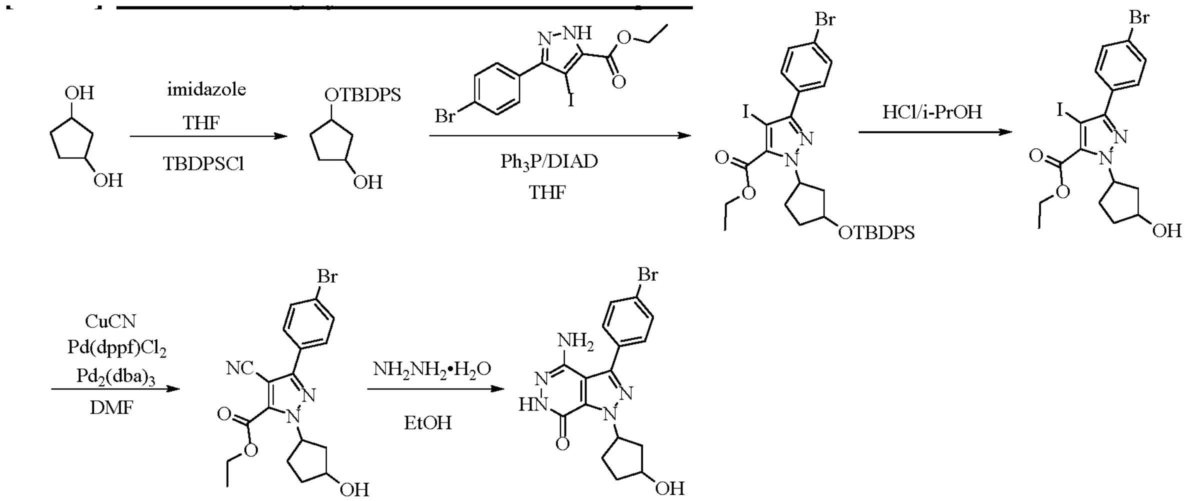


A-11

Step 1 Synthesis of Compound 3-((tert-butyldiphenylsilyl)oxy)cyclopentan-1-ol

THF (20 mL), 1,3-cyclopentanediol (1.1 g, 10.78 mmol), and imidazole (1.1 g, 16.18 mmol) were sequentially added to a 50 mL three-necked flask equipped with magnetic stirring, and stirred until dissolved. The solution was cooled to 0 °C, and TBDPSCl (2.97 g, 10.8 mmol) was added. The mixture was stirred for 10 min, and slowly warmed to room temperature. The reaction was stirred while maintaining the temperature for 2 h. Ethyl acetate (50 mL x 3) and water (50 mL) were added for extraction. The organic phase was separated, washed with water (50 mL), washed with saturated saline (50 mL), dried with anhydrous sodium sulfate, and filtered. The filtrate was concentrated to dryness. The residue was passed through a column with petroleum ether/ethyl acetate (10/1) to give 1.5 g of a colorless transparent oil, yield: 40.9%. LC-MS(APCI): m/z=341.2 $(M+1)^+$.

Step 2 Synthesis of compound ethyl 3-(4-bromophenyl)-1-(3-((tert-butyldiphenylsilyl)oxy)cyclopentanyl)-4-iodo-1H-pyrazole-5-carboxylate

Anhydrous THF (20 mL), ethyl 3-(4-bromophenyl)-4-iodo-1H-pyrazole-5-carboxylate (1.5 g, 3.57 mmol), 3-((tert-butyldiphenylsilyl)oxy)cyclopentan-1-ol (1.21 g, 3.56 mmol), and $Ph_3P$ (2.8 g, 10.68 mmol) were added to a 50 mL three-necked flask equipped with magnetic stirring. The flask was evacuated and the atmosphere was replaced with nitrogen three times. DIAD (1.44 g, 7.13 mmol) was added dropwise under ice-water bath cooling, stirred and dispersed. The reaction was stirred under an ice-water bath for 10 minutes, and slowly warmed to room temperature. The mixture was stirred and reacted overnight while maintaining the temperature. Water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 2.3 g of a white solid, yield: 87%. LC-MS(APCI): m/z=743.0$(M+1)^+$.

Step 3 Synthesis of compound ethyl 3-(4-bromophenyl)-1-(3-hydroxycyclopentanyl)-4-iodo-1H-pyrazole-5-carboxylate

To a 50 mL single-necked flask equipped with magnetic stirring, ethyl 3-(4-bromophenyl)-1-(3-((tert-butyldiphenylsilyl)oxy)cyclopentanyl)-4-iodo-1H-pyrazole-5-carboxylate (2.3 g, 3.09 mmol) and hydrochloric acid solution in isopropanol (5 mol/L, 10 mL) were added, and the reaction was stirred at room temperature for 0.5 h. The solvent was evaporated under reduced pressure. Saturated aqueous sodium bicarbonate solution (20 mL) and ethyl acetate (30 mL) were added. The organic phase was separated, and the aqueous phase was extracted with ethyl acetate (30 mLx2). The organic phases were combined, washed with saturated saline, dried with anhydrous sodium sulfate, and filtered. The filtrate was concentrated and passed through a silica gel column to obtain 1.28 g of a white solid, yield: 82.5%. LC-MS(APCI): m/z=504.0$(M+1)^+$.

Step 4 Synthesis of compound ethyl 3-(4-bromophenyl)-4-cyano-1-(3-hydroxycyclopentanyl)-1H-pyrazole-5-carboxylate

Ethyl 3-(4-bromophenyl)-1-(3-hydroxycyclopentanyl)-4-iodo-1H-pyrazole-5-carboxylate (1.284 g, 2.55 mmol) and DMF (15 mL) were charged to a 50 mL single-necked flask equipped with magnetic stirring and a condenser tube, and stirred until dissolved. Cuprous cyanide (0.343 g, 3.83 mmol), $Pd_2(dba)_3$ (0.234 g, 0.26 mmol), and $Pd(dppf)Cl_2$ (0.208 g, 0.26 mmol) were added. The flask was evacuated and the atmosphere was replaced with nitrogen three times. The mixture was heated to 100°C under nitrogen atmosphere, and the reaction was stirred overnight. At room temperature, water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 0.77g of a white solid, yield: 75%. LC-MS(APCI): m/z= 404.0$(M+1)^+$.

Step 4 Synthesis of Intermediate Compound A-1 1

Ethyl 3-(4-bromophenyl)-4-cyano-1-(3-hydroxycyclopentanyl)-1H-pyrazole-5-carboxylate (1 g, 2.48 mmol) and ethanol (43 mL) were charged to a 100 mL single-necked flask equipped with magnetic stirring and a condenser tube, and stirred until dissolved. Hydrazine hydrate (14 mL) was added, and the temperature was raised to 80 °C under nitrogen atmosphere. The mixture was stirred and reacted overnight while maintaining the temperature. The reaction was cooled to room temperature. Water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 0.52 g of a white solid, yield: 54%. LC-MS(APCI): m/z=390.0$(M+1)^+$.

**Preparation of intermediate compound A-12 4-amino-3-(4-bromophenyl)-1-(6-(hydroxymethyl)tetrahydro-2H-pyran-3-yl)-1,6-dihydro-7H-pyrazolo[3,4-d]pyridazin-7-one**

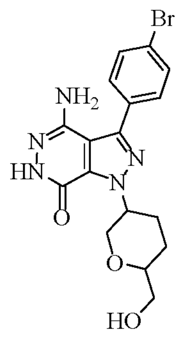

A-12

**The following synthetic route was adopted**

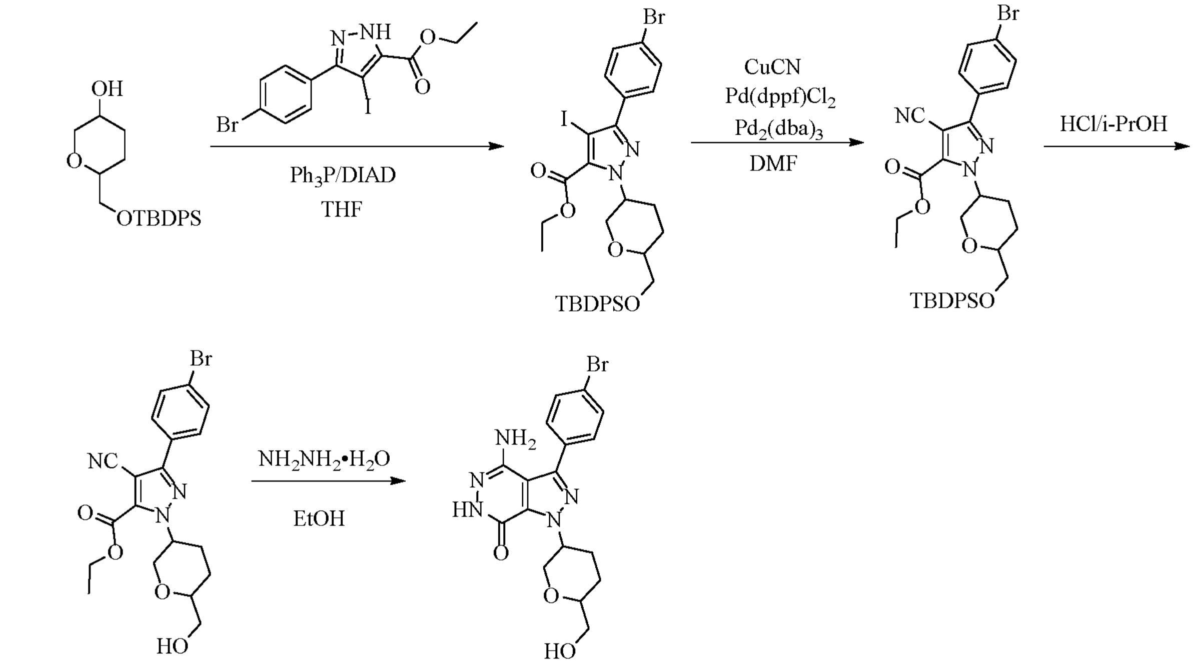


A-12

Step 1 Synthesis of compound ethyl 3-(4-bromophenyl)-1-(6-(((tert-butyldiphenylsilyl)oxy)methyl)tetrahydro-2H-pyran-3-yl)-4-iodo-1H-pyrazole-5-carboxylate

Anhydrous tetrahydrofuran (60 mL), 6-(((tert-butyldiphenylsilyl)oxy)methyl)tetrahydro-2H-pyran-3-ol (1 g, 2.7 mmol), ethyl 3-(4-bromophenyl)-4-iodo-1H-pyrazole-5-carboxylate (1.25 g, 3.0 mmol), and triphenylphosphine (1.06 g, 4.0 mmol) were sequentially added to a 150 mL three-necked flask equipped with magnetic stirring. The flask was evacuated and the atmosphere was replaced with nitrogen three times. The reaction was cooled in an ice bath. DIAD (0.82 g, 4.0 mmol) was added dropwise. After the addition was completed, the reaction was carried out at room temperature overnight. Water (60 mL) and ethyl acetate (100 mLx3) were added for extraction. The organic phases were combined, dried with anhydrous sodium sulfate, and filtered. The filtrate was concentrated and passed through a silica gel column to obtain 1 g of a colorless transparent oil, yield: 47.9%. LC-MS(APCI): m/z=773.1$(M+1)^+$.

Step 2 Synthesis of compound ethyl 3-(4-bromophenyl)-1-(6-(((tert-butyldiphenylsilyl)oxy)methyl)tetrahydro-2H-pyran-3-yl)-4-cyano-1H-pyrazole-5-carboxylate

DMF (30 mL), ethyl 3-(4-bromophenyl)-1-(6-(((tert-butyldiphenylsilyl)oxy)methyl)tetrahydro-2H-pyran-3-yl)-4-iodo-1H-pyrazole-5-carboxylate (1 g, 1.3 mmol), CuCN (0.173 g, 1.43 mmol), Pd(dppf)$Cl_2$DCM (0.106 g, 0.13 mmol), and $Pd_2(dba)_3$ (0.118 g, 0.13 mmol) were sequentially added to a 100 mL three-necked flask equipped with magnetic stirring and a condenser tube. The flask was evacuated and the atmosphere was replaced with nitrogen three times. The mixture was heated to 100 °C and the reacted overnight. Water (60 mL) and ethyl acetate (120 mLx3) were added for extraction. The organic phases were combined, washed with saturated saline (100 mL), dried with anhydrous sodium sulfate, and filtered. The filtrate was concentrated and passed through a silica gel column to obtain 600mg of a white solid, yield: 69%, LC-MS(APCI): m/z=672.2$(M+1)^+$.

Step 3 Synthesis of compound ethyl 3-(4-bromophenyl)-4-cyano-1-(6-(hydroxymethyl)tetrahydro-2H-pyran-3-yl)-1H-pyrazole-5-carboxylate

Ethyl 3-(4-bromophenyl)-1-(6-(((tert-butyldiphenylsilyl)oxy)methyl)tetrahydro-2H-pyran-3-yl)-4-cyano-1H-pyrazole-5-carboxylate (600 mg, 0.90 mmol), and hydrochloric acid solution in isopropanol (5 mL, 2 mol/L) were sequentially added to a 50 mL single-necked flask equipped with magnetic stirring and reacted overnight at room temperature. The solution was concentrated. The isopropanol was rotary evaporated to dryness. The concentrated solution was adjusted to a pH of 8 with saturated aqueous sodium bicarbonate solution. Water (20 mL) and dichloromethane (30 mL × 3) were added for extraction. The organic phases were combined, dried with anhydrous sodium sulfate, and filtered. The filtrate was concentrated and passed through a silica gel column to obtain 300 mg of a yellow solid, yield: 77%, LC-MS(APCI): m/z=434.2$(M+1)^+$.

Step 4 Synthesis of Intermediate Compound A-12

To a 10 mL microwave tube equipped with magnetic stirring, ethanol (8 mL), ethyl 3-(4-bromophenyl)-4-cyano-1-(6-(hydroxymethyl)tetrahydro-2H-pyran-3-yl)-1H-pyrazole-5-carboxylate (300 mg, 0.7 mmol), and hydrazine hydrate (2 mL, content 80%) were sequentially added. The mixture was heated to 80 °C, and reacted overnight while maintaining the temperature. The reaction solution was concentrated directly and passed through a silica gel column to obtain 90 mg of a white solid, yield: 31%, LC-MS(APCI): m/z=421.1(M+1)$^+$.

**Preparation of intermediate compound A-13 4-amino-3-(4-bromophenyl)-1-(3-hydroxycyclohexanyl)-1,6-dihydro-7H-pyrazolo[3,4-d]pyridazin-7-one**

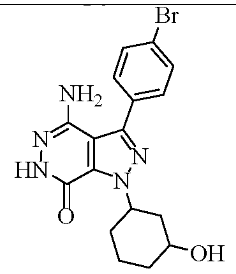

A-13

**The following synthetic route was adopted**

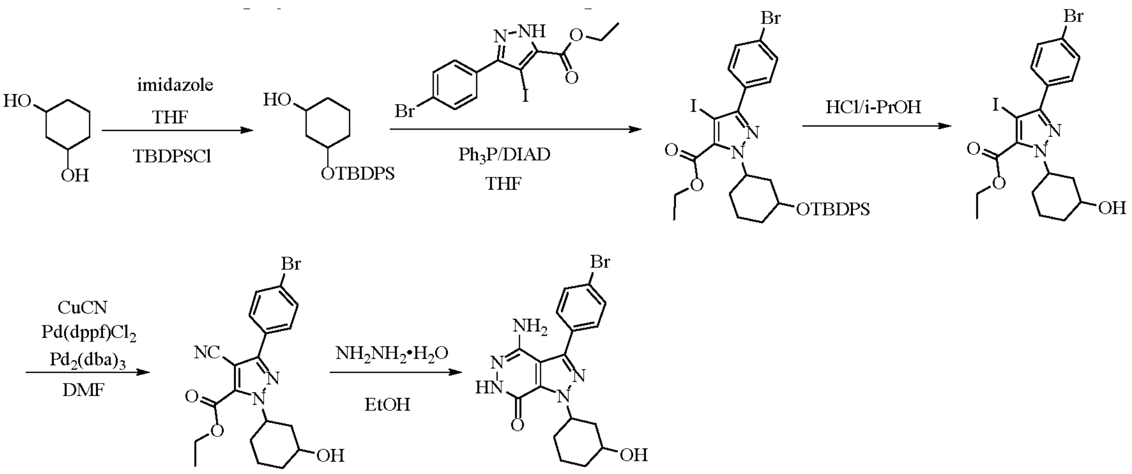


A-13

Step 1 Synthesis of compound 3-((tert-butyldiphenylsilyl)oxy)cyclohexan-1-ol

THF (20 mL), 1,3-cyclohexanediol (1.25 g, 10.78 mmol), and imidazole (1.1 g, 16.18 mmol) were sequentially added to a 50 mL three-necked flask equipped with magnetic stirring, and stirred until dissolved. The solution was cooled to 0 °C, and TBDPSCl (2.97 g, 10.8 mmol) was added. The mixture was stirred for 10 min, and slowly warmed to room temperature. The reaction was stirred while maintaining the temperature for 2 h. Ethyl acetate (50 mL × 3) and water (50 mL) were added for extraction. The organic phase was separated, washed with water (50 mL), washed with saturated saline (50 mL), dried with anhydrous sodium sulfate, and filtered. The filtrate was concentrated to dryness, and passed through a column with petroleum ether/ethyl acetate (10/1) to give 1.5 g of a colorless transparent oil, yield: 39.2%. LC-MS(APCI): m/z=355.2 (M+1)$^+$.

Step 2 Synthesis of compound ethyl 3-(4-bromophenyl)-1-(3-((tert-butyldiphenylsilyl)oxy)cyclohexanyl)-4-iodo-1H-pyrazole-5-carboxylate

Anhydrous THF (20 mL), ethyl 3-(4-bromophenyl)-4-iodo-1H-pyrazole-5-carboxylate (1.5 g, 3.57 mmol), 3-((tert-butyldiphenylsilyl)oxy)cyclohexan-1-ol (1.26 g, 3.56 mmol), and $Ph_3P$ (2.8 g, 10.68 mmol) were added to a 50 mL three-necked flask equipped with magnetic stirring. The flask was evacuated and the atmosphere was replaced with nitrogen three times. DIAD (1.44 g, 7.13 mmol) was added dropwise under ice-water bath cooling, stirred and dispersed. The reaction was stirred under ice-water bath for 10 minutes, and slowly warmed to room temperature. The mixture was stirred and reacted overnight while maintaining the temperature. Water (50 mL) was added to quench the reaction. The mixture was extracted with ethyl acetate (50 mL x3). The organic phase was separated, washed with saturated saline (50 mL), dried with anhydrous sodium sulfate, and filtered. The filtrate was concentrated and passed through a silica gel column to obtain 2.0 g of a white solid, yield: 75.7%. LC-MS(APCI): m/z=741.1$(M+1)^+$.

Step 3 Synthesis of compound ethyl 3-(4-bromophenyl)-1-(3-hydroxycyclohexanyl)-4-iodo-1H-pyrazole-5-carboxylate

To a 50 mL single-necked flask equipped with magnetic stirring, ethyl 3-(4-bromophenyl)-1-(3-((tert-butyldiphenylsilyl)oxy)cyclohexanyl)-4-iodo-1H-pyrazole-5-carboxylate (1.5 g, 2.02 mmol) and hydrochloric acid solution in isopropanol (5 mol/L 10 mL) were added, and the reaction was stirred at room temperature for 0.5 hours. The solvent was evaporated under reduced pressure. Saturated aqueous sodium bicarbonate (20 mL) and ethyl acetate (30 mL) were added. The organic phase was separated, and the aqueous phase was extracted with ethyl acetate (20 mLx2). The organic phases were combined, dried with anhydrous sodium sulfate, and filtered. The filtrate was concentrated and passed through a silica gel column to obtain 0.8 g of a white solid, yield: 76.5%. LC-MS(APCI): m/z=519.0$(M+1)^+$.

Step 4 Synthesis of compound ethyl 3-(4-bromophenyl)-4-cyano-1-(3-hydroxycyclohexanyl)-1H-pyrazole-5-carboxylate

Ethyl 3-(4-bromophenyl)-1-(3-hydroxycyclohexanyl)-4-iodo-1H-pyrazole-5-carboxylate (0.64 g, 1.26 mmol) and DMF (8 mL) were charged to a 50 mL single-necked flask equipped with magnetic stirring and a condenser tube, and stirred until dissolved. Cuprous cyanide (0.17 g, 1.94 mmol), $Pd_2(dba)_3$ (0.12 g, 0.13 mmol), and $Pd(dppf)Cl_2$ (0.21 g, 0.13 mmol) were added. The flask was evacuated and the atmosphere was replaced with nitrogen three times. The mixture was heated to 100 °C under a nitrogen atmosphere and the reaction was stirred overnight. At room temperature, water (20 mL) was added to quench the reaction. Ethyl acetate (30 mL × 3) was added for extraction. The organic phase was separated, washed with saturated saline (50 mL), dried with anhydrous sodium sulfate, and filtered. The filtrate was concentrated and passed through a silica gel column to obtain 0.40 g of a white solid, yield: 75.9%. LC-MS(APCI): m/z= 418.0$(M+1)^+$.

Step 5 Synthesis of Intermediate Compound A-13

Ethyl 3-(4-bromophenyl)-4-cyano-1-(3-hydroxycyclohexanyl)-1H-pyrazole-5-carboxylate (0.4 g, 0.95 mmol) and ethanol (20 mL) were charged to a 100 mL single-necked flask equipped with magnetic stirring and a condenser tube, and stirred until dissolved. Hydrazine hydrate (8 mL) was added, and warmed to 80 °C under nitrogen atmosphere. The mixture was stirred and reacted overnight while maintaining the temperature. The reaction was cooled to room temperature. Water (30 mL) was added to quench the reaction. Ethyl acetate (30 mL × 3) was added for extraction. The organic phase was separated, washed with saturated saline (50 mL), dried with anhydrous sodium sulfate, and filtered. The filtrate was concentrated and passed through a silica gel column to obtain 0.22 g of a white solid, yield: 57.5%. LC-MS(APCI): m/z=403.0$(M+1)^+$.

**<u>Preparation of intermediate compound A-14 (cis-4-(4-amino-3-iodo-1H-pyrazolo[3,4-d]pyrimidin-1-yl)cyclopent-2-en-1-yl) acetate</u>**

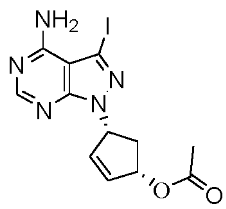
+
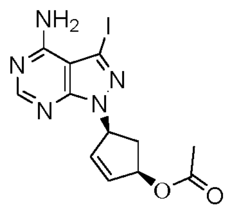
A-14

**<u>The following synthetic route was adopted</u>**

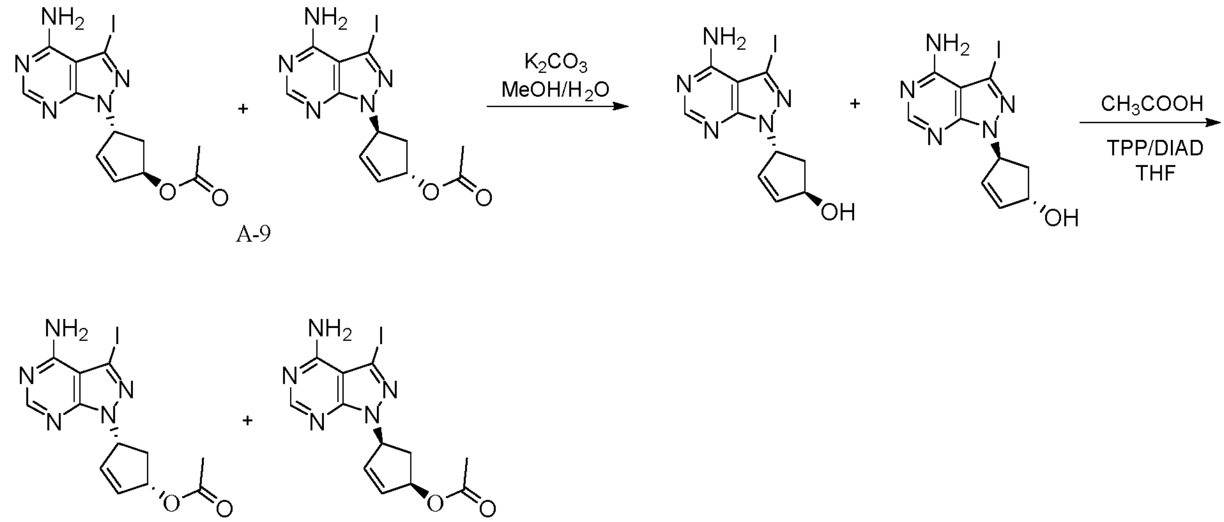


A-9

A-14

Step 1 Synthesis of compound trans-4-(4-amino-3-iodo-1H-pyrazolo[3,4-d]pyrimidin-1 -yl)cyclopent-2-en-1 -ol

Intermediate A-9 (1.5 g, 3.896 mmol), methanol (200 mL) and water (10 mL) were charged to a 100 mL three-necked flask equipped with magnetic stirring, and stirred until dissolved. Potassium carbonate (1.61 g, 11.688 mmol) was added. The flask was evacuated and the atmosphere was replaced with nitrogen three times. The reaction was stirred at 80 °C for 12 hours under nitrogen atmosphere. The reaction was cooled to room temperature. Water (30 mL) was added to quench the reaction. The mixture was extracted with ethyl acetate (30 mL × 2). The organic phases were combined, washed with saturated saline (50 mL), dried with anhydrous sodium sulfate, and filtered. The filtrate was concentrated and passed through a silica gel column to obtain 1.0 g of a white solid, yield: 74.8%. LC-MS(APCI): m/z=344.0$(M+1)^+$.

Step 2 Synthesis of Intermediate A-14

Trans-4-(4-amino-3-iodo-1H-pyrazolo[3,4-d]pyrimidin-1-yl)cyclopent-2-en-1-ol (1.0 g, 2.915 mmol) and THF (20 mL) were added to a 50 mL three-necked bottle equipped with magnetic stirring, and stirred until dissolved. Glacial acetic acid (210.0 mg, 3.498 mmol) and triphenylphosphine (2.291 g, 8.745 mmol) were added. The flask was evacuated and the atmosphere was replaced with nitrogen three times. The mixture was cooled with an ice-water bath, and DIAD (1.178 g, 5.830 mmol) was added dropwise. After the addition was completed, the ice bath was removed, and the reaction was stirred at room temperature under nitrogen atmosphere for 12 hours. Water (50 mL) was added to quench the reaction. The mixture was extracted with ethyl acetate (50 mL × 2). The organic phases were combined, washed with saturated saline (50 mL), dried with anhydrous sodium sulfate, and filtered. The filtrate was concentrated and passed through a silica gel column to obtain of a white solid 400 mg, yield: 35.6%. LC-MS(APCI): m/z=386.0$(M+1)^+$.

**Preparation of Intermediate Compound A-15 5-(4-bromophenyl)-7-(6-(((tert-butyldiphenylsilyl)oxy)methyl)tetrahydro-2H-pyran-3-yl)-5H-pyrrolo[3,2-d]pyrimidin-4-amine**

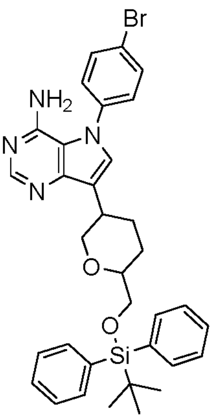

A-15

**The following synthetic route was adopted**

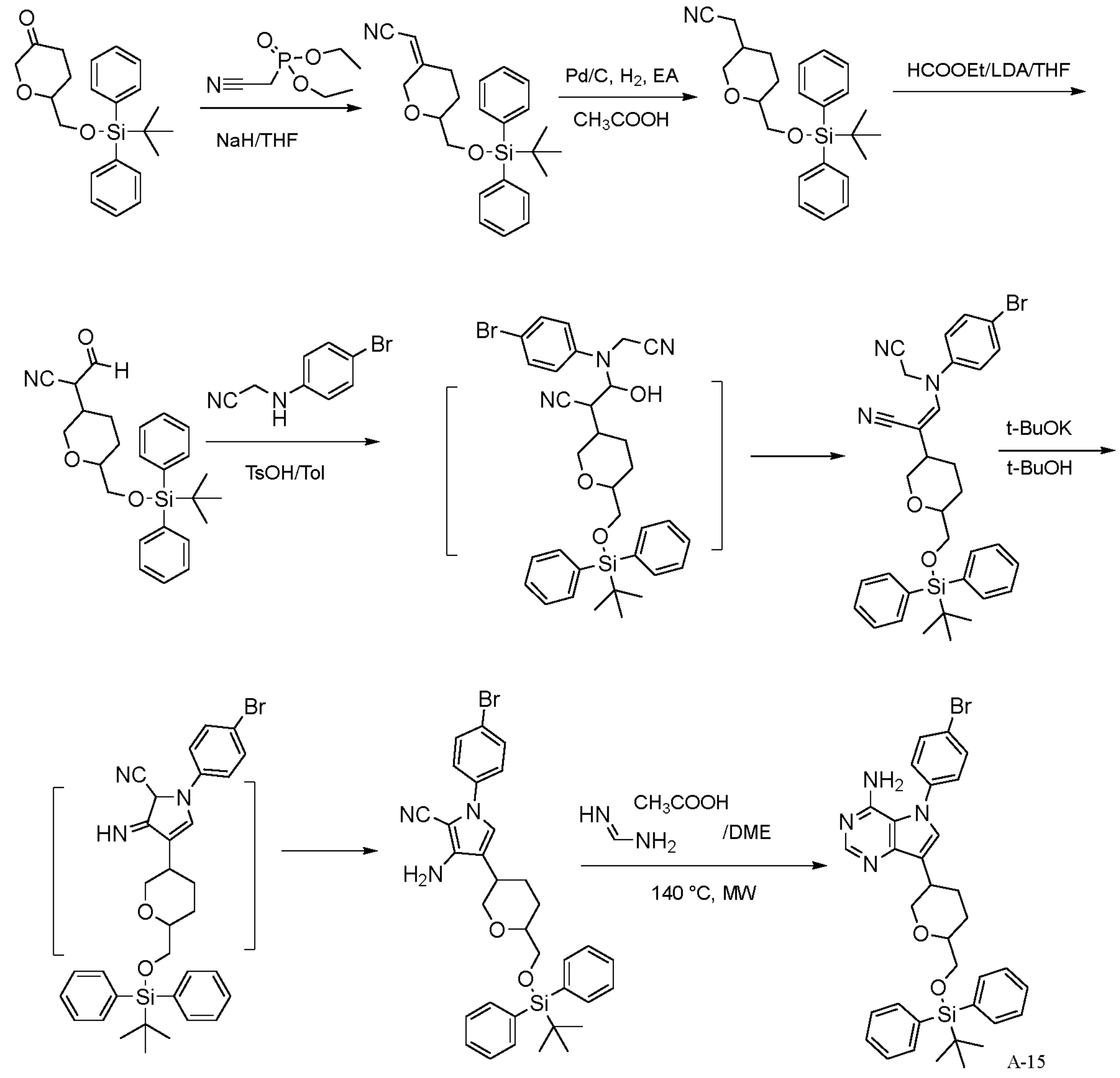


Step 1 Synthesis of Compound (Z)-2-(6-(((tert-butyldiphenylsilyl)oxy)methyl)dihydro-2H-pyran-3 (4H)-ylidene)acetonitrile

Tetrahydrofuran (170 mL) and NaH (2.1 g, 52.5 mmol, 60%) were sequentially added to to a 500 mL three-necked flask equipped with magnetic stirring. The flask was evacuated and the atmosphere was replaced with nitrogen three

times. The mixture was cooled to 0 °C, and a solution of diethyl cyanomethylphosphate (9.3 g, 52.5 mmol) in tetrahydrofuran (20 mL) was added dropwise. After the addition was completed, the mixture was reacted at 0 °C for 30 minutes. A solution of 6-(((tert-butyldiphenylsilyl)oxy)methyl)dihydro-2H-pyran-3(4H)-one (17.0 g, 47.7 mmol) in tetrahydrofuran (20 mL) was added dropwise, and the mixture was reacted at 0 °C for 30 minutes. The mixture was warmed to room temperature, and reacted overnight. Ethyl acetate (200 mL × 3) and water (100 mL) were added for extraction. The organic phase was separated, washed with saturated saline (150 mL), dried with anhydrous sodium sulfate, and filtered. The filtrate was concentrated and passed through a silica gel column to obtain 13.5 g of a yellow oil, yield: 72 %. LC-MS(APCI): m/z=392 $(M+1)^+$.

Step 2 Synthesis of Compound 2-(6-(((tert-butyldiphenylsilyl)oxy)methyl)tetrahydro-2H-pyran-3 -yl)acetonitrile

Ethyl acetate (100 mL), acetic acid (1 mL), (Z)-2-(6-(((tert-butyldiphenylsilyl)oxy)methyl)dihydro-2H-pyran-3(4H)-ylidene)acetonitrile (13.5 g, 34.5 mmol) and Pd/C (1.35 g, 10%) were sequentially added to a 250 mL single-necked flask equipped with magnetic stirring. The flask was evacuated and the atmosphere was replaced with nitrogen three times. The mixture was reacted at room temperature overnight. Pd/C was directly filtered off, and the reaction solution was concentrated. After the concentration, the residue was passed through a silica gel column to obtain 7.5 g of a yellow oil. yield: 56 %. LC-MS(APCI): m/z=394 $(M+1)^+$. $^1$H NMR (500 MHz, DMSO-$D_6$) δ (ppm): 7.63-7.60 (m, 4H), 7.45-7.40 (m, 6H), 3.67-3.61 (m, 2H), 3.58-3.52 (m, 2H), 3.45-3.41 (m, 1H), 2.70-2.62 (m, 2H), 1.95-1.90 (m, 1H), 1.80-1.68 (m, 2H), 1.51-1.43 (m, 2H), 0.98 (s, 9H).

Step 3 Synthesis of Compound 2-(6-(((tert-butyldiphenylsilyl)oxy)methyl)tetrahydro-2H-pyran-3-yl)-3-oxopropionitrile

Tetrahydrofuran (100 mL) and 2-(6-(((tert-butyldiphenylsilyl)oxy)methyl)tetrahydro-2H-pyran-3-yl)acetonitrile (6.2 g, 15.8 mmol) were sequentially added to a 250 mL three-necked flask equipped with magnetic stirring. The atmosphere was replaced with nitrogen three times. The mixture was cooled down to -78 °C, and a solution of LDA in tetrahydrofuran (7.9 mL, 15.8 mmol, 2 mol/L) was added dropwise. After the dropwise addition was completed, the mixture was reacted at -78 °C for 30 minutes. A solution of ethyl formate (2.3 g, 31.6 mmol) in tetrahydrofuran (20 mL) was added dropwise, and reacted at -78 °C for 30 minutes. The mixture was warmed to room temperature, and reacted overnight. Hydrochloric acid (1 mol/L) was added to adjust the pH to 3. Ethyl acetate (150 mL × 3) and water (70 mL) were added for extraction. The organic phase was separated, washed with saturated saline (100 mL), dried with anhydrous sodium sulfate, and filtered. The filtrate was concentrated and passed through a silica gel column to obtain 2.6 g of a yellow oil, yield: 40 %. LC-MS(APCI): m/z=422 $(M+1)^+$.

Step 4 Synthesis of compound (Z)-3-((4-bromophenyl)(cyanomethyl)amino)-2-(6-(((tert-butyldiphenylsilyl)oxy)methyl) tetrahydro-2H-pyran-3-yl)acrylonitrile

Toluene (15 mL), 2-(6-(((tert-butyldiphenylsilyl)oxy)methyl)tetrahydro-2H-pyran-3-yl)-3-oxopropionitrile (359 mg, 0.85 mmol), 2-((4-bromophenyl)amino)acetonitrile (150 mg, 0.85 mmol), and p-toluenesulfonic acid (13.5 mg, 0.085 mmol) were sequentially added to a 50 mL three-necked flask equipped with magnetic stirring. Water separator was equipped and the reaction was refluxed overnight. The pH was adjusted to 7-8 by adding saturated aqueous sodium bicarbonate solution. Ethyl acetate (30 mL × 3) and water (15 mL) were added for extraction. The organic phase was separated, washed with saturated saline (15 mL), dried with anhydrous sodium sulfate, and filtered. The filtrate was concentrated and passed through a silica gel column to obtain crude product 526 mg as a yellow oil. LC-MS(APCI): m/z=614 $(M+1)^+$.

Step 5 Synthesis of compound 3-amino-1-(4-bromophenyl)-4-(6-(((tert-butyldiphenylsilyl)oxy)methyl)tetrahydro-2H-pyran-3-yl)-1H-pyrrole-2-carbonitrile

Tert-butanol (15 mL), (Z)-3-((4-bromophenyl)(cyanomethyl)amino)-2-(6-(((tert-butyldiphenylsilyl)oxy)methyl) tetrahydro-2H-pyran-3-yl)acrylonitrile (523 mg, 0.85 mmol) and a solution of potassium tert-butoxide in tert-butanol (0.9 mL, 0.9 mmol, 1 mol/L) were sequentially charged to a 50 mL three-necked flask equipped with magnetic stirring, warmed to 80 °C and reacted for 30 minutes. The mixture was cooled to room temperature. Hydrochloric acid solution (1 mol/L) was added to adjust the pH to 7. Ethyl acetate (30 mL × 3) and water (15 mL) were added for extraction. The organic phase was separated, washed with saturated saline (15 mL), dried with anhydrous sodium sulfate, and filtered. The filtrate was concentrated and passed through a silica gel column to obtain 50 mg of a crude product as a yellow oil, yield: 9.5 %, LC-MS(APCI): m/z=614 $(M+1)^+$.

Step 6 Synthesis of Intermediate Compound A-15

DME (5 mL), 3-amino-1-(4-bromophenyl)-4-(6-(((tert-butyldiphenylsilyl)oxy)methyl)tetrahydro-2H-pyran-3-yl)-1H-pyrrole-2-carbonitrile (50 mg, 0.081 mmol) and formamidine acetate (42 mg, 0.41 mmol) were added in sequence to a microwave reaction tube (10 mL) equipped with a magnet. The tube was filled up with enough nitrogen. The cover of the microwave reaction tube was clamped. The microwave reaction tube was placed in a microwave reactor at 140 °C, and reacted for 1 hour. The reaction mixture was cooled to room temperature, directly concentrated and passed through a silica gel column to obtain 55 mg of a white solid, yield: 100 %, LC-MS(APCI): m/z=641 $(M+1)^+$.

**Preparation of intermediate compound A-16 6-chloro-N,N-bis(4-methoxybenzyl)-5-nitropyrimidin-4-amine**

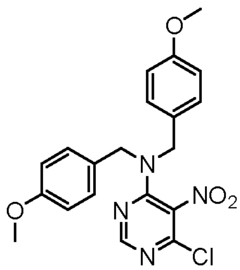

A-16

**The following synthetic route was adopted**

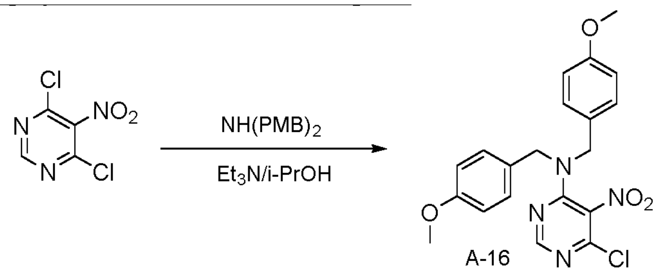


Isopropanol (50 mL), 4,6-dichloro-5-nitropyrimidine (8 g, 41.6 mmol), $NH(PMB)_2$ (10.68 g, 41.6 mmol) and triethylamine (5.44 g, 54 mmol) were sequentially added to a 100 mL single-necked flask equipped with magnetic stirring. The mixture was heated to 95 °C and reacted for 4 hours. The reaction mixture was directly concentrated and passed through a silica gel column to obtain 16.4 g of a yellow oil. yield: 96 %, LC-MS(APCI): m/z=415 $(M+1)^+$.

**Preparation of intermediate compound A-17 6-amino-7-(4-bromophenyl)-9-(3-((tert-butyldiphenylsilyl)oxy)cyclohexyl)-7,9-dihydro-8H-purin-8-one**

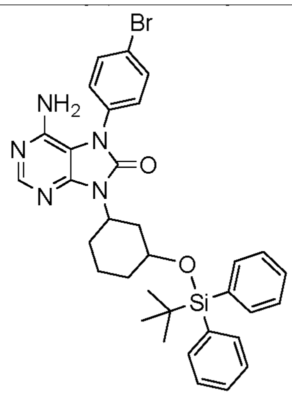

A-17

The following synthetic route was adopted

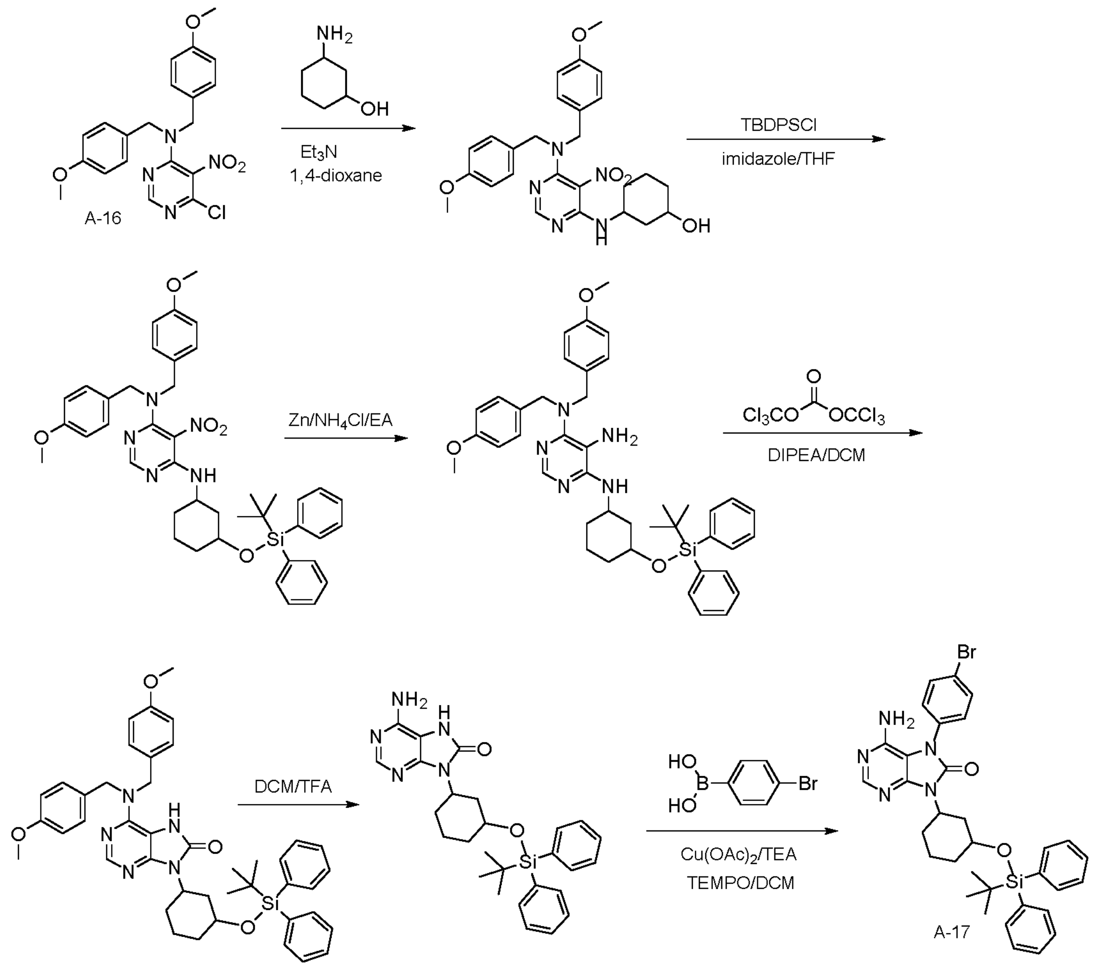


Step 1 Synthesis of compound 3-((6-(bis(4-methoxybenzyl)amino)-5-nitropyrimidin-4-yl)amino)cyclohexan-1-ol

1,4-dioxane (80 mL), intermediate compound A-16 (8.2 g, 19.8 mmol), 3-aminocyclohexanol (2.28 g, 19.8 mmol) and triethylamine (4.0 g, 39.6 mmol) were sequentially added to a 250 mL single-necked flask equipped with magnetic stirring, warmed to 50 °C, and reacted overnight. Water (100 mL) and ethyl acetate (150 mL × 3) were added for extraction. The organic phases were combined, washed with saturated saline (100 mL), dried with anhydrous sodium sulfate, and filtered. The filtrate was concentrated and passed through a silica gel column to obtain 8.88 g of a yellow oil, yield: 91 %, LC-MS(APCI): m/z=494 $(M+1)^+$.

Step 2 Synthesis of compound $N^4$-(3-((tert-butyldiphenylsilyl)oxy)cyclohexyl)-$N^6$,$N^6$-bis(4-methoxybenzyl)-5-nitropyrimidine-4,6-diamine

THF (100 mL), 3-((6-(bis(4-methoxybenzyl)amino)-5-nitropyrimidin-4-yl)amino)cyclohexan-1-ol (7.27 g, 14.7 mmol) and imidazole (1.5 g, 22.1 mmol) were sequentially added to a 250 mL three-necked flask equipped with magnetic stirring, and stirred until dissolved. The solution was cooled to 0 °C, and t-butyldiphenylchlorosilane (4.45 g, 16.2 mmol) was added. The mixture was stirred for 10 min. The reaction was slowly warmed to room temperature and stirred for 2 h. Ethyl acetate (150 mL × 3) and water (75 mL) were added for extraction. The organic phase was separated, washed with saturated saline (100 mL), dried with anhydrous sodium sulfate, and filtered. The filtrate was concentrated and passed through a silica gel column to obtain 10.35 g of a yellow oil, yield: 96 %. LC-MS(APCI): m/z=732 $(M+1)^+$.

Step 3 Synthesis of compound $N^4$-(3-((tert-butyldiphenylsilyl)oxy)cyclohexyl)-$N^6$,$N^6$-bis(4-methoxybenzyl)pyrimidine-4,5,6-triamine

Ethyl acetate (100 mL), $N^4$-(3-((tert-butyldiphenylsilyl)oxy)cyclohexyl)-$N^6$,$N^6$-bis(4-methoxybenzyl)-5-nitropyrimidine-4,6-diamine (10.35 g, 14.1 mmol), zinc powder (9.2 g, 141 mmol), and aqueous ammonium chloride (4.54 g, 84.9 mmol, 3.0 mol/L) were sequentially added to a 250 mL single-necked flask equipped with magnetic stirring, and reacted overnight at room temperature. The reaction mixture was filtered, concentrated and passed through a silica gel column to obtain 8.9 g of a yellow oil, yield: 90 %, LC-MS(APCI): m/z=702 $(M+1)^+$.

Step 4 Synthesis of compound 6-(bis(4-methoxybenzyl)amino)-9-(3-((tert-butyldiphenylsilyl)oxy)cyclohexyl)-7,9-dihydro-8H-purin-8-one

DCM (100 mL), $N^4$-(3-((tert-butyldiphenylsilyl)oxy)cyclohexyl)-$N^6$,$N^6$-bis(4-methoxybenzyl)pyrimidine-4,5,6-triamine (5 g, 7.1 mmol), DIPEA (2.0 g, 15.7 mmol) and triphosgene (2.1 g, 7.1 mmol, 3.0 mol/L) were sequentially added to a 250 mL single-necked flask equipped with magnetic stirring, and reacted at room temperature overnight. Ethyl acetate (100 mL × 3) and water (100 mL) were added for extraction. The organic phase was separated, washed with saturated saline (100 mL), dried with anhydrous sodium sulfate, and filtered. The filtrate was concentrated and passed through a silica gel column to obtain 2.035 g of a yellow oil, yield: 40.1 %. LC-MS(APCI): m/z=728 $(M+1)^+$.

Step 5 Synthesis of compound 6-amino-9-(3-((tert-butyldiphenylsilyl)oxy)cyclohexyl)-7,9-dihydro-8H-purin-8-one

DCM (3 mL), 6-(bis(4-methoxybenzyl)amino)-9-(3-((tert-butyldiphenylsilyl)oxy)cyclohexyl)-7,9-dihydro-8H-purin-8-one (200 mg, 0.27 mmol) and TFA (3 mL) were sequentially charged to a 50 mL single-necked flask equipped with magnetic stirring, warmed to 50 °C, and reacted for 4 hours. The reaction solution was adjusted to a pH of 7 with saturated aqueous sodium bicarbonate solution, and extracted with ethyl acetate (30 mL × 3) and water (15 mL). The organic phase was separated, washed with saturated saline (15 mL), dried with anhydrous sodium sulfate, and filtered. The filtrate was concentrated and passed through a silica gel column to obtain 80 mg of a yellow oil, yield: 59.8 %. LC-MS(APCI): m/z=488 $(M+1)^+$.

Step 6 Synthesis of Intermediate Compound A-17

DCM (15 mL), 6-amino-9-(3-((tert-butyldiphenylsilyl)oxy)cyclohexyl)-7,9-dihydro-8H-purin-8-one (80 mg, 0.16 mmol), 4-bromophenylboronic acid (43 mg, 0.21 mmol), copper acetate (15 mg, 0.08 mmol), TEMPO (28 mg, 0.176 mmol) and TEA (66 mg, 0.64 mmol) were sequentially added to a 50 mL single-necked flask equipped with magnetic stirring. The flask was evacuated and the atmosphere was replaced with oxygen three times. The mixture was reacted overnight at room temperature. The reaction mixture was directly concentrated and passed through a silica gel column to obtain 15 mg of a yellow oil, yield: 15 %, LC-MS(APCI): m/z=642 $(M+1)^+$.

**Preparation of intermediate compound A-18 compound ethyl (1R,4R)-4-(6-(bis(4-methoxybenzyl)amino)-8-oxo-7,8-dihydro-9H-purin-9-yl)cyclohexane-1-carboxylate**

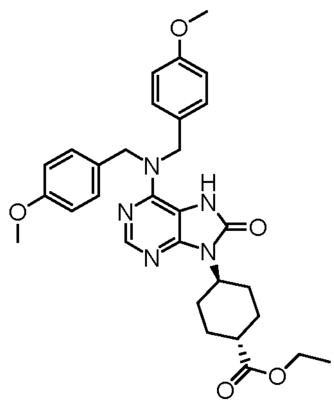

A-18

**The following synthetic route was adopted**

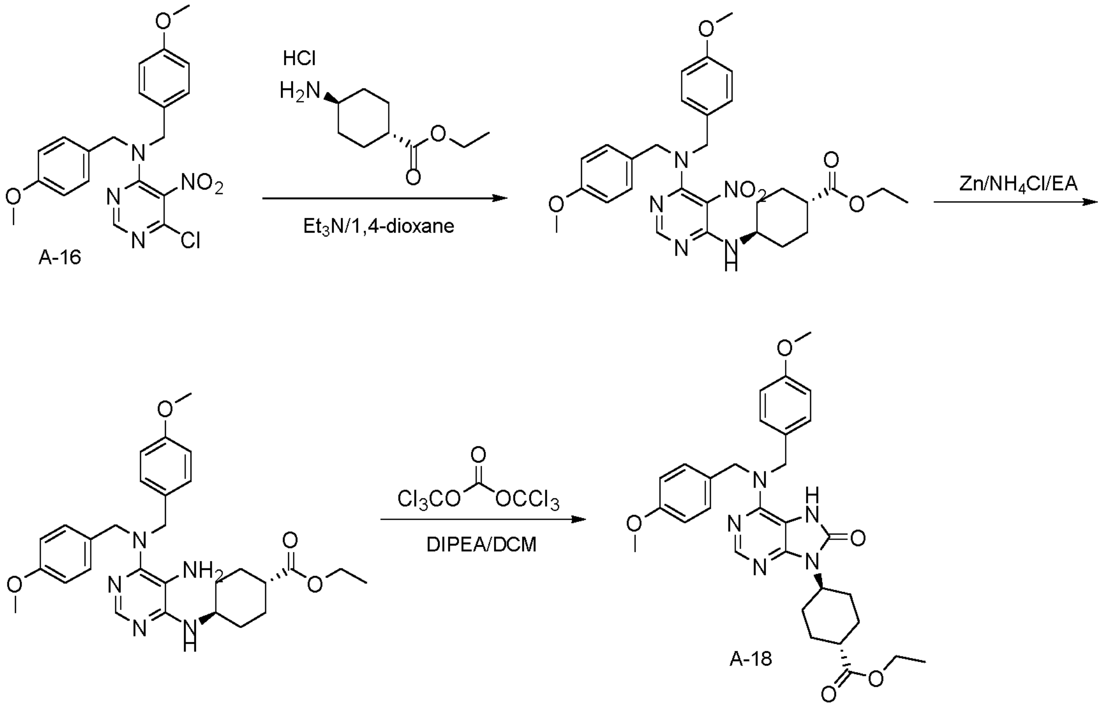


A-16

A-18

Step 1 Synthesis of compound ethyl (1R,4R)-4-((6-(bis(4-methoxybenzyl)amino)-5-nitropyrimidin-4-yl)amino)cyclohexane-1-carboxylate

1,4-dioxane (100 mL), intermediate compound A-16 (8.2 g, 19.8 mmol), ethyl (1R,4R)-4-aminocyclohexane-1-carboxylate hydrochloride (2.38 g, 19.8 mmol) and triethylamine (6.0 g, 59.4 mmol) were sequentially added to a 250 mL single-necked flask equipped with magnetic stirring, warmed to 50 °C, and reacted overnight. Water (100 mL) and ethyl acetate (150 mL × 3) were added for extraction. The organic phases were combined, washed with saturated saline (100 mL), dried with anhydrous sodium sulfate, and filtered. The filtrate was concentrated and passed through a silica gel column to obtain 10.67 g of a yellow oil, yield: 98 %, LC-MS(APCI): m/z=550 $(M+1)^+$.

Step 2 Synthesis of compound ethyl (1R,4R)-4-((5-amino-6-(bis(4-methoxybenzyl)amino)pyrimidin-4-yl)amino)cyclohexane-1-carboxylate

Ethyl acetate (100 mL), ethyl (1R,4R)-4-((6-(bis(4-methoxybenzyl)amino)-5-nitropyrimidin-4-yl)amino)cyclohexane-1-carboxylate (10.67 g, 19.4 mmol), zinc powder (12.7 g, 194 mmol), and an aqueous solution of ammonium chloride (6.3 g, 117 mmol, 3.0 mol/L) were sequentially charged to a 250 mL single-necked flask equipped with magnetic stirring, and reacted overnight at room temperature. The reaction mixture was filtered, concentrated and passed through a silica gel column to obtain 8.7 g of a yellow oil, yield: 86 %, LC-MS(APCI): m/z=520 $(M+1)^+$.

Step 3 Synthesis of Intermediate Compound A-18

DCM (100 mL), ethyl (1R,4R)-4-((5-amino-6-(bis(4-methoxybenzyl)amino)pyrimidin-4-yl)amino)cyclohexane-1-carboxylate (8.7 g, 16.7 mmol), DIPEA (4.9 g, 36.9 mmol) and triphosgene (4.97 g, 16.7 mmol, 3.0 mol/L) were sequentially charged to a 250 mL single-necked flask equipped with magnetic stirring, and reacted overnight at room temperature. Ethyl acetate (100 mL × 3) and water (100 mL) were added for extraction. The organic phase was separated, washed with saturated saline (100 mL), dried with anhydrous sodium sulfate, and filtered. The filtrate was concentrated and passed through a silica gel column to obtain 7.7 g of a yellow oil, yield: 84 %. LC-MS(APCI): m/z=546 $(M+1)^+$.

**Preparation of intermediate compound A-19 compound 1-(3-((tertbutyldimethylsilyl)oxy)cycloheptyl)-3-iodo-1H-pyrazolo[3,4-d]pyrimidin-4-amine**

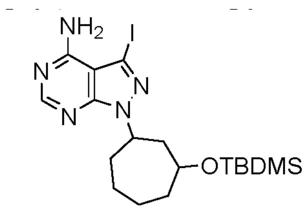

A-19

**The following synthetic route was adopted**

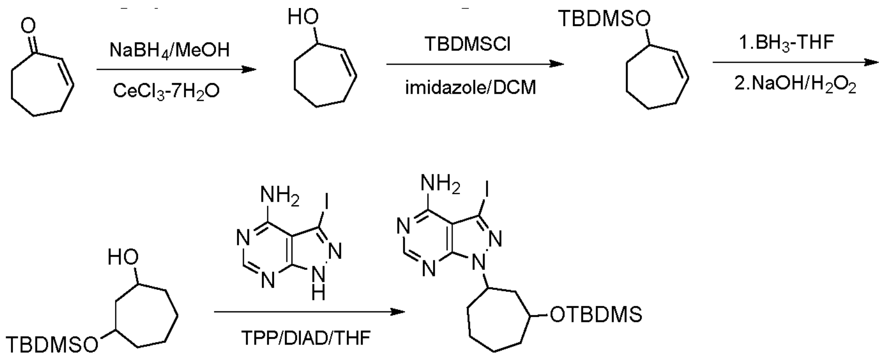


A-19

Step 1 Synthesis of compound 2-cyclohepten-1-ol

2-cyclohepten-1-one (2.0 g, 18.16 mmol) and MeOH (20 mL) were sequentially added to a 50 mL three-necked flask equipped with magnetic stirring, and stirred until dissolved. $CeCl_3$-$7H_2O$ (7.44 g, 19.98 mmol) was added. The mixture was stirred evenly at 0 °C. $NaBH_4$ (755.7 mg, 19.98 mmol) was added in batches, and stirred at room temperature overnight. Saturated ammonium chloride (20 mL) was added to quench the reaction. The organic solvent was removed by rotary evaporation under reduced pressure. Water (20 mL) was added and the mixture was extracted with ethyl acetate (20 mL x2). The organic phases were combined, washed with saturated saline (20 mL), dried with anhydrous sodium sulfate, and filtered. The filtrate was concentrated and passed through a silica gel column to obtain 1.02 g of a colorless oily liquid, yield: 50.1 %. LC-MS(APCI): m/z=113.0 $(M+1)^+$. $^1$H NMR (400MHz, $CDCl_3$) $\delta$ (ppm):5.80-5.72 (m, 2H), 4.42-4.40 (m, 1H), 2.23-2.16 (m, 1H), 2.08-2.00 (m, 1H), 1.96-1.91 (m, 1H), 1.72-1.53 (m, 4H), 1.42-1.32 (m, 1H).

Step 2 Synthesis of compound tert-butyl(cyclohept-2-en-1-yloxy)dimethylsilane

2-cyclohepten-1-ol (1.0 g, 8.90 mmol) and DCM (10 mL) were sequentially added to a 50 mL three-necked flask equipped with magnetic stirring, and stirred until dissolved. Imidazole (908.9 mg, 13.35 mmol) and tert-butyldimethylchlorosilane (1.48 g, 9.79 mmol) were added, and stirred at room temperature for 2 hours. The reaction was quenched by saturated saline (10 mL), and dichloromethane (10 mL × 2) was added for extraction. The organic phases were combined, washed with saturated saline (10 mL), dried with anhydrous sodium sulfate, and filtered. The filtrate was concentrated and passed through a silica gel column to obtain 1.7 g of a colorless oily liquid, yield: 84.5 %. LC-MS(APCI): m/z=227.0 $(M+1)^+$.

Step 3 Synthesis of Compound 3-((tert-butyldimethylsilyl)oxy)cycloheptan-1-ol

Tert-butyl(cyclohept-2-en-1-yloxy)dimethylsilane (1.65 g, 7.3 mmol) and $BH_3$-THF (1 M, 14.6 mL, 14.6 mmol) were sequentially added to a 50 mL three-necked flask equipped with magnetic stirring, and stirred at room temperature overnight. Aqueous NaOH (3 M, 14.6 mL, 43.8 mmol) and aqueous $H_2O_2$ (6.6 mL, 58.4 mmol) were added, and stirred at 55 °C for 1 hour. The reaction was cooled to room temperature. Saturated saline (20 mL) was added to quench the reaction. Dichloromethane (20 mL × 2) was added for extraction. The organic phases were combined, washed with saturated saline (20 mL), dried with anhydrous sodium sulfate, and filtered. The filtrate was concentrated and passed through a silica gel column to obtain 1.0 g of a colorless oily liquid, yield: 56.1 %. LC-MS(APCI): m/z=245.0 $(M+1)^+$.

Step 4 Synthesis of Intermediate Compound A-19

3-((Tert-butyldimethylsilyl)oxy)cycloheptan-1-ol (1.0 g, 4.1 mmol), 4-amino-3-iodo-1H-pyrrolo[3,4, b]pyrimidine

(1.07 g, 4.1 mmol), TPP (2.15 g, 8.2 mmol) and THF (15 mL) were sequentially added to a 50 mL three-necked flask equipped with magnetic stirring, and stirred evenly at 0 °C. DIAD (1.44 mL, 8.2 mmol) was slowly added dropwise, and stirred at room temperature for 6 hours. Saturated saline (20 mL) was added to quench the reaction. Ethyl acetate (20 mL × 2) was added for extraction. The organic phases were combined, washed with saturated saline (20 mL), dried with anhydrous sodium sulfate, and filtered. The filtrate was concentrated and passed through a silica gel column to obtain 480 mg of a white solid, yield: 24.0 %. LC-MS(APCI): m/z=488.0 $(M+1)^+$.

**Preparation of intermediate compound A-20 compound 5-bromo-7-(6-(((tert-butyldiphenylsilyl)oxy)methyl)tetrahydro-2H-pyran-3-yl)pyrrolo[2,1-f][1,2,4]triazin-4-**amine

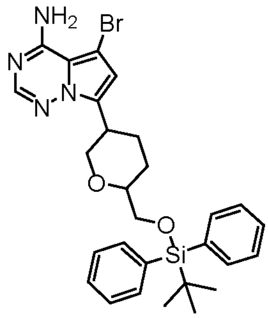

A-20

**The following synthetic route was adopted**

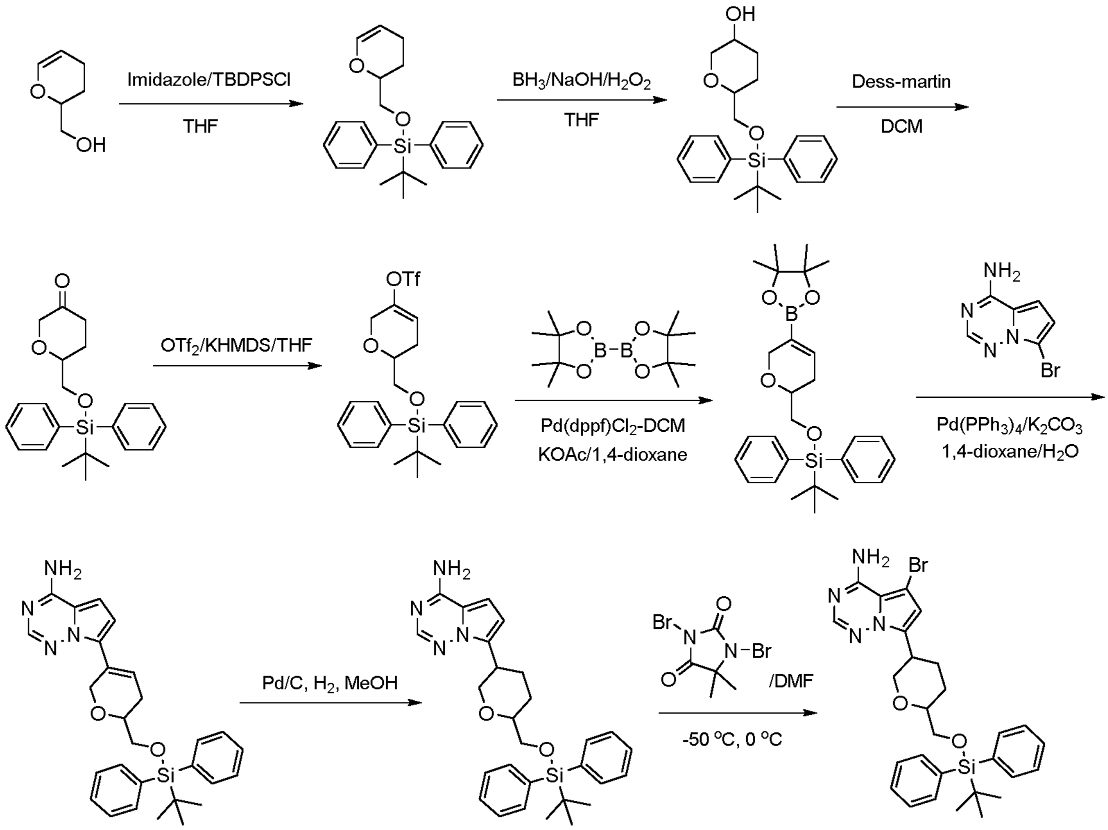


Step 1 Synthesis of compound tert-butyl ((3,4-dihydro-2H-pyran-2-yl)methoxy) diphenylsilane

THF (200 mL), (3,4-dihydro-2H-pyran-2-yl)methanol (20 g, 17.5 mmol) and imidazole (17.9 g, 26.3 mmol) were sequentially added to a 500 mL three-necked flask equipped with magnetic stirring, and stirred until dissolved. The solution was cooled to 0 °C, and tert-butyldiphenylchlorosilane (53.0 g, 19.3 mmol) was added. The mixture was stirred for 10 min,

and slowly warmed to room temperature. The reaction was stirred while maintaining the temperature for 2 hours. Ethyl acetate (200 mL × 3) and water (100 mL) were added for extraction. The organic phase was separated, washed with saturated saline (150 mL), dried with anhydrous sodium sulfate, and filtered. The filtrate was concentrated and passed through a silica gel column to obtain 60 g of a yellow oil, yield: 97 %. LC-MS(APCI): m/z=353 $(M+1)^+$.

Step 2 Synthesis of compound 6-(((tert-butyldiphenylsilyl)oxy)methyl)tetrahydro-2H-pyran-3-ol

THF (200 mL) and tert-butyl((3,4-dihydro-2H-pyran-2-yl)methoxy)diphenylsilane (60 g, 170 mmol) were sequentially added to a 1000 mL three-necked flask equipped with magnetic stirring, and nitrogen was discharged three times. The mixture was cooled to 0 °C, and a solution of $BH_3$ in tetrahydrofuran (255 mL, 255 mmol, 1.0 mol/L) was added dropwise. After the completion of addition, the mixture was reacted overnight at room temperature. The temperature was lowered to 0 °C, and a mixed solution of aqueous sodium hydroxide solution (151 mL, 302 mmol, 2 mol/L) and hydrogen peroxide solution (42 mL, 414 mmol, 30%) was added dropwise. After the dropwise addition was completed, the mixture was stirred at room temperature for 0.5 hours. The mixture was heated to 55 °C, and the reaction was performed for 1 hour. The mixture was cooled to room temperature. The reaction was quenched by adding saturated aqueous sodium bicarbonate solution (300 mL), and extracted by adding water (300 mL) and ethyl acetate (300 mL × 3). The organic phase was separated, washed with saturated saline (600 mL), dried with anhydrous sodium sulfate, and filtered. The filtrate was concentrated and passed through a silica gel column to obtain 45 g of a yellow oil, yield: 71.5 %. LC-MS(APCI): m/z=371 $(M+1)^+$.

Step 3 Synthesis of Compound 6-(((tert-butyldiphenylsilyl)oxy)methyl)dihydro-2H-pyran-3(4H)-one

To a 500 mL three-necked flask equipped with magnetic stirring were sequentially charged DCM (200 mL) and 6-(((tert-butyldiphenylsilyl)oxy)methyl)tetrahydro-2H-pyran-3-ol (45 g, 122 mmol), and stirred until dissolved. The atmosphere was replaced with nitrogen three times. The mixture was cooled to 0 °C. Dess-martin oxidant (56.7 g, 133 mmol) was added in batches, stirred for 10 min, slowly warmed to room temperature and stirred while maintaining the temperature for 1 hour. DCM (200 mL × 3) and water (100 mL) were added for extraction. The organic phase was separated, washed with saturated saline (150 mL), dried with anhydrous sodium sulfate, and filtered. The filtrate was concentrated and passed through a silica gel column to obtain 24.6 g of a yellow oil, yield: 55 %. LC-MS(APCI): m/z=369 $(M+1)^+$.

Step 4 Synthesis of compound 6-(((tert-butyldiphenylsilyl)oxy)methyl)-5,6-dihydro-2H-pyran-3 -yl trifluoromethanesulfonate

THF (70 mL), 6-(((tert-butyldiphenylsilyl)oxy)methyl)dihydro-2H-pyran-3(4H)-one (5.8 g, 16.3 mmol), and $OTf_2$ (7.22 g, 18.4 mmol) were sequentially added to a 100 mL three-necked flask equipped with magnetic stirring. The atmosphere was replaced with nitrogen three times. The mixture was cooled down to -78 °C. KHMDS (17.9 mL, 17.9 mmol, 1 mol/L) was added dropwise. After the dropwise addition was completed, the mixture was reacted while maintaining the temperature for 1 hour. Saturated aqueous ammonium chloride solution (126 mL) was added to quench the reaction. The mixture was extracted by adding ethyl acetate (100 mL × 3). The organic phase was separated, washed with saturated saline (150 mL), dried with anhydrous sodium sulfate, and filtered. The filtrate was concentrated and passed through a silica gel column to obtain 3.7 g of a yellow oil, yield: 45 %. LC-MS(APCI): m/z=501 $(M+1)^+$.

Step 5 Synthesis of compound tert-butyldiphenyl((5-(4,4,5,5-tetramethyl-1,3,2-dioxaborolan-2-yl)-3,6-dihydro-2H-pyran-2-yl)methoxy)silane

1,4-dioxane (50 mL), 6-(((tert-butyldiphenylsilyl)oxy)methyl)-5,6-dihydro-2H-pyran-3-yl trifluoromethanesulfonate (3.7 g, 7.4 mmol), bis(pinacolato)diborane (2.25 g, 8.8 mmol), $Pd(dppf)Cl_2 \cdot DCM$ (0.6 g, 0.74 mmol) and potassium acetate (2.17 g, 22.2 mmol) were sequentially added to a 100 mL three-necked flask equipped with magnetic stirring. The atmosphere was replaced with nitrogen three times. The mixture was heated to 95 °C and reacted while maintaining the temperature overnight. Ethyl acetate (100 mL × 3) and water (50 mL) were added for extraction. The organic phase was separated, washed with saturated saline (50 mL), dried with anhydrous sodium sulfate, and filtered. The filtrate was concentrated and passed through a silica gel column to obtain 2.2 g of a yellow oil, yield: 63 %. LC-MS(APCI): m/z=479 $(M+1)^+$. $^1H$ NMR (500 MHz, DMSO-$D_6$) δ (ppm): 7.63-7.60 (m, 4H), 7.48-7.40 (m, 6H), 6.09 (s, 1H), 4.20 (s, 2H), 3.71-3.67 (m, 3H), 2.26-2.24 (m, 2H), 0.98 (s, 9H).

Step 6 Synthesis of compound 7-(6-(((tert-butyldiphenylsilyl)oxy)methyl)-5,6-dihydro-2H-pyran-3-yl)pyrrolo[2,1-f][1,2,4]triazin-4-amine

1,4-dioxane (30 mL), water (7.5 mL), tert-butyldiphenyl((5-(4,4,5,5-tetramethyl-1,3,2-dioxaborolan-2-yl)-3,6-dihydro-2H-pyran-2-yl)methoxy)silane (2.2 g, 4.6 mmol), 4-amino-7-bromopyrrolo[2,1-F][1,2,4]triazine (0.817 g, 3.8 mmol), tetratriphenylphosphine palladium (0.22 g, 0.19 mmol), and potassium carbonate (1.57 g, 11.4 mmol) were sequentially added to a 100 mL three-necked flask equipped with magnetic stirring. The atmosphere was replaced with nitrogen three times. The mixture was heated to 100 °C, and reacted for 4 hours. Ethyl acetate (80 mL x 3) and water (50 mL) were added for extraction. The organic phase was separated, washed with saturated saline (50 mL), dried with anhydrous sodium sulfate, and filtered. The filtrate was concentrated and passed through a silica gel column to obtain 1.47 g of a yellow oil, yield: 80 %. LC-MS(APCI): m/z=485 $(M+1)^+$.

Step 7 Synthesis of compound 7-(6-(((tert-butyldiphenylsilyl)oxy)methyl)tetrahydro-2H-pyran-3-yl)pyrrolo[2,1-f][1,2,4]triazin-4-amine

Methanol (10 mL), 7-(6-(((tert-butyldiphenylsilyl)oxy)methyl)-5,6-dihydro-2H-pyran-3-yl)pyrrolo[2,1-f][1,2,4]triazin-4-amine (1 g, 2.0 mmol) and Pd/C (500 mg, 10%) were sequentially added to a 50 mL single-necked flask equipped with magnetic stirring. The flask was evacuated and the atmosphere was replaced with nitrogen three times. The mixture was reacted overnight at room temperature. Pd/C was directly filtered out. The reaction solution was concentrated to obtain 0.8 g of a crude product, yield: 80 %. LC-MS(APCI): m/z=487 $(M+1)^+$.

Step 8 Synthesis of Intermediate Compound A-20

DMF (10 mL) and 7-(6-(((tert-butyldiphenylsilyl)oxy)methyl)tetrahydro-2H-pyran-3-yl)pyrrolo[2,1-f][1,2,4]triazin-4-amine (0.4 g, 0.8 mmol) were sequentially added to a 50 mL three-necked flask equipped with magnetic stirring. The flask was evacuated and the atmosphere was replaced with nitrogen three times. The mixture was cooled down to -50 °C. Dibromohydantoin (0.26 g, 0.9 mmol) was added, slowly warmed to 0 °C and stirred while maintaining the temperature for 1 hour. Ethyl acetate (50 mL × 3) and water (15 mL) were added for extraction. The organic phase was separated, washed with saturated saline (15 mL), dried with anhydrous sodium sulfate, and filtered. The filtrate was concentrated and passed through a silica gel column to obtain 0.4 g of a yellow oil, yield: 87 %. LC-MS(APCI): m/z=565 $(M+1)^+$.

**Preparation of Intermediate Compound B-1 (4-((5-fluoro-2-methoxybenzoylamino)methyl)phenyl)boronic acid**

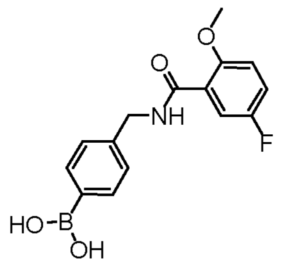

B-1

**The following synthetic route was adopted**

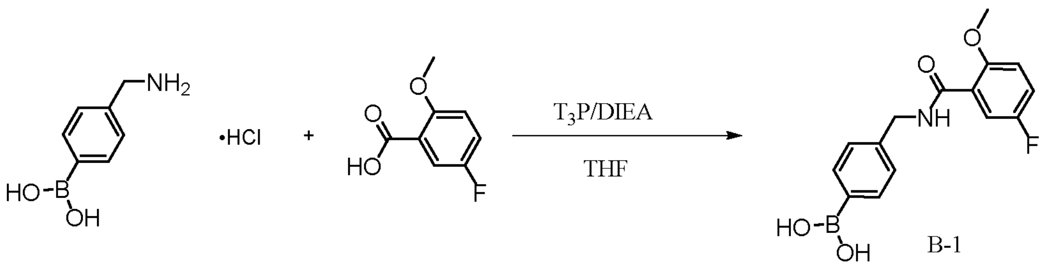


B-1

Tetrahydrofuran (120 mL), (4-(aminomethyl)phenyl)boronic acid hydrochloride (12 g, 64.1 mmol) and 5-fluoro-2-methoxybenzoic acid (10.9 g, 64.1 mmol) were sequentially charged to a 500 mL single-necked flask equipped with

magnetic stirring and a condenser tube, and stirred until dissolved. A solution of $T_3P$ in ethyl acetate (1.7 mol/L) (75.5 mL, 128.3 mmol) and DIEA (33 g, 256 mmol) were added with stirring. The flask was evacuated and the atmosphere was replaced with nitrogen three times. Temperature was raised to 70 °C. The mixture was stirred and reacted overnight while maintaining the temperature. The reaction was cooled to room temperature, and concentrated under reduced pressure. Water (120 mL) and dichloromethane (200 mL) were added. The organic phase was separated, and the aqueous phase was extracted with dichloromethane (200 mLx2). The organic phases were combined, dried with anhydrous sodium sulfate, and filtered. The filtrate was concentrated to dryness, and dichloromethane (100 mL) was added. The mixture was stirrd for 60 minutes, and filtered. The filter cake was washed with a small amount of dichloromethane, and the solid was dried by suction with a vacuum pump to obtain 17.6 g of a white solid, yield: 90.7%.

**Preparation of intermediate compound B-2 potassium (5-fluoro-2-methoxybenzoylamino)methyl)trifluoroborate**

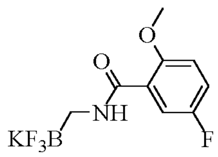

B-2

**The following synthetic route was adopted**

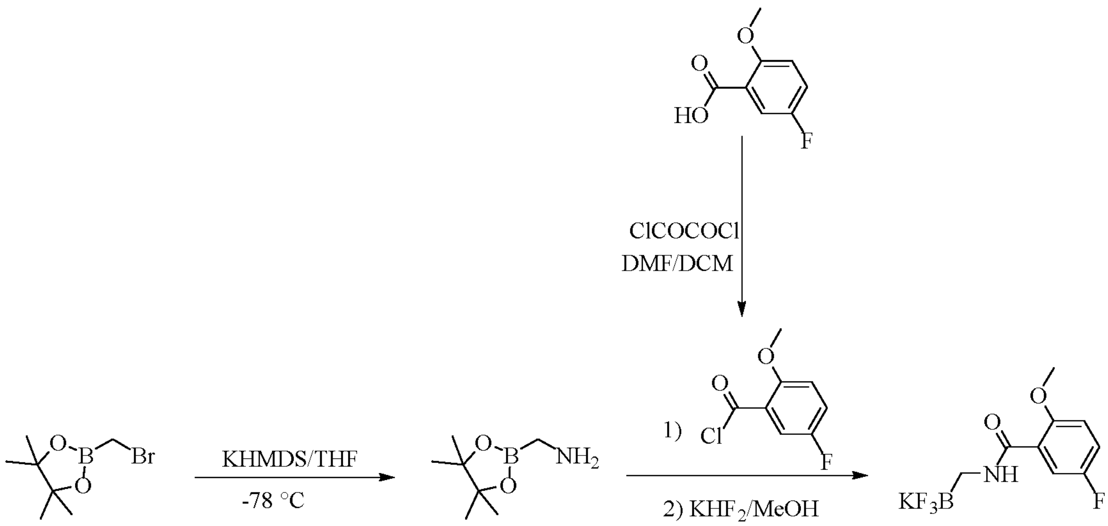


B-2

Step 1 Synthesis of compound 5-fluoro-2-methoxybenzoyl chloride

5-Fluoro-2-methoxybenzoic acid (2.6 g, 15 mmol) and anhydrous dichloromethane (30 mL) were added to a 100 mL three-necked flask equipped with magnetic stirring, and stirred until dissolved. The solution was cooled in an ice-water bath, and oxalyl chloride (3.8 g, 30 mmol) and anhydrous DMF (110 mgm, 1.5 mmol) were added dropwise under nitrogen atmosphere. After the addition was completed, the ice bath was removed, and the reaction was stirred at room temperature overnight. The solvent and uncompletely reacted oxalyl chloride were evaporated under reduced pressure, which was used later.

Step 3 Synthesis of Intermediate Compound B-2

2-(Bromomethyl)-4,4,5,5-tetramethyl-1,3,2-dioxaborolane (3.63 g, 16.5 mmol) and anhydrous THF (30 mL) were added to a 100 mL three-necked flask equipped with magnetic stirring, and cooled to -78 °C under nitrogen atmosphere. KHMDS (16.5 mL, 1M THF solution) was added dropwise, stirred for 30 minutes while maintaining the temperature, warmed to room temperature and stirred for 30 minutes. Anhydrous methanol (2.11 g, 66 mmol) was added. The mixture was stirred at room temperature for 1 hour. Insoluble solid was filtered off. The filtrate solvent was evaporated

at no more than 30 °C. Anhydrous THF (20 mL) was added and then the filtrate solvent was evaporated. The process was repeated twice. The residue was then dissolved in anhydrous THF (20 mL). The above 5-fluoro-2-methoxybenzoyl chloride solution in THF (20 mL) was slowly added dropwise. After the addition was completed, the reaction was stirred at room temperature overnight under a nitrogen atmosphere. The solvent was evaporated off, and the residue was dissolved in methanol (30 mL). An aqueous solution of potassium hydrogen fluoride (6.4 g, 82.5 mmol, 20 mL) was added, and the reaction was stirred at room temperature overnight. The solvent was evaporated under reduced pressure. Toluene (50 mL) was added and the solvent was distilled off. The process was repeated twice. The residue was washed with MTBE, and filtered. The filter cake was washed with hot methanol/acetone (1/3, 100 mL) solution, and the filtrate was concentrated to dryness. The residue was slurried with MTBE. The precipitated solid was filtered, and dried to obtain 3.5 g of a white solid, yield: 80.7%. $^1$H NMR (400MHz, DMSO-$D_6$)δ(ppm): 7.75(br s, 1H), 7.65-7.62(m, 1H), 7.30-7.25(m, 1H), 7.17-7.14(m, 1H), 3.87(s, 3H), 2.13-2.10(m, 2H).

**Example 1 Preparation of compound 5-amino-3-(4-((5-fluoro-2-methoxybenzoylamino)methyl)phenyl)-1-(3-hydroxycyclohexyl)-1H-pyrazole-4-carboxamide**

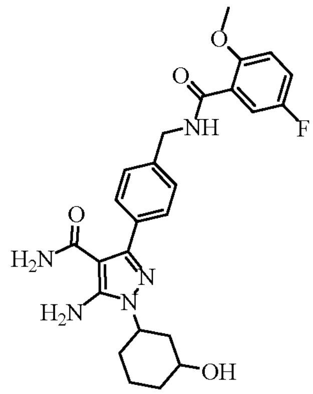

The following synthetic route was adopted

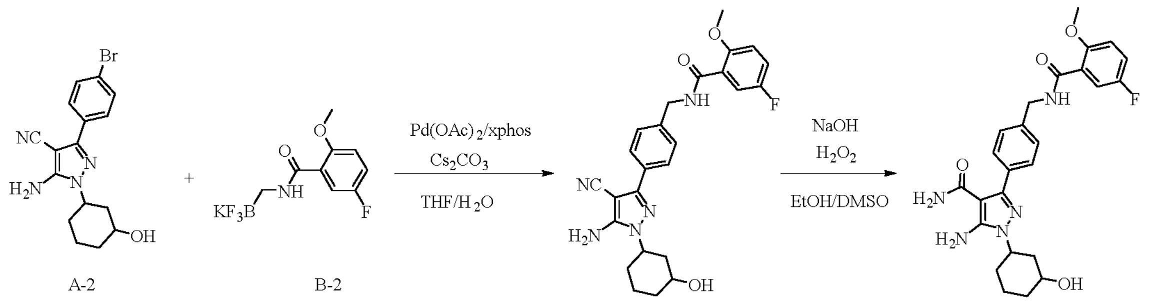


Step 1 Synthesis of compound N-(4-(5-amino-4-cyano-1-(3-hydroxycyclohexanyl)-1H-pyrazol-3-yl)benzyl)-5-fluoro-2-methoxybenzamide

Intermediate Compound A-2 (150 mg, 0.414 mmol), Intermediate Compound B-2 (144 mg, 0.497 mmol), $Cs_2CO_3$ (325 mg, 1.242 mmol), Xphos (20 mg, 0.04 mmol), Pd $(OAc)_2$ (5 mg, 0.02 mmol), THF (10 mL) and water (1 mL) were added to a microwave tube. The atmosphere was replaced with nitrogen for 2 minutes, and the reaction was allowed to react under microwave at 100 °C for 1.5 hours. The reaction was cooled to room temperature. Saturated saline (5 mL) and ethyl acetate (20 mL) were added. The organic phase was separated, and the aqueous phase was extracted with ethyl acetate (20 mLx2). The organic phases were combined, dried with anhydrous sodium sulfate, and filtered. The filtrate was concentrated and passed through a silica gel column to obtain 0.16g of a white solid, yield: 83.3%. LC-MS(APCI): m/z=465.2$(M+1)^+$. $^1$H NMR (400 MHz, $CDCl_3$)δ(ppm): 8.26-8.24(m, 1H), 7.98-7.95(dd, J=9.2 Hz, J=3.2 Hz, 1H), 7.88(d, J=8.0 Hz, 2H), 7.41(d, J=8.0 Hz, 2H), 7.18-7.13(m, 1H), 6.95-6.92(m, 1H), 4.71(d, J=5.6 Hz, 2H), 4.32(br s, 2H), 3.99-3.96(m, 1H), 3.93(s, 3H), 3.83-3.80(m, 1H), 2.28-2.25(m, 1H), 2.13-2.05(m, 1H), 1.99-1.95(m, 2H), 1.91-1.87(m, 2H), 1.43-1.36(m, 2H).

Step 2 Synthesis of compound 5-amino-3-(4-((5-fluoro-2-methoxybenzamido)methyl)phenyl)-1-(3-hydroxycyclohexyl)-1H-pyrazole-4-carboxamide

N-(4-(5-amino-4-cyano-1-(3-hydroxycyclohexanyl)-1H-pyrazol-3-yl)benzyl)-5-fluoro-2-methoxybenzamide (250 mg, 0.54 mmol), EtOH (20 mL) and DMSO (4 mL) were sequentially added to a 50 mL single-mouth bottle equipped with magnetic stirring, and stirred until dissolved. NaOH (86 mg, 2.16 mmol) was added under an ice bath. After the addition was completed, $H_2O_2$ (10 mL) was slowly added dropwise, and the reaction was stirred at room temperature overnight. 30 mL of water was added for dilution, and the mixture was extracted with dichloromethane (30 mLx3). The organic phases were combined, washed with saturated saline (50 mL), dried with anhydrous sodium sulfate, and filtered. The filtrate was concentrated and passed through a silica gel column to obtain 165mg of a white solid, yield: 63.5%, LC-MS(APCI): m/z=482.2(M+1)$^+$. $^1$H NMR (500 MHz, $CDCl_3$)δ(ppm): 8.30-8.28(m, 1H), 7.98-7.95(dd, J=9.5 Hz, J=3.5 Hz, 1H), 7.54(d, J=8.0 Hz, 2H), 7.44(d, J=8.0 Hz, 2H), 7.19-7.15(m, 1H), 6.96-6.94(m, 1H), 5.45(br s, 2H), 4.71(d, J=6.5 Hz, 2H), 4.05-4.02(m, 1H), 3.93(s, 3H), 3.83-3.80(m, 1H), 2.28-2.25(m, 1H), 2.13-2.05(m, 1H), 1.99-1.88(m, 3H), 1.52-1.40(m, 3H).

**Example 2 Preparation of compound 5-amino-3-(4-((5-fluoro-2-methoxybenzoylamino)methyl)phenyl)-1-(3-hydroxycyclohexyl)-1H-pyrazole-4-carboxamide (Isomer 1)**

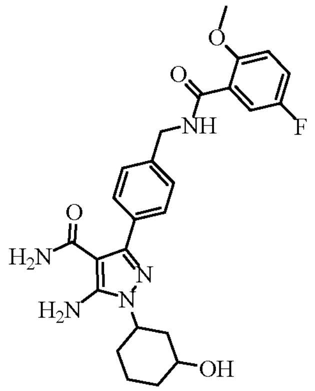

Compound 5-amino-3-(4-((5-fluoro-2-methoxybenzamido)methyl)phenyl)-1-(3-hydroxycyclohexyl)-1H-pyrazole-4-carboxamide (150 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325; 4.6mm*250mmL *5μm; batch No. ICOOCE-VL054; Part Number 83325;
Flow rate: 4 mL/min;

Mobile phase: methanol: n-hexane: ethanol =10: 75: 15.

The corresponding fractions were collected, and rotary evaporated to dryness to obtain 62 mg of the target compound. The corresponding retention time was 12.797 min. LC-MS(APCI): m/z=482.2(M+1)$^+$. $^1$H NMR (500 MHz, $CDCl_3$)δ(ppm): 8.30-8.28(m, 1H), 7.98-7.95(dd, J=9.5 Hz, J=3.5 Hz, 1H), 7.54(d, J=8.0 Hz, 2H), 7.44(d, J=8.0 Hz, 2H), 7.19-7.15(m, 1H), 6.96-6.94(m, 1H), 5.45(br s, 2H), 4.71(d, J=6.5 Hz, 2H), 4.05-4.02(m, 1H), 3.93(s, 3H), 3.83-3.80(m, 1H), 2.28-2.25(m, 1H), 2.13-2.05(m, 1H), 1.99-1.88(m, 3H), 1.52-1.40(m, 3H).

**Example 3 Preparation of compound 5-amino-3-(4-((5-fluoro-2-methoxybenzamido)methyl)phenyl)-1-(3-hydroxycyclohexyl)-1H-pyrazole-4-carboxamide**

**(Isomer 2)**

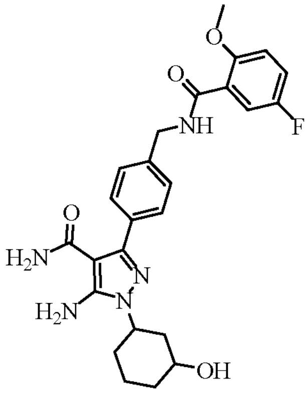

Compound 5-amino-3-(4-((5-fluoro-2-methoxybenzamido)methyl)phenyl)-1-(3-hydroxycyclohexyl)-1H-pyrazole-4-carboxamide (150 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325; 4.6mm*250mmL*5/μm; batch No. ICOOCE-VL054; Part Number 83325;
Flow rate: 4 mL/min;

Mobile phase: methanol: n-hexane: ethanol =10: 75: 15.

The corresponding fractions were collected, and rotary evaporated to dryness to obtain 61mg of the target compound. The corresponding retention time was 15.533 min. LC-MS(APCI): m/z=482.2$(M+1)^+$. $^1H$ NMR (500 MHz, $CDCl_3$)δ(ppm): 8.30-8.28(m, 1H), 7.98-7.95(dd, J=9.5 Hz, J=3.5 Hz, 1H), 7.54(d, J=8.0 Hz, 2H), 7.44(d, J=8.0 Hz, 2H), 7.19-7.15(m, 1H), 6.96-6.94(m, 1H), 5.45(br s, 2H), 4.71(d, J=6.5 Hz, 2H), 4.05-4.02(m, 1H), 3.93(s, 3H), 3.83-3.80(m, 1H), 2.28-2.25(m, 1H), 2.13-2.05(m, 1H), 1.99-1.88(m, 3H), 1.52-1.40(m, 3H).

## Example 4 Preparation of compound N-(4-(4-amino-1-(3-hydroxycyclohexanyl)-1H-pyrazolo[3,4-d]pyrimidin-3-yl)benzyl-5-fluoro-2-methoxybenzamide

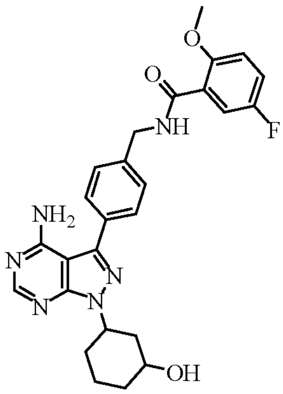

**The following synthetic route was adopted**

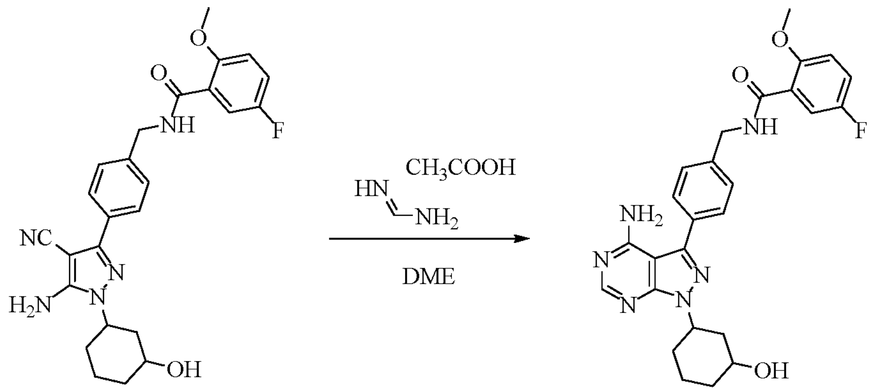


N-(4-(5-amino-4-cyano-1-(3-hydroxycyclohexanyl)-1H-pyrazol-3-yl)benzyl)-5-fluoro-2-methoxybenzamide (200 mg, 0.43 mmol), formamidine acetate (416.4 mg, 4.00 mmol) and DME (1 ml) were added to a 10 ml microwave tube equipped with a magnet, and the reaction was carried out in a microwave at 150° C under a nitrogen atmosphere for 2 hours. The reaction was cooled to room temperature. Water (30 mL) was added to quench the reaction. The mixture was extracted with ethyl acetate (30 mLx2). The organic phases were combined, washed with saturated saline (50 mL), dried with anhydrous sodium sulfate, and filtered. The filtrate was concentrated and passed through a silica gel column to obtain 134 mg of a white solid, yield: 63.6%. LC-MS(APCI): m/z=491.0$(M+1)^+$. $^1H$ NMR (400 MHz, $CDCl_3$)δ(ppm): 8.40(br s, 2H), 8.02-7.99(m, 1H), 7.71(d, J=8.0 Hz, 2H), 7.55(d, J=8.0 Hz, 2H), 7.21-7.19(m, 1H), 7.01-6.98(m, 1H), 5.63(br s, 2H), 4.96-4.93(m, 1H), 4.78(d, J=6.4 Hz, 2H), 3.99(s, 3H), 3.93-3.91(m, 1H), 2.39-2.37(m, 1H), 2.22-2.18(m, 1H), 2.07-1.96(m, 4H), 1.54-=1.46(m, 1H), 0.92-0.89(m, 1H).

## Example 5 Preparation of compound N-(4-(4-amino-1-(3-hydroxycyclohexanyl)-1H-pyrazolo[3,4-d]pyrimidin-3-yl)benzyl-5-fluoro-2-methoxybenzamide (Isomer 1)

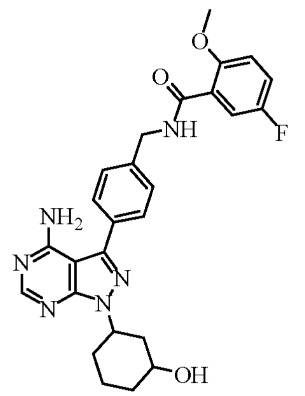

Compound N-(4-(4-amino-1-(3-hydroxycyclohexanyl)-1H-pyrazolo[3,4-d]pyrimidin-3-yl)benzyl-5-fluoro-2-methoxybenzamide (120 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4mL/min;
Mobile phase:dichloromethane:n-hexane:ethanol =45:45: 10;

The corresponding fractions were collected, and rotary evaporated to dryness to obtain 5 mg of the target compound. The corresponding retention time was 12.375 min. LC-MS(APCI): m/z=491.0$(M+1)^+$. $^1H$ NMR (400 MHz, $CDCl_3$)δ(ppm): 8.40(br s, 2H), 8.02-7.99(m, 1H), 7.71(d, J=8.0 Hz, 2H), 7.55(d, J=8.0 Hz, 2H), 7.21-7.19(m, 1H), 7.01-6.98(m, 1H), 5.63(br s, 2H), 4.96-4.93(m, 1H), 4.78(d, J=6.4 Hz, 2H), 3.99(s, 3H), 3.93-3.91(m, 1H), 2.39-2.37(m, 1H), 2.22-2.18(m, 1H), 2.07-1.96(m, 4H), 1.54-=1.46(m, 1H), 0.92-0.89(m, 1H).

**Example 6 Preparation of compound N-(4-(4-amino-1-(3-hydroxycyclohexanyl)-1H-pyrazolo[3,4-d]pyrimidin-3-yl)benzyl-5-fluoro-2-methoxybenzamide (Isomer 2)**

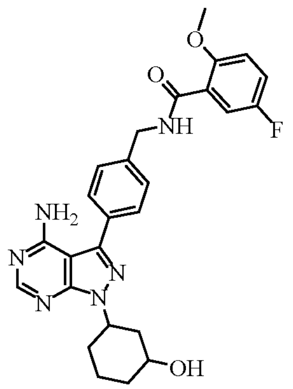

Compound N-(4-(4-amino-1-(3-hydroxycyclohexanyl)-1H-pyrazolo[3,4-d]pyrimidin-3-yl)benzyl-5-fluoro-2-methoxybenzamide (120 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4mL/min;
Mobile phase: dichloromethane: n-hexane: ethanol=45: 45: 10;

The corresponding fractions were collected, and rotary evaporated to dryness to obtain 5 mg of the target compound. The corresponding retention time was 13.903 min. LC-MS(APCI): m/z=491.0$(M+1)^+$. $^1$H NMR (400 MHz, $CDCl_3$)δ(ppm): 8.40(br s, 2H), 8.02-7.99(m, 1H), 7.71(d, J=8.0 Hz, 2H), 7.55(d, J=8.0 Hz, 2H), 7.21-7.19(m, 1H), 7.01-6.98(m, 1H), 5.63(br s, 2H), 4.96-4.93(m, 1H), 4.78(d, J=6.4 Hz, 2H), 3.99(s, 3H), 3.93-3.91(m, 1H), 2.39-2.37(m, 1H), 2.22-2.18(m, 1H), 2.07-1.96(m, 4H), 1.54-=1.46(m, 1H), 0.92-0.89(m, 1H).

**Example 7 Preparation of compound N-(4-(4-amino-1-(3-hydroxycyclohexanyl)-1H-pyrazolo[3,4-d]pyrimidin-3-yl)benzyl-5-fluoro-2-methoxybenzamide (Isomer 3)**

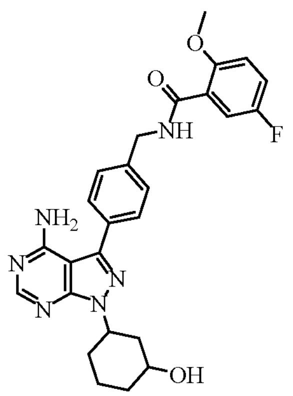

Compound N-(4-(4-amino-1-(3-hydroxycyclohexanyl)-1H-pyrazolo[3,4-d]pyrimidin-3-yl)benzyl-5-fluoro-2-methoxybenzamide (120 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4mL/min;
Mobile phase: dichloromethane: n-hexane: ethanol=45: 45: 10;

The corresponding fractions were collected, and rotary evaporated to dryness to obtain 42 mg of the target compound. The corresponding retention time was 15.742 min. LC-MS(APCI): m/z=491.0(M+1)$^+$. $^1$H NMR (400 MHz, $CDCl_3$)δ(ppm): 8.40(br s, 2H), 8.02-7.99(m, 1H), 7.71(d, J=8.0 Hz, 2H), 7.55(d, J=8.0 Hz, 2H), 7.21-7.19(m, 1H), 7.01-6.98(m, 1H), 5.63(br s, 2H), 4.96-4.93(m, 1H), 4.78(d, J=6.4 Hz, 2H), 3.99(s, 3H), 3.93-3.91(m, 1H), 2.39-2.37(m, 1H), 2.22-2.18(m, 1H), 2.07-1.96(m, 4H), 1.54-=1.46(m, 1H), 0.92-0.89(m, 1H).

**Example 8 Preparation of compound N-(4-(4-amino-1-(3-hydroxycyclohexanyl)-1H-pyrazolo[3,4-d]pyrimidin-3-yl)benzyl-5-fluoro-2-methoxybenzamide (Isomer 4)**

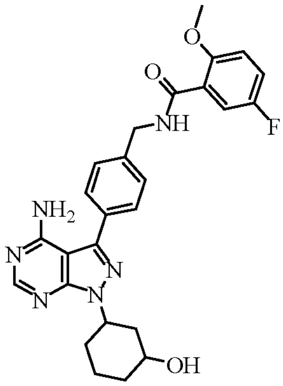

Compound N-(4-(4-amino-1-(3-hydroxycyclohexanyl)-1H-pyrazolo[3,4-d]pyrimidin-3-yl)benzyl-5-fluoro-2-methoxybenzamide (120 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: dichloromethane: n-hexane: ethanol=45: 45: 10;

The corresponding fractions were collected, and rotary evaporated to dryness to obtain 45 mg of the target compound. The corresponding retention time was 19.368 min. LC-MS(APCI): m/z=491.0(M+1)$^+$. $^1$H NMR (400 MHz, $CDCl_3$)δ(ppm): 8.40(br s, 2H), 8.02-7.99(m, 1H), 7.71(d, J=8.0 Hz, 2H), 7.55(d, J=8.0 Hz, 2H), 7.21-7.19(m, 1H), 7.01-6.98(m, 1H), 5.63(br s, 2H), 4.96-4.93(m, 1H), 4.78(d, J=6.4 Hz, 2H), 3.99(s, 3H), 3.93-3.91(m, 1H), 2.39-2.37(m, 1H), 2.22-2.18(m, 1H), 2.07-1.96(m, 4H), 1.54-=1.46(m, 1H), 0.92-0.89(m, 1H).

**Example 9 Preparation of compound trans-N-(4-(4-amino-1-(3-hydroxycyclohexanyl)-1H-pyrazolo[3,4-d]pyrimidin-3-yl)benzyl)-5-fluoro-2-methoxybenzamide**

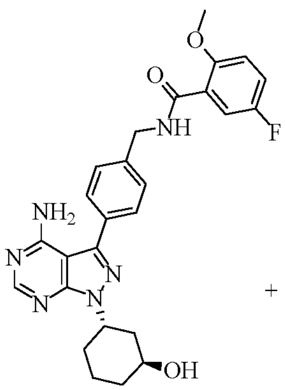
+
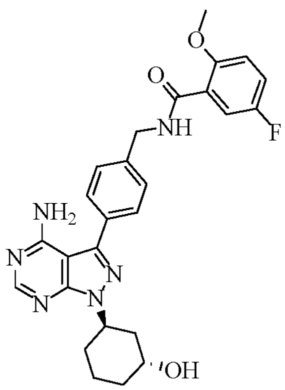

The following synthetic route was adopted

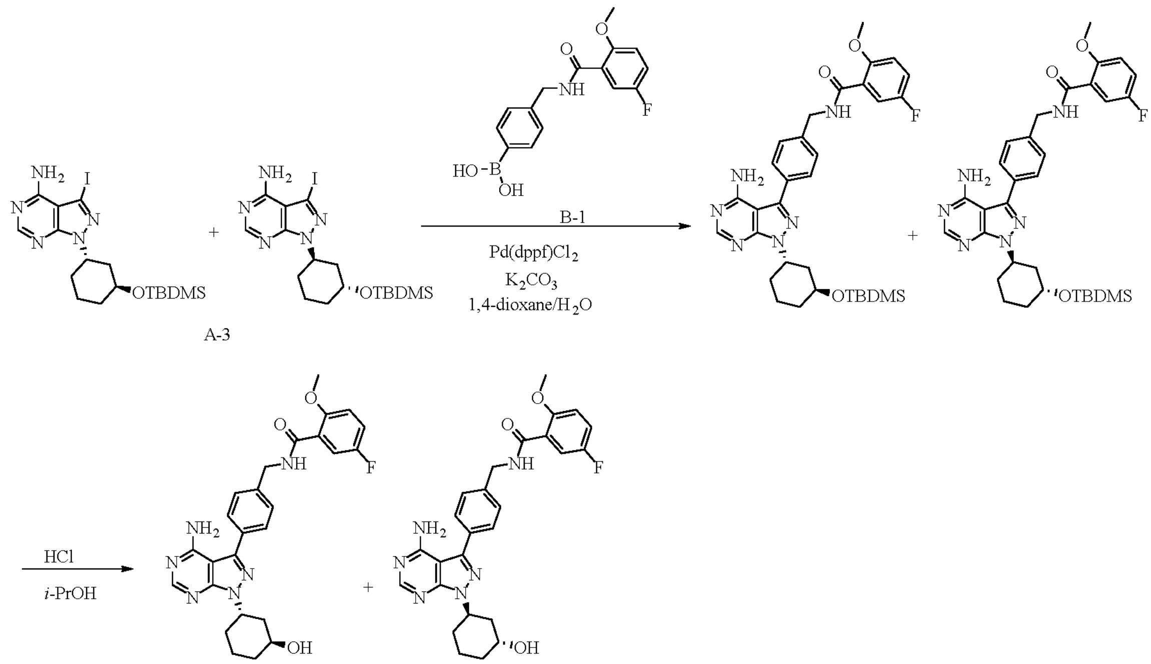


Step 1 Synthesis of compound trans-N-(4-(4-amino-1-(3-((tertbutyldimethylsilyl)oxy)cyclohexanyl)-1H-pyrazolo[3,4-d]pyrimidin-3-yl)benzyl)-5-fluoro-2-methoxybenzamide

Intermediate compound A-3 (1.0 g, 2.11 mmol), intermediate compound B-1 (833 mg, 2.75 mmol), $K_2CO_3$ (875 mg, 6.34 mmol), Pd (dppf) $Cl_2$ (162 mg, 0.21 mmol), 1,4-dioxane (10 mL) and water (3 mL) were added to a 20 mL microwave tube. The mixture was bubbled with nitrogen for 2 minutes. The mixture was heated by microwave to 120 °C and reacted for 0.5 hours. The reaction was cooled to room temperature, and saturated saline (5 mL) and DCM (20 mL) were added. The organic phase was separated, and the aqueous phase was extracted with DCM (20 mLx2). The organic phases were combined, dried with anhydrous sodium sulfate, and filtered. The filtrate was concentrated and passed through a silica gel column to obtain 1.0 g of a white solid, yield: 80.2%. LC-MS(APCI): m/z=605.2$(M+1)^+$.

Step 2 Synthesis of compound trans-N-(4-(4-amino-1-(3-hydroxycyclohexanyl)-1H-pyrazolo[3,4-d]pyrimidin-3-yl)benzyl)-5-fluoro-2-methoxybenzamide

Trans-N-(4-(4-amino-1-(3-((tert-butyldimethylsilyl)oxy)cyclohexanyl)-1H-pyrazolo[3,4-d]pyrimidin-3-yl)benzyl)-5-fluoro-2-methoxybenzamide (0.5 g, 0.828 mmol) and DCM (5 mL) were sequentially added to a 50 mL single-necked flask equipped with magnetic stirring, and stirred until dissolved. Hydrogen chloride solution in isopropanol (5 M, 20 mL) was added dropwise in an ice bath, and the reaction was stirred at room temperature for 2 hours under nitrogen atmosphere. The solvent was evaporated under reduced pressure. DCM (30 mL) and saturated $NaHCO_3$ solution (20 mL) were added and stirred for 2 minutes. The organic phase was separated, and the aqueous phase was extracted with dichloromethane (20 mLx2). The organic phases were combined, washed with saturated saline (30 mL), dried with anhydrous sodium sulfate, and filtered. The filtrate was concentrated and passed through a silica gel column. 373 mg of a white solid was obtained, yield: 91.9%. LC-MS(APCI): m/z=491.2$(M+1)^+$.

**Example 10 Preparation of compound trans-N-(4-(4-amino-1-(3-hydroxycyclohexanyl)-1H-pyrazolo[3,4-d]pyrimidin-3-yl)benzyl)-5-fluoro-2-methoxybenzamide (Isomer 1)**

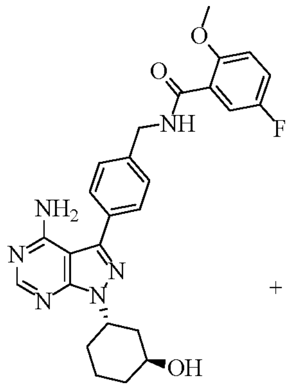

+

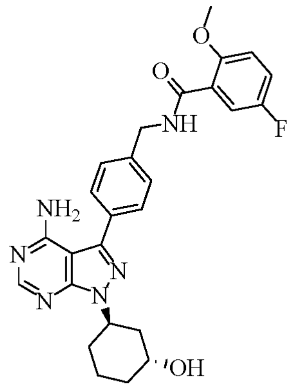

Compound trans-N-(4-(4-amino-1-(3-hydroxycyclohexanyl)-1H-pyrazolo[3,4-d]pyrimidin-3-yl)benzyl)-5-fluoro-2-methoxybenzamide (300 mg) was dissolved in methanol (30 mL) and the chiral preparation was carried out using the following conditions:

IC column: CHIRALPAKR IC; 4.6mm*250mmL*5μm; batch No. ICOOCE-VL054; Part Number 83325;
Flow rate: 4mL/min;
Mobile phase: dichloromethane: n-hexane: ethanol=45: 45: 10;

The corresponding fractions were collected, and rotary evaporated to dryness to obtain 128 mg of the target compound. The corresponding retention time was 12.877 min. LC-MS(APCI): m/z=491.2$(M+1)^+$.

**Example 11 Preparation of compound trans-N-(4-(4-amino-1-(3-hydroxycyclohexanyl)-1H-pyrazolo[3,4-d]pyrimidin-3-yl)benzyl)-5-fluoro-2-methoxybenzamide (Isomer 2)**

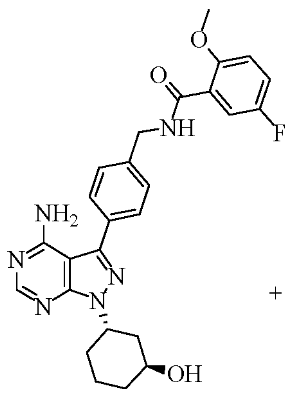

+

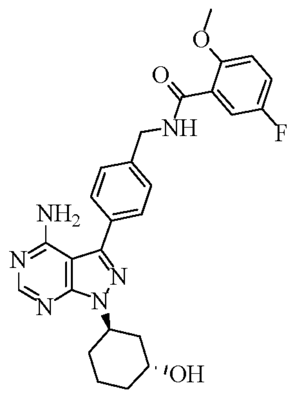

Compound trans-N-(4-(4-amino-1-(3-hydroxycyclohexanyl)-1H-pyrazolo[3,4-d]pyrimidin-3-yl)benzyl)-5-fluoro-2-methoxybenzamide (300 mg) was dissolved in methanol (30 mL) and the chiral preparation was carried out using the following conditions:

IC column: CHIRALPAKR IC; 4.6mm*250mmL*5μm; batch No. ICOOCE-VL054; Part Number 83325;
Flow rate: 4mL/min;
Mobile phase: dichloromethane: n-hexane: ethanol=45: 45: 10;

The corresponding fractions were collected, and rotary evaporated to dryness to obtain 125 mg of the target compound. The corresponding retention time was 14.505 min. LC-MS(APCI): m/z=491.2$(M+1)^+$.

**Example 12 Preparation of compound 5-amino-3-(4-((5-fluoro-2-methoxybenzamido)methyl)phenyl)-y-(6-(hydroxymethyl)tetrahydro-2H-pyran-3-yl)-1H-pyrazole-4-carboxamide**

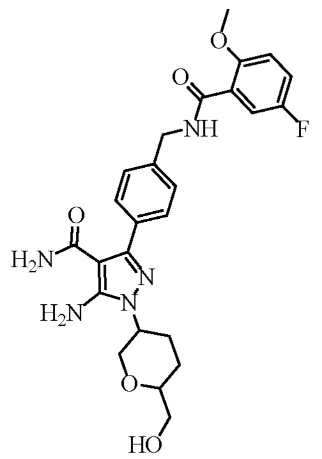

**The following synthetic route was adopted**

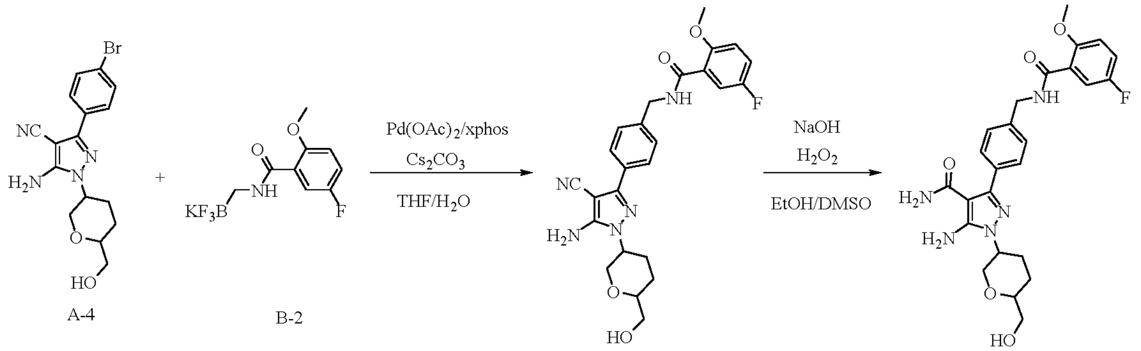


Step 1 Synthesis of compound N-(4-(5-amino-4-cyano-1-(6-(hydroxymethyl)tetrahydro-2H-pyran-3-yl)-1H-pyrazol-3-yl)benzyl)-5-fluoro-2-methoxybenzamide

Intermediate compound A-4 (500 mg, 1.33 mmol), intermediate compound B-2 (461 mg, 1.60 mmol), $Cs_2CO_3$ (1.3 g, 3.99 mmol), Xphos (80 mg, 0.13 mmol), Pd $(OAc)_2$ (30 mg, 0.07 mmol), THF (10 mL) and water (1 mL) were sequentially added to a 20 mL microwave tube, and bubbled with nitrogen for 2 minutes. The mixture was heated to 100°C in microwave and reacted for 1.5 h. The reaction was cooled to room temperature. The organic solvent was evaporated under reduced pressure. Saturated saline (5 mL) and ethyl acetate (20 mL) were added. The organic phase was separated, and the aqueous phase was extracted with ethyl acetate (20 mLx2). The organic phases were combined, dried with anhydrous sodium sulfate, and filtered. The filtrate was concentrated and passed through a silica gel column to obtain 510 mg of a white solid, yield: 80.3%. LC-MS(APCI): m/z=480.2$(M+1)^+$.

Step 2 Synthesis of compound 5-amino-3-(4-((5-fluoro-2-methoxybenzamido)methyl)phenyl)-1-(6-(hydroxymethyl)tetrahydro-2H-pyran-3-yl)-1H-pyrazole-4-carboxamide

In a 50 mL single-necked flask equipped with a magnetic stirrer, N-(4-(5-amino-4-cyano-1-(6-(hydroxymethyl) tetrahydro-2H-pyran-3-yl)-1H-pyrazol-3-yl)benzyl)-5-fluoro-2-methoxybenzamide (320 mg, 0.667 mmol), EtOH (20 mL) and DMSO (4 mL) were added, and stirred until dissolved. NaOH (2.67 mmol, 107 mg) was added under ice bath. After the addition was completed, $H_2O_2$ aqueous solution (5 mL, 33% by mass) was slowly added dropwise, slowly warmed to room temperature and stirred overnight. The organic solvent was evaporated under reduced pressure, diluted by adding water (30 mL), and extracted with ethyl acetate (30 mLx3). The organic phases were combined, washed with saturated saline (30 mLx2), dried with anhydrous sodium sulfate, and filtered. The filtrate was concentrated and passed through a silica gel column to obtain 221 mg of a white solid, yield: 66.3%, LC-MS(APCI): m/z=498.2$(M+1)^+$. $^1H$ NMR (400 MHz, $CDCl_3$) δ(ppm):8.33-8.31(m, 1H), 7.97(dd, J=9.2 Hz, 1H), 7.53(d, J=8.0 Hz, 2H), 7.46(d, J=8.0 Hz, 2H), 7.21-7.17(m, 1H), 6.98-6.95(m, 1H), 5.59(s, 2H), 5.27(s, 2H), 4.73(d, J=5.6 Hz, 2H), 4.14-4.10(m, 1H), 4.06-4.00(m, 1H), 3.96(s, 3H), 3.85(t, J=10.4Hz, 1H), 3.58-3.56(m, 2H), 2.38-2.34(m, 1H), 2.19-2.17(m, 2H), 1.82-1.78(m, 1H).

**Example 13 Preparation of compound 5-amino-3-(4-((5-fluoro-2-methoxybenzamido)methyl)phenyl)-1-(6-(hydroxymethyl)tetrahydro-2H-pyran-3-yl)-1H-pyrazole-4-carboxamide (Isomer 1)**

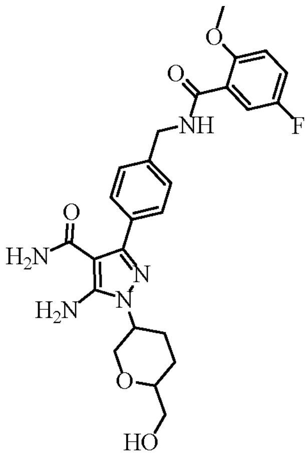

Compound 5-amino-3-(4-((5-fluoro-2-methoxybenzamido)methyl)phenyl)-1-(6-(hydroxymethyl)tetrahydro-2H-pyran-3-yl)-1H-pyrazole-4-carboxamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: methanol: n-hexane: ethanol =2: 63: 35(0.1% triethylamine);

The corresponding fractions were collected, and rotary evaporated to dryness to obtain 78 mg of the target compound. The corresponding retention time was 12.033 min. LC-MS(APCI): m/z=498.2(M+1)$^+$. $^1$H NMR (400 MHz, $CDCl_3$)δ(ppm): 8.33-8.31(m, 1H), 7.97(dd, J=9.2 Hz, 1H), 7.53(d, J=8.0 Hz, 2H), 7.46(d, J=8.0 Hz, 2H), 7.21-7.17(m, 1H), 6.98-6.95(m, 1H), 5.59(s, 2H), 5.27(s, 2H), 4.73(d, J=5.6 Hz, 2H), 4.14-4.10(m, 1H), 4.06-4.00(m, 1H), 3.96(s, 3H), 3.85(t, J=10.4Hz, 1H), 3.58-3.56(m, 2H), 2.38-2.34(m, 1H), 2.19-2.17(m, 2H), 1.82-1.78(m, 1H).

**Example 14 Preparation of compound 5-amino-3-(4-((5-fluoro-2-methoxybenzamido)methyl)phenyl)-1-(6-(hydroxymethyl)tetrahydro-2H-pyran-3-yl)-1H-pyrazole-4-carboxamide (Isomer 2)**

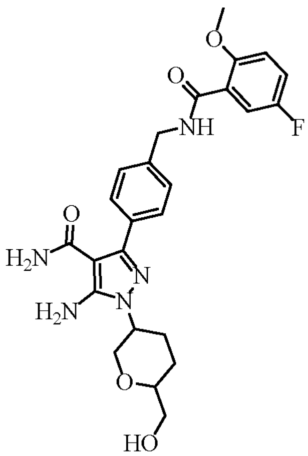

Compound 5-amino-3-(4-((5-fluoro-2-methoxybenzamido)methyl)phenyl)-1-(6-(hydroxymethyl)tetrahydro-2H-pyran-3-yl)-1H-pyrazole-4-carboxamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: methanol: n-hexane: ethanol =2: 63: 35(0.1% triethylamine);

The corresponding fractions were collected, and rotary evaporated to dryness to obtain 73 mg of the target compound. The corresponding retention time was 17.875 min. LC-MS(APCI): m/z=498.2(M+1)$^+$. $^1$H NMR (400 MHz, $CDCl_3$)δ(ppm):8.33-8.31(m, 1H), 7.97(dd, J=9.2 Hz, 1H), 7.53(d, J=8.0 Hz, 2H), 7.46(d, J=8.0 Hz, 2H), 7.21-7.17(m, 1H), 6.98-6.95(m, 1H), 5.59(s, 2H), 5.27(s, 2H), 4.73(d, J=5.6 Hz, 2H), 4.14-4.10(m, 1H), 4.06-4.00(m, 1H), 3.96(s, 3H), 3.85(t, J=10.4Hz, 1H), 3.58-3.56(m, 2H), 2.38-2.34(m, 1H), 2.19-2.17(m, 2H), 1.82-1.78(m, 1H).

**Example 15 Preparation of compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide**

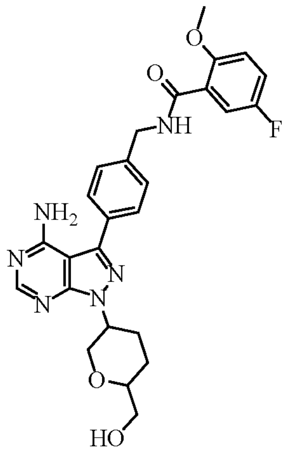

**The following synthetic route was adopted**

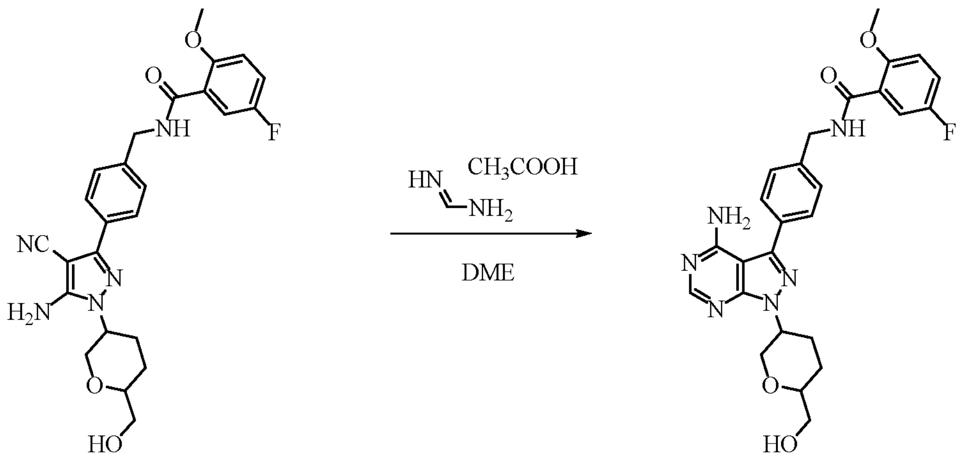


N-(4-(5-amino-4-cyano-1-(6-(hydroxymethyl)tetrahydro-2H-pyran-3-yl)-1H-pyrazol-3-yl)benzyl)-5-fluoro-2-methoxybenzamide (170 mg, 0.354 mmol) and ethylene glycol dimethyl ether (5 mL) were sequentially added to a 20 mL microwave tube, and stirred until dissolved. Formamidine acetate (368 mg, 3.54 mmol) was added, and the mixture was reacted in microwave at 120°C for 2 hours. The reaction was cooled to room temperature. The solvent was evaporated under reduced pressure. DCM (30 mL) and water (20 mL) were added to the residue. The organic phase was separated, and the aqueous phase was extracted with DCM (20 mLx2). The organic phases were combined, washed with saturated saline (20 mL), dried with anhydrous sodium sulfate, and filtered. The filtrate was concentrated and passed through a silica gel column to obtain125mg of a white solid, yield: 69.6%. LC-MS(APCI): m/z=507.2(M+1)$^+$. $^1$H NMR (400 MHz, $CDCl_3$) δ(ppm):8.37-8.35(m, 2H), 7.97-7.94(m, 1H), 7.65(d, J=8.0 Hz, 2H), 7.52(d, J=8.0 Hz, 2H), 7.19-7.14(m, 1H), 6.97-6.94(m, 1H), 5.73(br s, 2H), 4.98-4.91(m, 1H), 4.75(d, J=6.4 Hz, 2H), 4.18-4.15(m, 1H), 3.99(t, J=11.2 Hz, 1H), 3.95(s, 3H),

3.70-3.58(m, 3H), 2.46-2.38(m, 1H), 2.26-2.23(m, 1H), 1.82-1.79(m, 1H), 1.71-1.66(m, 1H).

**Example 16 Preparation of compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (Isomer 1)**

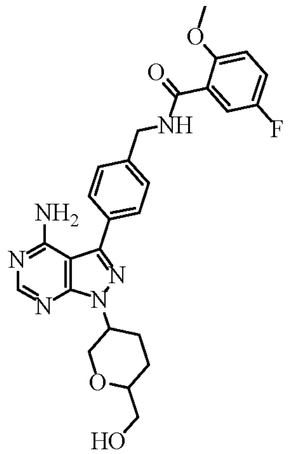

Compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: n-hexane: methanol: ethanol=65: 5: 30(0.1% triethylamine);

The corresponding fractions were collected, and rotary evaporated to dryness to obtain 6 mg of the target compound. The corresponding retention time was 21.672 min. LC-MS(APCI): m/z=507.2$(M+1)^+$. $^1$H NMR (400 MHz, $CDCl_3$)δ(ppm):8.37-8.35(m, 2H), 7.97-7.94(m, 1H), 7.65(d, J=8.0 Hz, 2H), 7.52(d, J=8.0 Hz, 2H), 7.19-7.14(m, 1H), 6.97-6.94(m, 1H), 5.73(br s, 2H), 4.98-4.91(m, 1H), 4.75(d, J=6.4 Hz, 2H), 4.18-4.15(m, 1H), 3.99(t, J=11.2 Hz, 1H), 3.95(s, 3H), 3.70-3.58(m, 3H), 2.46-2.38(m, 1H), 2.26-2.23(m, 1H), 1.82-1.79(m, 1H), 1.71-1.66(m, 1H).

**Example 17 Preparation of compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (Isomer 2)**

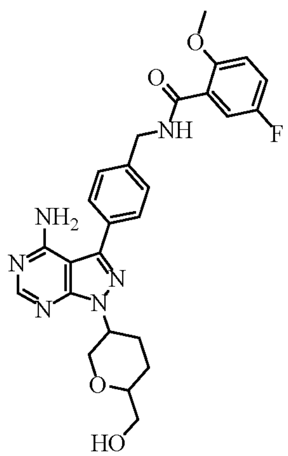

Compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: n-hexane: methanol: ethanol=65: 5: 30(0.1% triethylamine);

The corresponding fractions were collected, and rotary evaporated to dryness to obtain 5 mg of the target compound. The corresponding retention time was 25.023 min. LC-MS(APCI): m/z=507.2$(M+1)^+$. $^1H$ NMR (400 MHz, $CDCl_3$)δ(ppm):8.37-8.35(m, 2H), 7.97-7.94(m, 1H), 7.65(d, J=8.0 Hz, 2H), 7.52(d, J=8.0 Hz, 2H), 7.19-7.14(m, 1H), 6.97-6.94(m, 1H), 5.73(br s, 2H), 4.98-4.91(m, 1H), 4.75(d, J=6.4 Hz, 2H), 4.18-4.15(m, 1H), 3.99(t, J=11.2 Hz, 1H), 3.95(s, 3H), 3.70-3.58(m, 3H), 2.46-2.38(m, 1H), 2.26-2.23(m, 1H), 1.82-1.79(m, 1H), 1.71-1.66(m, 1H).

**Example 18 Preparation of compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (Isomer 3)**

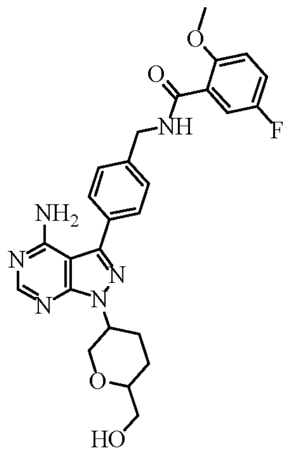

Compound N-(4-(4-amino-1-(6-(hydroxymethyl)tetrahydro-2H-pyran-3-yl)-1H-pyrazolo[3,4-d]pyrimidin-3-yl) benzyl)-5-fluoro-2-methoxybenzamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: n-hexane: methanol: ethanol=65: 5: 30(0.1% triethylamine);

The corresponding fractions were collected, and rotary evaporated to dryness to obtain 32 mg of the target compound. The corresponding retention time was 27.823 min. LC-MS(APCI): m/z=507.2$(M+1)^+$. $^1H$ NMR (400 MHz, $CDCl_3$)δ(ppm):8.37-8.35(m, 2H), 7.97-7.94(m, 1H), 7.65(d, J=8.0 Hz, 2H), 7.52(d, J=8.0 Hz, 2H), 7.19-7.14(m, 1H), 6.97-6.94(m, 1H), 5.73(br s, 2H), 4.98-4.91(m, 1H), 4.75(d, J=6.4 Hz, 2H), 4.18-4.15(m, 1H), 3.99(t, J=11.2 Hz, 1H), 3.95(s, 3H), 3.70-3.58(m, 3H), 2.46-2.38(m, 1H), 2.26-2.23(m, 1H), 1.82-1.79(m, 1H), 1.71-1.66(m, 1H).

**Example 19 Preparation of compound N-(4-(4-amino-1-(6-(hydroxymethyl)tetrahydro-2H-pyran-3-yl)-1H-pyrazolo[3,4-d]pyrimidin-3-yl)benzyl)-5-fluoro-2-methoxybenzamide (Isomer 4)**

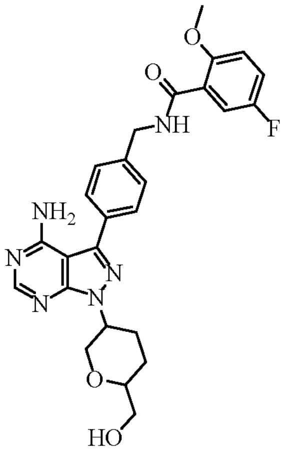

Compound N-(4-(4-amino-1-(6-(hydroxymethyl)tetrahydro-2H-pyran-3-yl)-1H-pyrazolo[3,4-d]pyrimidin-3-yl) benzyl)-5-fluoro-2-methoxybenzamide (200 mg) was dissolved in methanol (15 mL) and the chiral preparation was carried out using the following conditions:

AD-H column: CHIRALPAKR AD-H; 4.6mm*250mmL*5μm; batch No. ADHOCE-VB150; Part Number 19325;
Flow rate: 4 mL/min;
Mobile phase: n-hexane: methanol: ethanol=65: 5: 30(0.1% triethylamine);

The corresponding fractions were collected, and rotary evaporated to dryness to obtain 30 mg of the target compound. The corresponding retention time was 42.518 min. LC-MS(APCI): m/z=507.2$(M+1)^+$. $^1H$ NMR (400 MHz, $CDCl_3$)δ(ppm):8.37-8.35(m, 2H), 7.97-7.94(m, 1H), 7.65(d, J=8.0 Hz, 2H), 7.52(d, J=8.0 Hz, 2H), 7.19-7.14(m, 1H), 6.97-6.94(m, 1H), 5.73(br s, 2H), 4.98-4.91(m, 1H), 4.75(d, J=6.4 Hz, 2H), 4.18-4.15(m, 1H), 3.99(t, J=11.2 Hz, 1H), 3.95(s, 3H), 3.70-3.58(m, 3H), 2.46-2.38(m, 1H), 2.26-2.23(m, 1H), 1.82-1.79(m, 1H), 1.71-1.66(m, 1H).

**Example 20 Preparation of compound N-(4-(8-amino-3-(3-hydroxycyclohexanyl)imidazo[1,5-a]pyrazin-1-yl) benzyl)-5-fluoro-2-methoxybenzamide**

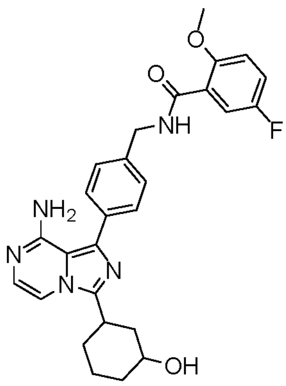

**The following synthetic route was adopted**

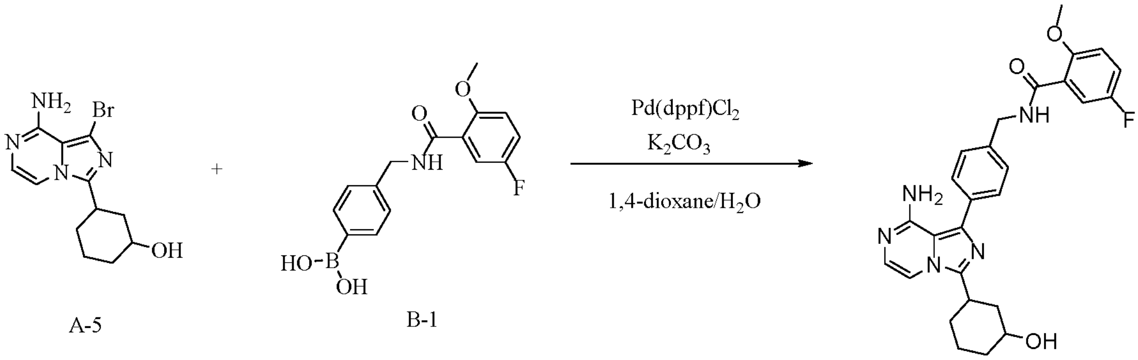


A-5

B-1

Compound intermediate compound A-5 (200 mg, 0.645 mmol), intermediate compound B-1 (254 mg, 0.839 mmol), $K_2CO_3$ (267 mg, 1.935 mmol), Pd(dppf)$Cl_2$ (43 mg, 0.06 mmol), 1,4-dioxane (10 mL), and water (3 mL) were sequentially added to a 20 mL microwave tube filled with a magnet, and the atmosphere was replaced with nitrogen for 2 minutes. The mixture was reacted in microwave at 120 °C for 0.5 hours. The reaction was cooled to room temperature, and saturated saline (5 mL) and DCM (20 mL) were added. The organic phase was separated, and the aqueous phase was extracted with DCM (20 mLx2). The organic phases were combined, dried with anhydrous sodium sulfate, and filtered. The filtrate was concentrated and passed through a silica gel column to obtain 0.27 g of a white solid, yield: 83.3%. LC-MS(APCI): m/z=490.1(M+1)$^+$. $^1$H NMR (400 MHz, $CDCl_3$)δ(ppm):8.29(s, 1H), 7.96(dd, J=9.2 Hz, J=3.2 Hz, 1H), 7.62(d, J=8.0 Hz, 2H), 7.45(d, J=8.0 Hz, 2H), 7.18-7.13(m, 2H), 7.07(d, J=5.2 Hz, 1H), 6.95-6.92(m, 1H), 5.16(br s, 2H), 4.72(d, J=5.6 Hz, 2H), 3.94(s, 3H), 3.93-3.90(m, 1H), 3.26-3.24(m, 1H), 2.26-2.22(m, 1H), 2.09-2.02(m, 1H), 1.90-1.83(m, 4H), 1.48-1.42(m, 1H).

**Example 21 Preparation** of **compound N-(4-(8-amino-3-(3-hydroxycyclohexanyl)imidazo[1,5-a]pyrazin-1-yl) benzyl)-5-fluoro-2-methoxybenzamide**

**(Isomer 1)**

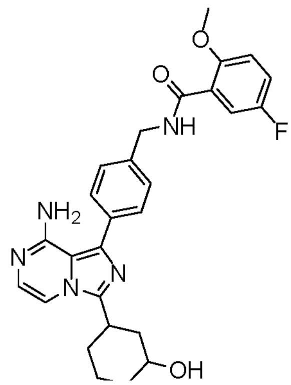

Compound N-(4-(8-amino-3-(3-hydroxycyclohexanyl)imidazo[1,5-a]pyrazin-1-yl)benzyl)-5-fluoro-2-methoxybenzamide (200 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: n-hexane: ethanol=60: 40(0.1% triethylamine);

The corresponding fractions were collected, and rotary evaporated to dryness to obtain 75 mg of the target compound. The corresponding retention time was 23.963 min. LC-MS(APCI): m/z=490.1(M+1)$^+$. $^1$H NMR (400 MHz, $CDCl_3$)δ(ppm):8.29(s, 1H), 7.96(dd, J=9.2 Hz, J=3.2 Hz, 1H), 7.62(d, J=8.0 Hz, 2H), 7.45(d, J=8.0 Hz, 2H), 7.18-7.13(m, 2H), 7.07(d, J=5.2 Hz, 1H), 6.95-6.92(m, 1H), 5.16(br s, 2H), 4.72(d, J=5.6 Hz, 2H), 3.94(s, 3H), 3.93-3.90(m, 1H), 3.26-3.24(m, 1H), 2.26-2.22(m, 1H), 2.09-2.02(m, 1H), 1.90-1.83(m, 4H), 1.48-1.42(m, 1H).

**Example 22 Preparation of compound N-(4-(8-amino-3-(3-hydroxycyclohexanyl)imidazo[1,5-a]pyrazin-1-yl) benzyl)-5-fluoro-2-methoxybenzamide**

**(Isomer 2)**

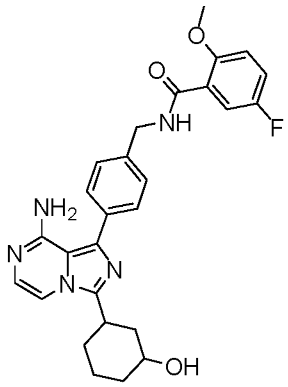

Compound N-(4-(8-amino-3-(3-hydroxycyclohexanyl)imidazo[1,5-a]pyrazin-1-yl)benzyl)-5-fluoro-2-methoxybenzamide (200 mg) was dissolved in methanol (15 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: n-hexane: ethanol=60: 40(0.1% triethylamine);

The corresponding fractions were collected, and rotary evaporated to dryness to obtain 81 mg of the target compound. The corresponding retention time was 27.885 min. LC-MS(APCI): m/z=490.1(M+1)$^+$. $^1$H NMR (400 MHz, $CDCl_3$)δ(ppm):8.29(s, 1H), 7.96(dd, J=9.2 Hz, J=3.2 Hz, 1H), 7.62(d, J=8.0 Hz, 2H), 7.45(d, J=8.0 Hz, 2H), 7.18-7.13(m, 2H), 7.07(d, J=5.2 Hz, 1H), 6.95-6.92(m, 1H), 5.16(br s, 2H), 4.72(d, J=5.6 Hz, 2H), 3.94(s, 3H), 3.93-3.90(m, 1H), 3.26-3.24(m, 1H), 2.26-2.22(m, 1H), 2.09-2.02(m, 1H), 1.90-1.83(m, 4H), 1.48-1.42(m, 1H).

**Example 23 Preparation of compound N-(4-(4-amino-1-(3-hydroxy-3-methylcyclohexanyl)-1H-pyrazolo[3,4-d] pyrimidin-3-yl)benzyl)-5-fluoro-2-methoxybenzamide (lower polarity); and**

**Example 24 Preparation of compound N-(4-(4-amino-1-(3-hydroxy-3-methylcyclohexanyl)-1H-pyrazolo[3,4-d] pyrimidin-3-yl)benzyl)-5-fluoro-2-methoxybenzamide (higher polarity)**

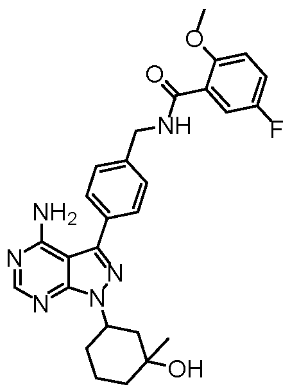

**The following synthetic route was adopted**

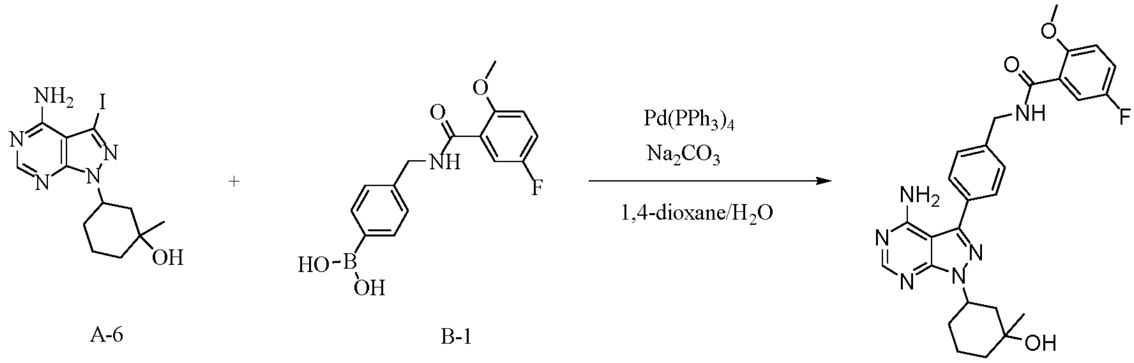


A-6 B-1

Intermediate compound A-6 (400 mg, 1.07 mmol) was added to a 50 mL single-necked flask equipped with magnetic stirring and a condenser tube. 1,4-Dioxane (8 mL) and $H_2O$ (2 mL) were added at room temperature. Tetrakis(triphenylphosphine)palladium (123 mg, 0.106 mmol), sodium carbonate (341 mg, 3.21 mmol), and intermediate compound B-1 (389.9 mg, 1.287 mmol) were added. The mixture was stirred and dispersed. The flask was evacuated and the atmosphere was replaced with nitrogen three times. Temperature was raised to 100 °C. The mixture was stirred and reacted overnight while maintaining the temperature. Aqueous solution (50 mL) was added to quench the reaction. The mixture was extracted with ethyl acetate (3x50 mL). The organic phases were combined, washed with saturated saline (2x50 mL), dried with anhydrous sodium sulfate, and filtered. The filtrate was concentrated under reduced pressure to dryness. The residue was passed through a silica gel column to obtain 105 mg of Example 23 with lower polarity as a white solid, yield: 19.5%, LC-MS(APCI): m/z=505.5$(M+1)^+$; and 98 mg of Example 24 with higher polarity as a white solid, yield: 18.2%, LC-MS(APCI): m/z=505.5$(M+1)^+$.

**Example 25 Preparation of compound N-(4-(4-amino-1-(3-hydroxy-3-methylcyclohexanyl)-1H-pyrazolo[3,4-d]pyrimidin-3-yl)benzyl)-5-fluoro-2-methoxybenzamide (Isomer 1)**

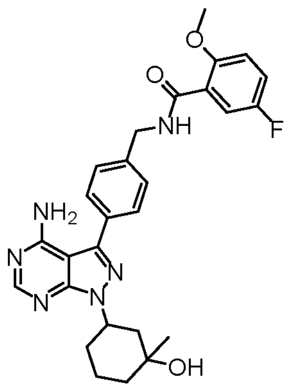

Example 23 (100 mg) was dissolved in methanol (10 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: dichloromethane: n-hexane: ethanol=40: 50: 10;

The corresponding fractions were collected, and rotary evaporated to dryness to obtain 38 mg of the target compound. The corresponding retention time was 16.013 min. LC-MS(APCI): m/z=505.5$(M+1)^+$.

**Example 26 Preparation of compound N-(4-(4-amino-1-(3-hydroxy-3-methylcyclohexanyl)-1H-pyrazolo[3,4-d]pyrimidin-3-yl)benzyl)-5-fluoro-2-methoxybenzamide (Isomer 2)**

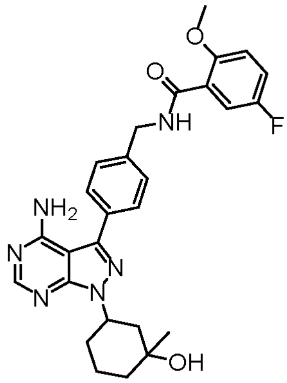

Example 23 (100 mg) was dissolved in methanol (10 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: dichloromethane: n-hexane: ethanol=40: 50: 10;

The corresponding fractions were collected, and rotary evaporated to dryness to obtain 42 mg of the target compound. The corresponding retention time was 20.367 min. LC-MS(APCI): m/z=505.5$(M+1)^+$.

**Example 27 Preparation of compound N-(4-(4-amino-l-(3-hydroxy-3-methylcyclohexanyl)-1H-pyrazolo[3,4-d]pyrimidin-3-yl)benzyl)-5-fluoro-2-methoxybenzamide (Isomer 1)**

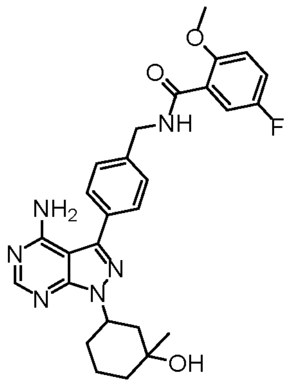

Example 24 (90 mg) was dissolved in methanol (10 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: dichloromethane: n-hexane: ethanol=40: 50: 10;

The corresponding fractions were collected, and rotary evaporated to dryness to obtain 35 mg of the target compound. The corresponding retention time was 13.253 min. LC-MS(APCI): m/z=505.5$(M+1)^+$.

**Example 28 Preparation of compound N-(4-(4-amino-1-(3-hydroxy-3-methylcyclohexanyl)-1H-pyrazolo[3,4-d]pyrimidin-3-yl)benzyl)-5-fluoro-2-methoxybenzamide (Isomer 2)**

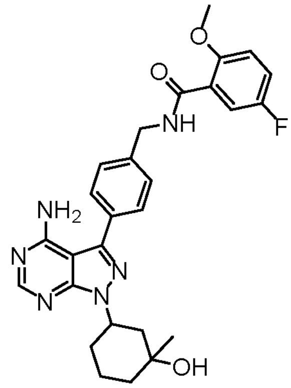

Example 24 (100 mg) was dissolved in methanol (10 mL) and the chiral preparation was carried out using the following conditions:

IC column: CHIRALPAKR IC; 4.6mm*250mmL*5μm; batch No. ICOOCE-VL054; Part Number 83325;
Flow rate: 4 mL/min;
Mobile phase: dichloromethane: n-hexane: ethanol=40: 50: 10;

The corresponding fractions were collected, and rotary evaporated to dryness to obtain 32 mg of the target compound. The corresponding retention time was 14.672 min. LC-MS(APCI): m/z=505.5(M+1)$^+$.

**Example 29 Preparation of compound N-(4-(4-amino-1-((1R,4R)-4-hydroxycyclohexylalkane)-1H-pyrazolo[3,4-d]pyrimidin-3-yl)benzyl)-5-fluoro-2-methoxybenzamide; and**

**Example 30 Preparation of compound N-(4-(4-amino-1-((1S,4S)-4-hydroxycyclohexylalkane)-1H-pyrazolo[3,4-d]pyrimidin-3-yl)benzyl)-5-fluoro-2-methoxybenzamide**

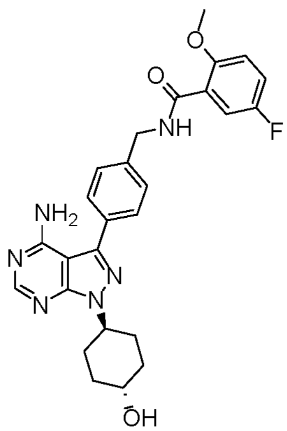

and

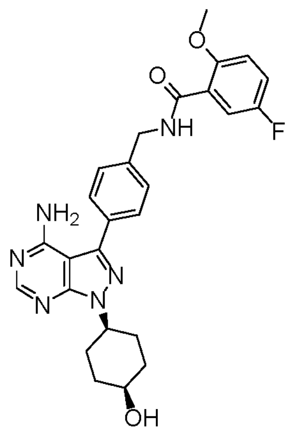

**The following synthetic route was adopted**

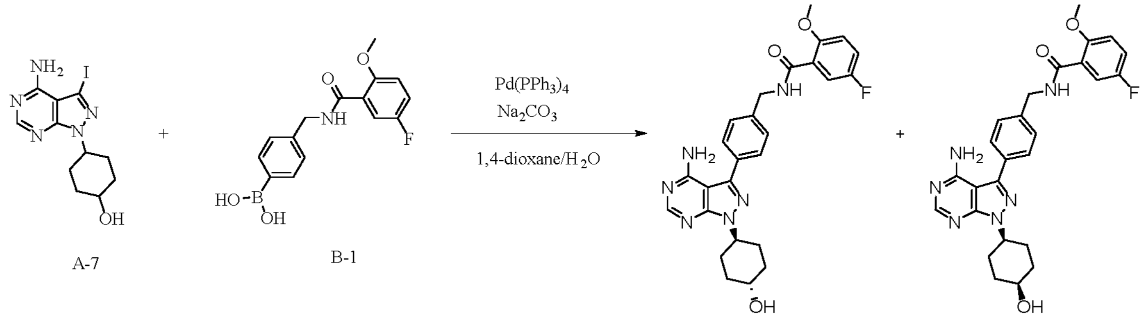


Intermediate compound A-7 (500 mg, 1.39 mmol) was added to a 50 mL single-necked flask equipped with magnetic stirring and a condenser tube. 1,4-Dioxane (8 mL) and water (2 mL) were added at room temperature. Tetrakis(triphenylphosphine)palladium (160 mg, 0.14 mmol), sodium carbonate (442.8 mg, 4.17 mmol), and intermediate compound B-1 (633 mg, 2.08 mmol) were added. The mixture was stirred and dispersed. The flask was evacuated and the atmosphere was replaced with nitrogen three times. The mixture was heated to 100 °C. The mixture was stirred and reacted overnight while maintaining the temperature. Water (50 mL) was added to quench the reaction. The mixture was extracted with ethyl acetate (3x50mL). The organic phases were combined, washed with saturated saline (2x50 mL), dried with anhydrous sodium sulfate, and filtered. The filtrate was concentrated under reduced pressure to dryness. The residue was passed through a silica gel column to obtain 232 mg of Example 29 with higher polarity as a white solid, yield: 34.09%, LC-MS(APCI): m/z=491.0 $(M+1)^+$, $^1H$ NMR (400MHz, $CDCl_3$)δ(ppm):8.38(s, 1H), 8.37-8.34(m, 1H), 8.00-7.97(m, 1H), 7.69(d, J=8.0 Hz, 2H), 7.52(d, J=8.0 Hz, 2H), 7.20-7.16(m, 1H), 6.98-6.95(m, 1H), 5.47(br s, 2H), 4.83-4.80(m, 1H), 4.76(d, J=5.6 Hz, 2H), 4.14(s, 1H), 3.96(s, 3H), 2.54-2.51(m, 2H), 2.05-2.00(m, 2H), 1.90-1.80(m, 4H).

75 mg of Example 30 with lower polarity was obtained as a white solid, yield: 11.01%. LC-MS(APCI): m/z=491.0 $(M+1)^+$. $^1H$ NMR (400MHz, $CDCl_3$)δ(ppm):8.38(s, 1H), 8.37-8.34(m, 1H), 8.00-7.97(m, 1H), 7.67(d, J=8.0 Hz, 2H), 7.52(d, J=8.0 Hz, 2H), 7.20-7.16(m, 1H), 6.98-6.95(m, 1H), 5.44(br s, 2H), 4.83-4.80(m, 1H), 4.76(d, J=5.6 Hz, 2H), 3.96(s, 3H), 3.83-3.81(m, 1H), 2.25-2.00(m, 8H).

## Example 31 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (lower polarity); and

## Example 32 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (higher polarity)

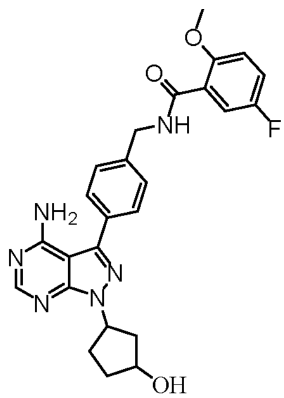

The following synthetic route was adopted

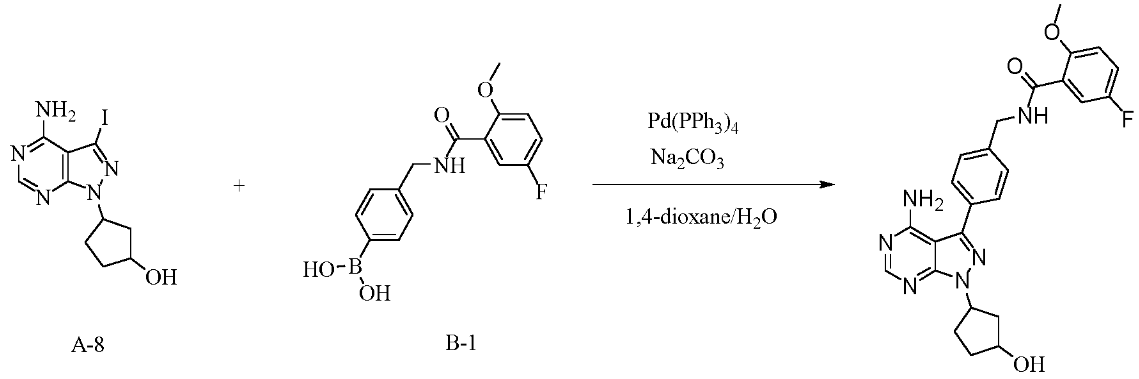


A-8 B-1

Intermediate compound A-8 (500 mg, 1.44 mmol) was added to a 50 mL single-necked flask equipped with magnetic stirring and a condenser tube. 1,4-Dioxane (8 mL) and $H_2O$ (2 mL) were added at room temperature. Tetrakis(triphenylphosphine)palladium (160 mg, 0.14 mmol), sodium carbonate (445 mg, 4.20 mmol), and intermediate compound B-1 (640 mg, 2.11 mmol) were added. The mixture was stirred and dispersed. The flask was evacuated and the atmosphere was replaced with nitrogen three times. The mixture was heated to 100 °C. The mixture was stirred and reacted overnight while maintaining the temperature. Water (50 mL) was added to quench the reaction. The mixture was extracted with ethyl acetate (3x50 mL). The organic phases were combined, washed with saturated saline (2x50 mL), dried with anhydrous sodium sulfate, and filtered. The filtrate was concentrated under reduced pressure to dryness. The residue was passed through a silica gel column to obtain Example 31 with lower polarity, 151 mg of a white solid, yield: 21.92%. LC-MS(APCI): m/z=476.2$(M+1)^+$.

Example 32 with higher polarity was obtained, 45 mg of a white solid, yield: 6.58%. LC-MS(APCI): m/z=476.2$(M+1)^+$.

**<u>Example 33 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (Isomer</u>** <u>1)</u>

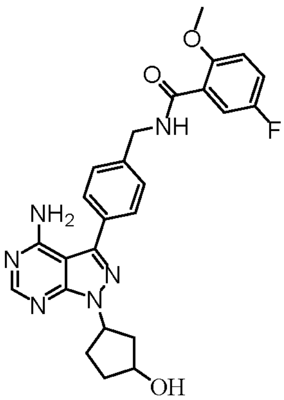

Example 31 (150 mg) was dissolved in methanol (10 mL) and the chiral preparation was carried out using the following conditions:

IG column: CHIRALPAKR IG; 4.6mmI.D*250mmL*5μm; batch No. IGOOCE-BR054; Part Number 87325;
Flow rate: 4ml/min;
Mobile phase: methyl tert-butyl ether: methanol: ethanol=95: 3: 2;

The corresponding fractions were collected, and rotary evaporated to dryness to obtain 58 mg of the target compound. The corresponding retention time was 30.460 min.

**Example 34 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (Isomer 2)**

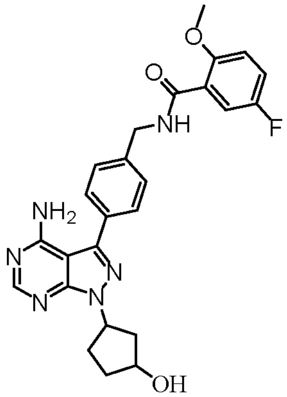

Example 31 (150 mg) was dissolved in methanol (10 mL) and the chiral preparation was carried out using the following conditions:

IG column: CHIRALPAKR IG; 4.6mmI.D*250mmL*5μm; batch No. IGOOCE-BR054; Part Number 87325;
Flow rate: 4ml/min;
Mobile phase: methyl tert-butyl ether: methanol: ethanol=95: 3: 2;

The corresponding fractions were collected, and rotary evaporated to dryness to obtain 52 mg of the target compound. The corresponding retention time was 34.478 min.

**Example 35 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (Isomer 1)**

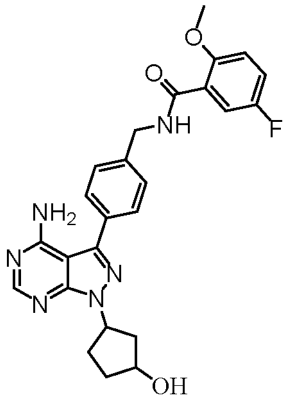

Example 32 (40 mg) was dissolved in methanol (6 mL) and the chiral preparation was carried out using the following conditions:

IG column: CHIRALPAKR IG; 4.6mmI.D*250mmL*5μm; batch No. IGOOCE-BR054; Part Number 87325;
Flow rate: 4ml/min;
Mobile phase: methyl tert-butyl ether: methanol: ethanol=95: 3: 2;

The corresponding fractions were collected, and rotary evaporated to dryness to obtain 13 mg of the target compound. The corresponding retention time was 24.480 min.

**Example 36 Preparation of compound N-(4-(4-amino-1-(3-hydroxycyclopentanyl)-1H-pyrazolo[3,4-d]pyrimidin-3-yl)benzyl)-5-fluoro-2-methoxybenzamide (Isomer 2)**

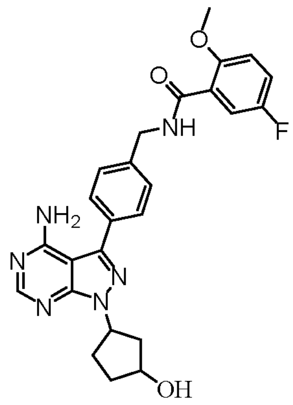

Example 32 (40 mg) was dissolved in methanol (6 mL) and the chiral preparation was carried out using the following conditions:

IG column: CHIRALPAKR IG; 4.6mmI.D*250mmL*5μm; batch No. IGOOCE-BR054; Part Number 87325;
Flow rate: 4ml/min;
Mobile phase: methyl tert-butyl ether: methanol: ethanol=95: 3: 2;

The corresponding fractions were collected, and rotary evaporated to dryness to obtain 15 mg of the target compound. The corresponding retention time was 27.155 min.

**Example 37 Preparation of compound trans-N-(4-(4-amino-1-(4-hydroxycyclopent-2-en-1-yl)-1H-pyrazolo[3,4-d]pyrimidin-3-yl)benzyl)-5-fluoro-2-methoxybenzamide (Isomer 1), and**

**Example 38 Preparation of compound trans-N-(4-(4-amino-1-(4-hydroxycyclopent-2-en-1-yl)-1H-pyrazolo[3,4-d]pyrimidin-3-yl)benzyl)-5-fluoro-2-methoxybenzamide (Isomer 2)**

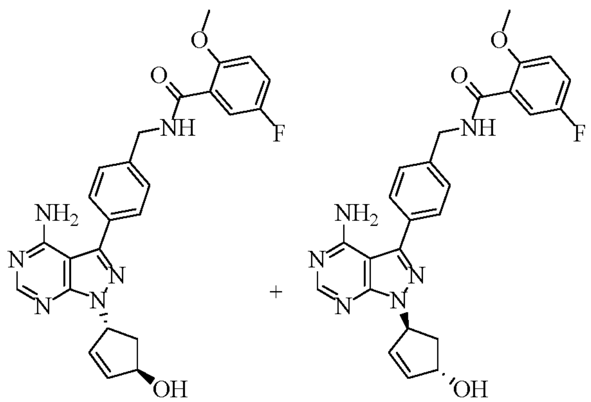

**The following synthetic route was adopted**

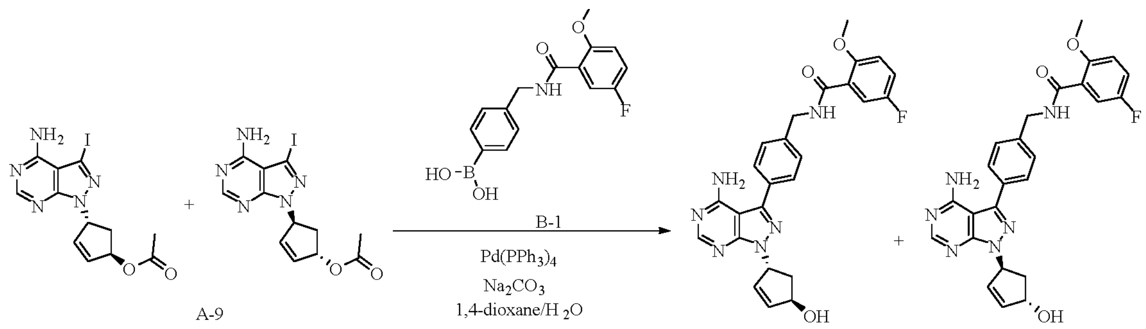


Intermediate compound A-9 (0.98 g, 2.54 mmol), intermediate compound B-1 (1.15 g, 3.81 mmol), sodium carbonate (0.81 g, 7.62 mmol), $Pd(pph_3)_4$ (146.8 mg, 0.127 mmol), 1,4-dioxane (20 ml) and water (5 ml) were added to a 100 ml three-necked bottle equipped with magnetic stirring and condensing tube, and the reaction was stirred at 100 °C under nitrogen atmosphere overnight. The reaction was cooled to room temperature. Water (30 mL) was added to quench the reaction. The mixture was extracted with ethyl acetate (30 mLx2). The organic phases were combined, washed with saturated saline (50 mL), dried with anhydrous sodium sulfate, and filtered. The filtrate was concentrated and passed through a silica gel column to obtain Example 37 with lower polarity, 71 mg of a white solid, yield: 5.8%, LC-MS(APCI): m/z=475.0$(M+1)^+$, $^1H$ NMR (400MHz, DMSO-$D_6$)δ(ppm):8.85(t, J=6.0 Hz, 1H), 8.23(s, 1H), 7.61(d, J=8.0 Hz, 2H), 7.52-7.48(m, 3H), 7.35-7.30(m, 1H), 7.19-7.16(m, 1H), 6.07-6.05(m, 1H), 5.90-5.88(m, 1H), 5.73(t, J=10.4 Hz, 1H), 5.25(d, J=6.8 Hz, 1H), 4.74-4.71(m, 1H), 4.56(d, J=6.0 Hz, 2H), 4.04-3.98(m, 1H), 3.89(s, 3H), 2.82-2.79(m, 1H); Example 38 with higher polarity was obtained, 165 mg of a white solid, yield: 13.7%, LC-MS(APCI): m/z=475.0$(M+1)^+$, $^1H$ NMR (400MHz, $CDCl_3$)δ(ppm):8.40(t, J=4.8 Hz, 1H), 8.26(s, 1H), 7.96(dd, J=9.6 Hz, J=3.2 Hz, 1H), 7.61-7.54(m, 4H), 7.20-7.16(m, 1H), 6.98-6.95(m, 1H), 6.28-6.24(m, 2H), 6.06-6.05(d, J=4.4 Hz, 1H), 5.34-5.33(m, 1H), 4.76(d, J=6.0 Hz, 2H), 3.98(s, 3H), 2.72-2.66(m, 1H), 2.43-2.37(m, 1H).

**Example 39 Preparation of compound cis-N-(4-(4-amino-1-((1S,4S)-4-hydroxycyclohexanyl)-7-oxo-6,7-dihydro-1H-pyrazolo[3,4-d]pyridazin-3-yl)benzyl)-5-fluoro-2-methoxybenzamide, and**

**Example 40 Preparation of compound trans-N-(4-(4-amino-1-((1R,4R)-4-hydroxycyclohexanyl)-7-oxo-6,7-dihydro-1H-pyrazolo[3,4-d]pyridazin-3-yl)benzyl)-5-fluoro-2-methoxybenzamide**

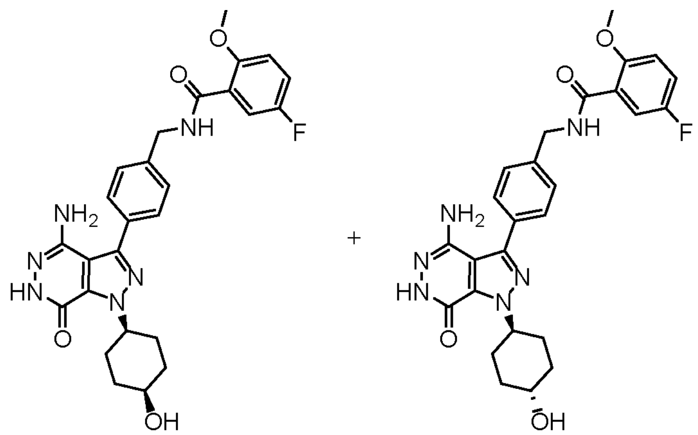

**The following synthetic route was adopted**

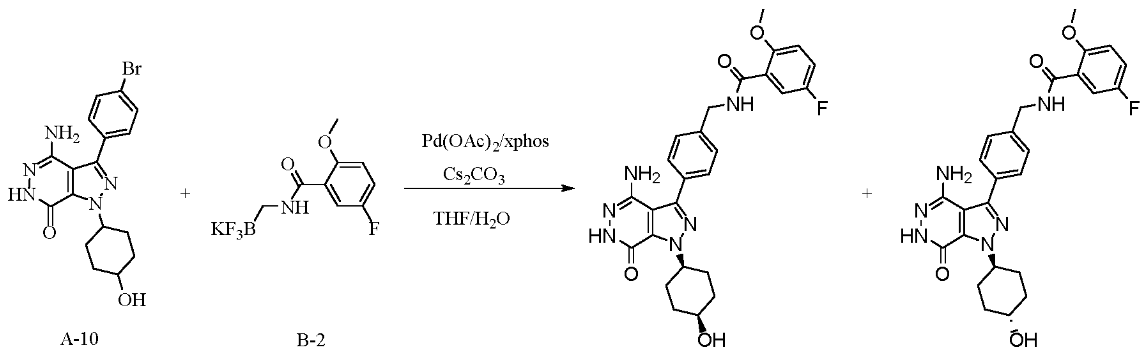


A-10 B-2

Intermediate compound A-10 (171 mg, 0.42 mmol), tetrahydrofuran (7 mL), water (0.7 mL), intermediate compound B-2 (153 mg, 0.53 mmol), $Pd(OAc)_2$ (5 mg, 0.02 mmol), XPhos (20 mg, 0.04 mmol), and cesium carbonate (415 mg, 0.12 mmol) were added to a 10 ml microwave tube. The atmosphere was replaced with nitrogen, and the mixture was reacted with microwave at 110 °C for one hour. The reaction was cooled to room temperature. Water (20 mL) was added to quench the reaction. Ethyl acetate (30 mL × 3) was added for extraction. The organic phase was separated, washed with saturated saline (30 mL), dried with anhydrous sodium sulfate, and filtered. The filtrate was concentrated and passed through a silica gel column to obtain the cis product Example 39 with lower polarity, 32 mg of a white solid, yield: 14.0%, LC-MS(APCI): m/z= 510.0(M+1)+; the trans product Example 40 with higher polarity was obtained, 75 mg of a white solid, yield: 35.0%, LC-MS(APCI): m/z= 510.0$(M+1)^+$.

**Example 41 Preparation of compound N-(4-(4-amino-1-(3-hydroxycyclopentanyl)-7-oxo-6,7-dihydro-1H-pyrazolo[3,4-d]pyridazin-3-yl)benzyl)-5-fluoro-2-methoxybenzamide (lower polarity); and**

**Example 42 Preparation of compound N-(4-(4-amino-1-(3-hydroxycyclopentanyl)-7-oxo-6,7-dihydro-1H-pyrazolo[3,4-d]pyridazin-3-yl)benzyl)-5-fluoro-2-methoxybenzamide (higher polarity)**

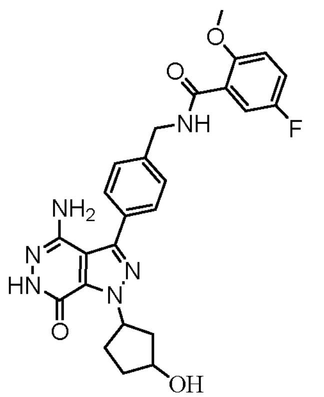

**The following synthetic route was adopted**

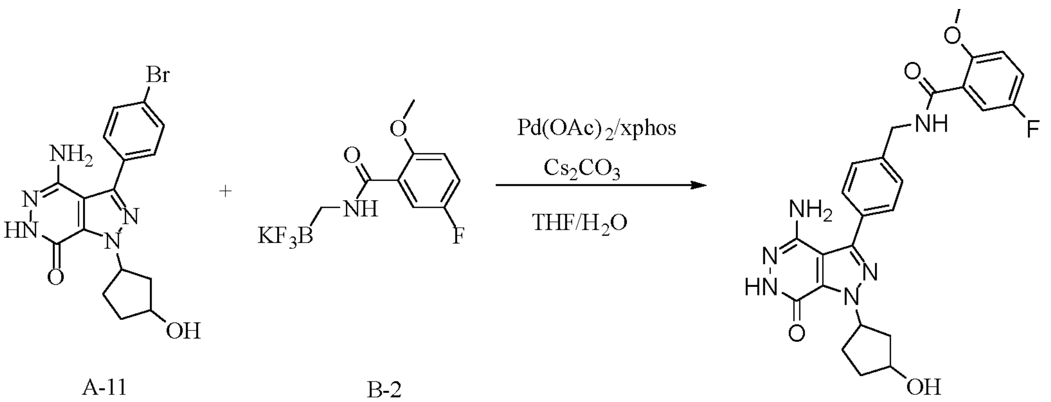


A-11 B-2

Intermediate compound A-11 (156 mg, 0.4 mmol), tetrahydrofuran (7 mL), water (0.7 mL), intermediate compound B-2 (184.5 mg, 0.63 mmol), $Pd(OAc)_2$ (9.5 mg, 0.04 mmol), XPhos (12.3 mg, 0.04 mmol), and cesium carbonate (416 mg, 0.12 mmol) were added to a 10 ml microwave tube. The atmosphere was replaced with nitrogen, and the mixture was reacted with microwave at 110 °C for one hour. The reaction was cooled to room temperature. Water (20 mL) was added to quench the reaction. Ethyl acetate (30 mL × 3) was added for extraction. The organic phase was separated, washed with saturated saline (30 mL), dried with anhydrous sodium sulfate, and filtered. The filtrate was concentrated and passed through a silica gel column to obtain Example 41 with lower polarity, 65 mg of a white solid, yield: 32.8%, LC-MS(APCI):
m/z= 496.0$(M+1)^+$; Example 42 with higher polarity was obtained, 58mg of a white solid, yield: 29.3%, LC-MS(APCI): m/z= 496.0$(M+1)^+$.

## Example 43 Preparation of compound N-(4-(4-amino-1-(6-(hydroxymethyl)tetrahydro-2H-pyran-3-yl)-7-oxo-6,7-dihydro-1H-pyrazolo[3,4-d]pyridazin-3-yl)benzyl)-5-fluoro-2-methoxybenzamide

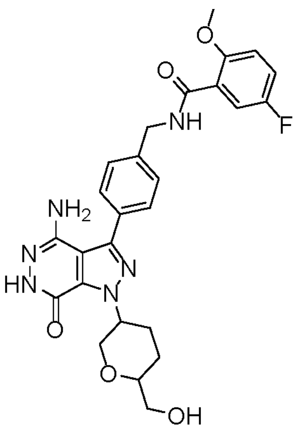

The following synthetic route was adopted

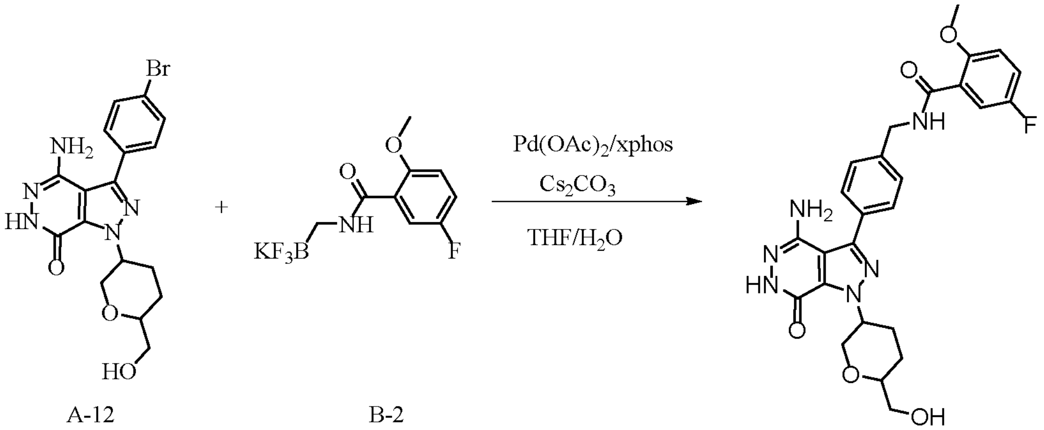


A-12 B-2

THF (10 mL), water (1 mL), intermediate compound A-12 (90 mg, 0.21 mmol), intermediate compound B-2 (80 mg, 0.27 mmol), Xphos (10.2 mg, 0.021 mmol), palladium acetate (2.4 mg, 0.01 mmol) and cesium carbonate (209 mg, 0.64 mmol) were sequentially added to a microwave reaction tube (10-20 mL) equipped with a magnet. The microwave reaction tube was filled with nitrogen and the lid was clamped tightly. The microwave reaction tube was placed in a microwave reactor for reaction at 110 °C for 1 hour. The mixture was cooled to room temperature, directly concentrated and passed through a silica gel column to obtain 32 mg of an off-white solid, yield: 29.2%, LC-MS(APCI): m/z=523.2 $(M+1)^+$.

**Example 44 Preparation of compound N-(4-(4-amino-1-(3-hydroxycyclohexanyl)-7-oxo-6,7-dihydro-1H-pyrazolo[3,4-d]pyridazin-3-yl)benzyl)-5-fluoro-2-methoxybenzamide**

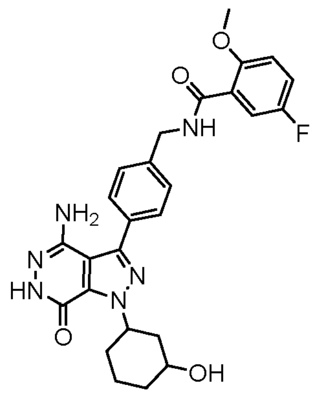

The following synthetic route was adopted

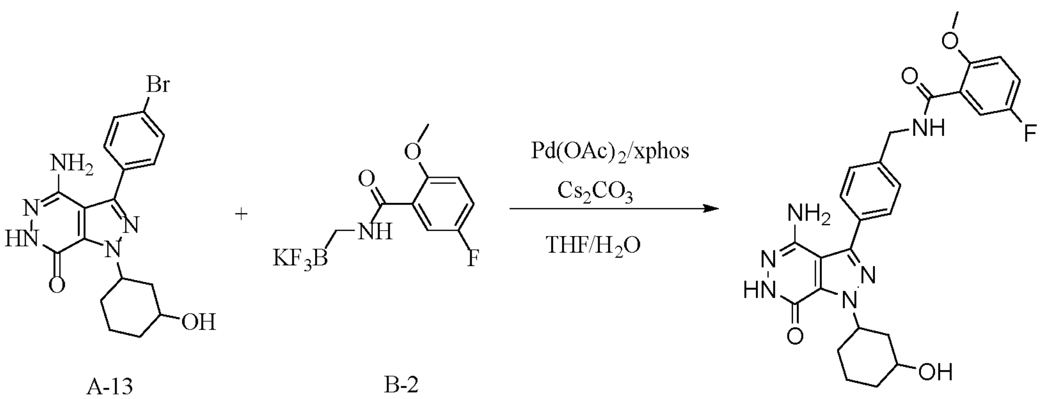


A-13 B-2

Intermediate compound A-13 (156 mg, 0.4 mmol), tetrahydrofuran (7 mL), water (0.7 mL), intermediate compound B-2 (184.5 mg, 0.63 mmol), Pd $(OAc)_2$ (9.5 mg, 0.04 mmol), XPhos (12.3 mg, 0.04 mmol), and cesium

carbonate (416 mg, 0.12 mmol) were added to a 10 ml microwave tube. The atmosphere was replaced with nitrogen, and the mixture was reacted with microwave at 110 °C for one hour. The reaction was cooled to room temperature. Water (20 mL) was added to quench the reaction. Ethyl acetate (30 mL × 3) was added for extraction. The organic phase was separated, washed with saturated saline (30 mL), dried with anhydrous sodium sulfate, and filtered. The filtrate was concentrated and passed through a silica gel column to obtain 124 mg of a white solid, yield: 61.3%, LC-MS(APCI): m/z= 507.0$(M+1)^+$.

**Example 45 Preparation of compound N-(4-(4-amino-1-(6-(hydroxymethyl)tetrahydro-2H-pyran-3-yl)-7-oxo-6,7-dihydro-1H-pyrazolo[3,4-d]pyridazin-3-yl)benzyl)-5-fluoro-2-methoxybenzamide (Isomer 1)**

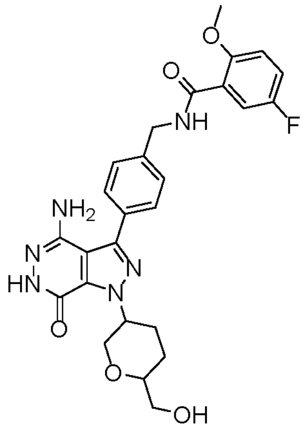

Example 43 (25 mg) was dissolved in methanol (10 mL) and the chiral preparation was carried out using the following conditions:

IG column: CHIRALPAKR IG; 4.6mm*250mmL*5μm;
Flow rate: 4ml/min;
Mobile phase: methanol: methyl tert-butyl ether = 8: 92;

The corresponding fractions were collected, and rotary evaporated to dryness to obtain 2 mg of the target compound. The corresponding retention time was 9.198 min.

**Example 46 Preparation of compound N-(4-(4-amino-1-(6-(hydroxymethyl)tetrahydro-2H-pyran-3-yl)-7-oxo-6,7-dihydro-1H-pyrazolo[3,4-d]pyridazin-3-yl)benzyl)-5-fluoro-2-methoxybenzamide (Isomer 2)**

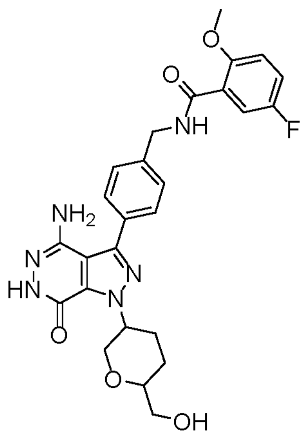

Example 43 (25 mg) was dissolved in methanol (10 mL) and the chiral preparation was carried out using the

following conditions:

IG column: CHIRALPAKR IG; 4.6mm*250mmL*5μm;
Flow rate: 4ml/min;
Mobile phase: methanol: methyl tert-butyl ether=8: 92;

The corresponding fractions were collected, and rotary evaporated to dryness to obtain 2 mg of the target compound. The corresponding retention time was 9.342 min.

**Example 47 Preparation of compound N-(4-(4-amino-1-(6-(hydroxymethyl)tetrahydro-2H-pyran-3-yl)-7-oxo-6,7-dihydro-1H-pyrazolo[3,4-d]pyridazin-3-yl)benzyl)-5-fluoro-2-methoxybenzamide** (Isomer 3)

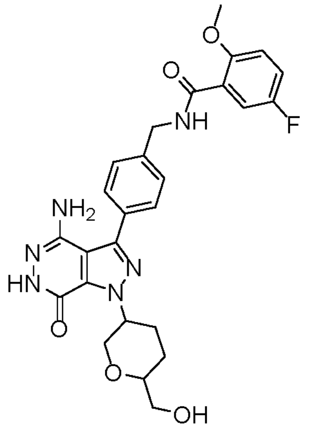

Example 43 (25 mg) was dissolved in methanol (10 mL), and the chiral preparation was carried out using the following conditions:

IG column: CHIRALPAKR IG; 4.6mm*250mmL*5μm;
Flow rate: 4ml/min;
Mobile phase: methanol: methyl tert-butyl ether=8: 92;

The corresponding fractions were collected, and rotary evaporated to dryness to obtain 8 mg of the target compound. The corresponding retention time was 22.452 min. $^1$H NMR (400 MHz, DMSO-$D_6$)δ(ppm): 11.76(s, 1H), 8.87(t, J=6.0 Hz, 1H), 7.62(d, J=8.0 Hz, 2H), 7.53-7.48(m, 3H), 7.36-7.31(m, 1H), 7.20-7.17(m, 1H), 5.49-5.43(m, 1H), 5.07(s, 2H), 4.70(t, J=6.0 Hz, 1H), 4.57(d, J=6.0 Hz, 2H), 4.08-4.04(m, 1H), 3.89(s, 3H), 3.64(t, J=10.8 Hz, 1H), 3.44-2.35(m, 3H), 2.27-2.20(m, 2H), 1.85-1.82(m, 1H), 1.46-1.42(m, 1H).

**Example 48 Preparation of compound N-(4-(4-amino-1-(6-(hydroxymethyl)tetrahydro-2H-pyran-3-yl)-7-oxo-6,7-dihydro-1H-pyrazolo[3,4-d]pyridazin-3-yl)benzyl)-5-fluoro-2-methoxybenzamide (Isomer 4)**

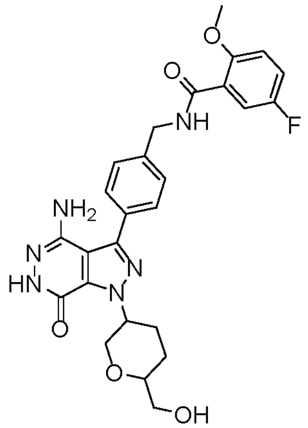

Example 43 (25 mg) was dissolved in methanol (10 mL), and the chiral preparation was carried out using the following conditions:

IG column: CHIRALPAKR IG; 4.6mm*250mmL*5μm;
Flow rate: 4ml/min;
Mobile phase: methanol: methyl tert-butyl ether=8: 92;

The corresponding fractions were collected, and rotary evaporated to dryness to obtain 8 mg of the target compound. The corresponding retention time was 27.243 min. $^1$H NMR (400 MHz, DMSO-$D_6$)δ(ppm): 11.76(s, 1H), 8.87(t, J=6.0 Hz, 1H), 7.62(d, J=8.0 Hz, 2H), 7.53-7.48(m, 3H), 7.36-7.31(m, 1H), 7.20-7.17(m, 1H), 5.49-5.43(m, 1H), 5.07(s, 2H), 4.70(t, J=6.0 Hz, 1H), 4.57(d, J=6.0 Hz, 2H), 4.08-4.04(m, 1H), 3.89(s, 3H), 3.64(t, J=10.8 Hz, 1H), 3.44-2.35(m, 3H), 2.27-2.20(m, 2H), 1.85-1.82(m, 1H), 1.46-1.42(m, 1H).

**Example 49 Preparation of compound trans-N-(4-(4-amino-1-(4-hydroxycyclopent-2-en-1-yl)-1H-pyrazolo[3,4-d]pyrimidin-3-yl)benzyl)-5-fluoro-2-methoxybenzamide (Isomer 1), and**

**Example 50 Preparation of compound trans-N-(4-(4-amino-1-(4-hydroxycyclopent-2-en-1-yl)-1H-pyrazolo[3,4-d]pyrimidin-3-yl)benzyl)-5-fluoro-2-methoxybenzamide (Isomer 2)**

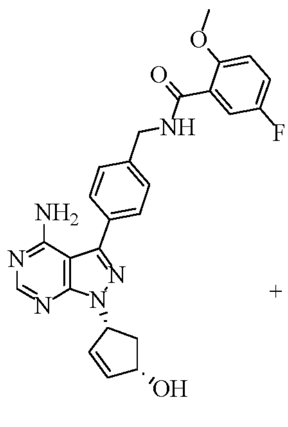

+

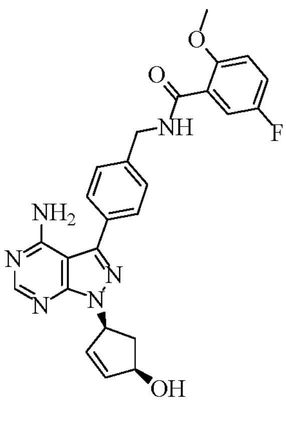

**The following synthetic route was adopted**

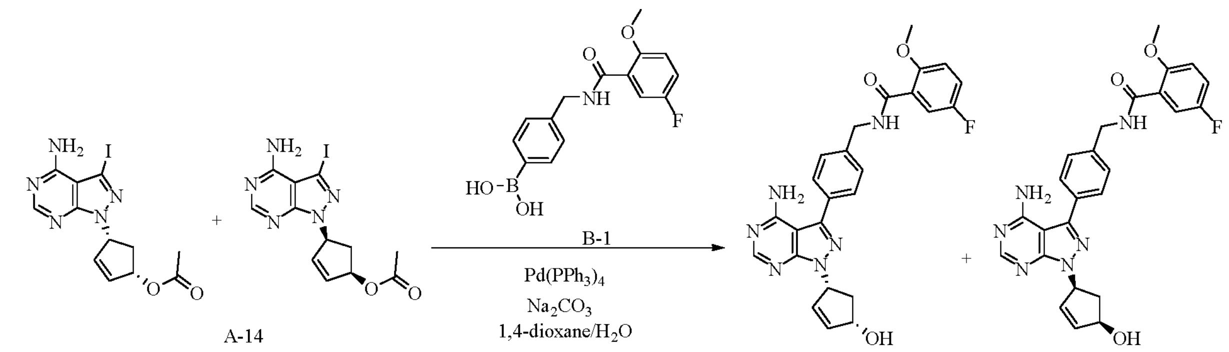


Intermediate A-14 (400 mg, 1.039 mmol), Intermediate B-1 (377.8 mg, 1.247 mmol), sodium carbonate (330.4 mg, 3.117 mmol), tetrakistriphenylphosphine palladium (120.1 mg, 0.104 mmol), 1,4-dioxane (8 mL) and water (2 mL) were added to a 50 mL three-necked flask equipped with magnetic stirring. The flask was evacuated and the atmosphere was replaced with nitrogen three times. The reaction was stirred at 100 °C under nitrogen atmosphere for 12 hours. The reaction was cooled to room temperature. Water (50 mL) and ethyl acetate (50 mL × 2) were added for extraction. The organic phases were combined, washed with saturated saline (50 mL), dried with anhydrous sodium sulfate, and filtered. The filtrate was concentrated and passed through a silica gel column to obtain Example 49 with lower polarity, 20 mg of a white solid, LC-MS(APCI): m/z=475.0(M+1)$^+$, $^1$H NMR (400 MHz, DMSO-$d_6$): 8.87(t, J=6.0 Hz, 1H), 8.25(s, 1H), 7.63(d, J=8.0 Hz, 2H), 7.54-7.49(m, 3H), 7.37-7.32(m, 1H), 7.21-7.17(m, 1H), 6.08-6.06(m, 1H), 5.92-5.90(m, 1H), 5.75(t, J=10.4 Hz, 1H), 5.27(d, J=6.8 Hz, 1H), 4.74-4.71(m, 1H), 4.56(d, J=6.0 Hz, 2H), 4.04-3.98(m, 1H), 3.90(s, 3H), 2.84-2.81(m, 1H), 2.02-1.95(m, 1H).

Example 50 with higher polarity was obtained, 80 mg of a white solid, LC-MS(APCI): m/z=475.0(M+1)$^+$, $^1$H NMR (400 MHz, DMSO-$d_6$): 8.85(t, J=6.0 Hz, 1H), 8.24(s, 1H), 7.59(d, J=8.8 Hz, 2H), 7.53-7.47(m, 3H), 7.35-7.30(m, 1H), 7.19-7.16(m, 1H), 6.10-6.05(m, 2H), 5.94-5.92(m, 1H), 4.99-4.97(m, 2H), 4.56(d, J=6.0 Hz, 2H), 3.98(s, 3H), 2.43-2.39(m, 1H), 2.17-2.13(m, 1H).

**Example 51 Preparation of compound N-(4-(4-amino-7-(6-(hydroxymethyl)tetrahydro-2H-pyran-3-yl)-5H-pyrrolo[3,2-d]pyrimidin-5-yl)benzyl)-5-fluoro-2-methoxybenzamide (Isomer 1), and**

**Example 52 Preparation of compound N-(4-(4-amino-7-(6-(hydroxymethyl)tetrahydro-2H-pyran-3-yl)-5H-pyrrolo[3,2-d]pyrimidin-5-yl)benzyl)-5-fluoro-2-methoxybenzamide (Isomer 2)**

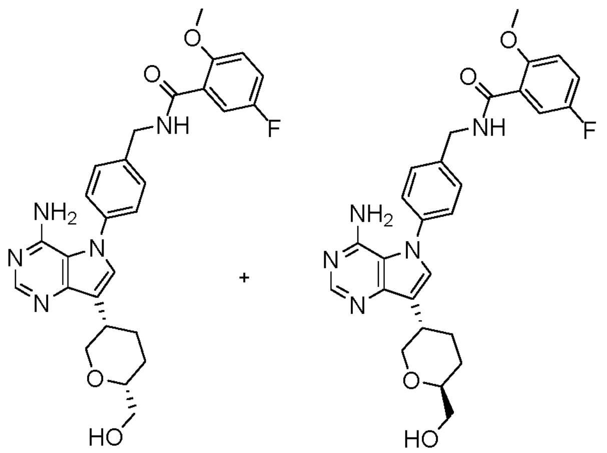

**The following synthetic route was adopted**

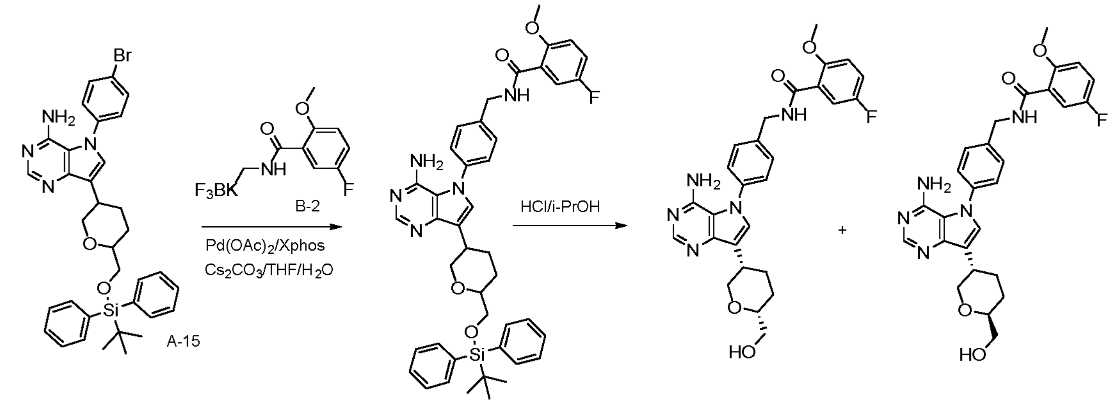


Step 1 Synthesis of compound N-(4-(4-amino-7-(6-(((tert-butyldiphenylsilyl)oxy)methyl)tetrahydro-2H-pyran-3-yl)-5H-pyrrolo[3,2-d]pyrimidin-5-yl)benzyl)-5-fluoro-2-methoxybenzamide

THF (10 mL), water (1 mL), intermediate compound A-15 (55 mg, 0.081 mmol), intermediate compound B-2 (30 mg, 0.106 mmol), Xphos (4 mg, 0.008 mmol), palladium acetate (1.83 mg, 0.008 mmol) and cesium carbonate (80 mg, 0.24 mmol) were added in sequence to a microwave reaction tube (10-20 mL) equipped with a magnet. The microwave reaction tube was filled with nitrogen and the lid was clamped tightly. The microwave reaction tube was placed in the microwave reactor at 110 °C, and reacted for 1 hour. The mixture was cooled to room temperature, directly concentrated and passed through a silica gel column to obtain 54 mg of an off-white solid, yield: 90 %, LC-MS(APCI): m/z=744 $(M+1)^+$.

Step 2 Synthesis of Example 51 Compound (Isomer 1) and Example 52 Compound (Isomer 2)

Hydrochloric acid solution in isopropanol (5 mol/L, 5 mL) and N-(4-(4-amino-7-(6-(((tert-butyldiphenylsilyl)oxy)methyl)tetrahydro-2H-pyran-3-yl)-5H-pyrrolo[3,2-d]pyrimidin-5-yl)benzyl)-5-fluoro-2-methoxybenzamide (54 mg, 0.072 mmol) were sequentially added to a 25 mL single-necked flask equipped with magnetic stirring and stirred at room temperature for 1 hour. The pH was adjusted to 7 with saturated aqueous sodium bicarbonate solution. Ethyl acetate (20 mL $\times$ 3) and water (10 mL) were added for extraction. The organic phase was separated, washed with saturated saline (15 mL), dried with anhydrous sodium sulfate, and filtered. The filtrate was concentrated and passed through a silica gel column to obtain Example 51 with lower polarity, 10 mg, yield: 27.4 %, a yellow solid, LC-MS(APCI): m/z=506 $(M+1)^+$, $^1$H NMR (400 MHz, DMSO-$D_6$) $\delta$ (ppm): 8.88 (t, J=6.0 Hz, 1H), 8.19 (s, 1H), 7.52-7.48 (m, 4H), 7.41 (d, J=8.4 Hz, 2H), 7.34-7.31 (m, 1H), 7.20-7.16 (m, 1H), 5.71 (br s, 2H), 4.61 (t, J=6.4 Hz, 1H), 4.57 (d, J=5.6 Hz, 2H), 4.12-4.09 (m, 1H), 3.89 (s, 3H), 3.44-3.38 (m, 2H), 3.35-3.33 (m, 1H), 3.08-3.00 (m, 1H), 2.11-2.05 (m, 1H), 1.88-1.82 (m, 1H), 1.74-1.70 (m, 1H), 1.39-1.32, (m, 1H).

Example 52 with higher polarity was obtained, 8 mg, yield: 22.0 %, LC-MS(APCI): m/z=506 $(M+1)^+$. $^1$H NMR (400 MHz, DMSO-$D_6$) $\delta$ (ppm): 8.89 (t, J=6.0 Hz, 1H), 8.20 (s, 1H), 7.58 (s, 1H), 7.53-7.50 (m, 3H), 7.43 (d, J=8.0 Hz, 2H), 7.36-7.31 (m, 1H), 7.20-7.16 (m, 1H), 5.76 (br s, 2H), 4.59-4.56 (m, 3H), 4.12 (d, J=10.4 Hz, 1H), 3.89 (s, 3H), 3.75 (dd, J=10.4 Hz, J=2.4 Hz, 1H), 3.41-3.34 (m, 3H), 3.22-3.20 (m, 1H), 2.02-1.96 (m, 2H), 1.52-1.39 (m, 2H).

**Example 53 Preparation of compound N-(4-(6-amino-9-(3-hydroxycyclohexyl)-8-oxo-8,9-dihydro-7H-purin-7-yl)benzyl)-5-fluoro-2-methoxybenzamide**

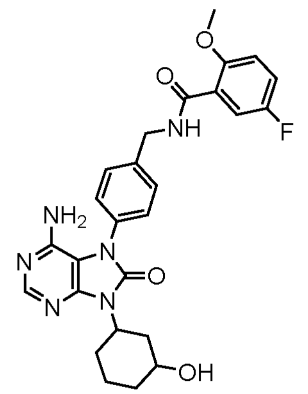

<u>**The following synthetic route was adopted**</u>

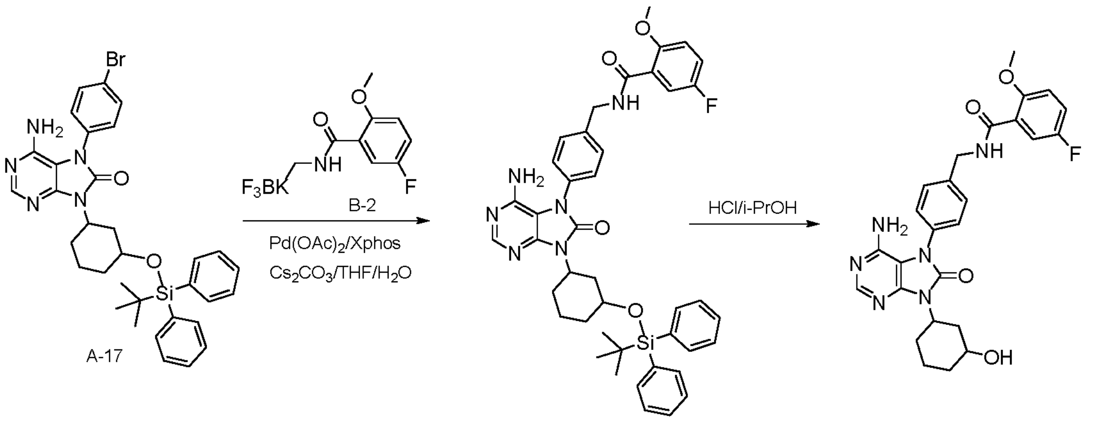


Step 1 Synthesis of compound N-(4-(6-amino-9-(3-((tert-butyldiphenylsilyl)oxy)cyclohexyl)-8-oxo-8,9-dihydro-7H-purin-7-yl)benzyl)-5-fluoro-2-methoxybenzamide

THF (10 mL), water (1 mL), intermediate compound A-17 (15 mg, 0.023 mmol), intermediate compound B-2 (9 mg, 0.03 mmol), Xphos (1.0 mg, 0.0023 mmol), palladium acetate (0.5 mg, 0.0023 mmol) and cesium carbonate (22.5 mg, 0.069 mmol) were sequentially added to a microwave reaction tube (10-20 mL) equipped with a magnet. The lid of the microwave reaction tube was clamped. The microwave reaction tube was placed in a microwave reactor at 110 °C, and reacted for 1 hour. The mixture was cooled to room temperature, directly concentrated and passed through a silica gel column to obtain 12 mg of an off-white solid, yield: 70 %, LC-MS(APCI): m/z=745 $(M+1)^+$.

Step 2 Synthesis of Example 53 Compound

Hydrochloric acid solution in isopropanol (5 mol/L, 5 mL) and N-(4-(6-amino-9-(3-((tert-butyldiphenylsilyl)oxy)cyclohexyl)-8-oxo-8,9-dihydro-7H-purin-7-yl)benzyl)-5-fluoro-2-methoxybenzamide (12 mg, 0.016 mmol) were sequentially added to a 25 mL single-necked flask equipped with a magnetic stirrer and stirred at room temperature for 1 hour. The pH was adjusted to 7 with saturated aqueous sodium bicarbonate solution. Ethyl acetate (20 mL × 3) and water (10 mL) were added for extraction. The organic phase was separated, washed with saturated saline (15 mL), dried with anhydrous sodium sulfate, and filtered. The filtrate was concentrated and passed through a silica gel column to obtain 5 mg of a white solid, yield: 61 %. LC-MS(APCI): m/z=507 $(M+1)^+$.

<u>**Example 54 Preparation of compound N-(4-(6-amino-9-((1R,4R)-4-(hydroxymethyl)cyclohexyl)-8-oxo-8,9-dihydro-7H-purin-7-yl)benzyl)-5-fluoro-2-methoxybenzamide**</u>

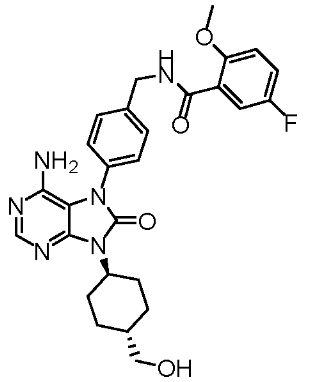

**The following synthetic route was adopted**

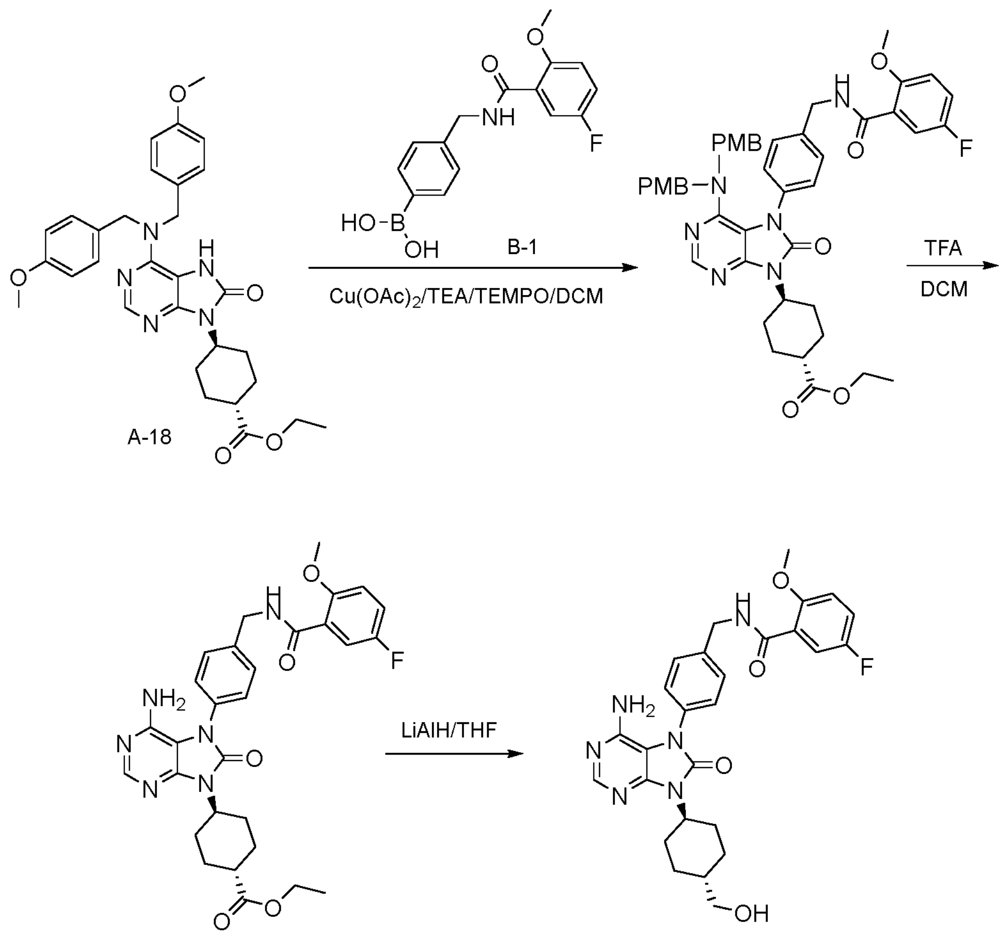


Step 1 Synthesis of compound ethyl (1R,4R)-4-(6-(bis(4-methoxybenzyl)amino)-7-(4-((5-fluoro-2-methoxybenzamido) methyl)phenyl)-8-oxo-7,8-dihydro-9H-purin-9-yl)cyclohexane-1-carboxylate

DCM (15 mL), intermediate compound A-18 (7.7 g, 14.1 mmol), intermediate compound B-1 (5.5 g, 18.3 mmol), copper acetate (1.28 g, 7.0 mmol), TEMPO (2.4 g, 15.5 mmol) and TEA (5.7 g, 56.5 mmol) were sequentially added to a 50 mL single-necked flask equipped with magnetic stirring. The flask was evacuated and the atmosphere was replaced with oxygen three times. The mixture was reacted overnight at room temperature. The reaction mixture was concentrated directly and passed through a silica gel column to obtain 1.13 g of a yellow oil, yield: 10 %, LC-MS(APCI): m/z=803 $(M+1)^+$.

Step 2 Synthesis of compound ethyl (1R,4R)-4-(6-amino-7-(4-((5-fluoro-2-methoxybenzamido)methyl)phenyl)-8-oxo-7,8-dihydro-9H-purin-9-yl)cyclohexane-1-carboxylate

DCM (5 mL), ethyl (1R,4R)-4-(6-(bis(4-methoxybenzyl)amino)-7-(4-((5-fluoro-2-methoxybenzamido)methyl) phenyl)-8-oxo-7,8-dihydro-9H-purin-9-yl)cyclohexane-1-carboxylate (1.13 g, 1.4 mmol) and TFA (5 mL) were sequentially added to a 50 mL single-necked flask equipped with magnetic stirring, warmed to 50 °C, and reacted for 4 hours. pH was adjusted to 7 with saturated aqueous sodium bicarbonate solution. The mixture was extracted with ethyl acetate (30 mL × 3) and water (15 mL), dried with anhydrous sodium sulfate, and filtered. The filtrate was concentrated and passed through a silica gel column to obtain 0.34 g of a yellow oil, yield: 43.0 %. LC-MS(APCI): m/z=563 $(M+1)^+$.

Step 3 Synthesis of Example 54 Compound

Anhydrous tetrahydrofuran (10 mL) and ethyl (1R,4R)-4-(6-amino-7-(4-((5-fluoro-2-methoxybenzoylamino) methyl)phenyl)-8-oxo-7,8-dihydro-9H-purin-9-yl)cyclohexane-1-carboxylate (340 mg, 0.6 mmol) were sequentially added to a 25 mL two-necked flask equipped with magnetic stirring. The atmosphere was replaced with nitrogen three times. The mixture was cooled to 0 °C. Lithium aluminum hydride (91 mg, 2.4 mmol) was added, and reacted at 0 °C for 1 hour. Water (30 mL) was added to quench the reaction, and the mixture was filtered. The filtrate was washed with ethyl acetate (20 mL). The organic phase was separated, and the aqueous phase was extracted with ethyl acetate (30 mL × 3). The organic phases were combined, washed with saturated saline (100 mL), dried with anhydrous sodium sulfate, and filtered. The filtrate was concentrated and passed through a silica gel column to obtain 165 mg of a white solid, yield: 52 %, LC-MS(APCI): m/z=521 $(M+1)^+$. $^1H$ NMR (400 MHz, DMSO-$D_6$) δ (ppm): 8.89 (t, J=6.0 Hz, 1H), 8.13 (s, 1H), 7.54-7.49 (m, 3H), 7.43-7.41 (m, 2H), 7.37-7.32 (m, 1H), 7.21-7.17 (m, 1H), 5.57 (br s, 2H), 4.58 (d, J=6.0 Hz, 2H), 4.45 (t, J=6.0 Hz, 1H), 4.24-4.18 (m, 1H), 3.90 (s, 2H), 3.28-3.24 (m, 2H), 2.38-2.29 (m, 2H), 1.87 (d, J=12.8 Hz, 2H), 1.78 (d, J=10.4 Hz, 2H), 1.45-1.42 (m, 1H), 1.10-1.01 (m, 2H).

**Example 55 Preparation of compound cis-N-(4-(4-amino-1-((1S,4S)-4-(hydroxymethyl)cyclohexyl)-1H-pyrazolo [3,4-d]pyrimidin-3-yl)benzyl)-5-fluoro-2-methoxybenzamide**

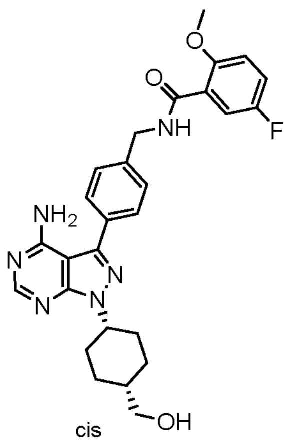

**The following synthetic route was adopted**

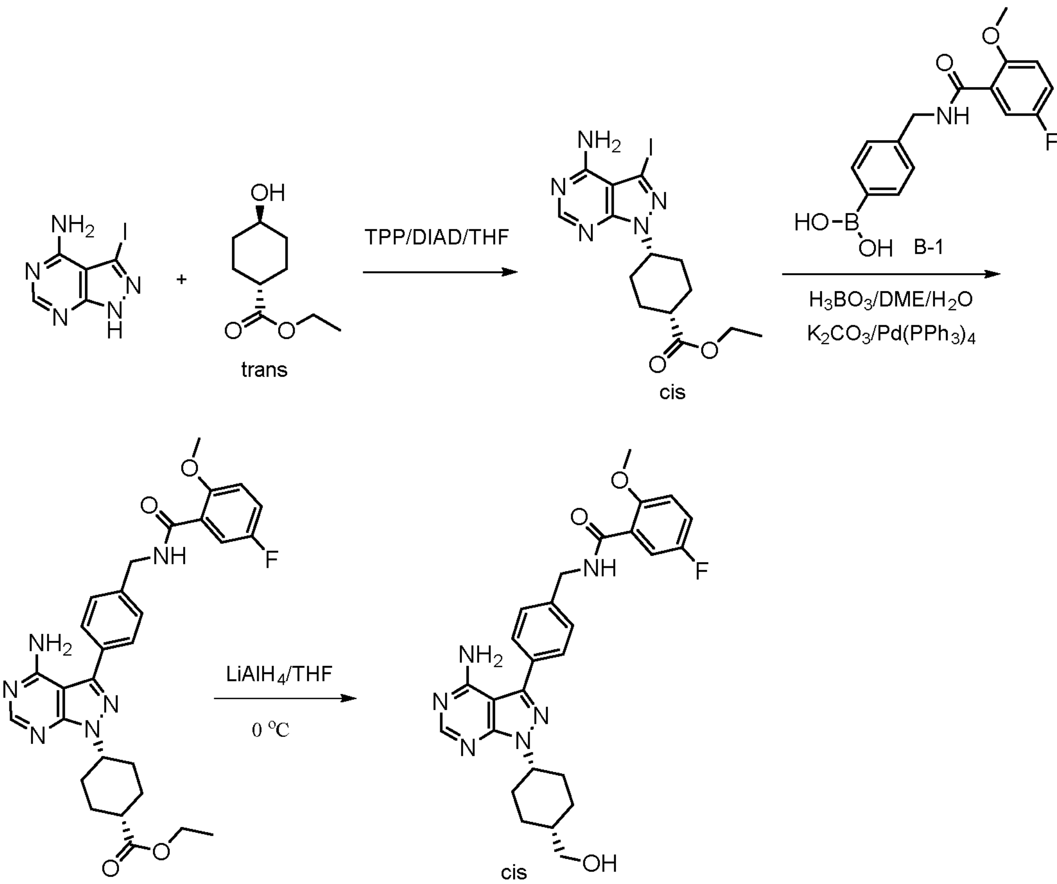


Step 1 Preparation of compound ethyl cis-(1S,4S)-4-(4-amino-3-iodo-1H-pyrazolo[3,4-d]pyrimidin-1-yl)cyclohexyl-1-carboxylate

Compound 3-iodo-1H-pyrazolo[3,4-d]pyrimidin-4-amine (2.0 g, 7.66 mmol), ethyl trans-4-hydroxycyclohexylcarboxylate (1.45 g, 8.43 mmol) and dry THF (30 mL) were sequentially added to a 100 mL three-necked flask equipped with magnetic stirring, and stirred until dissolved. $Ph_3P$ (4.01 g, 15.32 mmol) was added, and the mixture was cooled to 0 °C. DIAD (3.09 g, 15.32 mmol) was added dropwise slowly, and stirred overnight at room temperature after the completion of addition. Saturated saline (50 mL) was added to quench the reaction. The organic phase was separated, and the aqueous phase was extracted with ethyl acetate (50 mL × 2). The organic phases were combined, washed with saturated saline (50 mL), dried with anhydrous sodium sulfate, and filtered. The filtrate was concentrated and passed through a silica gel column to obtain 2.5 g of the titled product, yield: 78.6 %, LC-MS(APCI): m/z=416.0 $(M+1)^+$.

Step 2 Synthesis of compound ethyl (1s,4s)-4-(4-amino-3-(4-((5-fluoro-2-methoxybenzamido)methyl)phenyl)-1H-pyrazolo[3,4-d]pyrimidin-1-yl)cyclohexane-1-carboxylate

Ethyl cis-(1S,4S)-4-(4-amino-3-iodo-1H-pyrazolo[3,4-d]pyrimidin-1-yl)cyclohexyl-1-carboxylate (1 g, 2.41 mmol) and boronic acid (2.89 mmol, 876 mg) were dissolved in 30 mL of DME/$H_2O$ (V/V = 3/1). $K_2CO_3$ (7.23 mmol, 997 mg) was added, and then Pd($PPh_3$)$_4$ (0.241 mmol, 278 mg) and intermediate compound B-1 (1.1 g, 3.62 mmol) were quickly added. The atmosphere was replaced with nitrogen three times. The mixture was heated to 100 °C, and stirred for 4 hours. The reaction mixture was cooled to room temperature. Dichloromethane (40 mL) and water (30 mL) were added and stirred. The layers were separated. The organic phase was separated, and the aqueous phase was extracted with dichloromethane (20 mL × 2). The organic phases were combined, washed with saturated saline (20 mL), dried with anhydrous sodium sulfate, and filtered. The filtrate was concentrated and passed through a silica gel column to obtain 630 mg of the target product, yield: 48.1 %. LC-MS(APCI): m/z=547.2 $(M+1)^+$.

Step 3 Synthesis of Example 55 Compound

Compound (1S,4S)-ethyl 4-(4-amino-3-(4-((5-fluoro-2-methoxybenzoylamino)methyl)phenyl)-1H-pyrazolo [3,4-d]pyrimidin-1-yl)cyclohexane-1-carboxylate (300 mg, 0.548 mmol) was dissolved in 20 mL of anhydrous THF.

Compound $LiAlH_4$ (1.10 mmol, 42 mg) was added at 0 °C. After the addition was completed, the mixture was stirred while maintaining the temperature for 2 hours. Sodium sulfate decahydrate (10 g) was added under ice-water bath to quench the reaction. Ethyl acetate (20 mL) was added, and the mixture was filtered. The filtrate was concentrated and passed through a silica gel column to obtain 1 10 mg of the target product, yield: 39.7 %, LC-MS(APCI): m/z=505.1 $(M+1)^+$. $^1H$ NMR (400 MHz, MeOH-$d_4$) δ (ppm): 8.86 (t, J=6.0 Hz, 1H), 8.22 (s, 1H), 7.62 (d, J=8.0 Hz, 2H), 7.53-7.48 (m, 3H), 7.35-7.31 (m, 1H), 7.20-7.16 (m, 1H), 4.76-4.74 (m, 1H), 4.57 (d, J=6.0 Hz, 2H), 4.52-4.46 (m, 1H), 3.91 (s, 3H), 3.47 (d, J=6.8 Hz, 2H), 2.13-2.08 (m, 2H), 1.82-1.73 (m, 5H), 1.65-1.60 (m, 1H).

**Example 56 Preparation of compound trans-N-(4-(4-amino-1-((1R,4R)-4-(hydroxymethyl)cyclohexyl)-1H-pyrazolo[3,4-d]pyrimidin-3-yl)benzyl)-5-fluoro-2-methoxybenzamide**

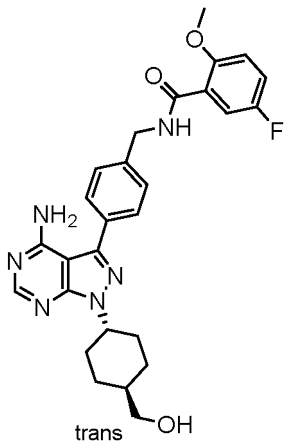

According to the synthetic route of Example 55, the raw material ethyl trans-4-hydroxycyclohexylcarboxylate was replaced with ethyl cis-4-hydroxycyclohexylcarboxylate to obtain the compound of Example 56 in trans configuration, LC-MS(APCI): m/z=505.1 $(M+1)^+$. $^1H$ NMR (400 MHz, MeOH-$d_4$) δ (ppm): 8.86 (t, J=6.0 Hz, 1H), 8.22 (s, 1H), 7.62 (d, J=8.0 Hz, 2H), 7.53-7.48 (m, 3H), 7.35-7.31 (m, 1H), 7.20-7.16 (m, 1H), 4.65-4.60 (m, 1H), 4.57 (d, J=6.0 Hz, 2H), 4.51-4.45 (m, 1H), 3.89 (s, 3H), 3.38-3.25 (m, 2H), 1.99-1.87 (m, 6H), 1.43-1.41 (m, 1H), 1.15-1.11 (m, 2H).

**Example 57 Preparation of compound N-(4-(4-amino-1-(3-hydroxycycloheptyl)-1H-pyrazolo[3,4-d]pyrimidin-3-yl)benzyl)-5-fluoro-2-methoxybenzamide**

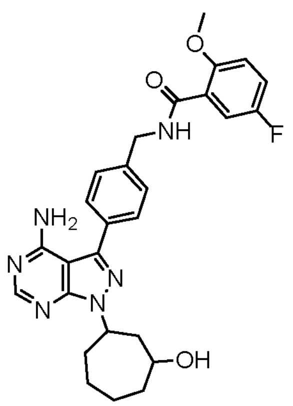

**The following synthetic route was adopted**

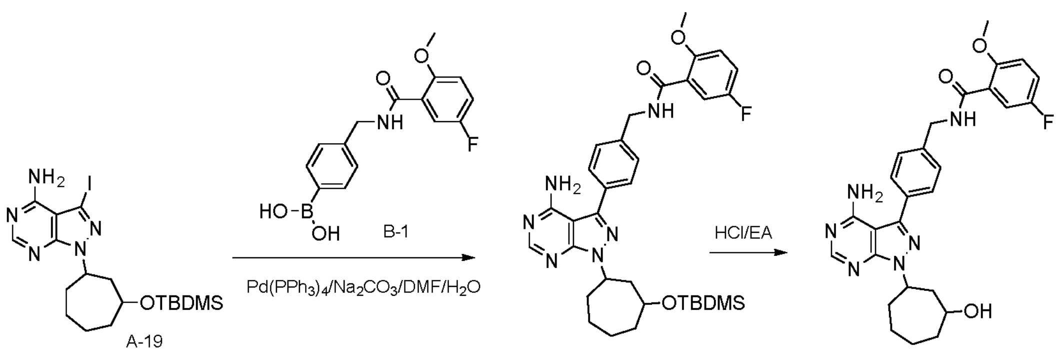


Step 1 Synthesis of compound N-(4-(4-amino-1-(3-((tertbutyldimethylsilyl)oxy)cycloheptyl)-1H-pyrazolo[3,4-d]pyrimidin-3-yl)benzyl)-5-fluoro-2-methoxybenzamide

Intermediate compound A-19 (400 mg, 0.82 mmol), intermediate compound B-1 (300 mg, 0.99 mmol), $Pd(PPh_3)_4$ (95 mg, 0.082 mmol), $Na_2CO_3$ (261 mg, 2.46 mmol), DMF (6 mL), and $H_2O$ (2 mL) were sequentially added to a 50 mL three-necked flask equipped with magnetic stirring. The system was evacuated. The atmosphere was replaced with $N_2$ three times. The mixture was stirred at 100 °C for 6 hours. The reaction was cooled to room temperature. The reaction was quenched by adding saturated saline (10 mL), and extracted with ethyl acetate (10 mL × 2). The organic phases were combined, washed with saturated saline (10 mL), dried with anhydrous sodium sulfate, and filtered. The filtrate was concentrated and passed through a silica gel column to obtain 410 mg of a white solid, yield: 82.8 %. LC-MS(APCI): m/z=619.0 $(M+1)^+$.

Step 2 Synthesis of Example 57 Compound

N-(4-(4-amino-1-(3-((tert-butyldimethylsilyl)oxy)cycloheptyl)-1H-pyrazolo[3,4-d]pyrimidin-3-yl)benzyl)-5-fluoro-2-methoxybenzamide (200 mg, 0.32 mmol) and HCl in ethyl acetate (4 M, 4 mL) were sequentially added to a 50 mL three-necked flask equipped with magnetic stirring, and stirred at room temperature for 1 hour. The mixture was cooled to 0 °C. Saturated $Na_2CO_3$ was added to adjust the pH to 10, and the mixture was extracted with ethyl acetate(10 mL x2). The organic phases were combined, washed with saturated saline (10 mL), dried with anhydrous sodium sulfate, and filtered. The filtrate was concentrated and passed through a silica gel column to obtain 120 mg of a white solid, yield: 74.4 %. LC-MS(APCI): m/z=505.0 $(M+1)^+$. $^1H$ NMR (400MHz, DMSO-$D_6$) δ (ppm): 8.87 (t, J=6.0 Hz, 1H), 8.23 (s, 1H), 7.63 (d, J=8.0 Hz, 2H), 7.54-7.49 (m, 3H), 7.35-7.30 (m, 1H), 7.21-7.18 (m, 1H), 4.88-4.85 (m, 1H), 4.61 (d, J=4.4 Hz, 1H), 4.58 (d, J=6.0 Hz, 2H), 3.91 (s, 3H), 2.32-2.22 (m, 2H), 2.12-2.05 (m, 2H), 1.98-1.90 (m, 3H), 1.76-1.73 (m, 1H), 1.63-1.53 (m, 3H).

**Example 58 Preparation of compound N-(4-(4-amino-7-(6-(hydroxymethyl)tetrahydro-2H-pyran-3-yl)pyrrolo[2,1-f][1,2,4]triazin-5-yl)benzyl)-5-fluoro-2-methoxybenzamide**

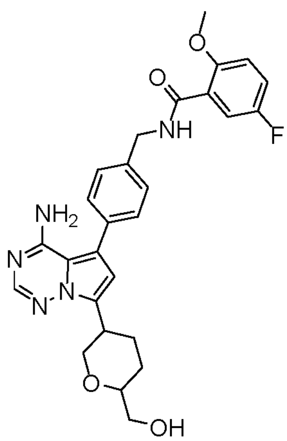

**The following synthetic route was adopted**

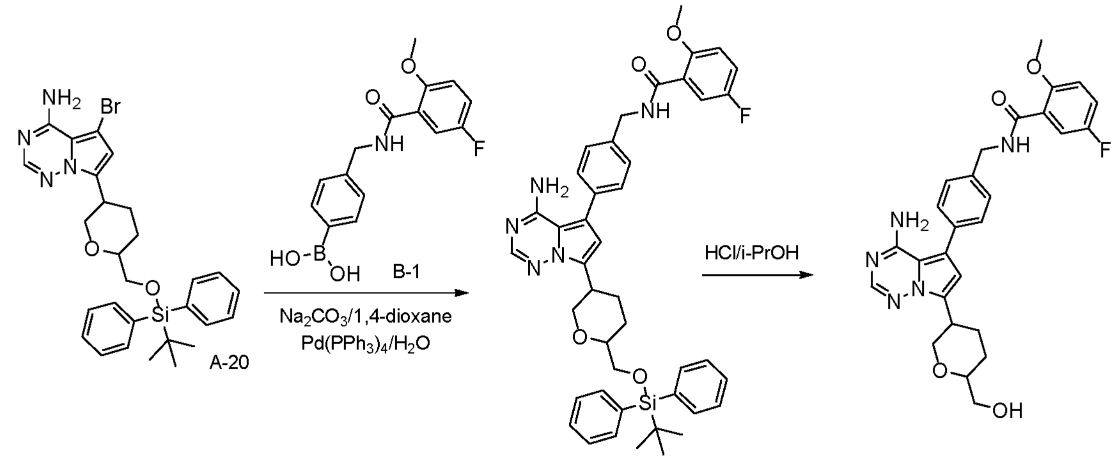

A-20

Step 1 Synthesis of compound N-(4-(4-amino-7-(6-(((tert-butyldiphenylsilyl)oxy)methyl)tetrahydro-2H-pyran-3-yl)pyrrolo[2,1-f][1,2,4]triazin-5-yl)benzyl)-5-fluoro-2-methoxybenzamide

1,4-Dioxane (20 mL), water (5 mL), intermediate compound A-20 (0.4 g, 0.7 mmol), intermediate compound B-1 (0.28 mg, 0.9 mmol), tetrakistriphenylphosphine palladium (0.16 g, 0.14 mmol), and sodium carbonate (0.23 g, 2.0 mmol) were sequentially charged to a 25 mL three-necked flask equipped with magnetic stirring. The atmosphere was replaced with $N_2$ three times. The mixture was heated to 100 °C, and reacted overnight. Ethyl acetate (80 mL × 3) and water (50 mL) were added for extraction. The organic phase was separated, washed with saturated saline (50 mL), dried with anhydrous sodium sulfate, and filtered. The filtrate was concentrated and passed through a silica gel column to obtain 0.43 g of a yellow oil, yield: 82 %. LC-MS(APCI): m/z=744 $(M+1)^+$.

Step 2 Synthesis of Example 58 Compound

Hydrochloric acid solution in isopropanol (5 mL), and N-(4-(4-amino-7-(6-(((tert-butyldiphenylsilyl)oxy)methyl) tetrahydro-2H-pyran-3-yl)pyrrolo[2,1-f][1,2,4]triazin-5-yl)benzyl)-5-fluoro-2-methoxybenzamide (430 mg, 0.57 mmol) were sequentially added to a 25 mL single-necked flask equipped with magnetic stirring, and stirred at room temperature for 1 hour. The pH was adjusted to 7 with saturated aqueous sodium bicarbonate solution. Ethyl acetate (20 mL × 3) and water (10 mL) were added for extraction. The organic phase was separated, washed with saturated saline (15 mL), dried with anhydrous sodium sulfate, and filtered. The filtrate was concentrated and passed through a silica gel column to obtain 119 mg of a yellow solid, yield: 41 %. LC-MS(APCI): m/z=506 $(M+1)^+$.

**Example 59 Preparation of compound N-(4-(4-amino-7-(6-(hydroxymethyl)tetrahydro-2H-pyran-3-yl)pyrrolo [2,1-f][1,2,4]triazin-5-yl)benzyl)-5-fluoro-2-methoxybenzamide (Isomer 1)**

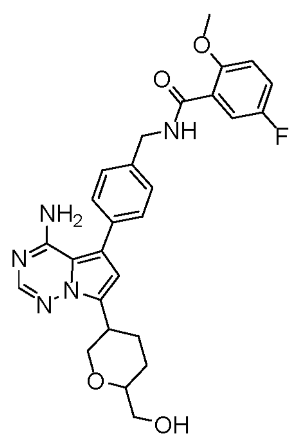

Example 58 (150 mg) was dissolved in methanol (15 mL), and the chiral preparation was carried out using the

following conditions:

AD-H column: CHIRALPAKR AD-H; 4.6mm*250mmL*5μm;
Flow rate: 4 ml/min;
Mobile phase: (ethyl acetate: n-hexane: ethanol=18: 72: 10) + 0.1 % triethylamine;

The target compound with a retention time of 28.923 min and a mass of 31 mg was obtained. LC-MS(APCI): m/z=506 $(M+1)^+$. $^1H$ NMR (400 MHz, DMSO-$D_6$): 8.84 (t, J=6.0 Hz, 1H), 7.89 (s, 1H), 7.53-7.49 (m, 1H), 7.43 (s, 4H), 7.36-7.30 (m, 1H), 7.19-7.16 (m, 1H), 6.77 (s, 1H), 4.59 (br s, 1H), 4.54 (d, J=6.4 Hz, 2H), 4.18 (d, J=11.2 Hz, 1H), 3.89 (s, 3H), 3.81-3.78 (m, 1H), 3.41-3.39 (m, 3H), 2.01-1.87 (m, 3H), 1.44-1.38 (m, 2H).

**Example 60 Preparation of compound N-(4-(4-amino-7-(6-(hydroxymethyl)tetrahydro-2H-pyran-3-yl)pyrrolo [2,1-f][1,2,4]triazin-5-yl)benzyl)-5-fluoro-2-methoxybenzamide (Isomer 2)**

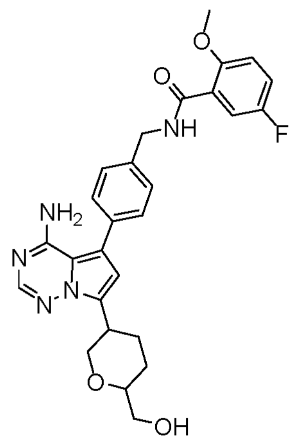

Example 58 (150 mg) was dissolved in methanol (15 mL), and the chiral preparation was carried out using the following conditions:

AD-H column: CHIRALPAKR AD-H; 4.6mm*250mmL*5μm;
Flow rate: 4 ml/min;
Mobile phase: (ethyl acetate: n-hexane: ethanol=18: 72: 10) + 0.1 % triethylamine;

The target compound with a peak time of 31.673 min and a mass of 30 mg was obtained, LC-MS(APCI): m/z=506 $(M+1)^+$. $^1H$ NMR (400 MHz, DMSO-$D_6$): 8.84 (t, J=6.0 Hz, 1H), 7.89 (s, 1H), 7.53-7.49 (m, 1H), 7.43 (s, 4H), 7.36-7.30 (m, 1H), 7.19-7.16 (m, 1H), 6.77 (s, 1H), 4.59 (br s, 1H), 4.54 (d, J=6.4 Hz, 2H), 4.13 (d, J=11.6 Hz, 1H), 3.89 (s, 3H), 3.81-3.78 (m, 1H), 3.41-3.39 (m, 3H), 2.01-1.87 (m, 3H), 1.44-1.38 (m, 2H).

**Example** 61 **Preparation of compound N-(4-(4-amino-7-(6-(hydroxymethyl)tetrahydro-2H-pyran-3-yl)pyrrolo [2,1-f][1,2,4]triazin-5-yl)benzyl)-5-fluoro-2-methoxybenzamide (Isomer 3)**

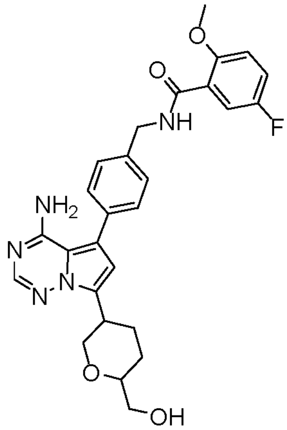

Example 58 (150 mg) was dissolved in methanol (15 mL), and the chiral preparation was carried out using the following conditions:

AD-H column: CHIRALPAKR AD-H; 4.6mm*250mmL*5μm;
Flow rate: 4 ml/min;
Mobile phase: (ethyl acetate:n-hexane: ethanol=18: 72: 10) + 0.1 % triethylamine;

The target compound with a peak time of 40.873 min and a mass of 20 mg was obtained, LC-MS(APCI): m/z=506 $(M+1)^+$. $^1H$ NMR (400 MHz, DMSO-$D_6$): 8.84 (t, J=6.0 Hz, 1H), 7.89 (s, 1H), 7.53-7.49 (m, 1H), 7.43 (s, 4H), 7.36-7.30 (m, 1H), 7.19-7.16 (m, 1H), 6.55 (s, 1H), 4.66 (br s, 1H), 4.54 (d, J=6.4 Hz, 2H), 4.13 (d, J=6.0 Hz, 1H), 3.89 (s, 3H), 3.42-3.39 (m, 2H), 3.41-3.39 (m, 3H), 2.13-1.10 (m, 1H), 1.83-1.73 (m, 3H), 1.38-1.35 (m, 1H).

**Example 62 Preparation of compound N-(4-(4-amino-7-(6-(hydroxymethyl)tetrahydro-2H-pyran-3-yl)pyrrolo [2,1-f][1,2,4]triazin-5-yl)benzyl)-5-fluoro-2-methoxybenzamide (Isomer 4)**

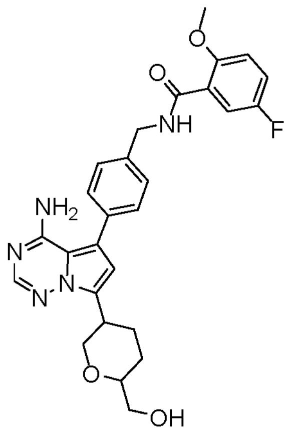

Example 58 (150 mg) was dissolved in methanol (15 mL), and the chiral preparation was carried out using the following conditions:

AD-H column: CHIRALPAKR AD-H; 4.6mm*250mmL*5μm;
Flow rate: 4 ml/min;
Mobile phase: (ethyl acetate:n-hexane: ethanol=18: 72: 10) + 0.1 % triethylamine;

The target compound with a peak time of 47.310 min and a mass of 21 mg was obtained, LC-MS(APCI): m/z=506 $(M+1)^+$. $^1H$ NMR (400 MHz, DMSO-$D_6$): $^1H$ NMR (400 MHz, DMSO-$D_6$) δ (ppm): 8.84 (t, J=6.0 Hz, 1H), 7.89 (s, 1H), 7.53-7.49 (m, 1H), 7.43 (s, 4H), 7.36-7.30 (m, 1H), 7.19-7.16 (m, 1H), 6.55 (s, 1H), 4.64 (br s, 1H), 4.54 (d, J=6.4 Hz, 2H), 4.10 (d, J=6.0 Hz, 1H), 3.89 (s, 3H), 3.42-3.39 (m, 2H), 3.41-3.39 (m, 3H), 2.13-1.10 (m, 1H), 1.83-1.73 (m, 3H), 1.38-1.35 (m, 1H).

**Biological activity assay**

(1) Kinase Activity Inhibition Assay

In this assay, the HTRF TK Kit (Catalog No. 62TKOPEC) provided by Cisbio was used to detect the inhibitory activity of compounds on BTK kinase and BTK C481S kinase and the selectivity of compounds relative to EGFR, ITK and TEC kinases.

Reagents and consumables: BTK kinase (Invitrogen, Catalog No. PV3587), BTK C481S (Carna, Product No. 08-547), ITK (Carna, Product No. 08-181), EGFR (Carna, Product No. 08-115), TEC (Carna, Product No. 08-182), HTRF-TK kit (Cisbio, Product No. 62TK0PEC), $MgCl_2$ (Sigma, Product No. M1028), $MnCl_2$ (Sigma, Product No. M1787), DTT (Sigma, Product No. D0632), ATP (Sigma, Product No. A7699-1g), DMSO (Sigma, Product No. D2650), 384-well plate (PE, Product No. 6008280).

Kinase activity analysis: the required compounds were perpared, and the final concentration of DMSO in the kinase detection system did not exceed 1%. The entire operation steps were carried out on ice, and the total reaction volume was 10μL. The compound (4μL), substrate and ATP (4μL), kinase (2μL) were added in sequence to a 384-well plate, and shaked for 30 seconds. After an appropriate time at room temperature, the detection reagent was added. After 1 hour at room temperature, the plate was detected with a microplate reader. The specific steps were as follows:

a) 1 × kinase buffer solution required for kinase detection was calculated according to the amount of the detection compound, and prepared as needed;
b) compounds with 2.5 × working concentration were prepared, the 100 times gradient concentration solution of compounds was diluted by 40 × to the desired concentration, mixed well, and added to each well at 4 μL per well;
c) 5 × working concentration of substrate and ATP were prepared, mixed in a ratio of 1:1 before adding to a 384-well plate, and added to each well at 4 μL per well;
d) 5 × working concentration of kinase was prepared, and 2 μL was added to each well;
e) the plate was shaked on a microplate oscillator for 30 seconds, sealed with a sealing membrane, and reacted at room temperature for an appropriate time;
f) 4 × working concentrations of streptavidin XL-665 and TK antibody-cryptate compound were prepared, mixed in a ratio of 1:1 before adding to a 384-well plate, and added to each well at 10 μL per well;
g) the plate was read after reacting at room temperature for 1 hour;
h) the wells with only substrate, ATP and 1% DMSO added but no kinase were blank control wells, and the wells with only substrate, ATP, 1% DMSO and kinase were positive control wells. Readings: The values of 620 nm (cryptate) and 665 nm (XI,665) were read with a BioTek Synerg Neo2 microplate reader, the ratio of 665/620 per well was calculated, and the data were exported. Data analysis: $IC_{50}$ was calculated using GraphPad Prism 7.0 software.

The following nonlinear fitting formula was used to obtain the $IC_{50}$ (half inhibitory concentration) of compounds:

$$Y=Bottom + (Top-Bottom)/(1+10^{\wedge}((LogIC_{50}-X)*HillSlope))$$

X: Compound concentration log value
Y: Inhibition rate (% inhibition)

The kinase inhibition rate (% inhibition rate) was calculated as:

Inhibition rate% = (1-(each concentration well-blank well)/(positive control well-blank well))* 100%

The assay results show that the compounds of the present disclosure have strong activity against both wild-type BTK kinase and drug-resistant mutated BTK C481S kinase and excellent selectivity over EGFR, ITK and TEC kinases. The results of representative Example compounds are summarized in Table 1 below, wherein A represents $IC_{50}$ <_ 10 nM, B represents $IC_{50}$ is 10-50nM, C represents $IC_{50}$ is 50-100 nM, D represents $IC_{50}$ is 100-1000 nM, E represents $IC_{50}$ is 1000-10000 nM, F represents $IC_{50}$ > 10000 nM.

| Example compound No. | BTK | BTK C481S | EGFR | ITK | TEC |
|---|---|---|---|---|---|
| 4 | A | A | D | F | D |
| 5 | B | A | C | F | D |

(continued)

| Example compound No. | BTK | BTK C481S | EGFR | ITK | TEC |
|---|---|---|---|---|---|
| 6 | A | A | C | F | D |
| 7 | | A | D | F | D |
| 8 | | B | D | F | E |
| 18 | A | A | C | F | D |
| 20 | A | B | B | F | D |
| 22 | A | A | B | F | D |
| 29 | A | A | C | F | D |
| 30 | A | A | C | F | D |
| 31 | A | A | D | F | D |
| 33 | A | A | C | F | D |
| 34 | A | A | C | F | D |
| 35 | A | A | C | F | D |
| 36 | A | A | E | F | D |
| 55 | A | A | B | F | D |
| 56 | A | A | B | F | D |
| 59 | B | B | B | F | E |
| 60 | A | A | A | F | D |

(2) Assay of Cell Growth Inhibitory Activity

In this assay, the CellTiter-Glo® luminescence cell viability detection kit provided by Promega Company was used, which is a homogeneous cell viability detection method. The cell viability of cultured cells (TMD-8 cells: human diffuse large B lymphoma cell line, REC1 cells: human mantle cell lymphoma cells, DOHH2 cells: human follicular lymphoma cells, Pfeiffer cells: human diffuse large cell lymphoma cells, Raji cells: human Burkitt lymphoma cells, RPMI-8226 cells: human multiple myeloma cells) was measured by quantifying ATP. The specific assay steps were as follows:

a) cells in logarithmic growth phase were collected, cell suspension was prepared and cells were counted; cell concentration was calculated.
b) The cell suspension was diluted to the required concentration and then added to the middle wells of a 96-well plate, and PBS was added to the edge wells. The wells with no cells and only culture medium was used as blank controls.
c) In addition to the cell plate used for compound analysis, another 96-well plate was prepared as a T0 plate, and 3-6 blank wells and 3-6 cell wells were added. The next day, CTG was used to detect the cell viability as the T0 viability value of the cells.
d) The cell plates were cultured overnight in a 5% $CO_2$ incubator at 37 °C.
e) The next day, gradient dilution of each concentration of compounds was added, and the cell plate was cultured in a 5% $CO_2$ incubator at 37 °C for 72 h. The wells with no compounds and only 0.5% DMSO were used as positive control for cell growth.
f) CTG reagent (CellTiter-Glo kit) was added according to the manufacturer's instructions to detect the cell viability value.
g) The plate was read with a Biotek cytation3.0 microplate reader and the data were exported.
h) IC50 was calculated using GraphPad Prism 7.0 software.

The following nonlinear fitting formula was used to obtain the $IC_{50}$ (half inhibitory concentration) of compounds:

$$Y=Bottom + (Top-Bottom)/(1+10\^{}((LogIC_{50}-X)*HillSlope))$$

X: Compound concentration log value
Y: Inhibition rate (%inhibition)

The formula for calculating cell inhibition rate (% inhibition rate) was:

Inhibition rate% = 1- (Lum of drug to be tested - Lum of vehicle control)/(Lum of cell control - --> Lum of vehicle control)×100%.

The assay results show that the compounds of the present disclosure have stronger activity on TMD-8 cells, REC1 cells, DOHH2 cells, Pfeiffer cells, Raji cells and RPMI-8226 cells.

(3) Phosphor-BTK(Y223) Assay

The purpose of this assay is to simply, quickly and directly detect the endogenous level of BTK in cells. The specific assay steps are as follows:

Day 1: NIH/3T3 BTK WT cells (7.5 k/well) and NIH/3T3 BTK C481S cells (10 k/well) were plated in 96-well plates, respectively, and cultured overnight at 37 °C and 5% $CO_2$.
Day 2:

1. A 3-fold final concentration gradient dilution of the compound was prepared, and 50 μL was added to 100 μL of cell culture; 0.5% DMSO was used as a positive control for cell growth.
2. The plate was incubated at 37°C, 5% $CO_2$ for 0.5 h.
3. $Na_3VO_4$ with a final concentration of 1mM was added, and incubated at 37 °C and 5% $CO_2$ for 2 hours.
4. During the incubation, the following reagents were prepared:

1x added lysis buffer: 3 vol ddH2O + 1 vol 4x lysis buffer + 100x blocking reagent.
p-BTK (Y223) cryptate-antibody and p-BTK (Y223) d2-antibody working solutions: 1 volume of each cryopreserved antibody (stored at - 20 °C) was added to 19 times volumes of assay buffer (stored at 4 °C).

5. After the incubation, the medium was removed and 50 μL of 1x added lysis buffer was added; The mixture was centrifuged at 500 rpm for 30 min at room temperature.
6. The lysate was mixed well and 16 μL was transferred to a 384-well plate; 1 × added lysis buffer was used as a blank control.
7. 4 μL of pre-mixed working solutions of the two antibodies (cryptate Ab:d2 Ab = 1:1) were added and the plate was sealed.
8. The plate was incubated overnight at room temperature.

Day 3:

Biotek cytation3.0 microplate reader was used to read the plate at 665nm and 620nm, and the ratio of 665/620nm per well was calculated.
Data analysis:
$IC_{50}$ was calculated using GraphPad Prism 7.0 software.

The following nonlinear fitting formula was used to obtain the phosphorylated BTK Y223 $IC_{50}$ (half phosphorylation inhibitory concentration) of the compounds:

$$Y=Bottom + (Top-Bottom)/(1+10^{\wedge}((LogIC_{50}-X)*HillSlope))$$

X: Compound concentration log value
Y: Inhibition rate (% inhibition)

The formula for calculating cell phosphorylation inhibition rate (% inhibition rate) was:

$$\% \text{ inhibition} = 1 - (\text{each concentration well-blank well})/(\text{positive control well-blank well})$$

The results of the above Phosphor-BTK (Y223) assay are summarized in Table 2 below, wherein A represents an $IC_{50} \leq 25$ nM, B represents an $IC_{50}$ of 25-50 nM, C represents an $IC_{50}$ of 50-100 nM, and D represents an $IC_{50}$ of 100-500 nM:

| Example No. | NIH/3T3 BTK-WT pBTK | NIH/3T3 BTK-C481S pBTK |
|---|---|---|
| 1 | D | D |
| 2 | C | C |
| 3 | D | D |
| 4 | A | B |
| 5 | A | A |
| 6 | A | A |
| 7 | A | B |
| 8 | A | C |
| 9 | A | A |
| 12 | D | D |
| 13 | C | D |
| 14 | C | D |
| 15 | A | C |
| 16 | C | D |
| 17 | D | D |
| 18 | A | A |
| 19 | B | C |
| 20 | A | A |
| 21 | B | C |
| 22 | A | A |
| 23 | B | B |
| 24 | A | B |
| 25 | B | B |
| 26 | B | C |
| 27 | A | B |
| 28 | A | B |
| 29 | A | A |
| 30 | A | A |
| 31 | A | A |
| 32 | A | A |
| 33 | A | A |
| 34 | A | A |
| 35 | A | A |
| 36 | A | A |
| 37 | B | C |
| 38 | A | B |
| 39 | C | D |

(continued)

| Example No. | NIH/3T3 BTK-WT pBTK | NIH/3T3 BTK-C481S pBTK |
|---|---|---|
| 40 | B | C |
| 41 | B | C |
| 42 | C | C |
| 43 | D | D |
| 44 | C | D |
| 47 | D | D |
| 48 | D | D |
| 49 | A | B |
| 50 | A | B |
| 51 | B | C |
| 52 | C | D |
| 55 | A | A |
| 56 | A | A |
| 57 | B | C |
| 58 | A | B |
| 59 | B | C |
| 60 | A | A |
| 61 | A | B |
| 62 | A | B |

(4) Metabolic Stability Evaluation

Metabolic stability is generally used to describe the rate and degree of compounds being metabolized, and is one of the main factors affecting pharmacokinetic properties. Many compounds are substrates of CYP450 enzymes and other drug metabolizing enzymes, and liver microsomes are rich in CYP450. The purpose of this assay is to study the in vitro metabolic stability by incubating the compounds of the present disclosure with human and SD rat liver microsomes respectively and detecting the remaining proportion of the compounds by LC-MS/MS.

1) Preparation of solutions

Phosphate buffer (PBS): 150mL of pre-prepared $K_2HPO_4$ (0.5 M) solution was mixed with 700 mL of $K_2HPO_4$ (0.5 M) solution; $K_2HPO_4$ (0.5 M) solution was then used to adjust the pH value of the mixed solution to 7.4. The mixture was used as 5 times concentration of PBS, and stored at 4 °C for later use. The mixture was diluted 5 times with ultrapure water before use, and 3.3 mM magnesium chloride was added to obtain phosphate buffer solution PBS (100 mM).

NADPH regeneration system solution: A NADPH solution containing 6.5 mM NADP, 16.5 mM G-6-P, and 3U/mL G-6-P D was prepared with 5 mL of PBS.

Internal standard stop solution: 50 ng/mL propranolol hydrochloride and 200 ng/mL tolbutamide were prepared with acetonitrile as an internal standard working solution.

Human liver microsome solution: 0.31 mL of human liver microsomes (25mg/mL) were added to 0.961 mL of PBS (pH7.4) and mixed well to obtain a human liver microsome dilution with a protein concentration of 0.625 mg/mL.

SD rat liver microsome solution: 0.31 mL of SD rat liver microsomes (25 mg/mL) were added to 0.961 mL of PBS (pH7.4) and mixed well to obtain SD rat liver microsome dilution with a protein concentration of 0.625 mg/mL.

Sample working solution: The powder of the compound of the present disclosure, the positive control dextromethorphan powder and omeprazole powder were prepared with DMSO to 10 mM as the sample stock solution, and then diluted with 70% acetonitrile-water to obtain a 0.25 mM sample working solution.

2) Sample incubation

398 μL of human liver microsome dilution was added to a 96-well incubation plate (N = 2); 2 μL of 0.25 mM test compound and dextromethorphan were added respectively, and mixed well.

398 μL of SD rat liver microsome dilution was added to a 96-well incubation plate (N = 2); 2 μL of 0.25 mM test compound and omeprazole were added respectively, and mixed well.

300 μL of pre-cooled stop solution per well was added to a 96-well deep well plate as a stop plate and placed on ice.

The 96-well incubation plate and the NADPH regeneration system were placed in a 37 °C water bath, shaked at 100 rpm, and pre-incubated for 5 minutes. 80μL of incubation solution was taken out from each well of the incubation plate, added to the stop plate, and mixed well. 20μL of NADPH regeneration system solution was supplemented, as a 0 min sample. Then 80μL of NADPH regeneration system solution was added to each well of the incubation plate. The reaction was initiated, and timing was started. The reaction concentration of the compound to be tested was 1μM, and the protein concentration was 0.5 mg/mL.

At 10, 30, and 90 minutes of the reaction, respectively, 100 μL of the reaction solution was added to the stop plate, and vortexed for 3 minutes to stop the reaction.

The stop plate was centrifuged at 5000rpm and 4 °C for 15 minutes. 200μL of the supernatant was added into a 96-well plate pre-added with 200μL of ultrapure water, and mixed well. LC-MS/MS was used for sample analysis, and 10 μL was injected.

3) Sample Analysis Method

In this assay, LC-MS/MS system was used to detect the peak areas of the compound to be tested, dextromethorphan, omeprazole and internal standard, and the ratio of the peak areas of the compound to the internal standard was calculated.

4) Data Processing

The peak area of the sample and internal standard was obtained by mass spectrometer and Analyst software. The substrate elimination rate constant K was obtained by plotting the residual amount of compound (R%) against time using the single exponential degradation model of Graphpad prism7.0 software.

$$Ct/C0 = \exp(-K*t)$$

The half-life $T_{1/2}$ and intrinsic clearance $CL_{int}$ were calculated according to the following formula, where V/M is equal to 1/C (protein).

$$T_{1/2} = -\frac{0.693}{Slope},\ CL_{int} = \frac{0.693}{t_{1/2}} \cdot \frac{V}{M}\quad , t_{1/2}(\text{min});\ CL_{int}(\mu L/\text{min}/\text{mg})$$

The assay results show that the compounds of the present disclosure have excellent metabolic stability.

(5) Pharmacokinetic assays in rats

Six male Sprague-Dawley rats, 7-8 weeks old, weighing about 210g, were divided into two groups, three rats in each group. A single dose of the compound (oral 10mg/kg) was administered intravenously or orally. The pharmacokinetic differences were compared.

Rats were fed on standard diet and given water. Fasting started 16 hours before the test. The drug was dissolved with PEG400 and dimethyl sulfoxide. Blood was collected from the orbit. The time points of blood collection were 0.083 hours, 0.25 hours, 0.5 hours, 1 hour, 2 hours, 4 hours, 6 hours, 8 hours, 12 hours and 24 hours after administration.

The rats were briefly anesthetized after inhaling ether, and a 300μL blood sample was collected from the orbit in the test tube. There was 30μL of 1% heparin salt solution in the test tube. Before use, the test tube was dried at 60 °C overnight. After the blood sample collection was completed at the last time point, the rats were sacrificed after ether anesthesia.

Immediately after the blood sample was collected, the test tube was gently inverted at least 5 times to ensure sufficient mixing and placed on ice. The blood sample was centrifuged at 4 °C at 5000 rpm for 5 minutes to separate the plasma from the red blood cells. A pipette was used to add 100 μL of the plasma into a clean plastic centrifuge tube. The

name of the compound and time point were marked. The plasma was stored at -80 °C before analysis. The concentration of the compound of the present disclosure in the plasma was determined by LC-MS/MS. The pharmacokinetic parameters were calculated based on the plasma drug concentration of each animal at different time points.

The assay results show that the compounds of the present disclosure have better pharmacokinetic properties in animals, and thus have better pharmacodynamics and therapeutic effects.

The above content is a further detailed description of the present disclosure in conjunction with specific alternative embodiments, and it cannot be considered that the specific implementation of the present disclosure is limited to these descriptions. For those skilled in the technical field to which the present disclosure belongs, some simple deductions or substitutions can be made without departing from the concept of the present disclosure, and should be regarded as belonging to the scope of protection of the present disclosure.

## Claims

1. A compound of formula (I), or a tautomer, stereoisomer, prodrug, crystal form, pharmaceutically acceptable salt, hydrate or solvate thereof:

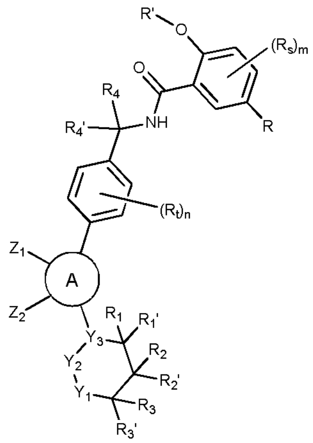

(I)

wherein

ring A is

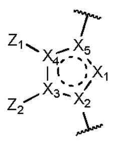

or

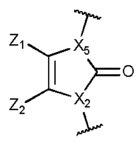

;

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O, S, NH or $C(R_{Y1})_2$; wherein $R_{Y1}$ is independently H, D, halogen, $C_{1-6}$ alkyl or $C_{1-6}$ haloalkyl;
$Y_2$ is bond, $C(R_{Y2})_2$ or $C(R_{Y2})_2C(R_{Y2})_2$; wherein $R_{Y2}$ is independently H, D, halogen, $C_{1-6}$ alkyl or $C_{1-6}$ haloalkyl;
$Y_3$ is N, CD or CH;
$Z_1$ is $C(O)NH_2$;
$Z_2$ is $NH_2$;
or $Z_1$ and $Z_2$ are taken together with the atoms to which they are attached to form

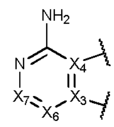;

wherein $X_6$ is N, CD, CH or C-OH;
$X_7$ is N, CD or CH;
$R_1$ is H, D, halogen or OH;
$R_1$' is H, D or halogen;
$R_2$ is H, D, OH or $NH_2$;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, halogen, $C_{1-6}$ alkyl, $C_{1-6}$ haloalkyl, $C_{3-6}$ cycloalkyl or 3- to 7-membered heterocyclyl;
or $-C(R_3)(R_3')-C(R_{Y1})_2-$ or $-C(R_1)(R_1')-C(R_2)(R_2')-$ is taken together to form -CH=CH-; or $R_1$ and $R_2$ are attached to form $C_{1-4}$ alkylene;
$R_4$ is H, D, halogen, $C_{1-6}$ alkyl or $C_{1-6}$ haloalkyl;
$R_4$' is H, D, halogen, $C_{1-6}$ alkyl or $C_{1-6}$ haloalkyl;
R is halogen;
R' is $C_{1-6}$ alkyl or $C_{1-6}$ haloalkyl;
$R_s$ is H, D, halogen, $C_{1-6}$ alkyl or $C_{1-6}$ haloalkyl;
$R_t$ is H, D, halogen, $C_{1-6}$ alkyl or $C_{1-6}$ haloalkyl;
m=0, 1, 2 or 3;
n=0, 1, 2, 3 or 4;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups may be substituted with D, up to full deuteration;
provided that at least one of $R_2$ and $R_3$ is OH or $CH_2OR_a$.

**2.** The compound of claim 1, or a tautomer, stereoisomer, prodrug, crystal form, pharmaceutically acceptable salt, hydrate or solvate thereof, wherein ring A is

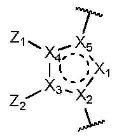,

alternatively is

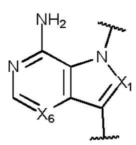, 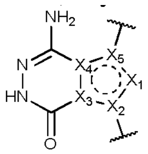, 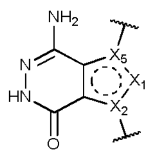, 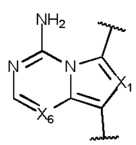, 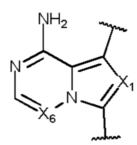, 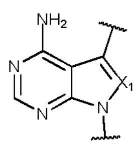

or

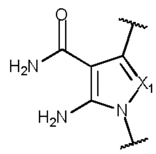,

yet alternatively is

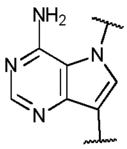, 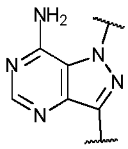, 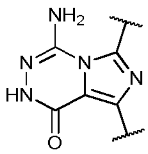, 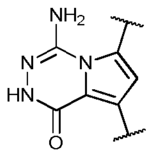, 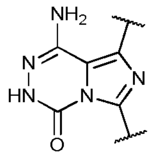,

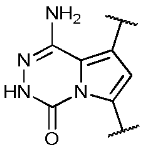, 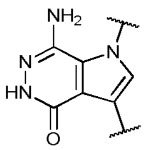, 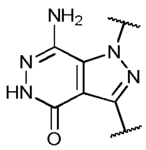, 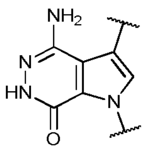, 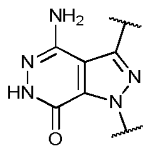, 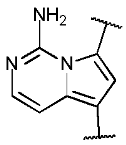, 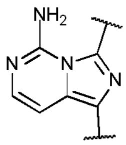,

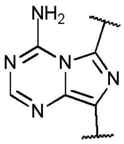, 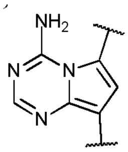, 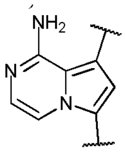, 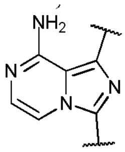, 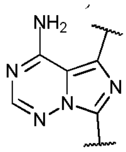, 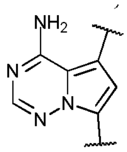, 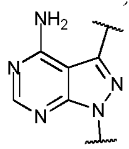, 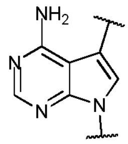,

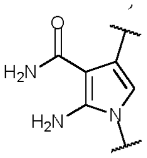 or 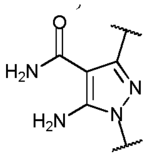.

3. The compound of claim 1, or a tautomer, stereoisomer, prodrug, crystal form, pharmaceutically acceptable salt, hydrate or solvate thereof, wherein ring A is

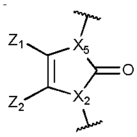,

alternatively is

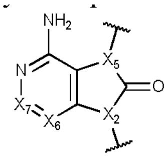, 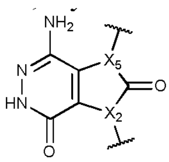, 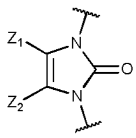 or 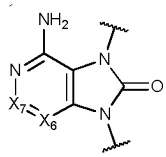,

yet alternatively is

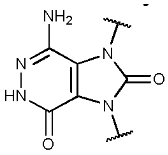, 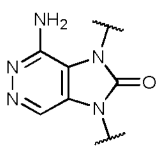, 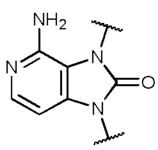 or 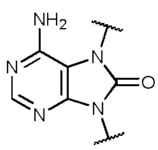

4. The compound of any one of claims 1-3, or a tautomer, stereoisomer, prodrug, crystal form, pharmaceutically acceptable salt, hydrate or solvate thereof, wherein $Y_1$ is O.

5. The compound of any one of claims 1-3, or a tautomer, stereoisomer, prodrug, crystal form, pharmaceutically acceptable salt, hydrate or solvate thereof, wherein $Y_1$ is $C(R_{Y1})_2$, alternatively $CH_2$.

6. The compound of any one of claims 1-5, or a tautomer, stereoisomer, prodrug, crystal form, pharmaceutically acceptable salt, hydrate or solvate thereof, wherein $Y_2$ is bond.

7. The compound of any one of claims 1-5, or a tautomer, stereoisomer, prodrug, crystal form, pharmaceutically acceptable salt, hydrate or solvate thereof, wherein $Y_2$ is $C(R_{Y2})_2$ or $C(R_{Y2})_2C(R_{Y2})_2$, alternatively $CH_2$ or $CH_2CH_2$.

8. The compound of any one of claims 1-7, or a tautomer, stereoisomer, prodrug, crystal form, pharmaceutically acceptable salt, hydrate or solvate thereof, wherein $Y_3$ is CH.

9. The compound of any one of claims 1-8, or a tautomer, stereoisomer, prodrug, crystal form, pharmaceutically acceptable salt, hydrate or solvate thereof, wherein $R_2$ is OH and $R_3$ is H, or $R_2$ is H and $R_3$ is OH, or $R_2$ is H and $R_3$ is $CH_2OH$.

10. The compound of any one of claims 1-9, or a tautomer, stereoisomer, prodrug, crystal form, pharmaceutically acceptable salt, hydrate or solvate thereof, wherein R is F.

11. The compound of any one of claims 1-10, or a tautomer, stereoisomer, prodrug, crystal form, pharmaceutically acceptable salt, hydrate or solvate thereof, wherein R' is methyl.

12. The compound of any one of claims 1-11, or a tautomer, stereoisomer, prodrug, crystal form, pharmaceutically acceptable salt, hydrate or solvate thereof, which has the following general formula:

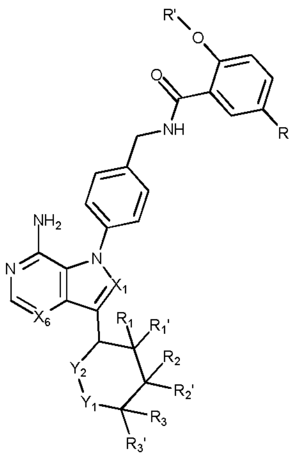

(II),

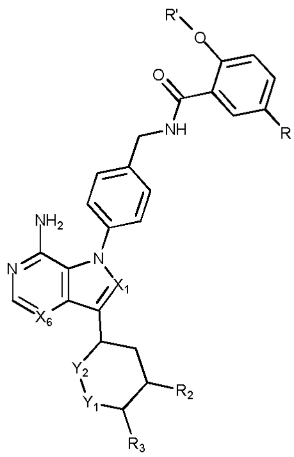

(II-1),

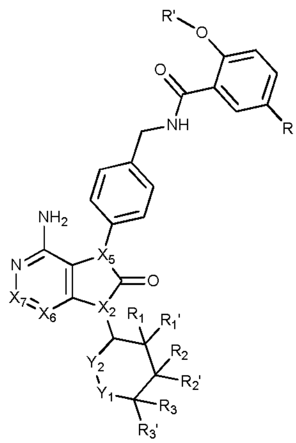

(III),

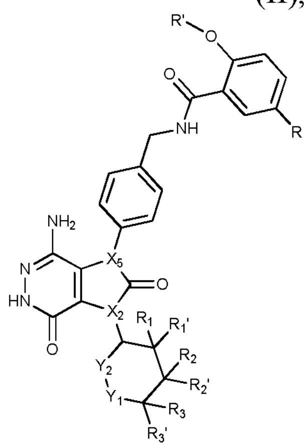

(III-1),

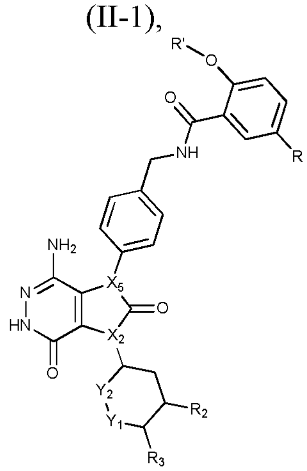

(III-2),

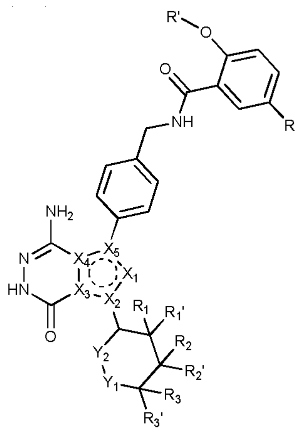 (IV), 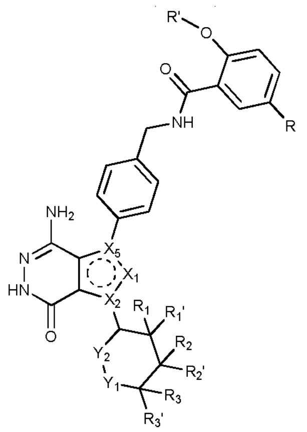 (IV-1), 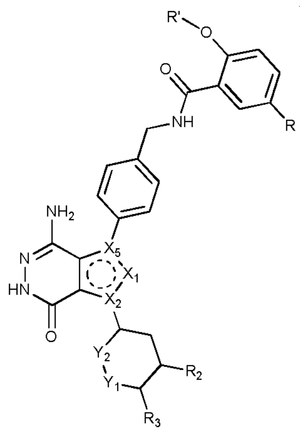 (IV-

2), 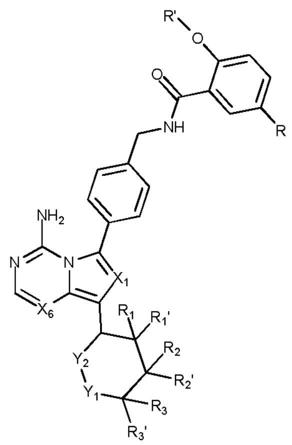 (V), 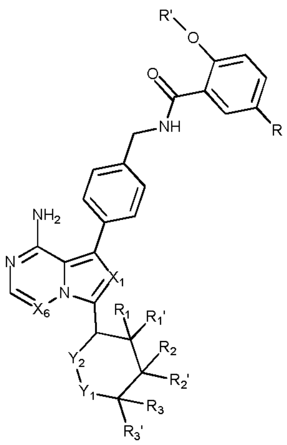 (VI), 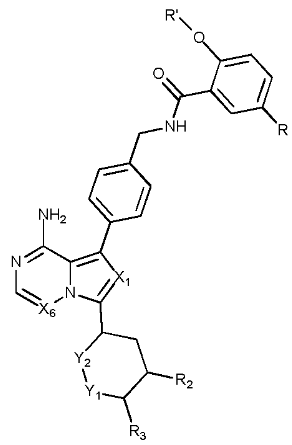 (VI-1),

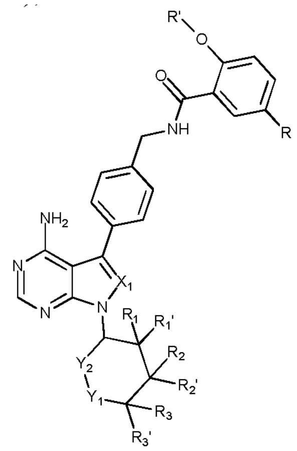 (VII), 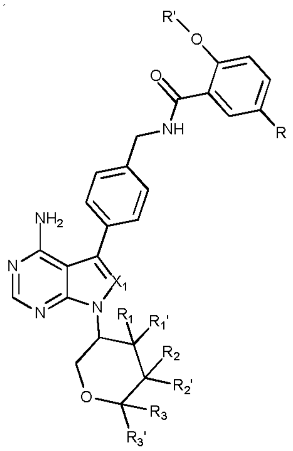 (VII-1), 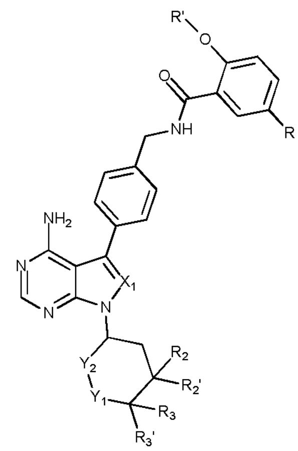 (VII-2),

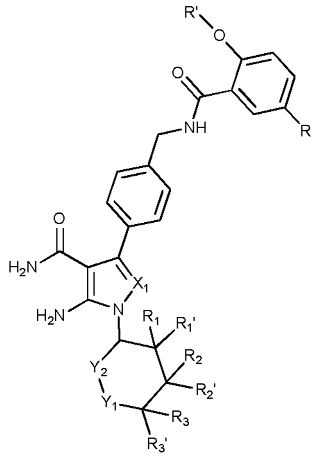

(VIII) or

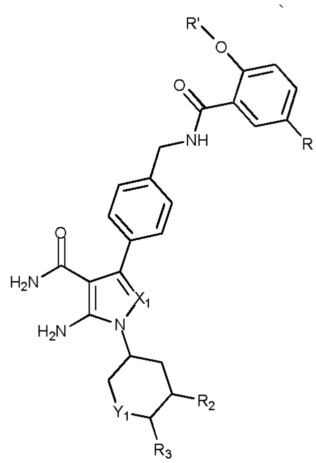

(VIII-1),

wherein each group is as defined in any one of claims 1-11.

**13.** The compound of claim 12, or a tautomer, stereoisomer, prodrug, crystal form, pharmaceutically acceptable salt, hydrate or solvate thereof, which is a compound of formula (II):

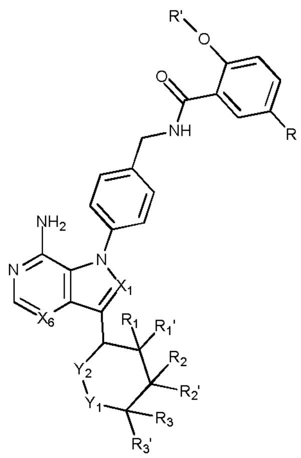

(II)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**14.** The compound of claim 12, or a tautomer, stereoisomer, prodrug, crystal form, pharmaceutically acceptable salt, hydrate or solvate thereof, which is a compound of formula (II-1):

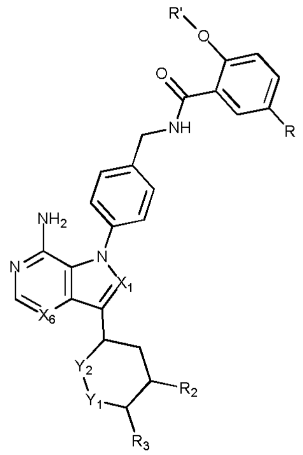

(II-1)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OH$.

**15.** The compound of claim 14, or a tautomer, stereoisomer, prodrug, crystal form, pharmaceutically acceptable salt, hydrate or solvate thereof, wherein:

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

**16.** The compound of claim 12, or a tautomer, stereoisomer, prodrug, crystal form, pharmaceutically acceptable salt, hydrate or solvate thereof, which is a compound of formula (III):

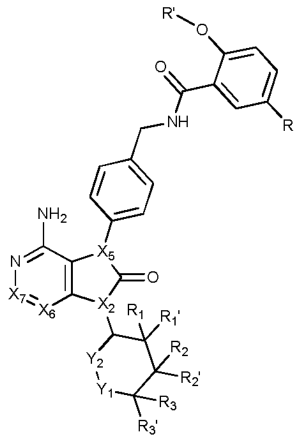

(III)

wherein

$X_2$ is N, CD or CH;
$X_5$ is N, CD or CH;
$X_6$ is N, CD, CH or C-OH;
$X_7$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**17.** The compound of claim 12, or a tautomer, stereoisomer, prodrug, crystal form, pharmaceutically acceptable salt, hydrate or solvate thereof, which is a compound of formula (III-1):

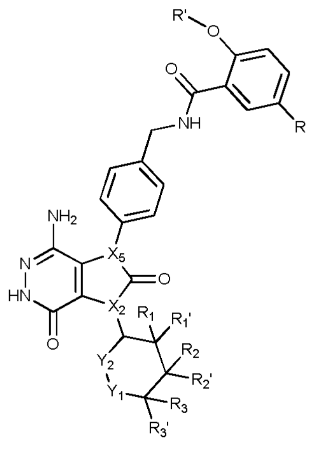

(III-1)

wherein

$X_2$ is N, CD or CH;
$X_5$ is N, CD or CH;
$Y_1$ is O or $C(R_{Y1})_2$; wherein $R_{Y1}$ is independently H, D or halogen;

$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**18.** The compound of claim 12, or a tautomer, stereoisomer, prodrug, crystal form, pharmaceutically acceptable salt, hydrate or solvate thereof, which is a compound of formula (III-2):

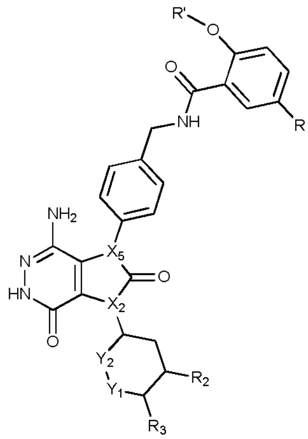

(III-2)

wherein

$X_2$ is N, CD or CH;
$X_5$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OH$.

**19.** The compound of claim 18, or a tautomer, stereoisomer, prodrug, crystal form, pharmaceutically acceptable salt, hydrate or solvate thereof, wherein:

$X_2$ is N;
$X_5$ is N;
$Y_1$ is O or $C(R_{Y1})_2$; wherein $R_{Y1}$ is independently H, D or halogen;
$Y_2$ is $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

**20.** The compound of claim 12, or a tautomer, stereoisomer, prodrug, crystal form, pharmaceutically acceptable salt, hydrate or solvate thereof, which is a compound of formula (IV):

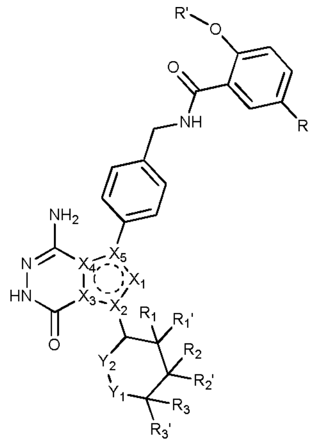

(IV)

wherein

the ring where $X_1$-$X_5$ are located is an aromatic ring;
$X_1$ is N, CD or CH;
$X_2$ is a N atom or a C atom;
$X_3$ is a N atom or a C atom;
$X_4$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**21.** The compound of claim 12, or a tautomer, stereoisomer, prodrug, crystal form, pharmaceutically acceptable salt, hydrate or solvate thereof, which is a compound of formula (IV-1):

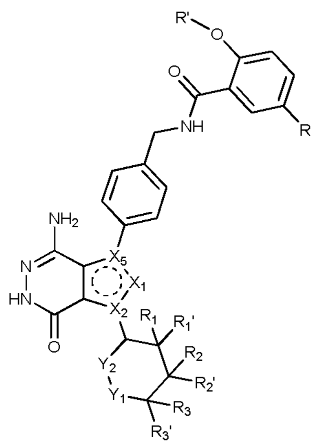

(IV-1)

wherein

the ring where $X_1$, $X_2$ and $X_5$ are located is an aromatic ring;
$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**22.** The compound of claim 12, or a tautomer, stereoisomer, prodrug, crystal form, pharmaceutically acceptable salt, hydrate or solvate thereof, which is a compound of formula (IV-2):

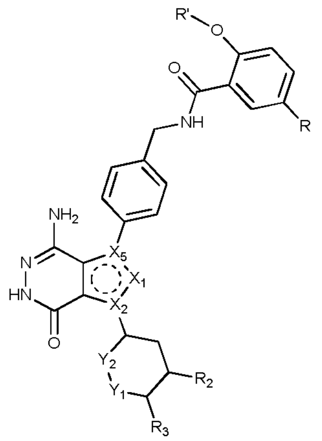

(IV-2)

wherein

the ring where $X_1$, $X_2$ and $X_5$ are located is an aromatic ring;
$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is halogen;
R' is $C_{1-6}$ alkyl or $C_{1-6}$ haloalkyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OH$.

**23.** The compound of claim 22, or a tautomer, stereoisomer, prodrug, crystal form, pharmaceutically acceptable salt, hydrate or solvate thereof, wherein:

the ring where $X_1$, $X_2$ and $X_5$ are located is an aromatic ring;

$X_1$ is N, CD or CH;
$X_2$ is a N atom or a C atom;
$X_5$ is a N atom or a C atom;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

**24.** The compound of claim 12, or a tautomer, stereoisomer, prodrug, crystal form, pharmaceutically acceptable salt, hydrate or solvate thereof, which is a compound of formula (V):

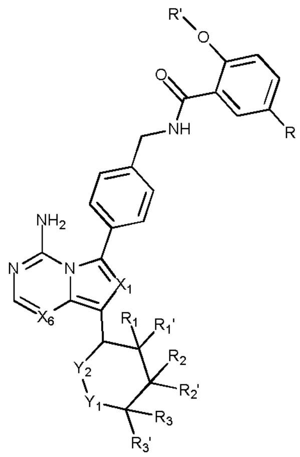

(V)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**25.** The compound of claim 12, or a tautomer, stereoisomer, prodrug, crystal form, pharmaceutically acceptable salt, hydrate or solvate thereof, which is a compound of formula (VI):

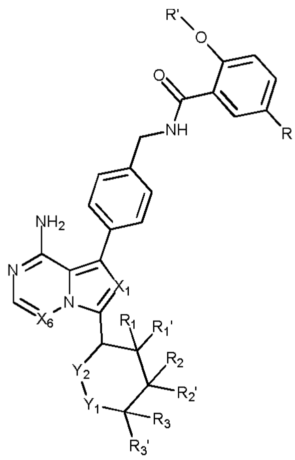

(VI)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**26.** The compound of claim 12, or a tautomer, stereoisomer, prodrug, crystal form, pharmaceutically acceptable salt, hydrate or solvate thereof, which is a compound of formula (VI-1):

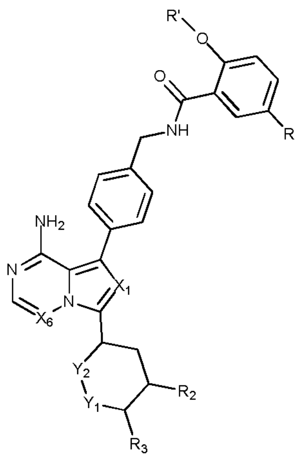

(VI-1)

wherein

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;

$R_2$ is H, D or OH;
$R_3$ is H, D, OH or $CH_2OH$;
R is halogen;
R' is $C_{1-6}$ alkyl or $C_{1-6}$ haloalkyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OH$.

**27.** The compound of claim 26, or a tautomer, stereoisomer, prodrug, crystal form, pharmaceutically acceptable salt, hydrate or solvate thereof, wherein:

$X_1$ is N, CD or CH;
$X_6$ is N, CD or CH;
$Y_1$ is O or $C(R_{Y1})_2$; wherein $R_{Y1}$ is independently H, D or halogen;
$Y_2$ is bond or $C(R_{Y2})_2$; wherein $R_{Y2}$ is independently H, D or halogen;
$R_2$ is H, D or OH;
$R_3$ is H, D, OH or $CH_2OH$;
R is F;
R' is methyl;
provided that at least one of $R_2$ and $R_3$ is OH or $CH_2OH$.

**28.** The compound of claim 12, or a tautomer, stereoisomer, prodrug, crystal form, pharmaceutically acceptable salt, hydrate or solvate thereof, which is a compound of formula (VII):

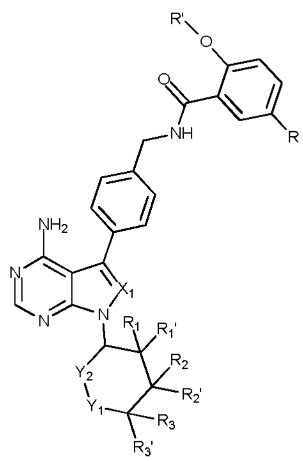

(VII)

wherein

$X_1$ is N, CD or CH;
$Y_1$ is O;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_1$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_1)(R_1')-C(R_2)(R_2')$ is taken together to form -CH=CH-;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl, $C_{3-6}$ cycloalkyl and $-CH_2-$ in the above groups may be substituted with D, up to full deuteration;
provided that at least one of $R_2$ and $R_3$ is OH or $CH_2OR_a$.

**29.** The compound of claim 12, or a tautomer, stereoisomer, prodrug, crystal form, pharmaceutically acceptable salt, hydrate or solvate thereof, which is a compound of formula (VII-1):

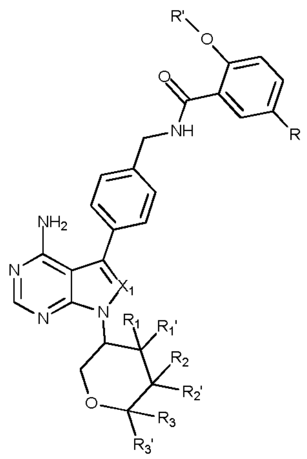

(VII-1)

wherein

$X_1$ is N, CD or CH;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OH$;
$R_3$' is H or D;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl and $-CH_2-$ in the above groups may be substituted with D, up to full deuteration;
provided that at least one of $R_2$ and $R_3$ is OH or $CH_2OH$.

**30.** The compound of claim 29, or a tautomer, stereoisomer, prodrug, crystal form, pharmaceutically acceptable salt, hydrate or solvate thereof, wherein:

$X_1$ is N;
$R_1$ is H, D, halogen or OH;
$R_1$' is H, D or halogen;
$R_2$ is H, D or OH;
$R_2$' is H or D;
$R_3$ is H, D, OH or $CH_2OH$;
$R_3$' is H or D;
or $R_1$ and $R_2$ are attached to form $-CH_2-$;
R is F;
R' is methyl;
provided that at least one of $R_2$ and $R_3$ is OH or $CH_2OH$.

**31.** The compound of claim 12, or a tautomer, stereoisomer, prodrug, crystal form, pharmaceutically acceptable salt, hydrate or solvate thereof, which is a compound of formula (VII):

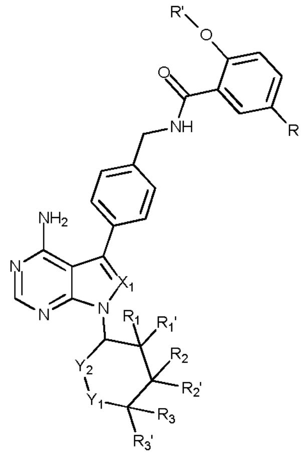

(VII)

wherein

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_1$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_3)(R_3')-C(R_{Y1})_2-$ is taken together to form -CH=CH-;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_1$.

**32.** The compound of claim 12, or a tautomer, stereoisomer, prodrug, crystal form, pharmaceutically acceptable salt, hydrate or solvate thereof, which is a compound of formula (VII-2):

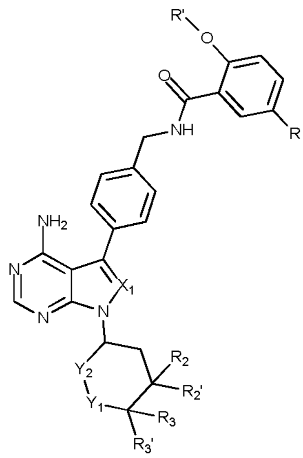

(VII-2)

wherein

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_2$ is H, D or OH;
$R_2$' is H, D, $C_{1-6}$ alkyl or $C_{1-6}$ haloalkyl;

$R_3$ is H, D, OH or $CH_2OR_1$; wherein $R_a$ is H, $C_{1-6}$ alkyl or $C_{1-6}$ haloalkyl;
$R_3$' is H or D;
or $-C(R_3)(R_3')-C(R_{Y1})_2-$ is taken together to form -CH=CH-;
R is halogen;
R' is $C_{1-6}$ alkyl or $C_{1-6}$ haloalkyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OR_1$.

**33.** The compound of claim 32, or a tautomer, stereoisomer, prodrug, crystal form, pharmaceutically acceptable salt, hydrate or solvate thereof, wherein:

$X_1$ is N;
$Y_1$ is $C(R_{Y1})_2$; wherein $R_{Y1}$ is independently H or D;
$Y_2$ is bond, $C(R_{Y2})_2$ or $C(R_{Y2})_2C(R_{Y2})_2$; wherein $R_{Y2}$ is independently H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H or D;
or $-C(R_3)(R_3')-C(R_{Y1})_2-$ is taken together to form -CH=CH-;
R is F;
R' is methyl;
wherein one or more H in $C_{1-6}$ alkyl and $C_{1-6}$ haloalkyl in the above groups may be substituted with D, up to full deuteration;
provided that at least one of $R_2$ and $R_3$ is OH or $CH_2OR_a$.

**34.** The compound of claim 12, or a tautomer, stereoisomer, prodrug, crystal form, pharmaceutically acceptable salt, hydrate or solvate thereof, which is a compound of formula (VIII):

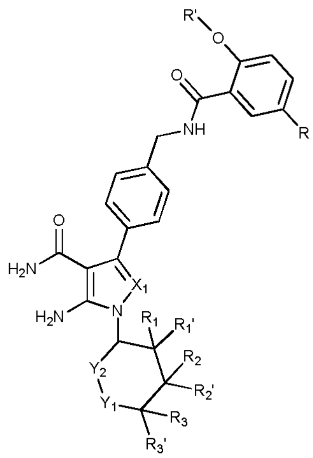

(VIII)

wherein

$X_1$ is N, CD or CH;
$Y_1$ is O or $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen; $R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl, $C_{3-6}$ cycloalkyl or 3- to 7-membered heterocyclyl; or $-C(R_3)(R_3')-C(R_{Y1})_2-$ or $-C(R_1)(R_1')-C(R_2)(R_2')-$ is taken together to form -CH=CH-;
or $R_1$ and $R_2$ are attached to form $C_{1-4}$ alkylene;
R is halogen;

R' is $C_{1-6}$ alkyl or $C_{1-6}$ haloalkyl;
wherein one or more H in $C_{1-6}$ alkyl, $C_{1-6}$ haloalkyl, $C_{3-6}$ cycloalkyl, 3- to 7-membered heterocyclyl and $C_{1-4}$ alkylene in the above groups may be substituted with D, up to full deuteration;
provided that at least one of $R_2$ and $R_3$ is OH or $CH_2OR_a$.

**35.** The compound of claim 34, or a tautomer, stereoisomer, prodrug, crystal form, pharmaceutically acceptable salt, hydrate or solvate thereof, wherein:

$X_1$ is N, CD or CH;
$Y_1$ is O;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_1)(R_1')-C(R_2)(R_2')$ is taken together to form -CH=CH-;
or $R_1$ and $R_2$ are attached to form $-CH_2-$;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**36.** The compound of claim 34, or a tautomer, stereoisomer, prodrug, crystal form, pharmaceutically acceptable salt, hydrate or solvate thereof, wherein:

$X_1$ is N, CD or CH;
$Y_1$ is $C(R_{Y1})_2$; wherein $R_{Y1}$ is independently H, D or halogen;
$Y_2$ is bond, $C(R_{Y2})_2$ or $C(R_{Y2})_2C(R_{Y2})_2$; wherein $R_{Y2}$ is independently H, D or halogen;
$R_1$ is H, D, halogen or OH;
$R_1$' is H, D or halogen;
$R_2$ is H, D or OH;
$R_2$' is H, D, $C_{1-6}$ alkyl or $C_{1-6}$ haloalkyl;
$R_3$ is H, D, OH or $CH_2OR_a$; wherein $R_a$ is H, $C_{1-6}$ alkyl or $C_{1-6}$ haloalkyl;
$R_3$' is H, D, $C_{1-6}$ alkyl, $C_{1-6}$ haloalkyl or $C_{3-6}$ cycloalkyl;
or $-C(R_3)(R_3')-C(R_{Y1})_2-$ is taken together to form -CH=CH-;
R is halogen;
R' is $C_{1-6}$ alkyl or $C_{1-6}$ haloalkyl;
wherein one or more H in $C_{1-6}$ alkyl, $C_{1-6}$ haloalkyl and $C_{3-6}$ cycloalkyl in the above groups may be substituted with D, up to full deuteration;
provided that at least one of $R_2$ and $R_3$ is OH or $CH_2OR_a$.

**37.** The compound of claim 12, or a tautomer, stereoisomer, prodrug, crystal form, pharmaceutically acceptable salt, hydrate or solvate thereof, which is a compound of formula (VIII-1):

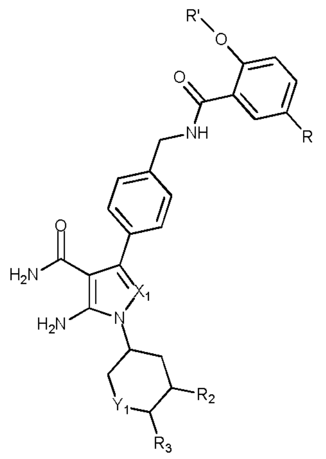

(VIII-1)

wherein

$X_1$ is N, CD or CH;
$Y_1$ is O, CHD, $CD_2$ or $CH_2$;
$R_2$ is H, D or OH;
$R_3$ is H, D or $CH_2OH$;
R is halogen;
R' is $C_{1-6}$ alkyl or $C_{1-6}$ haloalkyl; wherein one or more H in $C_{1-6}$ alkyl and $C_{1-6}$ haloalkyl in the above groups may be substituted with D, up to full deuteration;
provided that $R_2$ and $R_3$ are not both H.

**38.** The compound of claim 37, or a tautomer, stereoisomer, prodrug, crystal form, pharmaceutically acceptable salt, hydrate or solvate thereof, wherein:

$X_1$ is N;
$Y_1$ is O, CHD, $CD_2$ or $CH_2$;
$R_2$ is H, D or OH;
$R_3$ is H, D or $CH_2OH$;
R is F;
R' is methyl;
provided that $R_2$ and $R_3$ are not both H.

**39.** A compound, or a tautomer, stereoisomer, prodrug, crystal form, pharmaceutically acceptable salt, hydrate or solvate thereof, wherein the compound is:

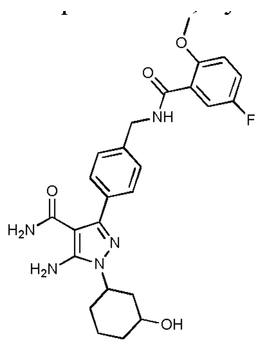 , 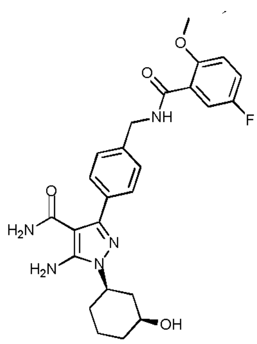 , 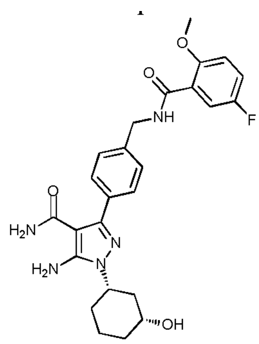 , 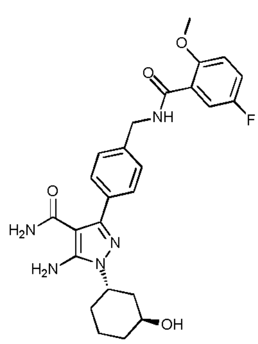 ,

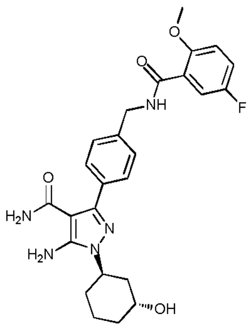,
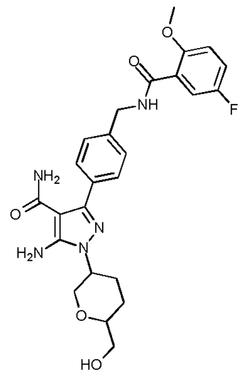,
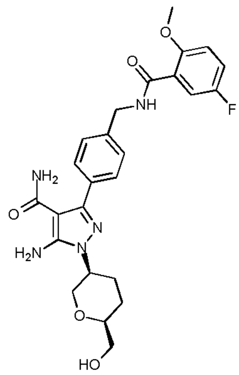,
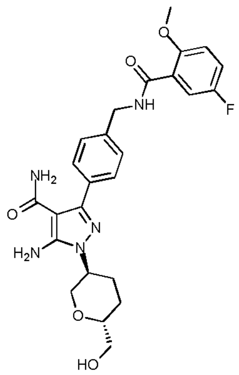,

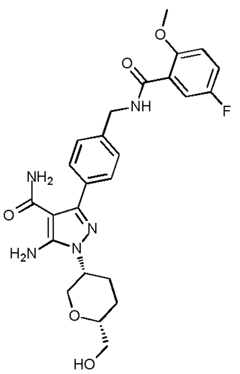,
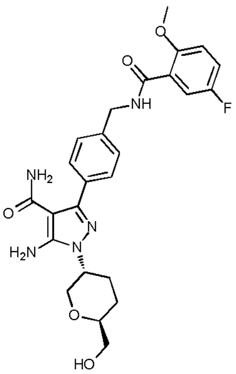,
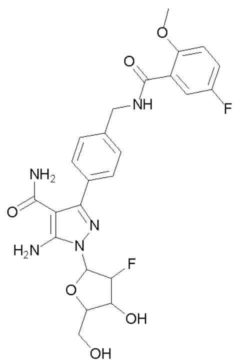,
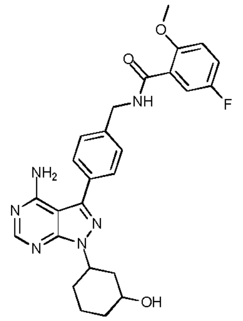,

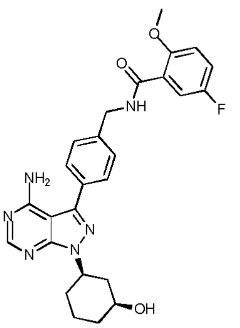,
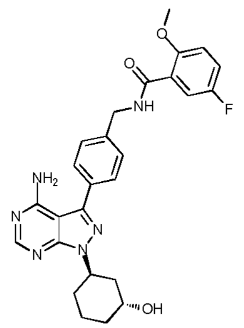,
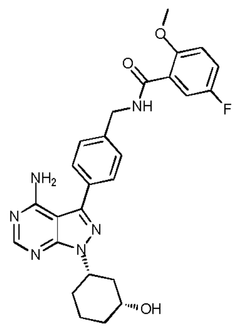,
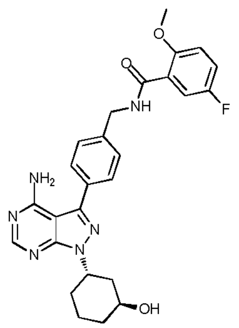,

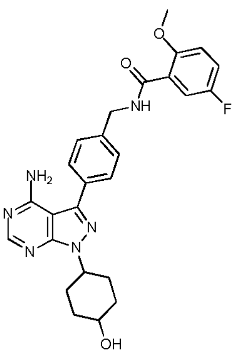,
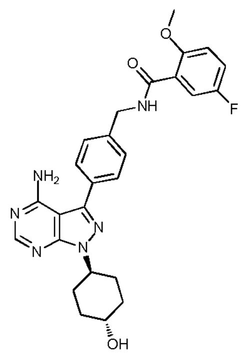,
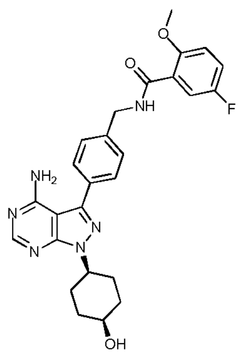,
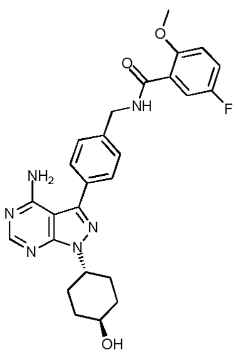,

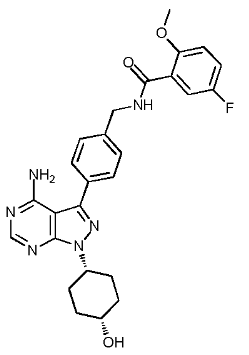,
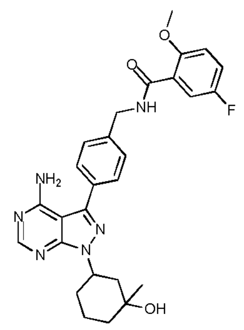,
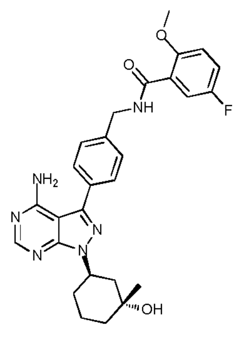,
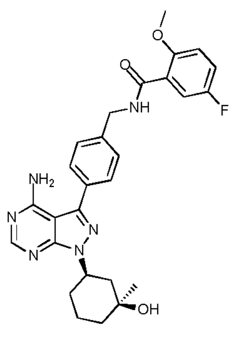,

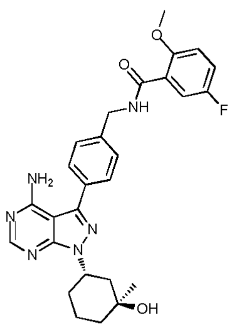,
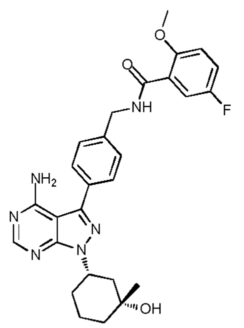,
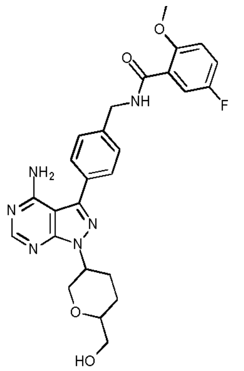,
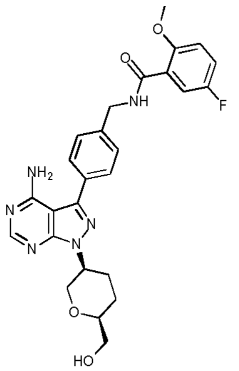,

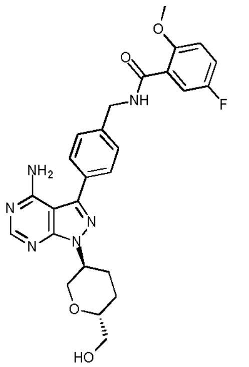,
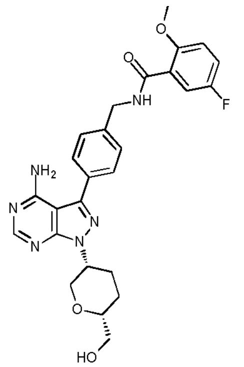,
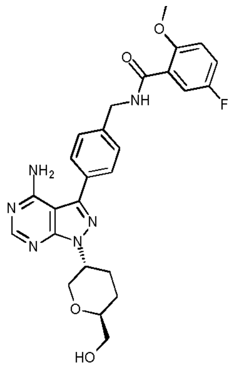,
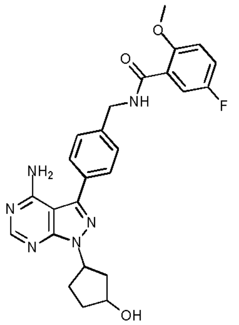,

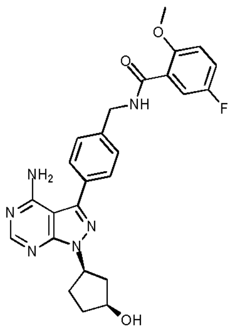,
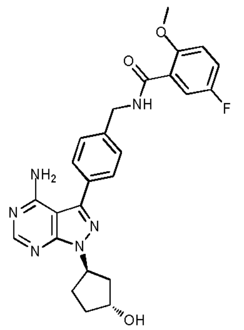,
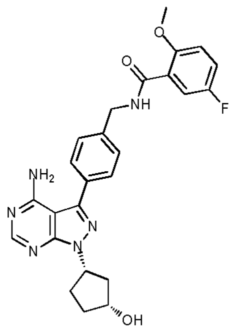,
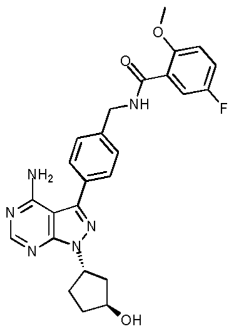,

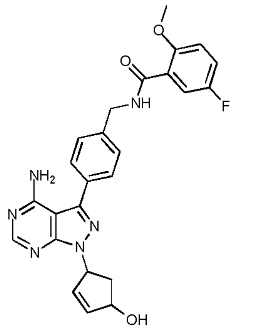 , 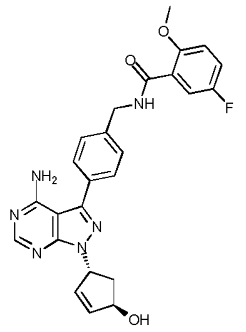 , 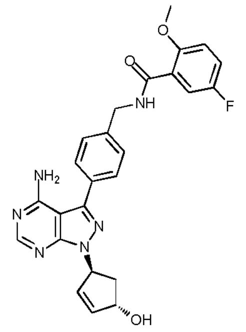 , 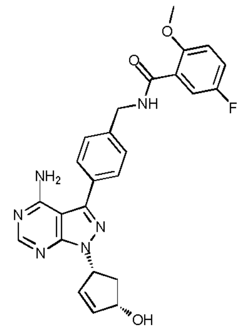 ,

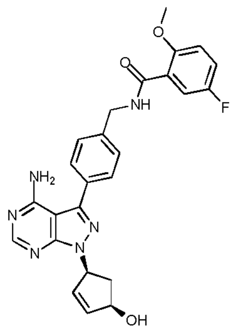 , 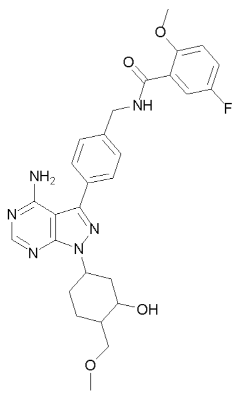 , 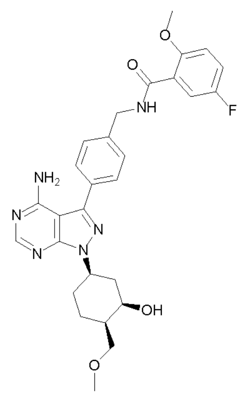 , 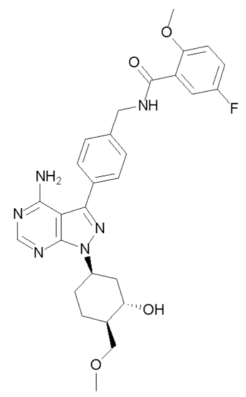 ,

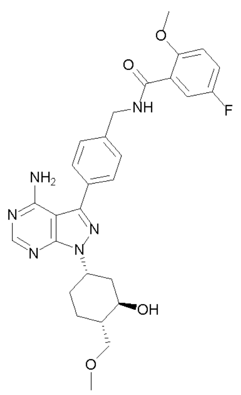 , 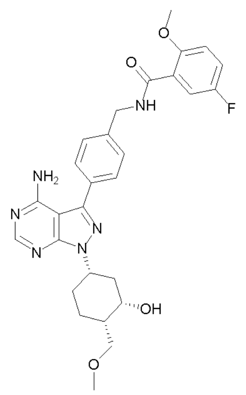 , 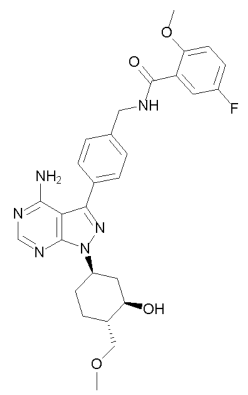 , 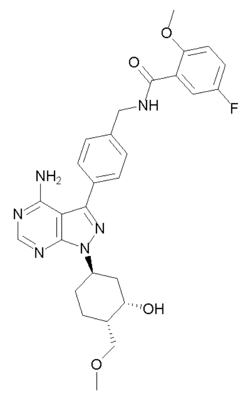 ,

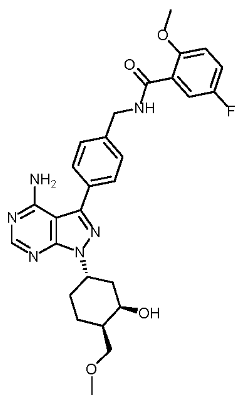 , 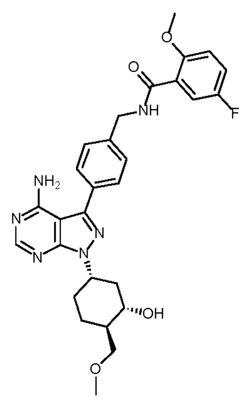 , 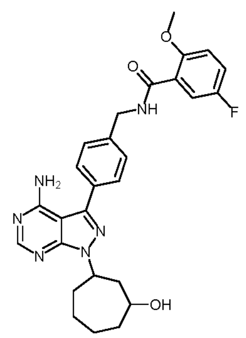 , 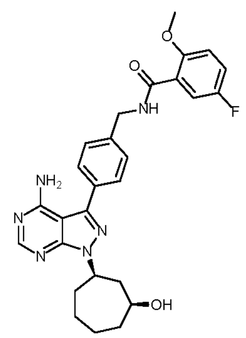 ,

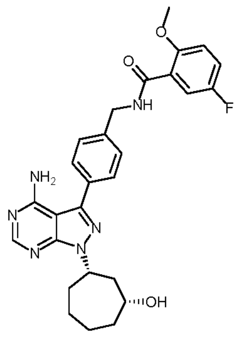 , 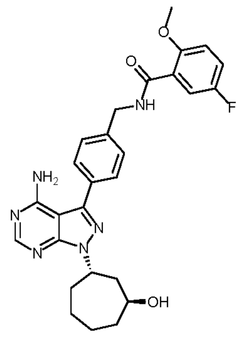 , 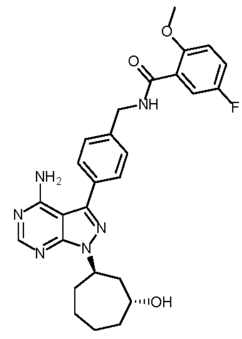 , 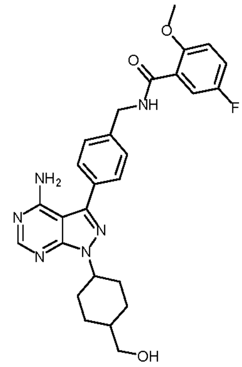 ,

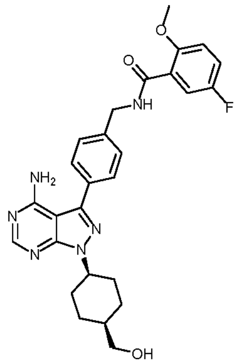 , 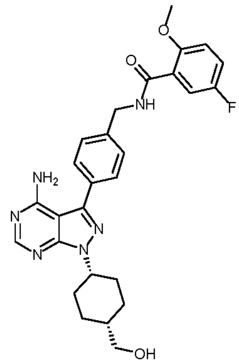 , 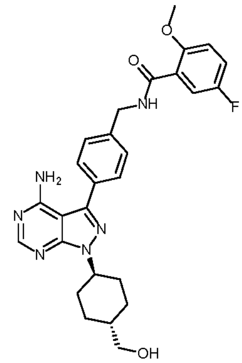 , 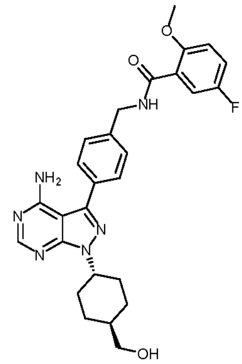 ,

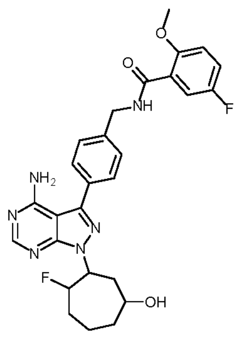 , 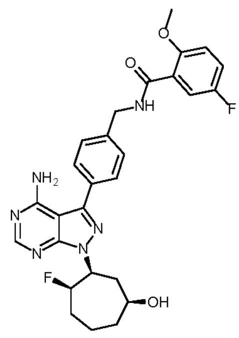 , 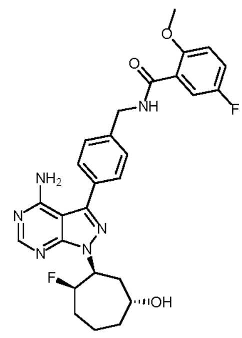 , 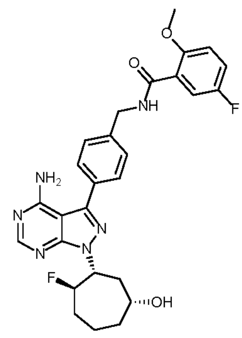 ,

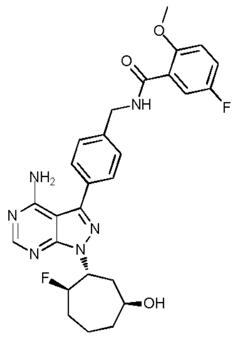 , 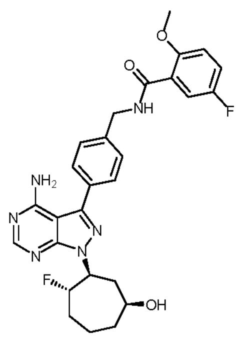 , 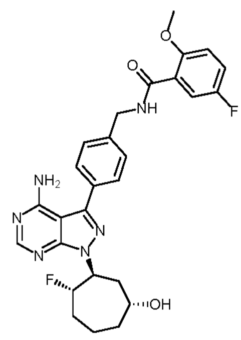 , 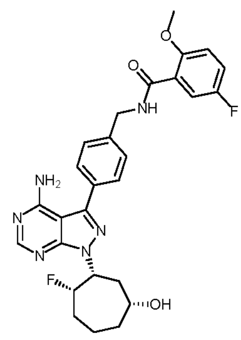 ,

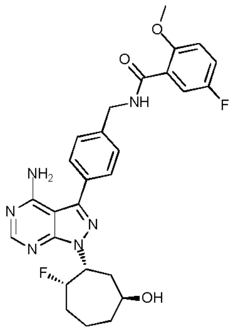,

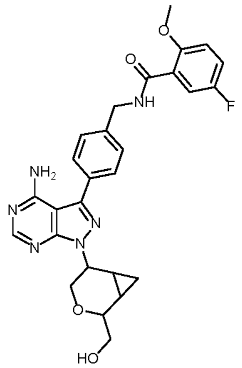,

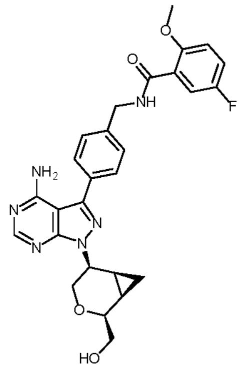,

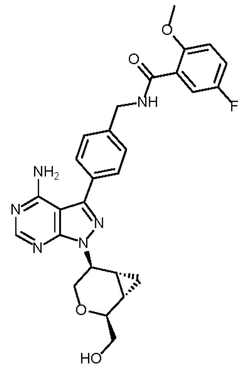,

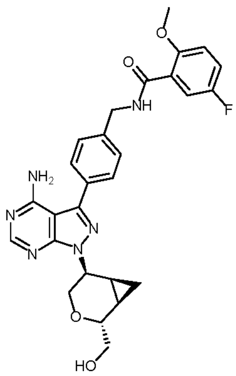,

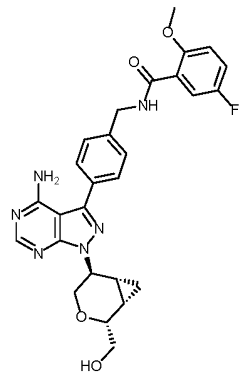,

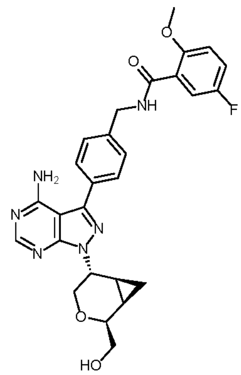,

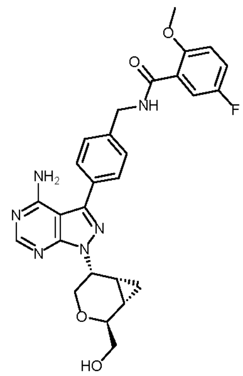,

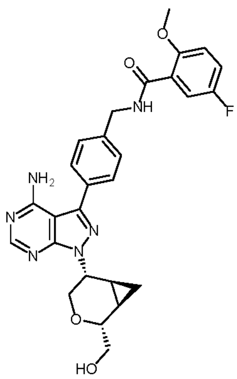,

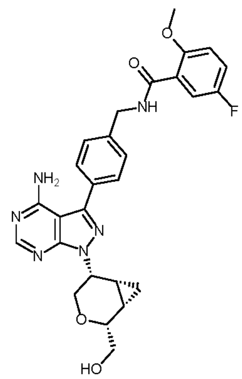,

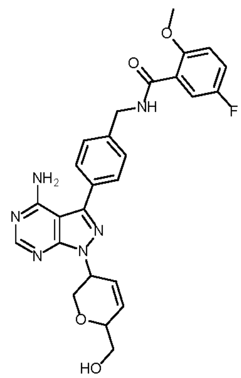,

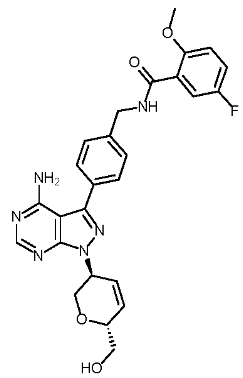,

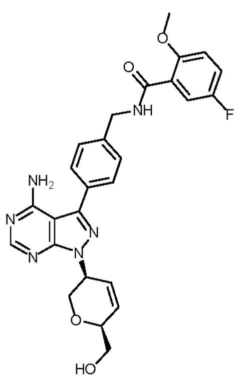,

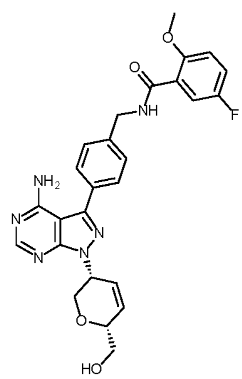,

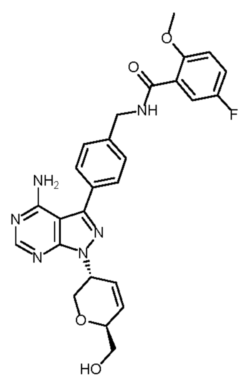,

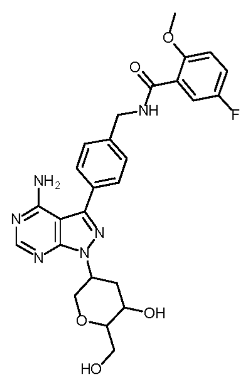,

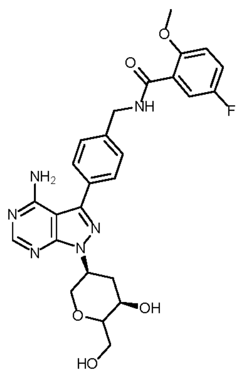

,

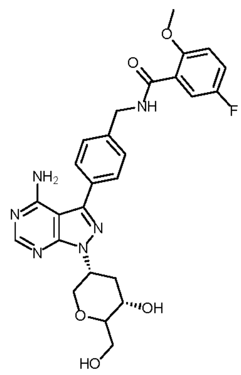

,

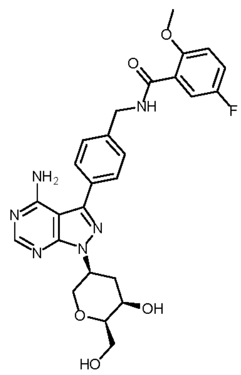

,

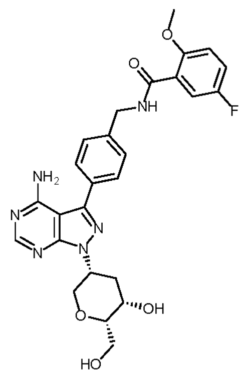

,

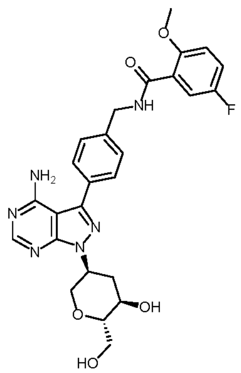

,

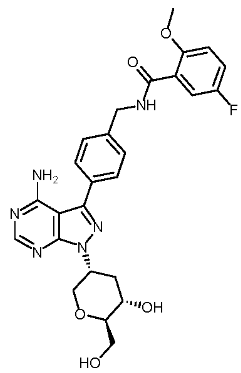

,

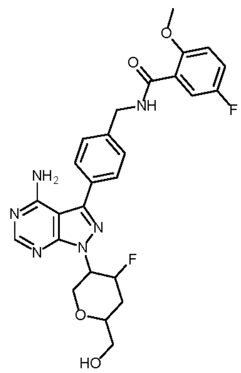

,

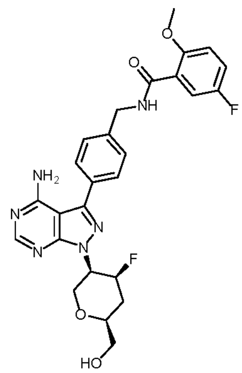

,

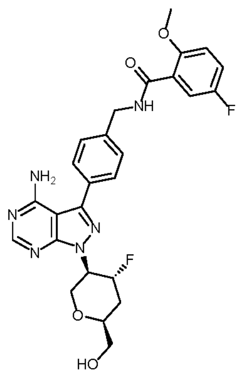

,

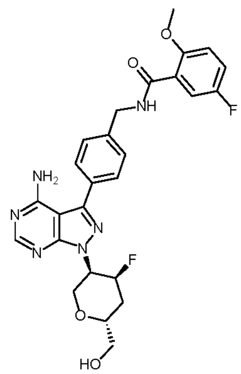

,

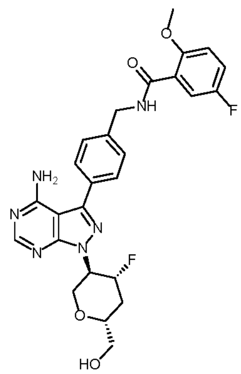

,

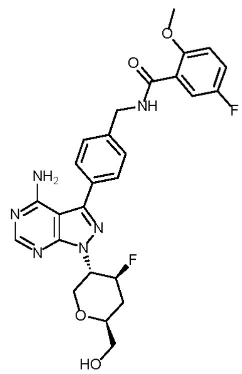

,

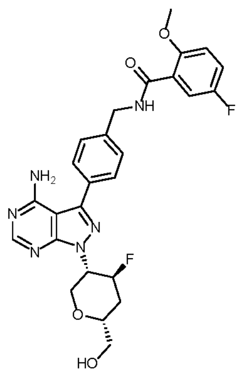

,

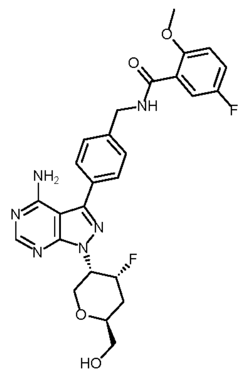

,

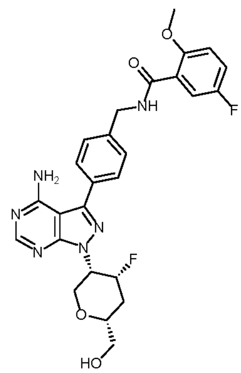

,

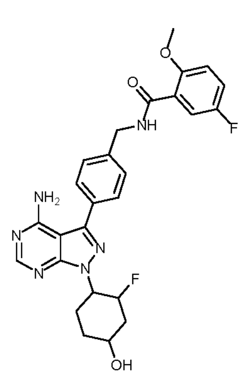

,

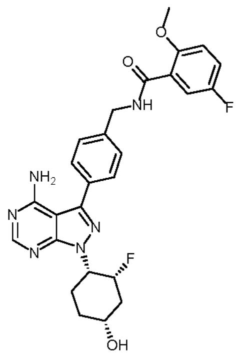, 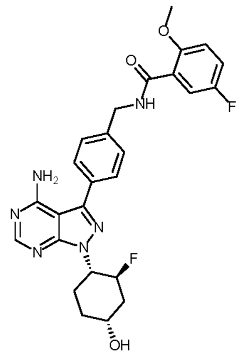, 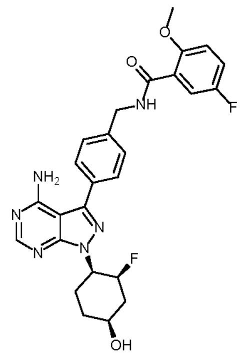, 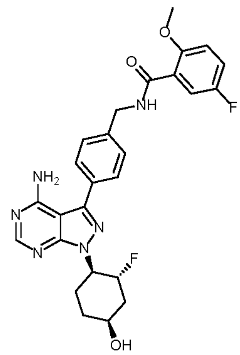,

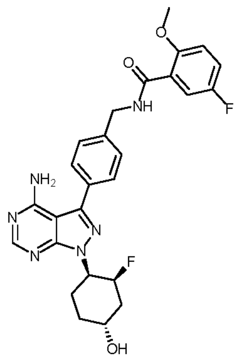, 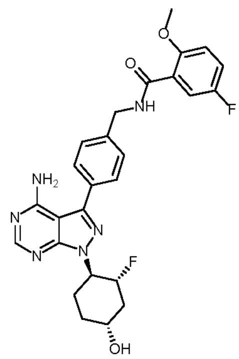, 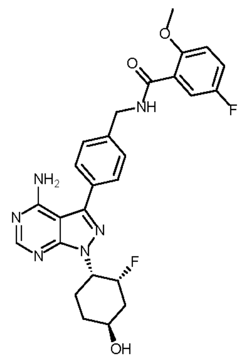, 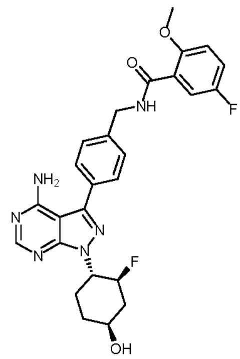,

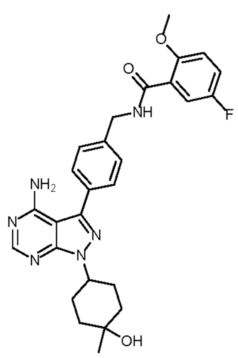, 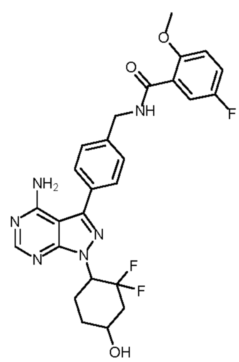, 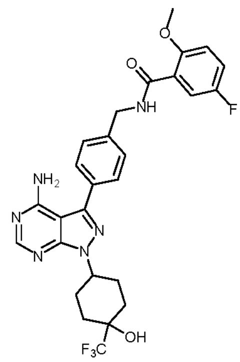, 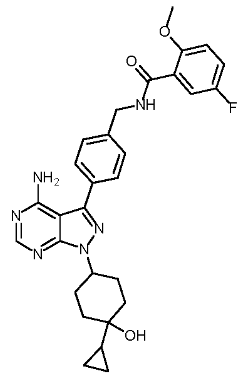,

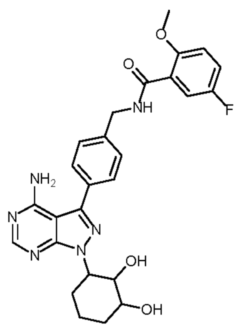, 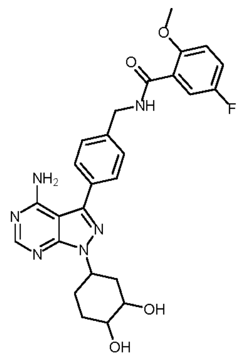, 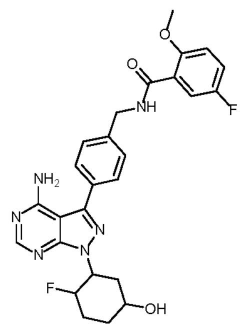, 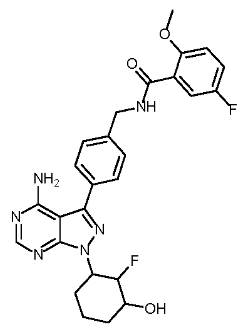,

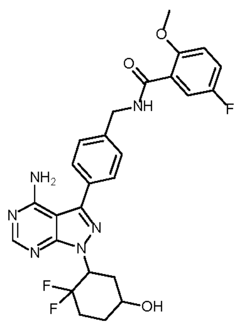,
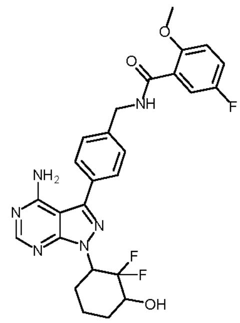,
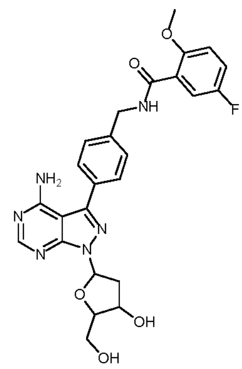,
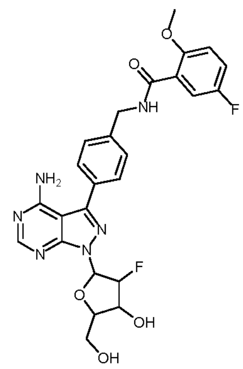,

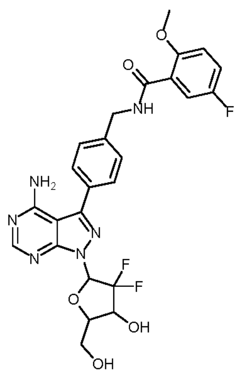,
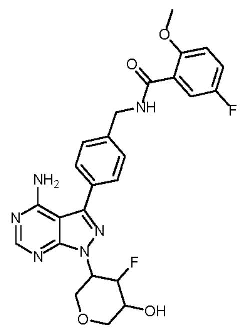,
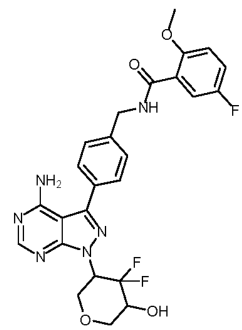,
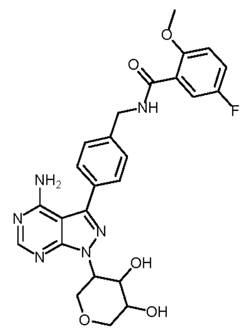,

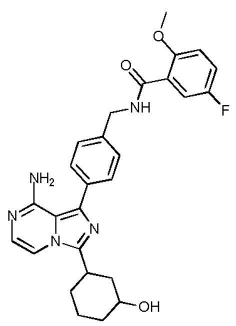,
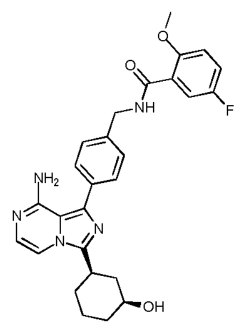,
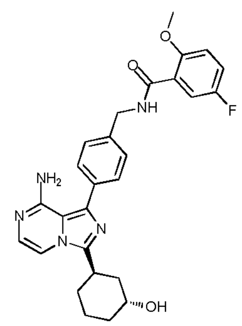,
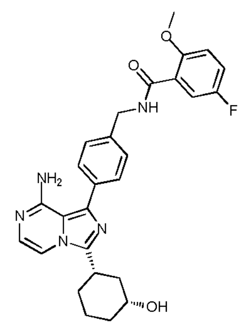,

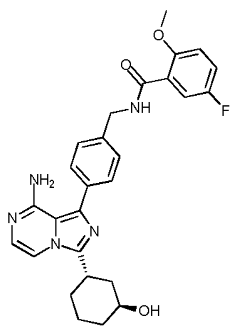,
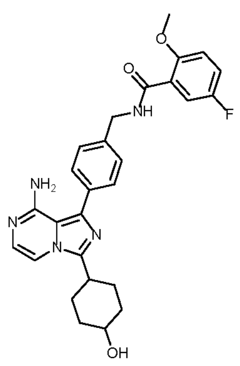,
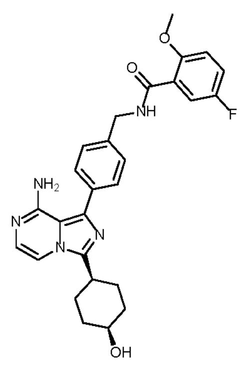,
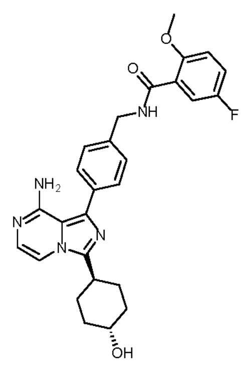,

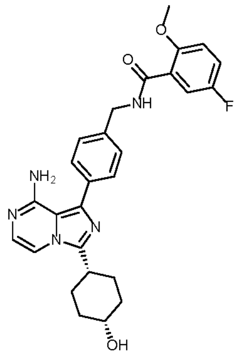 ,
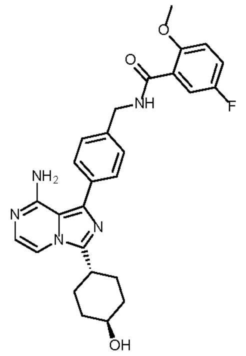 ,
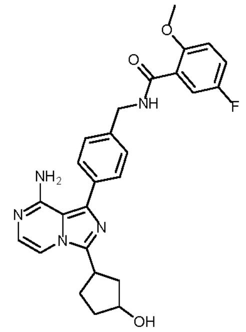 ,
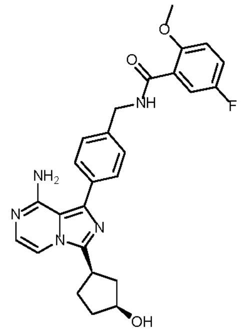 ,

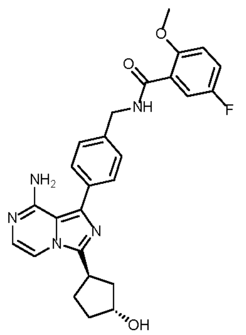 ,
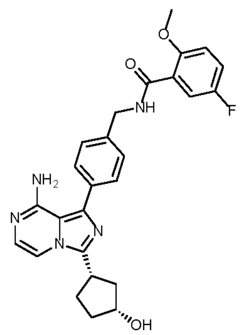 ,
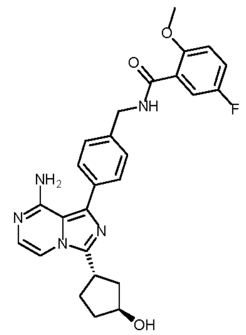 ,
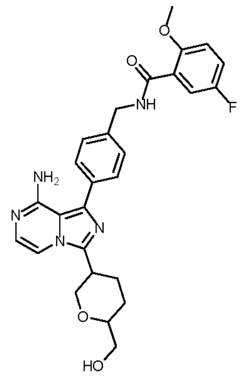 ,

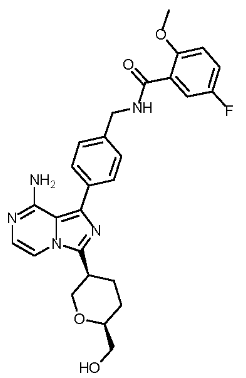 ,
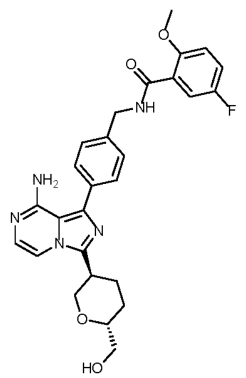 ,
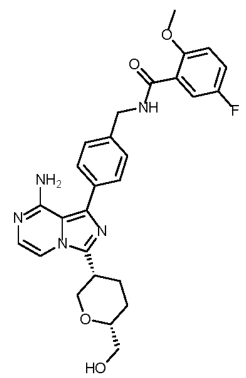 ,
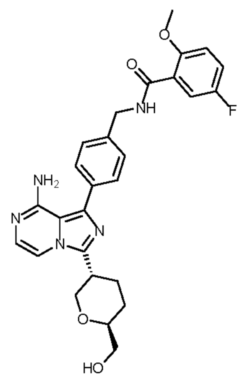 ,

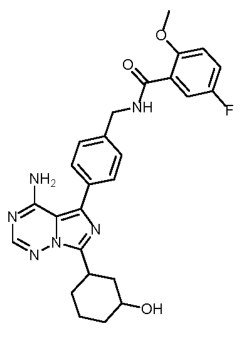 ,
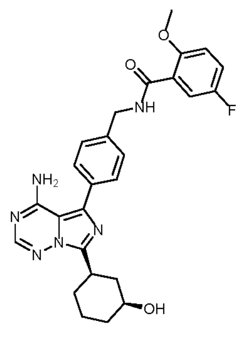 ,
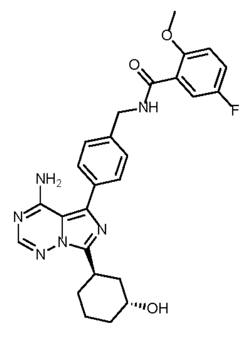 ,
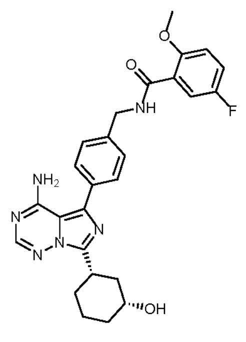 ,

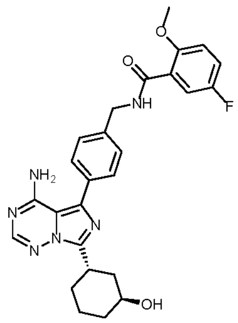 , 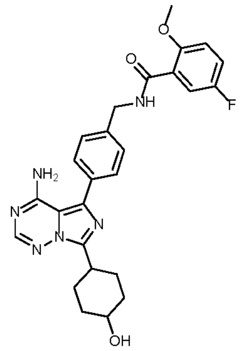 , 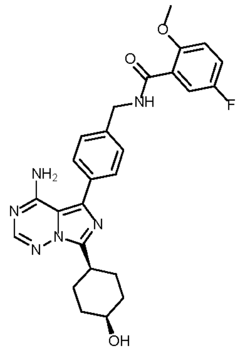 , 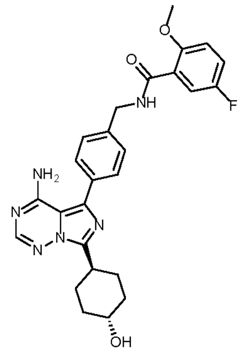 ,

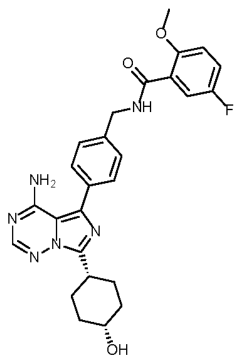 , 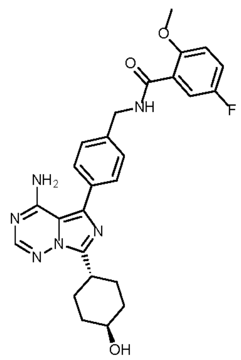 , 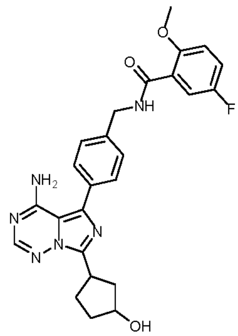 , 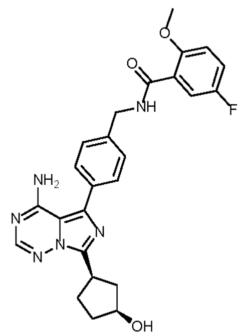 ,

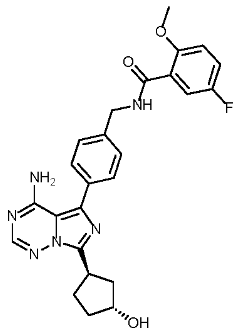 , 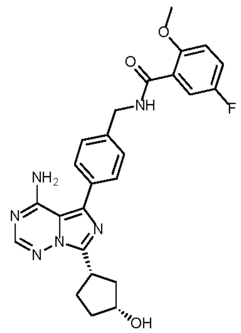 , 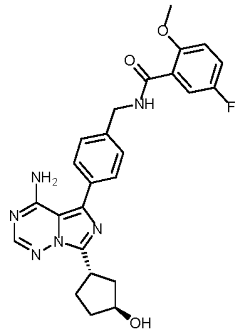 , 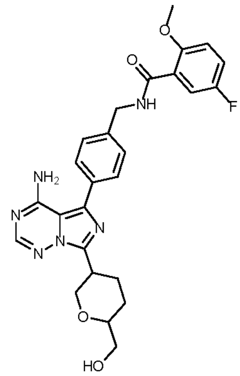 ,

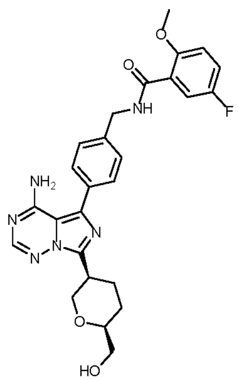 , 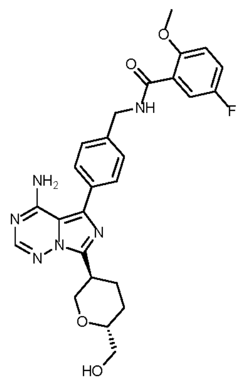 , 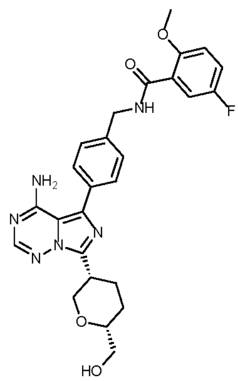 , 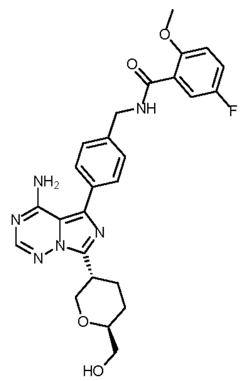 ,

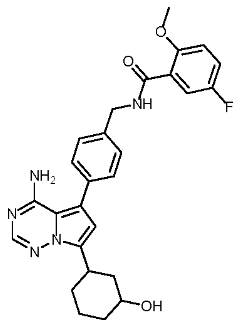 , 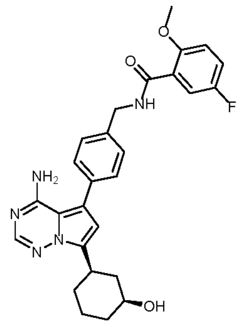 , 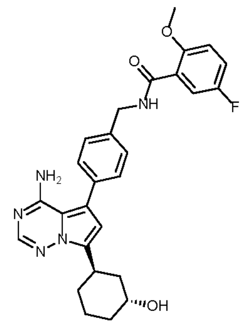 , 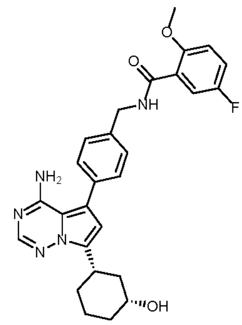 ,

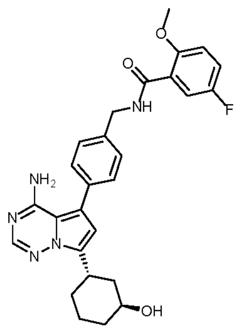 , 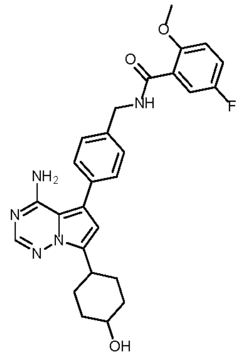 , 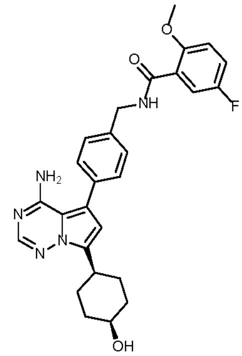 , 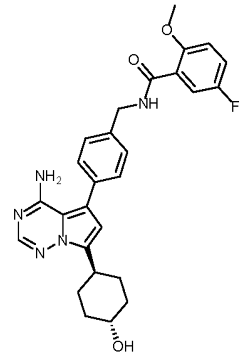 ,

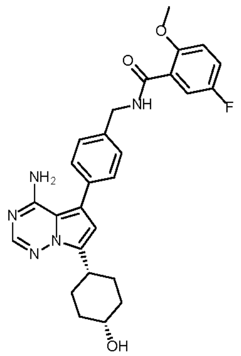 , 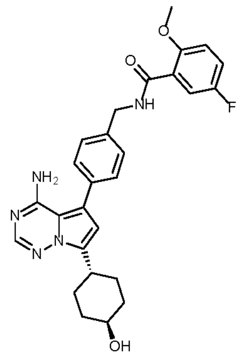 , 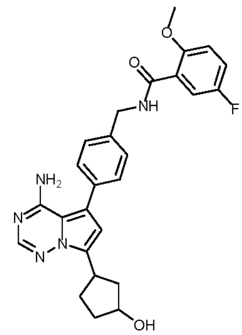 , 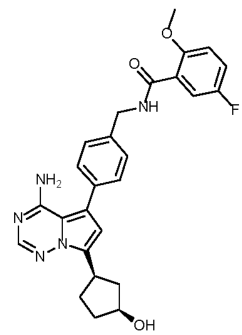 ,

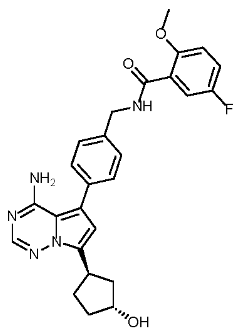 , 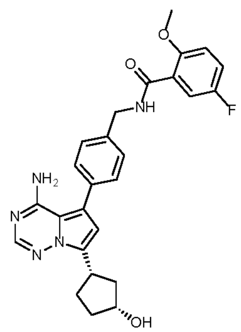 , 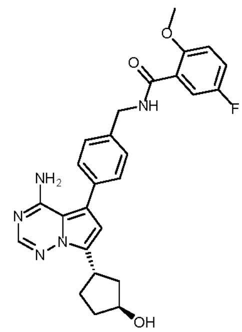 , 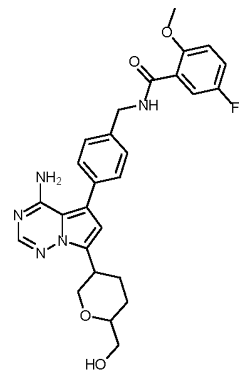 ,

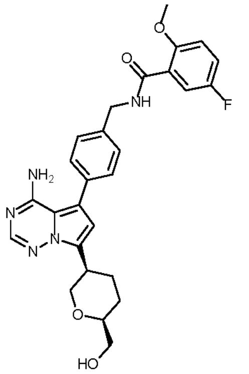 ,

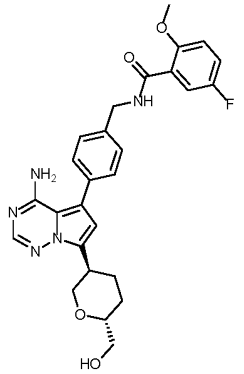 ,

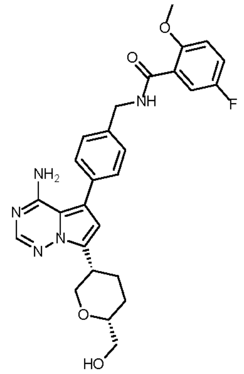 ,

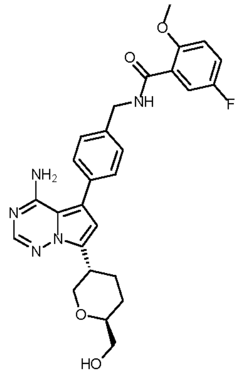 ,

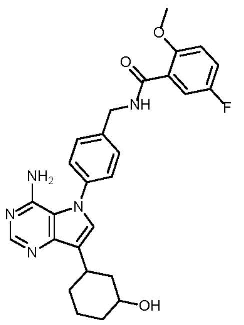 ,

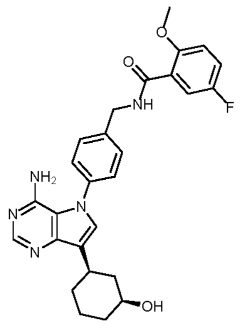 ,

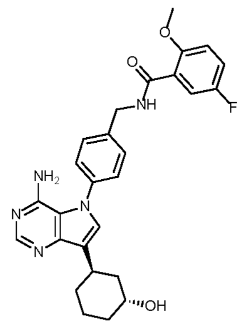 ,

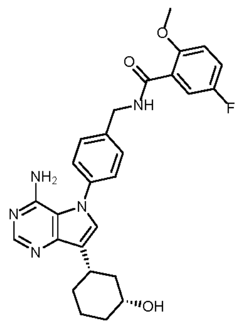 ,

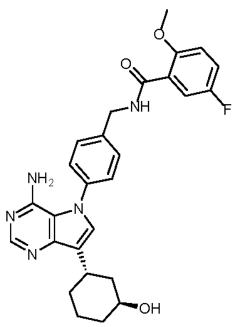 ,

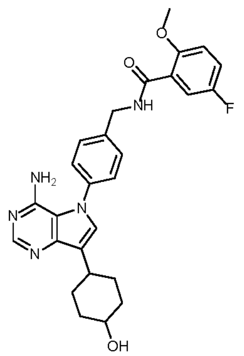 ,

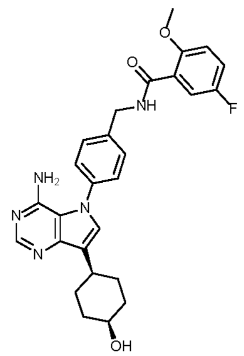 ,

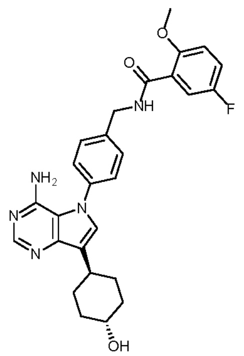 ,

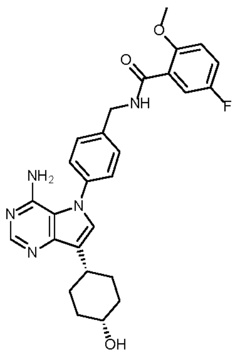 ,

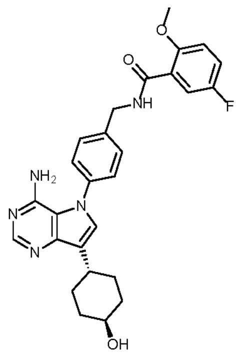 ,

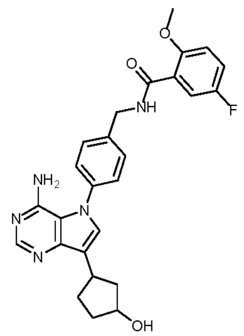 ,

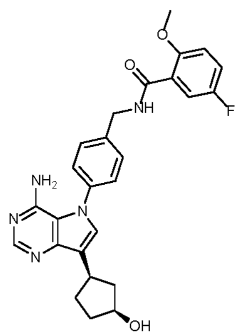 ,

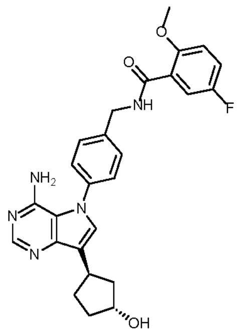 , 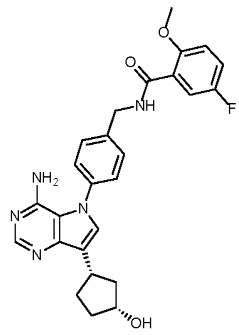 , 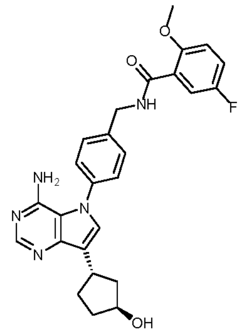 , 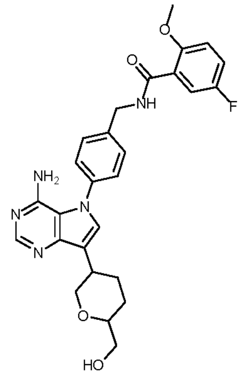 ,

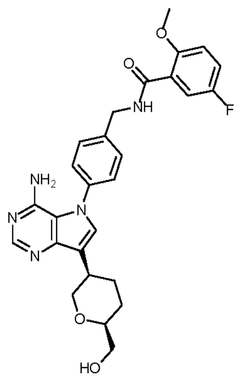 , 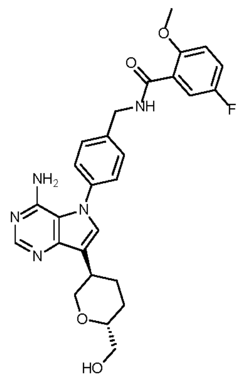 , 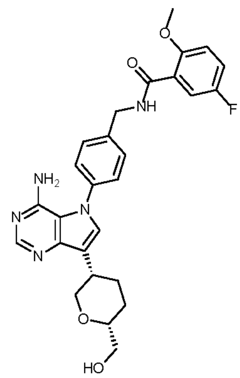 , 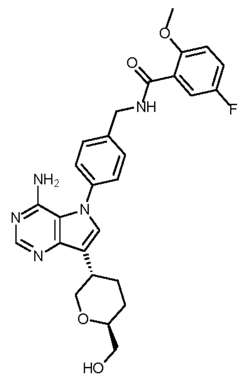 ,

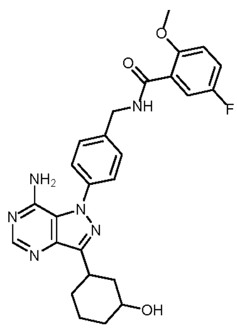 , 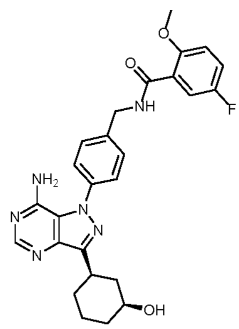 , 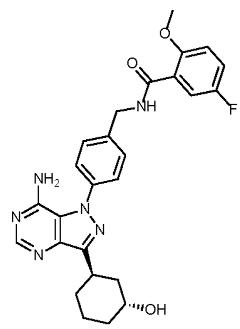 , 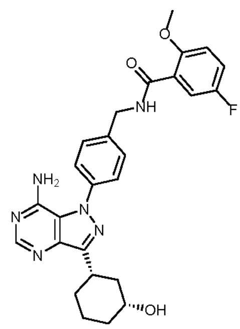 ,

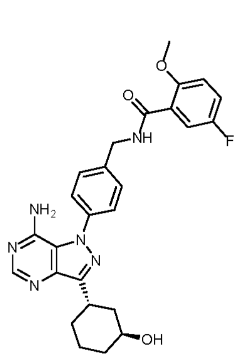 , 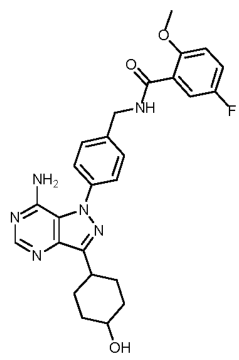 , 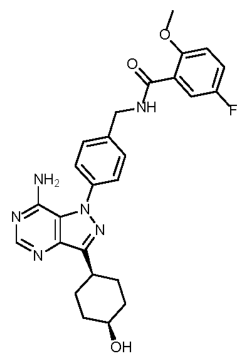 , 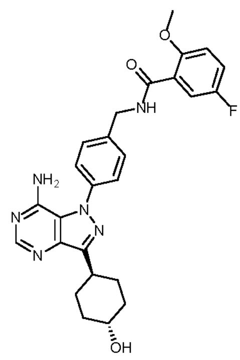 ,

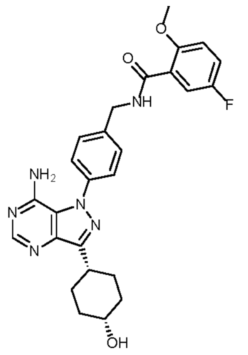,
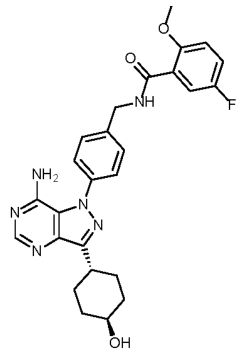,
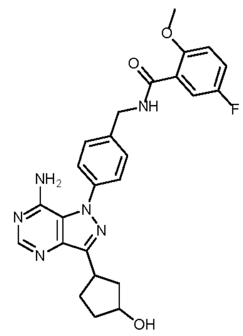,
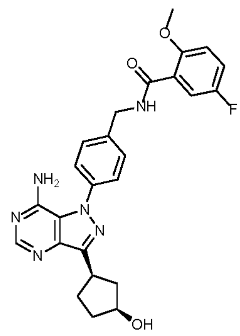,

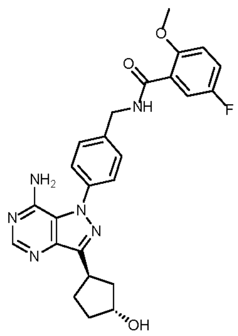,
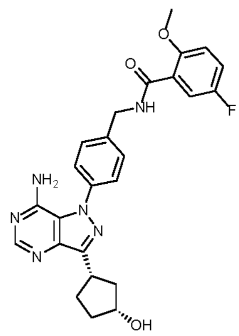,
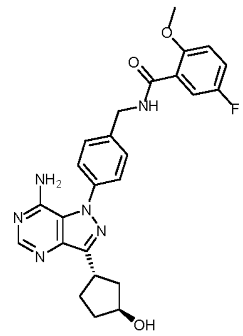,
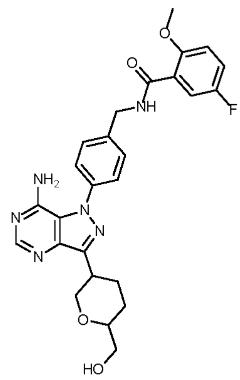,

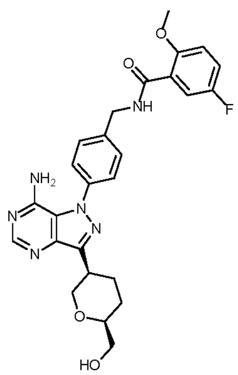,
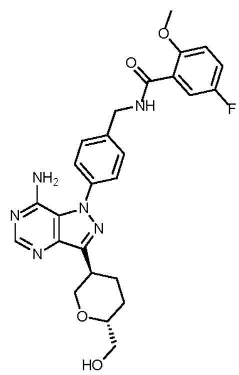,
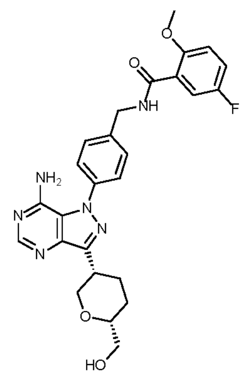,
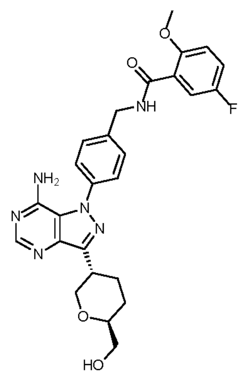,

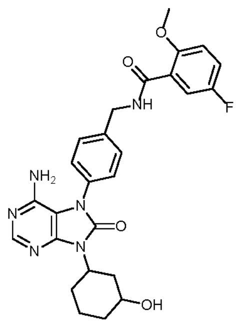,
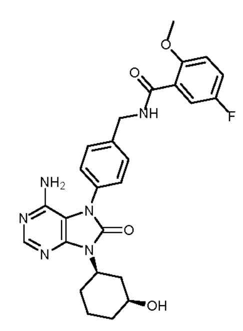,
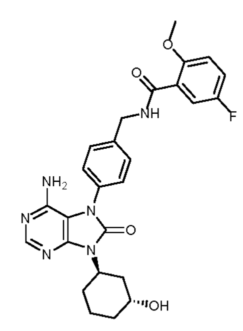,
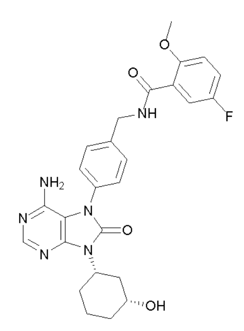,

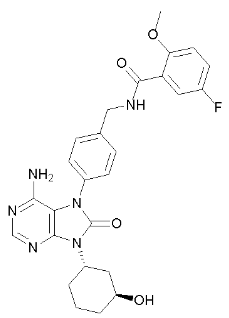 ,

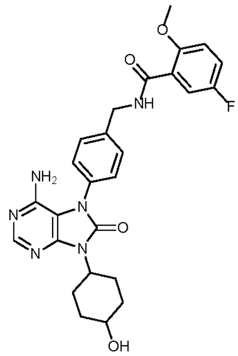 ,

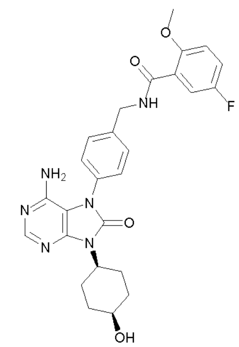 ,

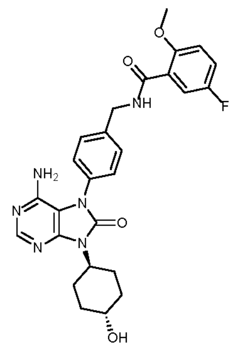 ,

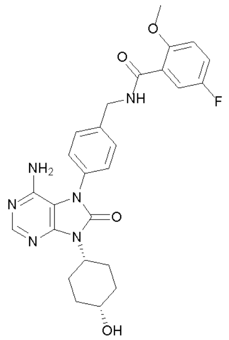 ,

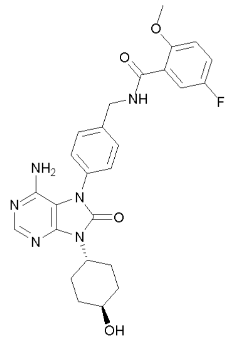 ,

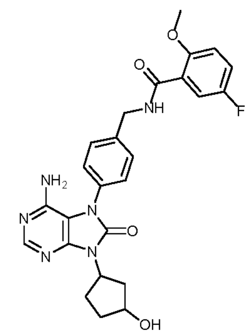 ,

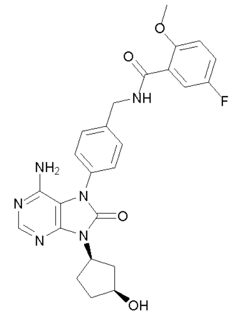 ,

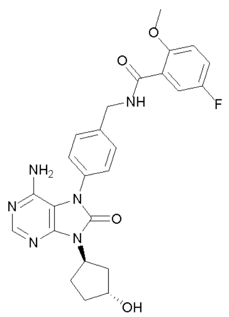 ,

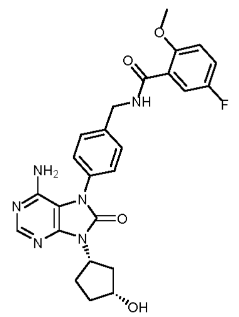 ,

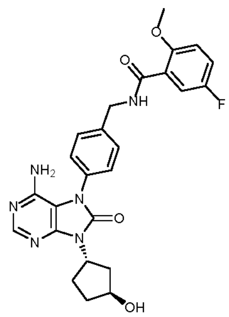 ,

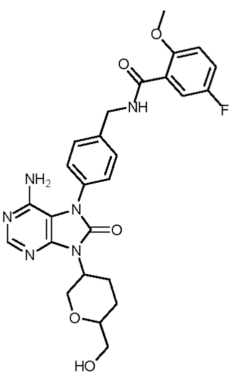 ,

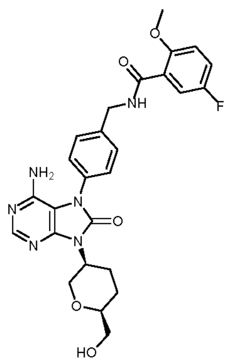 ,

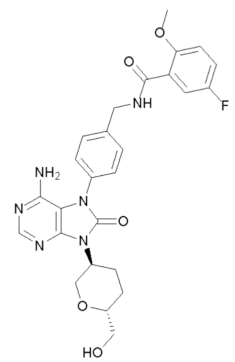 ,

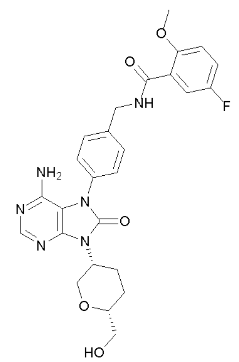 ,

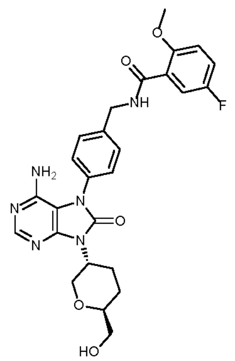 ,

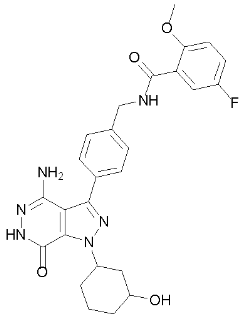 , 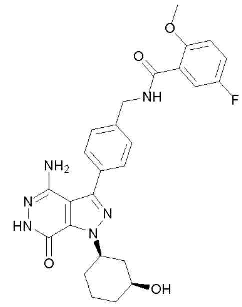 , 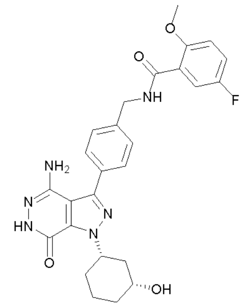 , 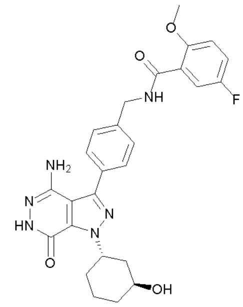 ,

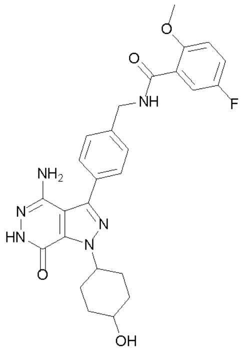 , 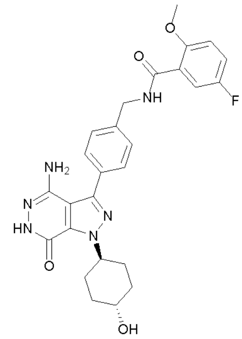 , 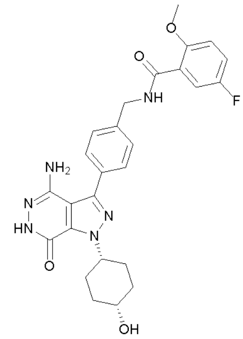 , 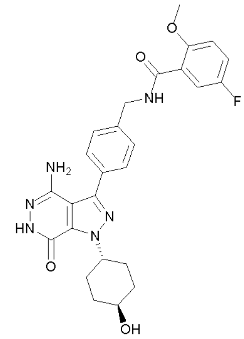 ,

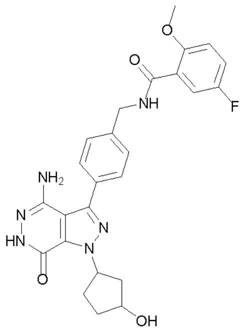 , 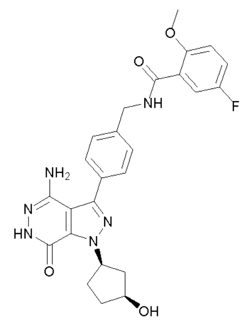 , 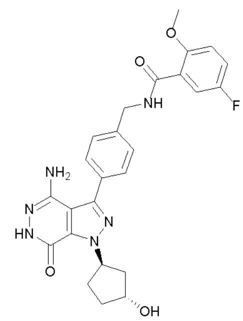 , 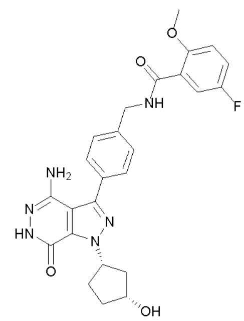 ,

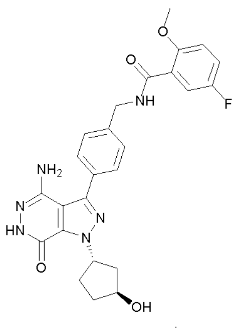 , 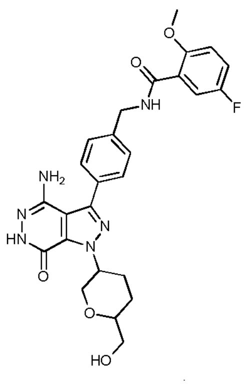 , 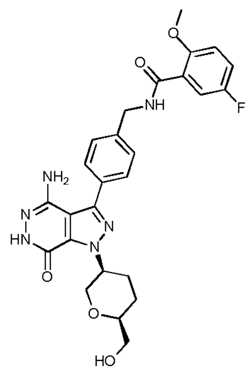 , 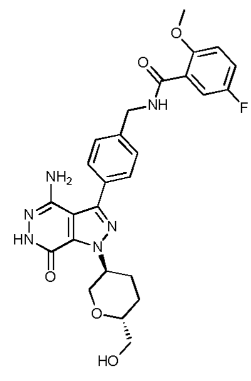 ,

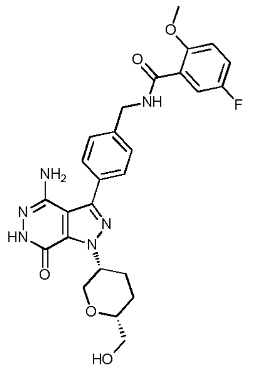 , 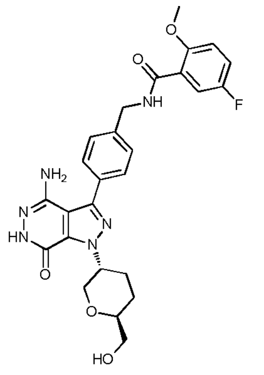 , 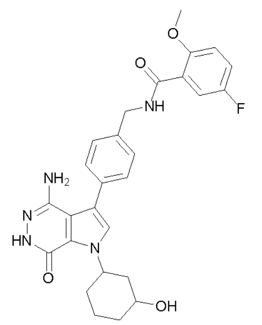 , 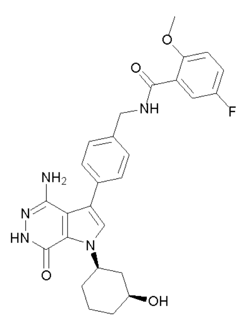 ,

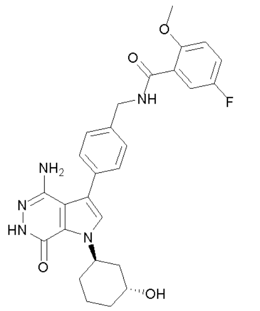 , 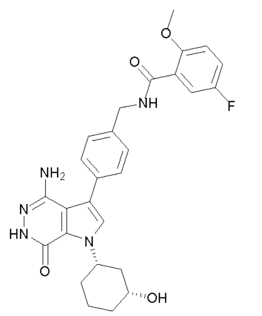 , 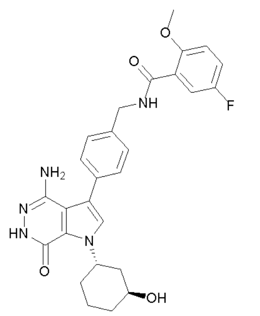 , 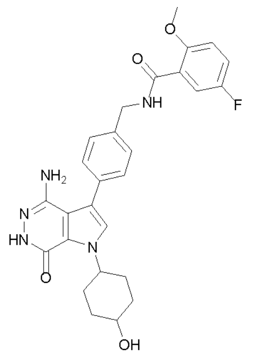 ,

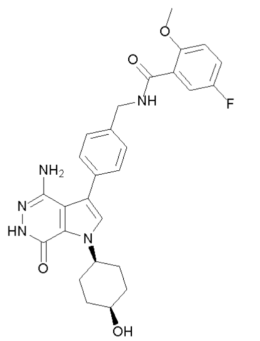 , 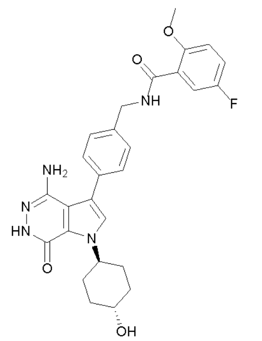 , 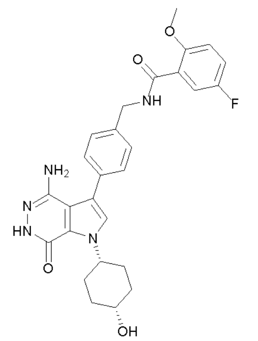 , 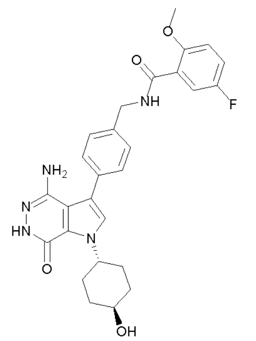 ,

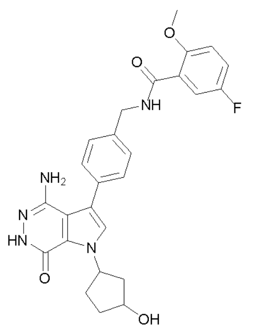 , 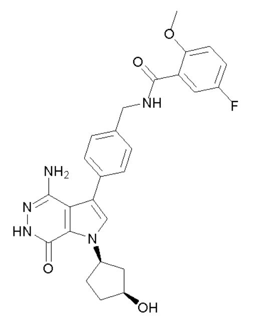 , 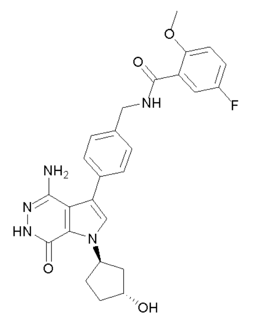 , 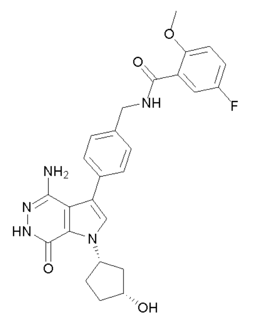 ,

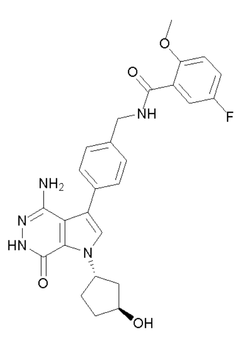 , 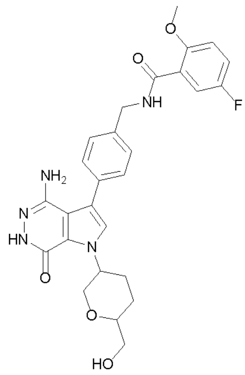 , 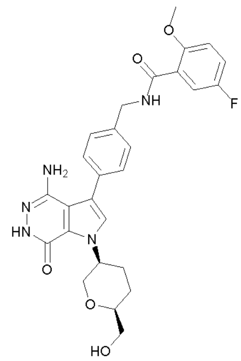 , 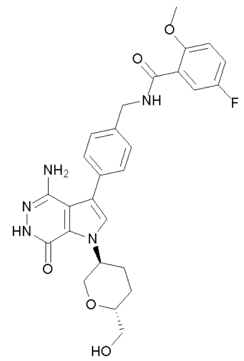 ,

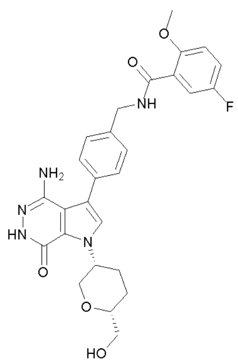 , 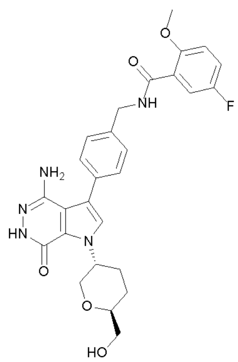 , 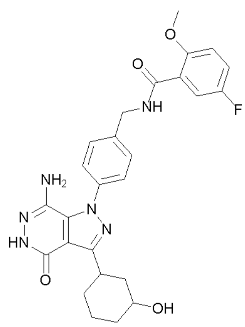 , 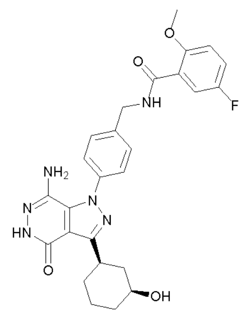 ,

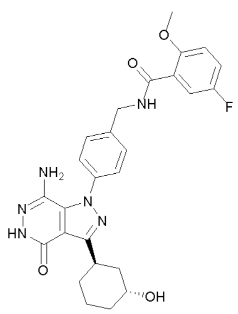 , 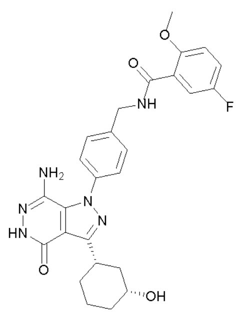 , 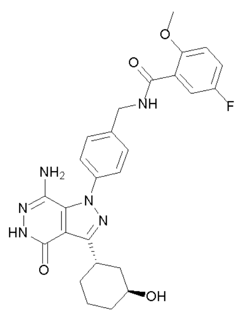 , 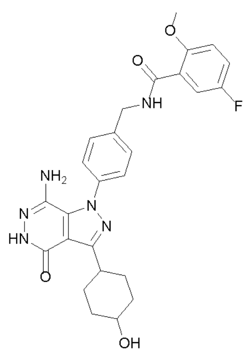 ,

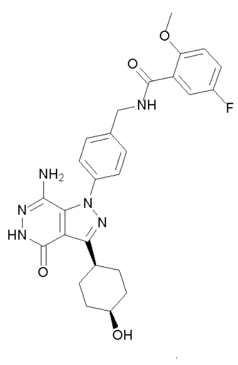 , 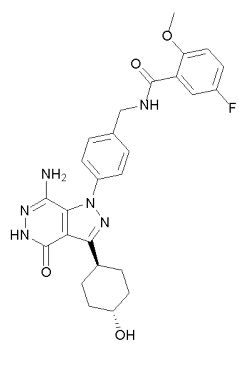 , 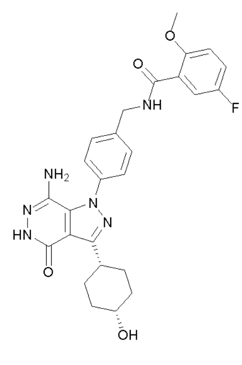 , 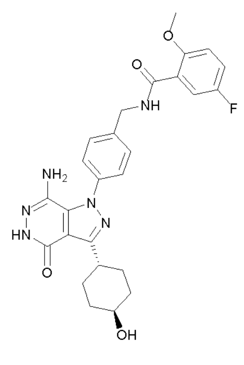 ,

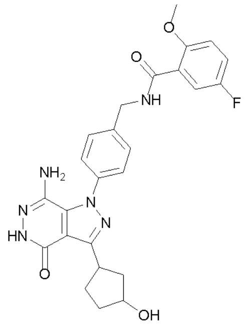,
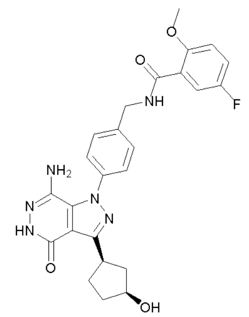,
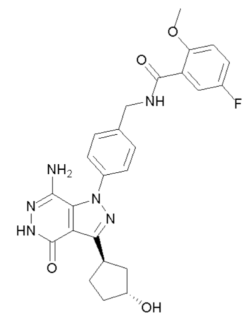,
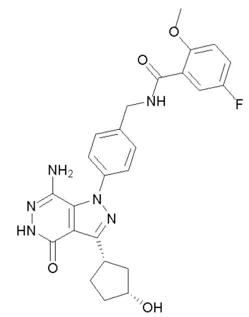,

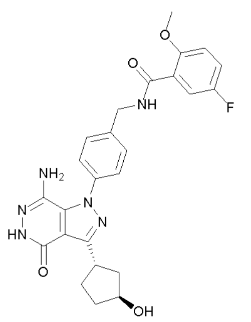,
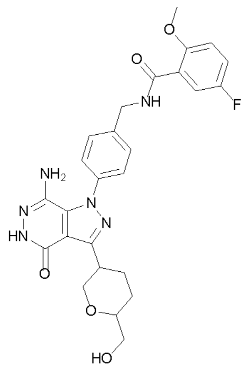,
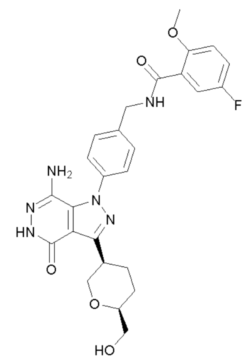,
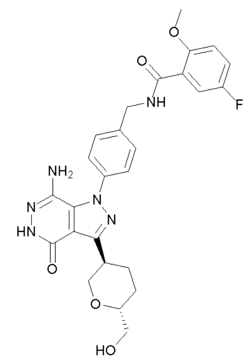,

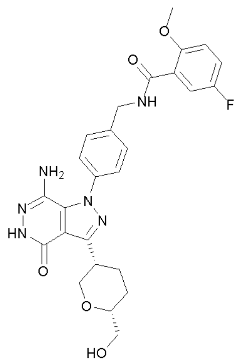,
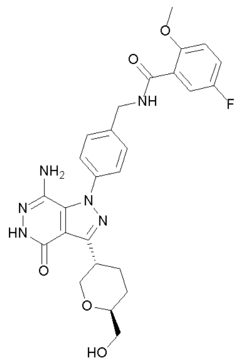,
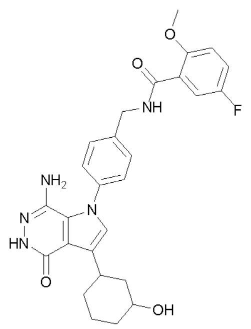,
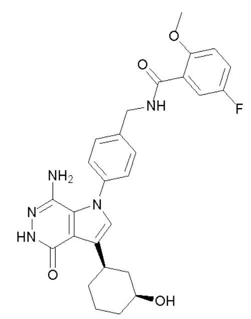,

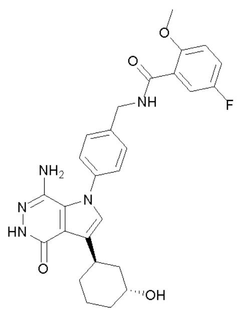,
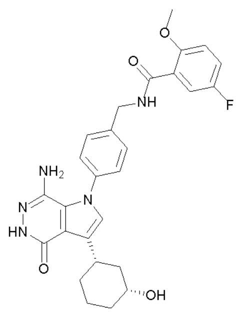,
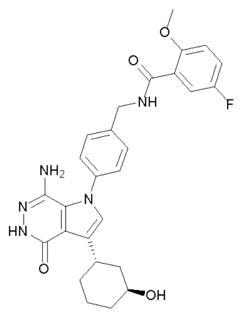,
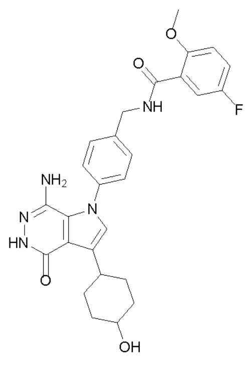,

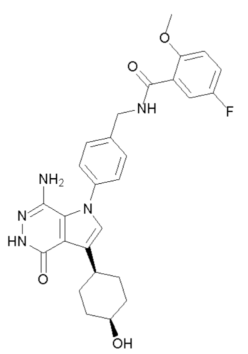 , 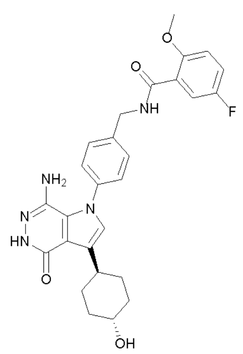 , 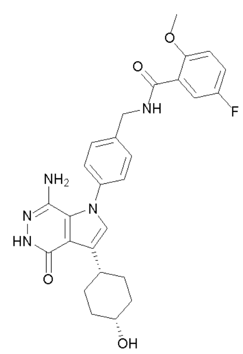 , 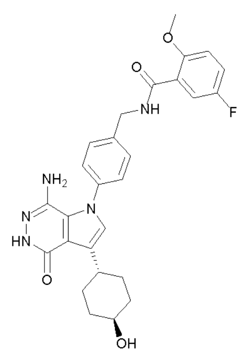 ,

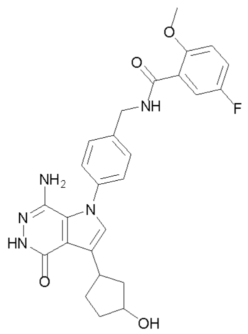 , 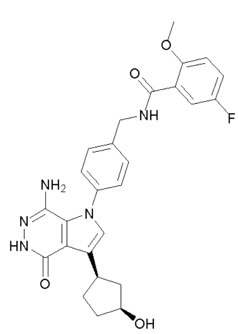 , 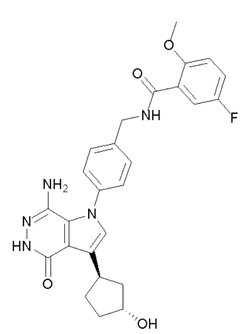 , 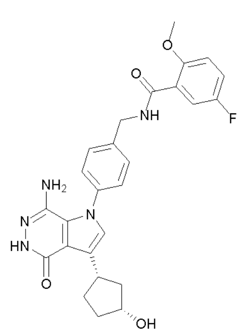 ,

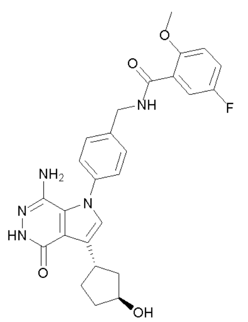 , 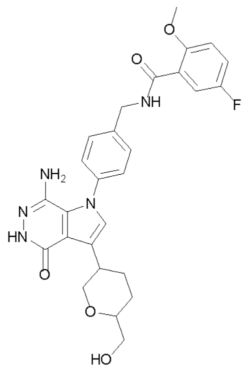 , 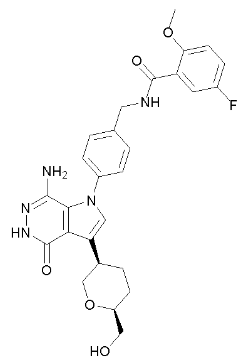 , 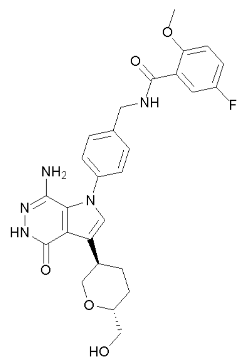 ,

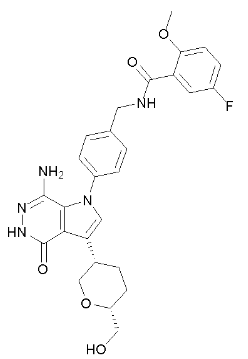 , 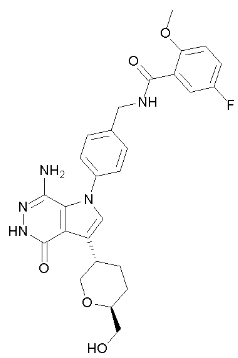 , 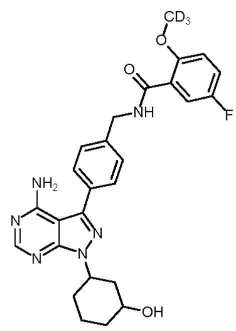 , 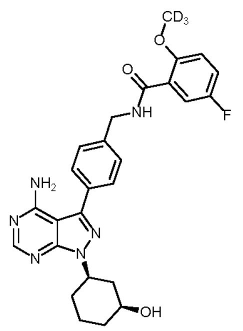 ,

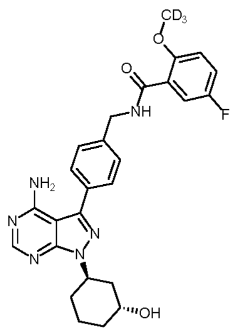 , 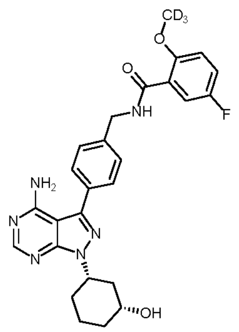 , 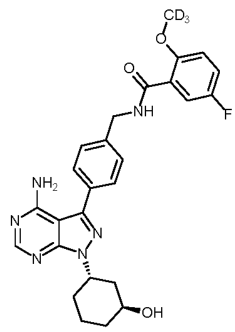 , 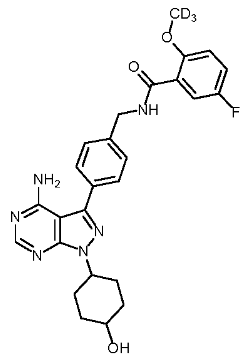 ,

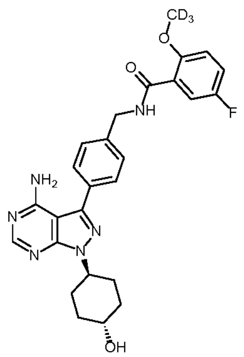 , 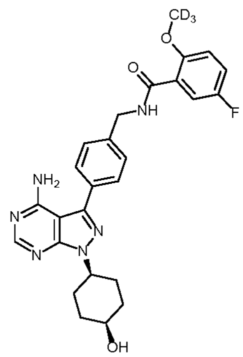 , 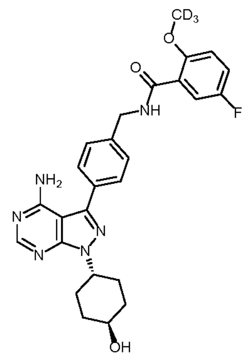 , 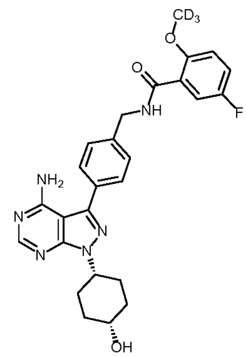 ,

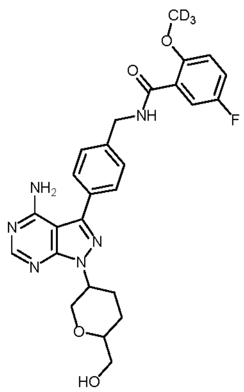 , 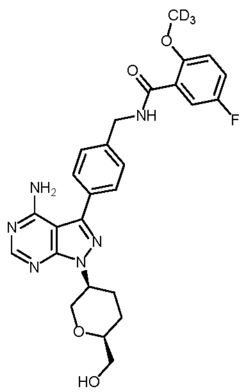 , 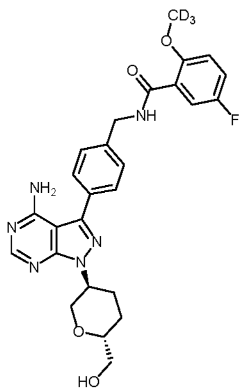 , 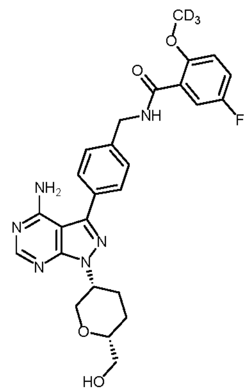

or

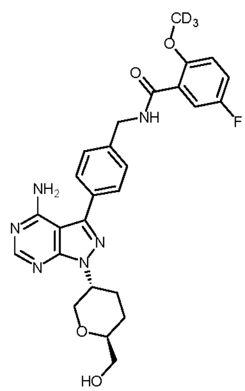

**40.** A pharmaceutical composition comprising a compound according to any one of claims 1 to 39, or a tautomer, stereoisomer, prodrug, crystal form, pharmaceutically acceptable salt, hydrate or solvate thereof, and pharmaceutically acceptable excipients, and optionally, other therapeutic agents.

**41.** Use of a compound of any one of claims 1 to 39, or a tautomer, stereoisomer, prodrug, crystal form, pharmaceutically acceptable salt, hydrate or solvate thereof, or a pharmaceutical composition according to claim 40 in the manufacture of a medicament for treating and/or preventing a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

**42.** A method of treating and/or preventing a wild and/or mutated BTK kinase-mediated disease in a subject, the method comprising administering to the subject a compound of any one of claims 1-39, or a pharmaceutically acceptable salt, stereoisomer, solvate, hydrate, polymorph, prodrug, or isotopic variant thereof, or a pharmaceutical composition of claim 40; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

**43.** A compound of any one of claims 1-39, or a tautomer, stereoisomer, prodrug, crystal form, pharmaceutically acceptable salt, hydrate or solvate thereof, or a pharmaceutical composition of claim 40, for use in the treatment and/or prevention of a wild and/or mutated BTK kinase-mediated disease; optionally, the mutated BTK kinase is selected from BTK C481S, BTK C481F, BTK C481Y, BTK C481R, BTK C481T, BTK C481G and BTK C481W; optionally, the mutated BTK kinase is BTK C481S.

**44.** The use of claim 41, or the method of claim 42, or the compound or composition for use according to claim 43, wherein the BTK-mediated disease is selected from an allergic disease, an autoimmune disease, an inflammatory disease, and a cancer.

**45.** The use of claim 41, or the method of claim 42, or the compound or composition for use according to claim 43, wherein the BTK-mediated disease is a B-cell proliferative disease selected from chronic lymphocytic lymphoma, non-Hodgkin's lymphoma, diffuse large B-cell lymphoma, mantle cell lymphoma, follicular lymphoma, chronic lymphocytic leukemia, acute lymphocytic leukemia, acute myeloid leukemia, acute promyelocytic leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplastic disorder, mixed lineage leukemia, myelodysplastic syndrome, myeloproliferative disease, marginal zone lymphoma, and Waldenstrom's macroglobulinemia.

**46.** The use of claim 41 or the method of claim 42 or the compound or composition for use according to claim 43, wherein the BTK-mediated disease is multiple myeloma.

**INTERNATIONAL SEARCH REPORT**

International application No.
**PCT/CN2023/079360**

**A. CLASSIFICATION OF SUBJECT MATTER**

C07D231/38(2006.01)i; C07D471/04(2006.01)i; C07D487/04(2006.01)i; A61K31/522(2006.01)i; A61K31/437(2006.01)i; A61K31/53(2006.01)i; A61K31/519(2006.01)i; A61P35/00(2006.01)i; A61P37/02(2006.01)i; A61P35/02(2006.01)i; A61P29/00(2006.01)i; A61P19/02(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN; ISI Web of Science: 深圳市塔吉瑞生物医药有限公司, 王义汉, 李焕银, 艾义新, 酰胺, 癌症, 淋巴瘤, 白血病, 自身免疫疾病, 炎性疾病, BTK, amide, cancer, lymphoma, leukemia, autoimmune disease, inflammatory disease, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 114075190 A (BEIJING NUOCHENG JIANHUA MEDICINE TECHNOLOGY CO., LTD.) 22 February 2022 (2022-02-22)<br>description paragraphs [0037-0055], [0069-0071], claim 9 | 1-41, 44-46 (in part) |
| X | CN 108431007 A (REDX PHARMA PLC) 21 August 2018 (2018-08-21)<br>claim 14, description paragraphs [0185]-[0189], [0930] | 1-41, 44-46 (in part) |
| PX | CN 114380820 A (HEIXIDAE MEDICINE GROUP STOCK LIMITED COMPANY) 22 April 2022 (2022-04-22)<br>claims 1-10 | 1-41, 44-46 (in part) |
| PX | CN 114573586 A (HANGZHOU HERTZ PHARMACEUTICAL CO., LTD.) 03 June 2022 (2022-06-03)<br>claims 1-15 | 1-41, 44-46 (in part) |
| PX | CN 115707705 A (SICHUAN KELUN-BIOTECH BIOPHARMACEUTICAL CO., LTD.) 21 February 2023 (2023-02-21)<br>claims 1-13 | 1-41, 44-46 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 May 2023** | **11 June 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/079360**

**C. DOCUMENTS CONSIDERED TO BE RELEVANT**

| Category* | Citation of document, with indication, where appropriate, of the relevant passages | Relevant to claim No. |
|---|---|---|
| PX | US 2022169645 A1 (ETERNITY BIOSCIENCE INC.) 02 June 2022 (2022-06-02)<br>claims 1-25 | 1-41, 44-46 (in part) |
| A | CN 109851620 A (SHENZHEN TARGETRX, INC.) 07 June 2019 (2019-06-07)<br>entire document | 1-41, 44-46 (in part) |
| A | CN 109111439 A (SHENZHEN TARGETRX, INC.) 01 January 2019 (2019-01-01)<br>entire document | 1-41, 44-46 (in part) |

Form PCT/ISA/210 (second sheet) (July 2022)

**INTERNATIONAL SEARCH REPORT**

International application No.
**PCT/CN2023/079360**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **42, 43, 44-46 (in part)**
because they relate to subject matter not required to be searched by this Authority, namely:

Claims 42, 43 and 44-46 (when referring to the method of claim 42 or 43) set forth a method for treating and/or preventing a wild and/or mutated BTK kinase-mediated disease in a subject, which belongs to a method for treatment of a living human or animal body, that is, belongs to the cases set forth in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/079360**

| Patent document cited in search report | | | Publication date (day/month/year) | Patent family member(s) | | | Publication date (day/month/year) |
|---|---|---|---|---|---|---|---|
| CN | 114075190 | A | 22 February 2022 | WO | 2022037649 | A1 | 24 February 2022 |
| CN | 108431007 | A | 21 August 2018 | EP | 4116303 | A1 | 11 January 2023 |
| | | | | AU | 2016322063 | A1 | 12 April 2018 |
| | | | | US | 2018362537 | A1 | 20 December 2018 |
| | | | | US | 10399990 | B2 | 03 September 2019 |
| | | | | US | 2018298008 | A1 | 18 October 2018 |
| | | | | US | 10611766 | B2 | 07 April 2020 |
| | | | | CA | 2998796 | A1 | 23 March 2017 |
| | | | | KR | 20180109842 | A | 08 October 2018 |
| | | | | US | 2018362533 | A1 | 20 December 2018 |
| | | | | US | 10399989 | B2 | 03 September 2019 |
| | | | | PH | 12018500587 | A1 | 15 October 2018 |
| | | | | EP | 3350184 | A1 | 25 July 2018 |
| | | | | EP | 3350184 | B1 | 19 October 2022 |
| | | | | WO | 2017046604 | A1 | 23 March 2017 |
| | | | | SG | 11201802155 | A1 | 27 April 2018 |
| | | | | IN | 201837012921 | A | 18 May 2018 |
| | | | | HK | 1258779 | A0 | 22 November 2019 |
| | | | | IL | 258154 | A | 30 April 2018 |
| | | | | MX | 2018003351 | A1 | 17 September 2018 |
| | | | | JP | 2018527384 | W | 20 September 2018 |
| | | | | BR | 112018005189 | A2 | 02 October 2018 |
| | | | | CN | 108431007 | B | 07 June 2022 |
| | | | | CN | 114685516 | A | 01 July 2022 |
| CN | 114380820 | A | 22 April 2022 | | None | | |
| CN | 114573586 | A | 03 June 2022 | | None | | |
| CN | 115707705 | A | 21 February 2023 | | None | | |
| US | 2022169645 | A1 | 02 June 2022 | | None | | |
| CN | 109851620 | A | 07 June 2019 | EP | 3492471 | A1 | 05 June 2019 |
| | | | | EP | 3492471 | A4 | 04 September 2019 |
| | | | | EP | 3492471 | B1 | 02 December 2020 |
| | | | | JP | 6737952 | B2 | 12 August 2020 |
| | | | | WO | 2018033091 | A1 | 22 February 2018 |
| | | | | US | 2021070758 | A1 | 11 March 2021 |
| | | | | US | 11186578 | B2 | 30 November 2021 |
| | | | | JP | 2019528270 | W | 10 October 2019 |
| | | | | CN | 108368119 | A | 03 August 2018 |
| | | | | CN | 108368119 | B | 23 April 2019 |
| | | | | CN | 109851620 | B | 07 August 2020 |
| CN | 109111439 | A | 01 January 2019 | WO | 2019001307 | A1 | 03 January 2019 |
| | | | | CN | 109111439 | B | 18 September 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *J. Pharmaceutical Sciences*, 1977, vol. 66, 1-19
- Bioreversible Carriers in Drug Design. **T. HIGUCHI** ; **V. STELLA**. Prodrugs as Novel Delivery Systems, A.C.S. Symposium Series. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985